# EUROPEAN PATENT APPLICATION

(11) **EP 1 650 201 A1**
(43) Date of publication of application: **26.04.2006**
(21) Application number: 04748264.1
(22) Date of filing: 30.07.2004
(51) Int. Cl.: C07D 267/14

(54) **BENZOXAZEPINE COMPOUND**

(30) Priority: 01.08.2003 JP 2003285341
(71) Applicant: Takeda Pharmaceutical Company Limited, Osaka 541-0045 (JP)
(72) Inventor: MARUI, Shogo Takeda Pharmaceutical Company Limited, Osaka-shi, Osaka (JP); MIKI, Takashi Takeda Pharmaceutical Company Ltd., Osaka-shi, Osaka (JP); MIURA, Shoutarou Takeda Pharmaceutical Comp. Ltd., Osaka-shi, Osaka (JP); NISHIMOTO, Tomoyuki Takeda Pharmaceut. Comp. Ltd., Osaka-shi, Osaka (JP); NAKADA, Yoshihisa Takeda Pharmaceutical Comp. Ltd., Osaka-shi, Osaka (JP)
(74) Representative: Lewin, John Harvey
(86) International application number: PCT/JP2004/011293
(87) International publication number: WO 2005/012272

(57) **Abstract**

A compound represented by the formula [1]: 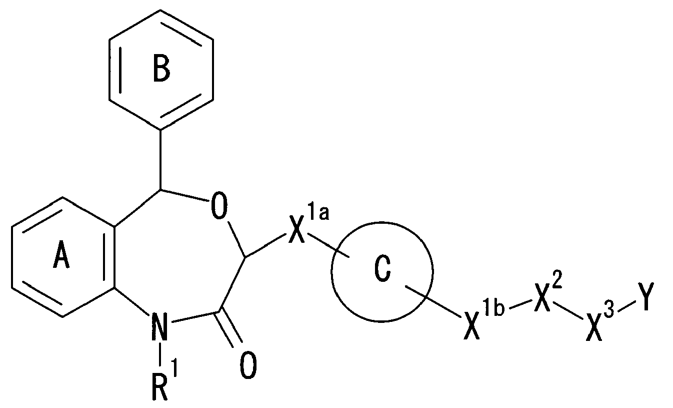 wherein ring A and ring B each represent an optionally substituted benzene ring; ring C represents an optionally further substituted aromatic ring; R¹ represents a lower alkyl group optionally substituted with an optionally substituted hydroxyl group; X^{1a} represents a bond or optionally substituted lower alkylene; X^{1b} represents a bond or optionally substituted lower alkylene; X² represents a bond, -O- or -S-; X³ represents a bond or an optionally substituted divalent hydrocarbon group; Y represents an optionally esterified or amidated carboxyl group, or a salt thereof. The compound of the formula [I] is safer and has more potent lipid lowering activity such as squalene synthase inhibitory activity (cholesterol lowering activity) and triglyceride lowering activity, and thus it is a compound useful as an agent for preventing or treating hyperlipemia.

## Description

### Technical Field

The present invention relates to a novel benzoxazepine compound having squalene synthase inhibitory activity, cholesterol lowering activity and triglyceride lowering activity, which is useful for preventing or treating hyperlipemia and the like.

### Background Art

Abnormal increase of the concentration of blood serum lipids is called hyperlipidemia or hyperlipemia. Blood serum lipids include cholesterol (cholesterol ester, free cholesterol), phospholipid (lecithin, sphingomyelin and the like), triglyceride (neutral lipid), free fatty acid, other sterols and the like, and specifically, the increase of cholesterol and triglyceride is a clinical issue (COMMON DISEASE SERIES No.19 hyperlipemia, Haruo Nakamura ed., published on October 10, 1991, by Nankodo).

Examples of a drug for lowering cholesterol level in blood include drugs that trap bile acid and inhibit its absorption such as Cholestyramine and Colestipol (for example, disclosed in U.S.P. 4,027,009) and drugs that inhibit acyl coenzyme A cholesterol acyltransferase (ACAT) and suppress intestinal absorption of cholesterol, such as Melinamide. In addition, drugs that suppress cholesterol biosynthesis, such as Lovastatin (disclosed in U.S.P. 4,231,938), Simvastatin (disclosed in U.S.P. 4,444,784) and Pravastatin (disclosed in U.S.P. 4,346,227) which inhibit 3-hydroxy-3-methylglutaryl coenzyme A (HMG-CoA) reductase, are used as medicaments.

Furthermore, as triglyceride lowering agents, fibric acid derivatives such as clofibrate (British Patent No. 860303), fenofibrate (German Patent No. 2250327) and the like are used as medicaments.

On the other hand, as compounds having inhibitory effect on biosynthesis of choresterol by inhibiting squalene synthase are disclosed in non-Patent Publications such as Journal of Medicinal Chemistry, 1988, vol.31, p.1869-1871; Expert Opinion on Therapeutic Patents, 1998, vol.8, p.521-530; Bioorganic Medicinal Chemistry, 2002, vol.10, p.385-400; Bioorganic Medicinal Chemistry, 2002, vol.10, p.401-412; Chemical & Pharmaceutical Bulletin, 2002, vol.50, p.53-58; Chemical & Pharmaceutical Bulletin, 2002, vol.50, p.59-65; Journal of Medicinal Chemistry, 2002, vol.45, p.4571-4580; and Patent Publications such as JP-A 1-213288, JP-A 2-101088, JP-A 2-235820, JP-A 2-235821, JP-A 3-20226, JP-A 3-68591, JP-A 3-148288, JP-A 9-087260, USP 5135935, USP 5726306, USP 5698691, EP 0645377, WO 92/15579, WO 93/09115, WO 95/021834, WO 97/10224, WO 2001/98282 and USP 6537987.

### Disclosure of Invention

It is very important to control the concentration of blood serum lipids properly for preventing or treating various diseases associated with arteriosclerosis including ischemic cardiac diseases and cerebral infarction. In addition, it is thought that hypertriglyceridemia induces pancreatic disorder. When HMG-CoA reductase is inhibited with a HMG-CoA reductase inhibitor, not only biosynthesis of cholesterol, but also biosynthesis of other components essential to living organism such as ubiquinone, dolichol and heme A are inhibited, resulting in side effects of concern. In addition, combination use of a triglyceride lowering agent and a statin compound is contraindicated due to hepatic toxicity. On the other hand, squalene synthase is an enzyme participating in an essential stage of the cholesterol biosynthesis pathway. This enzyme catalyzes reductive dimerization of two molecules of farnesyl pyrophosphate to form squalene.

Under such circumstances, an object of the present invention is to provide a compound which is safer and has more potent lipid lowering activity such as squalene synthase inhibitory activity (cholesterol lowering activity) and triglyceride lowering activity, and thus is useful as a drug for preventing or treating hyperlipemia.

The present inventors intensively studied and, as a result, for the first time, synthesized a 4,1-benzoxazepine compound characterized by a chemical structure having a particular substituent at the 3-position and found that this compound has unexpectedly excellent pharmaceutical effects such as lipid lowering effect based on its unique chemical structure, has high delivery in transference to a target organ and has a wide safety margin, which resulted in completion of the present invention.

That is, the present invention relates to:
(1) a compound represented by the formula [1]: 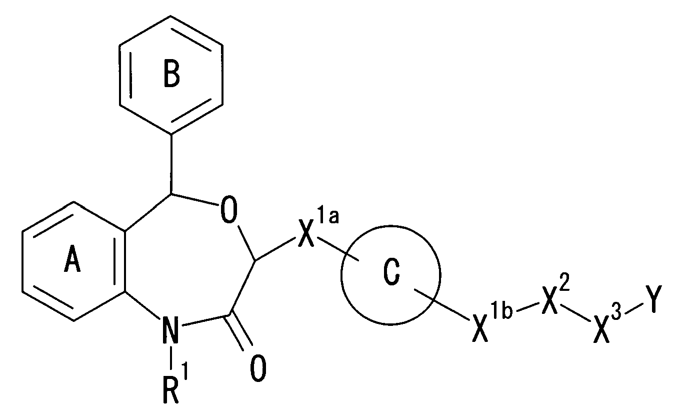 wherein ring A and ring B each represent an optionally substituted benzene ring, ring C represents an optionally further substituted aromatic ring, R¹ represents a lower alkyl group optionally substituted with an optionally substituted hydroxyl group, X^{1a} represents a bond or optionally substituted lower alkylene, X^{1b} represents a bond or optionally substituted lower alkylene, X² represents a bond, -O- or -S-, X³ represents a bond or an optionally substituted divalent hydrocarbon group, and Y represents an optionally esterified or amidated carboxyl group, or a salt thereof;
(2) the compound according to the above (1), wherein X^{1b} is a bond and Y is an optionally esterified carboxyl group;
(3) the compound according to the above (1), wherein ring A is a benzene ring substituted with halogen atom(s);
(4) the compound according to the above (1), wherein ring B is a benzene ring substituted with lower alkoxy group(s);
(5) the compound according to the above (1), wherein ring C is an optionally further substituted monocyclic aromatic heterocyclic ring;
(6) the compound according to the above (1), wherein ring C is an optionally further substituted benzene ring;
(7) the compound according to the above (1), wherein ring C is an optionally further substituted aromatic ring having no hydrogen atom that may be deprotonated;
(8) the compound according to the above (1), wherein X^{1a} is C₁₋₃ alkylene;
(9) the compound according to the above (1), wherein X² is a bond;
(10) the compound according to the above (1), wherein X³ is C₁₋₄ alkylene;
(11) the compound according to the above (1), wherein the formula [I] is the formula [Ia]: 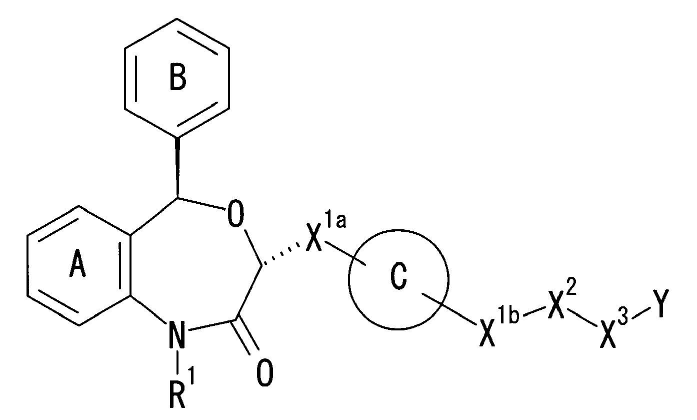 wherein respective symbols are as defined in the above (1);
(12) 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid, or a salt thereof;
(13) (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid, (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid, or a salt thereof;
(14) 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-5-yl)pentanoic acid, or a salt thereof;
(15) a prodrug of the compound according to the above (1);
(16) a medicine comprising the compound according to the above (1) or a prodrug thereof;
(17) a medicine comprising a combination of the compound according to the above (1) or a prodrug thereof and a cholesterol lowering agent;
(18) the medicine according to the above (16) or (17), which is a squalene synthase inhibitor;
(19) the medicine according to the above (16) or (17), which is a triglyceride lowering agent;
(20) the medicine according to the above (16) or (17), which is a lipid lowering agent;
(21) the medicine according to the above (16) or (17), which is an agent for preventing or treating hyperlipemia;
(22) the medicine according to the above (16) or (17), which is a high density lipoprotein-cholesterol level elevating agent;
(23) a process for preparing a compound represented by the formula [I']: 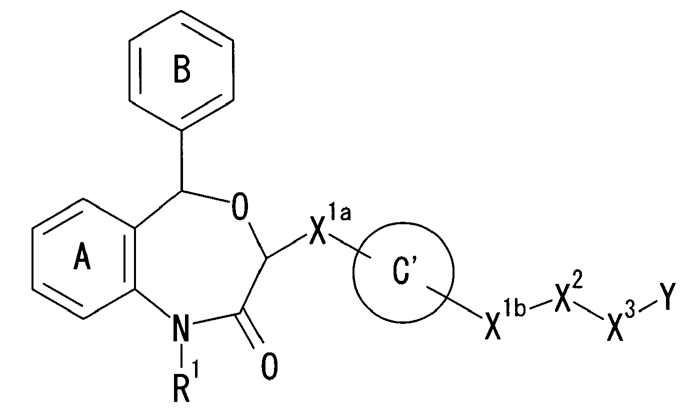 wherein ring C' represents an optionally further substituted aromatic heterocyclic ring and other symbols are as defined in the above (1), or a salt thereof, which comprises reacting a compound represented by the formula: 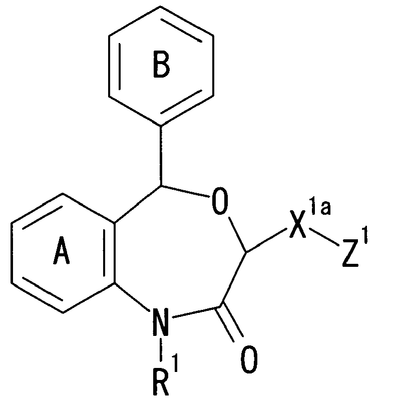 wherein Z¹ represents a functional group involved in an aromatic heterocyclic ring forming reaction and other symbols are as defined in the above (1), or a salt thereof, with a compound represented by the formula: 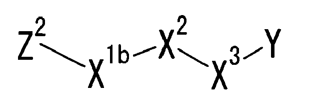 wherein Z² represents a functional group involved in an aromatic heterocyclic ring forming reaction and other symbols are as defined in the above (1), or a salt thereof;
(24) a method of inhibiting squalene synthase in a mammal, which comprises administering an effective amount of the compound according to the above (1) or a prodrug thereof to said mammal;
(25) a method of lowering triglyceride level in a mammal, which comprises administering an effective amount of the compound according to the above (1) or a prodrug thereof to said mammal;
(26) a method of lowering lipid level in a mammal, which comprises administering an effective amount of the compound according to the above (1) or a prodrug thereof to said mammal;
(27) a method of preventing or treating hyperlipemia in a mammal, which comprises administering an effective amount of the compound according to the above (1) or a prodrug thereof to said mammal;
(28) a method of elevating high density lipoprotein-cholesterol level in a mammal, which comprises administering an effective amount of the compound according to the above (1) or a prodrug thereof to said mammal;
(29) use of the compound according to the above (1) or a prodrug for manufacture of a squalene synthase inhibitor;
(30) use of the compound according to the above (1) or a prodrug thereof for manufacture of a triglyceride lowering agent;
(31) use of the compound according to the above (1) or a prodrug thereof for manufacture of a lipid lowering agent;
(32) use of the compound according to the above (1) or a prodrug thereof for manufacture of an agent for preventing or treating hyperlipemia;
(33) use of the compound according to the above (1) or a prodrug thereof for manufacture of a high density lipoprotein-cholesterol level elevating agent; and the like.

### Detailed Explanation of the Invention

The substituent of the "optionally substituted benzene ring" represented by ring A includes halogen (e.g. fluorine, chlorine, bromine, iodine), an optionally substituted lower alkyl group having 1 to 4 carbon atoms (e.g. methyl, ethyl, propyl, butyl, tert-butyl etc.), an optionally substituted lower alkoxy group having 1 to 4 carbon atoms (e.g. methoxy, ethoxy, propoxy, isopropoxy, butoxy, tert-butoxy etc.), a hydroxyl group, a nitro group and cyano. The ring A may have 1 to 3, preferably 1 to 2 of these substituents. The adjacent substituents of these substituents may be taken together to form a ring. The substituent of the optionally substituted lower alkyl group having 1 to 4 carbon atoms or the optionally substituted lower alkoxy group having 1 to 4 carbon atoms includes halogen (e.g. fluorine, chlorine, bromine, iodine), and 1 to 3 substituents may be at optional substitutable positions. The ring A is preferably a benzene ring substituted with halogen atoms, etc., more preferably a benzene ring substituted with a chlorine atom. The ring A is preferably a benzene ring represented by the formula: 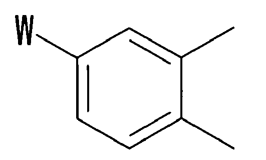 wherein W represents a halogen atom (e.g. fluorine, chlorine, bromine, iodine) and inter alia, W is preferably a chlorine atom.

The substituent of the "optionally substituted benzene ring" represented by ring B includes the same number of the same groups as those exemplified above as the substituent of the "optionally substituted benzene ring" represented by ring A. The ring B is preferably a benzene ring substituted with a lower alkoxy group having 1 to 4 carbon atoms, and inter alia, preferably a benzene ring represented by the formula: 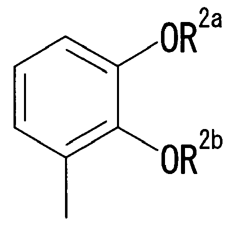 wherein R^{2a} and R^{2b} represent independently a hydrogen atom or a lower alkyl group having 1 to 4 carbon atoms (e.g. methyl, ethyl, propyl, butyl etc.) and particularly preferably R^{2a} and R^{2b} are both methyl groups.

The aromatic ring of the "optionally further substituted aromatic ring" represented by ring C includes an aromatic hydrocarbon ring and an aromatic heterocyclic ring. The aromatic hydrocarbon ring includes, for example, a benzene ring, a naphthalene ring and the like, and preferred is a benzene ring. The aromatic heterocyclic ring (the aromatic heterocyclic ring of the "optionally further substituted aromatic heterocyclic ring" represented by ring C') includes, for example, an aromatic heterocyclic ring containing at least one (preferably 1 to 4, more preferably 1 to 2) of 1 to 3 kinds (preferably 1 or 2 kinds) of heteroatoms selected from an oxygen atom, a sulfur atom, a nitrogen atom and the like, as atoms constituting the ring system (ring atoms).

The aromatic heterocyclic ring includes 5- to 6-membered monocyclic aromatic heterocyclic rings such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine and the like; 8- to 12-membered fused aromatic heterocyclic rings such as benzofuran, isobenzofuran, benzo[b]thiophene, indole, isoindole, 1H-indazole, benzimidazole, benzoxazole, 1,2-benzisoxazole, benzothiazole, benzopyrane, 1,2-benzisothiazole, 1H-benzotriazole, quinoline, isoquinoline, cinnoline, quinazoline, quinoxaline, phthalazine, naphthyridine, purine, pteridine, carbazole, α-carboline, β-carboline, γ-carboline, acridine, phenoxazine, phenothiazine, phenazine, phenoxathiine, thianthrene, phenanthridine, phenanthroline, indolizine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridine, 1,2,4-triazolo[4,3-b]pyridazine and the like (preferably a heterocyclic ring in which the aforementioned 5- to 6-membered monocyclic aromatic heterocyclic ring is fused with a benzene ring, or a heterocyclic ring in which the same or different two of the aforementioned 5- to 6-membered monocyclic aromatic heterocyclic rings are fused, more preferably a heterocyclic ring in which the aforementioned 5- to 6-membered monocyclic aromatic heterocyclic ring is fused with a benzene ring) and the like.

The ring C is preferably a monocyclic aromatic heterocyclic ring, a benzene ring or the like, and inter alia, preferred is a 5-membered monocyclic aromatic heterocyclic ring such as pyrazole, imidazole, thiazole, oxazole, isoxazole, 1,2,4-oxadiazole, 1,3,4-oxadiazole or the like.

Although the ring C may be an aromatic ring having a hydrogen atom that may be deprotonated or an aromatic ring having no hydrogen atom that may be deprotonated, an aromatic ring having no hydrogen atom that may be deprotonated is preferred. The aromatic ring having no hydrogen atom that may be deprotonated includes, in addition to an aromatic ring originally having no hydrogen atom that may be deprotonated (e.g. benzene ring, thiazole, oxazole, isoxazole, 1,2,4-oxadiazole, 1,3,4-oxadiazole etc.), an aromatic ring in which a hydrogen atom that may be deprotonated is substituted (e.g. pyrrole, pyrazole, imidazole etc. whose hydrogen atom on the ring-constituting nitrogen atom is substituted or which is bound to X^{1a} or/and X^{1b} via the ring-constituting nitrogen atom).

The substituent, which the aromatic ring of the "optionally further substituted aromatic ring" represented by ring C may have, includes (i) a carboxyl group optionally esterified with an optionally halogenated C₁₋₆ alkyl group or an optionally halogenated C₆₋₁₀ aryl-C₁₋₄ alkyl group (e.g. methyl, ethyl, propyl, isopropyl, butyl, tert-butyl, phenyl, benzyl etc.), (ii) a phosphoric acid group optionally mono- or di-substituted with optionally halogenated C₁₋₆ alkyl (e.g. methyl, ethyl, n-propyl, isopropyl, n-butyl, isobutyl, n-pentyl, isopentyl, neopentyl, hexyl etc.) or C₂₋₇ alkanoyloxy-C₁₋₆ alkyl such as acetoxymethyl or pivaloyloxymethyl, (iii) a sulfonic acid group, (iv) a sulfonamide group optionally substituted with an optionally halogenated C₁₋₆ alkyl group or an optionally halogenated C₆₋₁₀ aryl-C₁₋₄ alkyl group (e.g. methyl, ethyl, propyl, isopropyl, butyl, tert-butyl, benzyl etc.), (v) a hydroxyl group and a sulfhydryl group, which may be optionally substituted with an optionally halogenated C₁₋₃ alkyl group (e.g. methyl, ethyl, propyl etc.), (vi) a carbamoyl group, (vii) a phenyl group optionally substituted with 1 to 5 substituents [e.g. hydroxyl group, chlorine, fluorine, aminosulfonyl group, amino group optionally substituted with C₁₋₃ alkyl group (e.g. methyl, ethyl, propyl etc.)] and optionally bound to the aromatic ring via 0 or S, (viii) an amino group optionally mono- or di-substituted with an optionally halogenated C₁₋₃ alkyl group (e.g. methyl, ethyl, propyl etc.), (ix) a cyclic amino group optionally substituted with 1 to 3 C₁₋₃ alkyl (e.g. methyl, ethyl etc.), benzyl, phenyl and the like (e.g. a 5- to 6-membered cyclic amino group optionally containing an oxygen atom or a sulfur atom in addition to nitrogen atoms as ring-constituting atoms, such as a cyclic amino group derived (by removing one hydrogen atom) from cyclic amine such as piperidine, pyrrolidine, morpholine, thiomorpholine, piperazine, 4-methylpiperazine, 4-benzylpiperazine, 4-phenylpiperazine, 1,2,3,4-tetrahydroisoquinoline or phthalimide), (x) a 5- to 6-membered aromatic heterocyclic group containing 1 to 4 heteroatoms selected from N, O and S and optionally bound to the aromatic ring via O or S (e.g. pyridyl, imidazolyl, indolyl, tetrazolyl etc.), (xi) a halogen atom (e.g. chlorine, fluorine, bromine, iodine etc.), (xii) a C₁₋₄ alkyl group (e.g. methyl, ethyl, propyl, isopropyl, butyl, tert-butyl etc.), a C₁₋₄ alkoxy group (e.g. methoxy, ethoxy, propoxy, isopropoxy, butoxy, tert-butoxy etc.) or a C₁₋₄ alkylthio group (e.g. methylthio, ethylthio, propylthio, isopropylthio, butylthio, tert-butylthio etc.), each of which may be optionally substituted with a substituent selected from a halogen atom, a C₁₋₄ alkoxy group, a C₁₋₄ alkylthio group, carboxyl and phenyl, (xiii) a C₅₋₇ cycloalkyl group (e.g. cyclopentyl, cyclohexyl, cycloheptyl etc.), and (xiv) optionally halogenated C₁₋₇ alkanoyloxy (e.g. formyloxy, acetoxy, propionyloxy, butyryloxy, tert-butoxycarbonyloxy, isobutyryloxy, valeryloxy, pivaloyloxy etc.). The "optionally further substituted aromatic ring" may be substituted with 1 to 6, preferably 1 to 3 such substituents at substitutable positions. Two of such substituents may be taken together to form C₃₋₆ alkylene, C₃₋₆ alkyleneoxy, C₃₋₆ alkylenedioxy or the like. For example, when two adjacent substituents on a phenyl group are linked each other to form C₄ alkylene, a tetrahydronaphthyl group is formed.

The lower alkyl group of the "lower alkyl group optionally substituted with an optionally substituted hydroxyl group" represented by R¹ includes, for example, C₁₋6 alkyl such as methyl, ethyl, n-propyl, isopropyl, n-butyl, isobutyl, n-pentyl, isopentyl, neopentyl, hexyl and the like. Among them, a C₃₋₆ alkyl group is preferred and a C₄₋₅ alkyl group is more preferred. Inter alia, a branched C₄₋₅ alkyl group such as isobutyl, neopentyl or the like is preferred.

The substituent, which the lower alkyl group of the "lower alkyl group optionally substituted with an optionally substituted hydroxyl group" represented by R¹ may have, includes a hydroxyl group optionally substituted with C₂₋₂₀ alkanoyl or C₁₋₇ alkyl. Such substituent includes, for example, a hydroxyl group, acetyloxy (acetoxy), propionyloxy, tert-butoxycarbonyloxy, palmitoyloxy, dimethylaminoacetyloxy, 2-aminopropionyloxy and the like. The lower alkyl group may be substituted with 1-3 such substituents at substitutable positions.

Examples of R¹ include 1-propyl, 1-isopropyl, 1-isobutyl, 1-neopentyl, 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl, 3-acetoxy-2-acetoxymethyl-2-methylpropyl, [1-(hydroxymethyl)cyclobutyl]methyl and the like. Among them, preferred are 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methylpropyl, 3-acetoxy-2-acetoxymethyl-2-methylpropyl and the like.

The lower alkylene of the "optionally substituted lower alkylene" represented by X^{1a} includes, for example, C₁₋₆ alkylene such as methylene, dimethylene, trimethylene, tetramethylene, pentamethylene, hexamethylene and the like. Among them, preferred is straight chain C₁₋₄ alkylene such as methylene, dimethylene, trimethylene, tetramethylene or the like, and more preferred is straight chain C₁₋₃ alkylene.

The substituent, which the lower alkylene of the "optionally substituted lower alkylene" represented by X^{1a} may have, includes the same groups as those exemplified above as the substituent, which the aromatic ring of the "optionally further substituted aromatic ring" represented by ring C may have, an oxo group and the like. The "lower alkylene" may be substituted with 1 to 6, preferably 1 to 3 such substituents at substitutable positions.

X^{1a} is preferably a bond or straight chain C₁₋₃ alkylene, and particularly preferably methylene.

The lower alkylene of the "optionally substituted lower alkylene" represented by X^{1b} includes the same groups as those exemplified as the lower alkylene of the "optionally substituted lower alkylene" represented by X^{1a}. The substituent, which the lower alkylene of the "optionally substituted lower alkylene" represented by X^{1b} may have, includes the same number of the same groups as those exemplified as the substituent which the lower alkylene of the "optionally substituted lower alkylene" represented by X^{1a} may have.

X^{1b} is preferably a bond or straight chain C₁₋₃ alkylene, and particularly preferably a bond.

X² is preferably a bond.

The "divalent hydrocarbon group" of the "optionally substituted divalent hydrocarbon group" represented by X³ includes a group formed by removing one hydrogen atom from a hydrocarbon group. The hydrocarbon group includes a C₁₋₇ straight or branched chain alkyl group (e.g. methyl, ethyl, n-propyl, isopropyl, n-butyl, isobutyl, 1,1-dimethylethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), a C₃₋₇ cycloalkyl group (cyclopropyl, cyclobutyl, cyclopentyl, cyclohexyl, cyclohexylmethyl etc.), a straight or branched chain C₂₋₆ alkenyl group (e.g. vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc.), a C₆₋₁₀ aryl group (e.g. phenyl, naphthyl), a C₇₋₁₄ arylalkyl group (e.g. benzyl, phenethyl, naphthylmethyl) and the like.

The substituent, which the "divalent hydrocarbon group" of the "optionally substituted divalent hydrocarbon group" represented by X³ may have, includes the same group as those exemplified above as the substituent which the lower alkylene of the "optionally substituted lower alkylene" represented by X^{1a} may have, optionally halogenated C₁₋₆ alkylidene (e.g. methylidene, ethylidene, propylidene, isopropylidene, butenylidene etc.), vinylidene, cyclohexylidene, benzylidene and the like. The "divalent hydrocarbon group" may be substituted with 1 to 6, preferably 1 to 3 such substituents at substitutable positions.

The "divalent hydrocarbon group" of the "optionally substituted divalent hydrocarbon group" represented by X³ preferably includes (1) straight or branched chain alkylene in which the number of carbon atoms constituting the straight chain part is 1 to 7 (preferably 1 to 4) (e.g. methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene etc.), (2) a double bond- containing carbon chain in which the number of carbon atoms constituting the straight chain part is 2 to 7 (preferably 2 to 4) (e.g. vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene etc.), (3) phenylene (e.g. 1,2-phenylene, 1,3-phenylene, 1,4-phenylene etc.) and (4) a divalent group in which phenylene and alkylene and/or alkenylene are combined (e.g. -CH₂-C₆H₄-, -CH₂CH₂-C₆H₄-, -CH₂-C₆H₄-CH₂- etc.).

X³ is preferably C₁₋₄ alkylene such as methylene, ethylene, trimethylene, tetramethylene or the like, vinylene, propenylene, phenylene or the like.

The "optionally esterified or amidated carboxyl group" represented by Y includes carboxyl, lower alkoxycarbonyl having 2 to 7 carbon atoms (e.g. methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl etc.), C₇₋₁₄ aryloxycarbonyl (e.g. phenoxycarbonyl, 1-naphthoxycarbonyl), C₈₋₁₂ aralkyloxycarbonyl (e.g. benzyloxycarbonyl etc.), carbamoyl, N-C₁₋₆ alkylcarbamoyl, N,N-diC₁₋₆ alkylcarbamoyl, N-C₈₋₁₂ aralkylcarbamoyl, N,N-diC₈₋₁₂ aralkylcarbamoyl, 1-pyrrolidinylcarbonyl, piperidinocarbonyl, morpholinocarbonyl and the like. Among them, Y is preferably carboxyl, methoxycarbonyl, ethoxycarbonyl or the like, and particularly preferably carboxyl.

The compound represented by the formula [1] includes, particularly preferably, 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid, (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid, (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid, 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxodiazol-5-yl)pentanoic acid and the like.

The compound represented by the formula [I] may be in a free form or a pharmacologically acceptable salt form, and both forms are included in the scope of the present invention. When the compound represented by the formula [I] has an acidic group such as a carboxyl group, etc., the compound may form a salt with inorganic bases (e.g. alkali metal such as sodium, potassium etc., alkaline earth metal such as calcium, magnesium etc., transition metal such as zinc, iron, copper etc.) or organic bases (e.g. organic amines such as trimethylamine, triethylamine, pyridine, picoline, ethanolamine, diethanolamine, triethanolamine, tris(hydroxymethyl)methylamine, dicyclohexylamine, N,N'-dibenzylethylenediamine and t-butylamine, basic amino acids such as arginine, lysine ornithine, and the like).

When the compound represented by the formula [I] of the present invention has a basic group such as an amino group or the like, the compound may form a salt with inorganic acids or organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.) or acidic amino acids such as aspartic acid or glutamic acid.

The compound represented by the formula [I] or a salt thereof has asymmetric carbon atoms at the 3-position and the 5-position, and may be a mixture of stereoisomers. The isomers may be separated by a known means. Preferred is the trans form in which the substituents at the 3-position and 5-position are directed to the opposite direction each other relative to the plane of the 7-membered ring, and specifically preferred is a compound having the absolute configuration represented by the formula [Ia]. In addition, the compound represented by the formula [I] or a salt thereof may be a racemic form or an optically active form, and the optically active form can be separated from the racemic form by a known optical resolution means.

A prodrug of the compound represented by the formula [I] or a salt thereof refers to a compound which is converted into the compound represented by the formula [I] or a salt thereof by a reaction with an enzyme or gastric acid under the physiological condition in vivo, that is, a compound which is changed into the compound represented by the formula [I] or a salt thereof by enzymatic oxidation, reduction, hydrolysis or the like, or a compound which is changed into the compound represented by the formula [I] or a salt thereof by hydrolysis with gastric acid or the like. The prodrug of the compound represented by the formula [I] or a salt thereof includes, when the compound represented by the formula [I] or a salt thereof has an amino group, a compound whose amino group is acylated, alkylated or phosphorylated (e.g. the compound represented by the formula [I] or a salt thereof whose amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated or tert-butylated, etc.); when the compound represented by the formula [I] or a salt thereof has a hydroxyl group, a compound whose hydroxyl group is acylated, alkylated, phosphorylated or borated (e.g. a compound whose hydroxyl group is acetylated, palmitoylated, propanoylated, pivaloylated, succinylated, fumarylated, alanylated or dimethylaminomethylcarbonylated, etc.); and when the compound represented by the formula [I] or a salt thereof has a carboxyl group, a compound whose carboxyl group is esterified or amidated (e.g. a compound whose carboxyl group is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified or methylamidated, etc.). These prodrugs can be prepared from the compound represented by the formula [I] or a salt thereof by a method known per se.

A prodrug of the compound represented by the formula [I] or a salt thereof may be a compound that is changed into the compound represented by the formula [I] or a salt thereof under the physiological condition as described in Development of Pharmaceutical Products, vol. 7, Molecular Design, 163-198, Hirokawa Shoten (1990).

The compound represented by the formula [I] or a salt thereof may be hydrous or anhydrous.

The compound represented by the formula [I] or a salt thereof may be labeled with an isotope (e.g. ³H, ¹⁴C, ³⁵S, ¹²⁵I etc.).

The compound represented by the formula [I] or a salt thereof can be prepared, for example, by reacting a compound represented by the formula [II]: 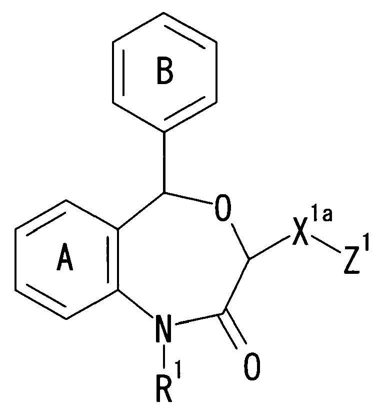 wherein symbols are as defined above, or a derivative thereof with a compound represented by the formula: 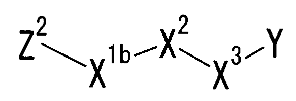 wherein symbols are as defined above, or a salt thereof according to a method known per se (e.g. "Comprehensive Heterocyclic Chemistry", ed. by A.R.Katritzky and C.W.Rees, vol.4-6, Pergamon Press, Oxford, 1984), or thereafter forming an aromatic heterocyclic ring by cyclization reaction.

A combination of the "functional group involved in an aromatic heterocyclic ring forming reaction" represented by Z¹ and Z² includes, for example:
(1) one is a carboxyl group and the other is 2-aminoalkanoyl group,
(2) one is a carboxyl group and the other is hydrazide,
(3) one is a thiocarbamoyl group and the other is an alkanoyl group substituted with a halogen atom at the 2-position, and
(4) one is a N-hydroxyamidino group and the other is carboxylic acid halide, or the like.

A method of preparing the compound represented by the formula [I'] includes Process A, Process B and the like, wherein a compound represented by the formula [II'] or a derivative thereof is used as the starting material. 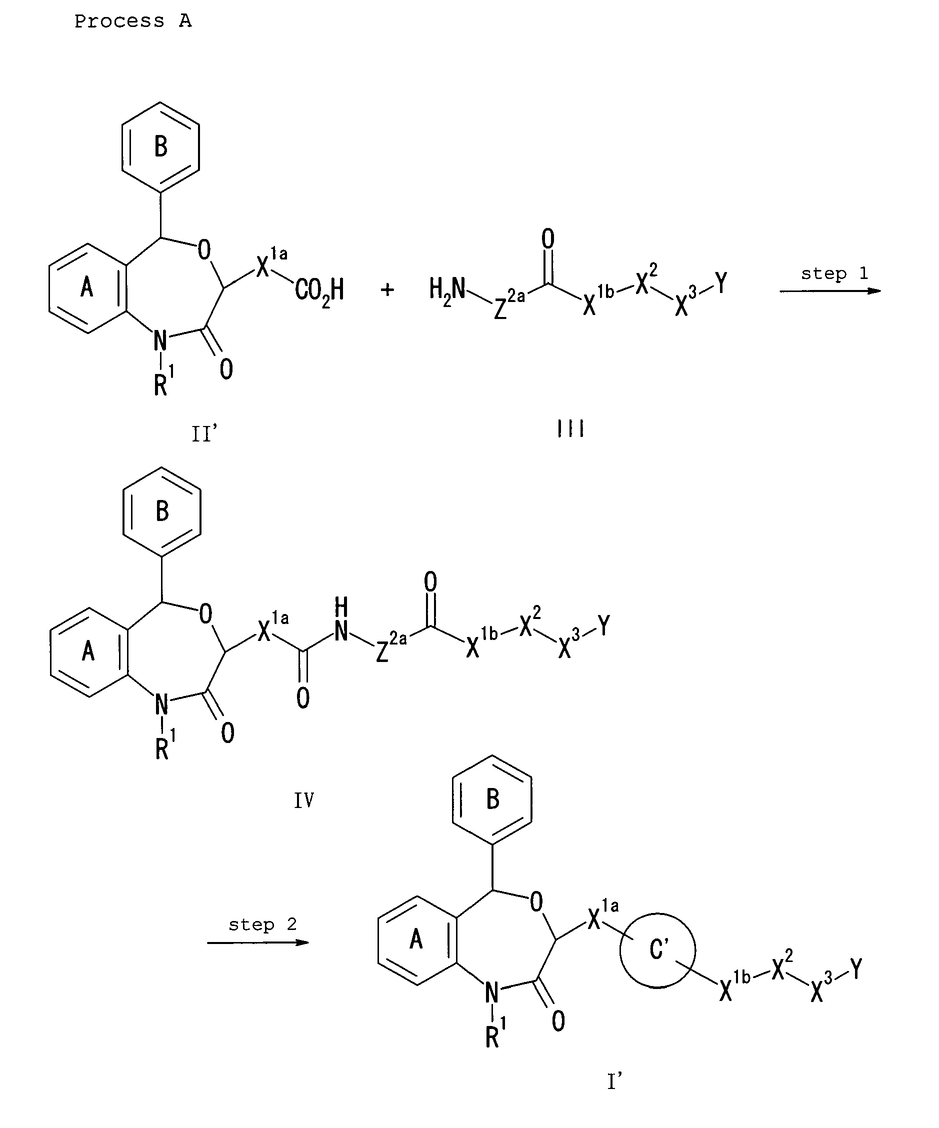 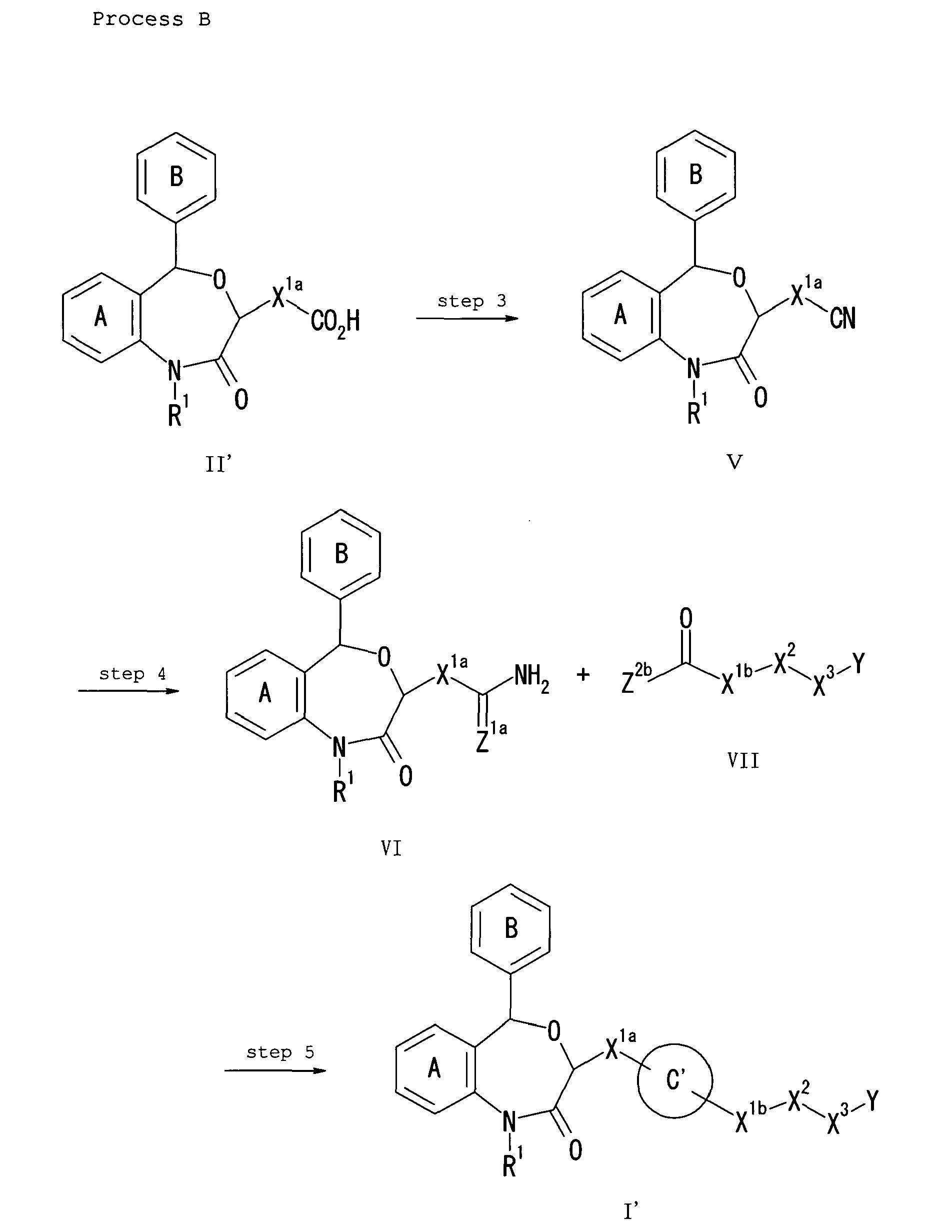 wherein, Z^{1a} represents a hydroxyimino group or a sulfur atom, Z^{2a} represents a methylene group or an imino group, and Z^{2b} represents a halogen atom, or a lower alkyl group in which the carbon atom adjacent to the carbonyl group is substituted with one halogen atom.

In the step 1, a method of preparing the compound represented by the formula [IV] from the compound represented by the formula [II'] or a derivative thereof and the compound represented by the formula [III] includes, for example, a method comprising condensing the compound represented by the formula [II'] and the compound represented by the formula [III] with a generally known dehydration condensing agent in the presence of both compounds simultaneously, a method comprising activating the carboxylic acid of the compound represented by the formula [II'] by a generally known activating method and then reacting it with the compound represented by the formula [III], and a method comprising reacting a derivative of the compound represented by the formula [II'] with the compound represented by the formula [III]. The dehydration condensing agent includes, for example, N,N'-dicyclohexylcarbodiimide, 1-ethyl-3-(3-dimethylaminopropyl)carbodiimide, carbonyldiimidazole, 1H-benzotriazol-1-yloxytris(dimethylamino)phosphonium hexafluorophosphate and the like.

A method of activating carboxylic acid includes, for example, a method comprising converting carboxylic acid into acid anhydride with chloroformate, pivaloyl chloride or the like, a method comprising converting carboxylic acid into acid chloride with oxalyl chloride, thionyl chloride or the like, method comprising esterifying carboxylic acid with 1-hydroxybenzotriazole using a dehydration condensing agent or the like, and the like.

A method of reacting a derivative of the compound represented by the formula [II'] with the compound represented by the formula [III] includes a method comprising heating an ester derivative of the compound represented by the formula [II'] in the presence of the compound represented by the formula [III].

The compound represented by the formula [III] can be prepared, for example, according to Convenient Syntheses of δ-Aminolevulinic Acid, Ayelet Nudelman and Abraham Nodelman, Synthesis 1999, No. 4, pp568-570.

In the step 2, a method of preparing the compound represented by the formula [I] from the compound represented by the formula [IV] includes a method comprising treatment with Lawesson's reagent or phosphorus oxychloride (POCl₃).

In the step 3, a method of preparing the compound represented by the formula [V] from the compound represented by the formula [II'] or a derivative thereof includes, for example:
1) a method of preparing the compound represented by the formula [V] using, as an intermediate, a compound represented by the formula [VIII]: 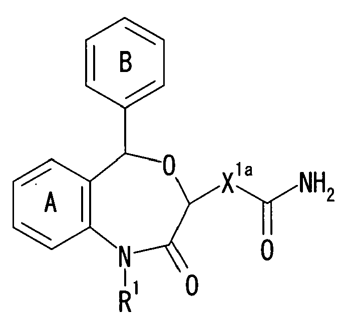 obtained from the compound represented by the formula [II'] by a generally known amidation method;
2) a method of preparing the compound represented by the formula [V] using, as an intermediate, a compound represented by the formula [IX]: 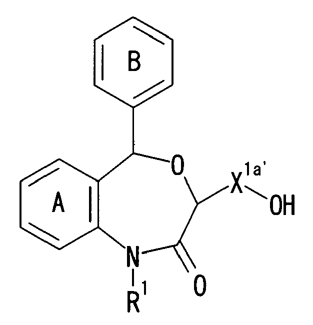 wherein X^{1a'} represents an optionally substituted lower alkylene, obtained by a reduction reaction of carboxylic acid or a derivative thereof from the compound represented by the formula [II'] or a derivative thereof;
3) a method of preparing the compound represented by the formula [V] using, as an intermediate, a compound represented by the formula [X] : 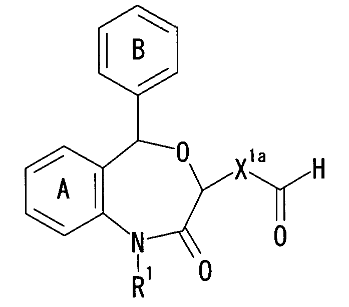 obtained by a generally known reduction reaction of carboxylic acid or a derivative thereof from the compound represented by the formula [II'] or a derivative thereof, or by a generally known oxidation reaction of alcohol from the compound represented by the formula [IX]; or the like.

An amidation method includes a method comprising condensation with ammonia or 1-hydroxybenzotriazole ammonia (HOBt·NH₃) using a dehydration condensing agent and a method comprising preparation of amide represented by the formula [XI]: 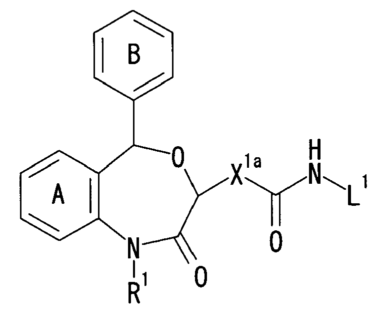 wherein L¹ represents a protecting group for an amino group, followed by removal of the protecting group for an amino group.

A reduction reaction of carboxylic acid or a derivative thereof includes a reduction reaction of carboxylic acid or a derivative of carboxylic acid using a reducing agent such as lithium aluminum hydride, diisobutylaluminum hydride sodium borohydride, or the like.

An oxidation reaction of alcohol includes, for example, a method comprising treatment with a sulfur trioxide pyridine complex or oxalyl chloride in dimethyl sulfide, an oxidation method using an oxidizing agent such as pyridinium chlorochromate or Dess-martin reagent, etc. and the like.

A method of preparing the compound represented by the formula [V] from the compound represented by the formula [VIII] includes, for example, a method comprising treatment with anhydride such as trifluoromethanesulfonic acid anhydride, a method comprising treatment with a dehydration condensing agent such as carbonyldiimidazole in the presence of allyl bromide and the like.

A method of preparing the compound represented by the formula [V] from the compound represented by the formula [IX] includes, for example, a method comprising convertion of the hydroxyl group into a substitutable group followed by treatment with cyanide, a method comprising Mitsunobu reaction in the presence of cyanide such as acetone cyanohydrin (a method comprising treatment with an azodicarboxylic acid derivative such as 1,1'-(azodicarbonyl)dipiperidine and an organic phosphorus compound such as tri-n-butylphosphine or triphenylphosphine) and the like.

A method of preparing the compound represented by the formula [V] from the compound represented by the formula [X] includes, for example, a method comprising reaction with an oxidizing agent such as iodine in aqueous ammonia, a method comprising Wittig reaction with phosphoric acid ester containing a cyano group in its molecule such as cyanomethylphosphoric acid diethylester followed by hydrogenation reaction and the like.

In the step 4, a method of preparing the compound represented by the formula [VI] from the compound represented by the formula [V] includes, for example, a method comprising treatment of the compound represented by the formula [V] with dithiophosphoric acid diethylester or the like, a method comprising treatment with hydroxylammonium chloride in the presence of an alkali such as sodium hydrogen carbonate.

In addition, a method of preparing the compound represented by the formula [VI] includes, for example, a method comprising treatment of the compound represented by the formula [VIII] with a sulfurizing agent such as diphosphorus pentasulfide and the like.

In the step 5, a method of preparing the compound represented by the formula [I] from the compound represented by the formula [VI] and the compound represented by the formula [VII] includes, for example, a method comprising heating the compound represented by the formula [VI] in the presence of the compound represented by the formula [VII] and the like.

Among the compounds represented by the formula [VII], a compound represented by the formula [XII]: 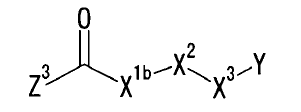 wherein Z³ represents a lower alkyl group in which it is substituted with a halogen atom on the carbon atom adjacent to the carbonyl group, may be prepared by, for example, Process C or the like. 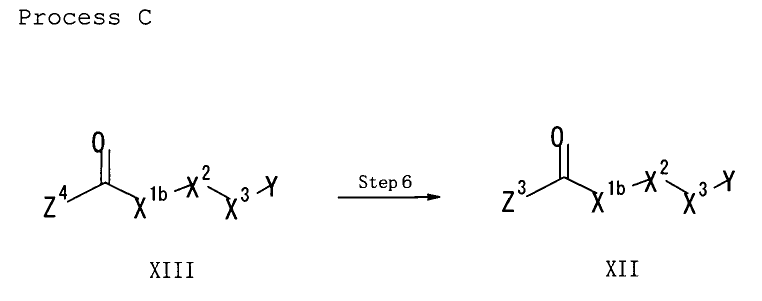 wherein Z⁴ represents a halogen atom.

In the step 6, a method of preparing the compound represented by the formula [XII] from the compound represented by the formula [XIII] includes, for example, a method comprising a reaction with diazomethane or trimethylsilyldiazomethane in a solution of hydrobromic acid in acetic acid and the like.

In each reaction of the process for preparing the compound [I] or a salt thereof and each reaction for synthesis of the starting compounds described above, when the starting compound has an amino group, a carboxyl group or a hydroxy group as a substituent, such a substituent may be protected with a protecting group which is usually used in peptide chemistry or the like. After reaction, if necessary, the protecting group can be removed to obtain the desired compound.

A protecting group for an amino group includes, for example, formyl, C₁₋₆ alkylcarbonyl (e.g. acetyl, ethylcarbonyl etc.), phenylcarbonyl, C₁₋₆ alkyl-oxycarbonyl (e.g. methoxycarbonyl, ethoxycarbonyl etc.), phenyloxycarbonyl, C₇₋₁₀ aralkyl-carbonyl (e.g. benzylcarbonyl etc.), trityl, phthaloyl, N,N-dimethylaminomethylene and the like, each of which may be optionally substituted. As such a substituent, a halogen atom (e.g. fluorine, chlorine, bromine, iodine etc.), C₁₋₆ alkyl-carbonyl (e.g. methylcarbonyl, ethylcarbonyl, butylcarbonyl etc.), a nitro group or the like is used and the number of substituents is about 1 to 3.

A protecting group for a carboxyl group includes, for example,C₁₋₆ alkyl (e.g. methyl, ethyl, n-propyl, i-propyl, n-butyl, tert-butyl etc.), phenyl, trityl, silyl and the like, each of which may be optionally substituted. As such a substituent, a halogen atom (e.g. fluorine, chlorine, bromine, iodine etc.), formyl, C₁₋₆ alkyl-carbonyl (e.g. acetyl, ethylcarbonyl, butylcarbonyl etc.), a nitro group or the like is used and the number of substituents is about 1 to 3.

A protecting group for a hydroxy group includes, for example, C₁₋₆ alkyl (e.g. methyl, ethyl, n-propyl, i-propyl, n-butyl, tert-butyl etc.), phenyl, C₇₋₁₀ aralkyl (e.g. benzyl etc.), formyl, C₁₋₆ alkyl-carbonyl (e.g. acetyl, ethylcarbonyl etc.), phenyloxycarbonyl, benzoyl, C₇₋₁₀ aralkyl-carbonyl (e.g. benzylcarbonyl etc.), pyranyl, furanyl, silyl and the like, each of which may be optionally substituted. As such a substituent, a halogen atom (e.g. fluorine, chlorine, bromine, iodine etc.), C₁₋₆ alkyl (e.g. methyl, ethyl, n-propyl etc.), phenyl, C₇₋₁₀ aralkyl (e.g. benzyl etc.), a nitro group or the like is used and the number of substituents is about 1 to 4.

A method of removing a protecting group may be a method known per se or the similar method, and for example, a method comprising treatment with acid, base, reduction, ultraviolet light, hydrazine, phenylhydrazine, sodium N-methyldithiocarbamate, tetrabutylammonium fluoride, palladium acetate or the like is used.

In addition, in each reaction of the process for preparing the compound [I] or a salt thereof and each reaction for synthesizing the starting compounds described above, a generally known solvent may be used during the reaction.

A generally known solvent includes, for example, ethers such as tetrahydrofuran, diethyl ether, 1,2-dimethoxyethane and 1,4-dioxane; esters such as ethyl acetate and butyl acetate; aromatic hydrocarbons such as benzene and toluene; aromatic heterocyclic compounds such as pyridine and lutidine; amides such as N,N-dimethylformamide and N-methylpyrrolidone; halides such as chloroform and methylene chloride; alcohols such as methanol, ethanol, 2-propanol and 2,2-dimethylethanol; hydrocarbon compounds such as hexane, heptane and petroleum ether; carboxylic acids such as formic acid and acetic acid; water; and the like.

A solvent used in the reaction may be a single solvent or a mixture of 2 to 6 solvents.

The reaction may be also performed in the presence of a base such as amines such as triethylamine, N,N-diisopropylamine, pyridine or N-methylmorpholine, sodium hydroxide, potassium carbonate or the like.

The reaction may be also performed in the presence of an acid such as hydrochloric acid, sulfuric acid, acetic acid or the like.

The compound represented by the formula [I] or a salt thereof obtained by the above methods can be isolated or purified by a conventional separation means such as recrystallization, distillation, chromatography or the like. When the compound represented by the formula [I] of the present invention thus obtained is in the free form, it can be converted into a salt form by a method known per se or the similar method (e.g. neutralization, etc.). Conversely, when the compound is obtained as a salt, it can be converted into the free form or another salt by a method known per se or the similar method. When the resulting compound is in the racemic form, it can be separated into the d-form and the 1-form by a conventional optical resolution means.

The starting compounds of the compound represented by the formula [I] or a salt thereof may be also in a salt form similar to a salt of the compound represented by the formula [I], but the kind of the salt is not particularly limited as long as the reaction is not disturbed.

The compound represented by the formula [II] or a derivative thereof can be prepared by, for example, a method described in EPA567026, WO95/21834 (PCT application based on Japanese Patent Application No. 6-15531), EPA645377 (application based on Japanese Patent Application No. 6-229159), EPA645378 (application based on Japanese Patent Application No. 6-229160) or JP-A 9-136880, or the similar method.

Since the compound represented by the formula [I] or a salt or a prodrug thereof (hereinafter sometimes referred to merely as the compound (I), including a salt thereof and a prodrug thereof) is low toxic and has squalene synthase inhibitory activity, LDL cholesterol lowering activity, triglyceride lowering activity, lipid lowering activity, non-HDL cholesterol lowering activity and HDL cholesterol level elevating activity, it is useful as a safe medicament for prevention or treatment of hyperlipemia such as hypercholesterolemia, hypertriglyceridemia or hypoHDLemia in a mammal (e.g., mouse, rat, rabbit, dog, cat, cattle, pig, monkey, human and the like) and is also useful for prevention or treatment of metabolic syndrome. Moreover, the compound is useful as a safe medicament for prevention or treatment of kidney disease such as nephritis or nephropathy, arteriosclerosis, ischemic disease, heart infarction, angina pectoris, heart failure, aneurysm, cerebral arteriosclerosis, cerebral apoplexy, transient ischemic stroke, brain infarction, peripheral arteriosclerosis, intermittent claudication, thrombosis, hypertension, osteoporosis, diabetes (e.g., insulin resistance-dependent type and the like), diabetic complications (e.g. diabetic nephropathy, diabetic neuropathy, diabetic retinopathy, diabetic angiopathy and the like), pancreatic disorder, or restenosis after percutaneous coronary plasty (PTCA) or stent placement.

Hereinafter the utility of the present invention is explained in detail.

The compound (I) has superior triglyceride lowering activity and cholesterol lowering activity as well as biological characteristics thereof, and is specifically suitable for treatment or prevention of hyperlipemia, in particular hypertriglyceridemia, hyperlipoproteinemia and hypercholesterolemia, as well as metabolic syndrome and atherosclerotic vascular lesion arising therefrom and secondary disease thereof such as coronary artery disease, cardiac infarction, angina pectoris, brain infarction, brain ischemia, intermittent claudication, peripheral arteriosclerosis, gangrene and the like.

For treatment of these diseases, the compound (I) may be used alone or may be used in combination with other drug ingredients such as a lipid lowering agent, a cholesterol lowering agent, an HDL level elevating agent or a cholesterol absorption inhibitor (administered simultaneously or administered at a certain interval). In this case, these compounds are preferably administered as oral preparations (including intraoral disintegrating tablets) or they may be optionally administered in the form of suppository as rectal preparations. In this case, possible ingredients for the combination use include, for example, a PPAR_{α} agonist such as fibrates [e.g., Clofibrate, Benzafibrate, Gemfibrozil, Fenofibrate, Wy-1463, GW9578 and the like]; and a cholesterol lowering agent such as nicotinic acid and a derivative and analogue thereof [e.g., Acipimox and Probucol], a bile acid-binding resin [e.g., Cholestyramine, Colestipol and the like], a compound that suppresses absorption of cholesterol [e.g., Ezetimibe, Sitosterol, Neomycin and the like], a compound that inhibits biosynthesis of cholesterol [e.g., an HMG-CoA reductase inhibitor such as Lovastatin, Simvastatin, Pravastatin, Atorvastatin, ZD-4522 (Rosuvastatin), Itavastatin and the like], a squalene epoxidase inhibitor [e.g., NB-598 and an analogue compound thereof and the like] or an HDL level elevating agent due to inhibition of cholesterol ester-transporting protein [e.g. JTT-705, CP-529-414 and the like]. In addition, a combination with an acyl-Coenzyme A cholesterol acyltransferase (ACAT) inhibitor (e.g. melinamide etc.) or a lipid rich plaque regressing agent (e.g. compounds described in WO 02/06264, WO 03/059900 etc.) is possible. Further, a combination with a body fat lowering agent such as a pancreatic lipase inhibitor (e.g.orlistat etc.) is also possible.

Another possible ingredient for the combination use includes an oxide squalene-lanosterol cyclase inhibitor, for example, a decalin derivative, an azadecalin derivative or an indan derivative.

In particular, by combining with an HMG-CoA reductase inhibitor, myalgia and myolysis which are also the side effect of an HMG-CoA reductase inhibitor may be alleviated. Further, this combination not only has lipid lowering activity but also is effective against osteoporosis and Alzheimer's disease, and may improve the prognosis of ischemic disease (cerebral apoplexy, cardiac infarction or the like).

In addition, the compound (I) is suitable for treatment of disease associated with hyperchylomicronemia, for example, acute pancreatitis. Regarding the mechanism of onset of pancreatitis, it is said that chylomicrons cause microembolization in a pancreatic capillary blood vessel, or hyperchylomicronemia results in increase in production of free fatty acid by digestion of triglyceride with pancreatic lipase and then the free fatty acid stimulates strongly a local place, whereby pancreatitis is developed. Therefore, since the compound (I) has triglyceride lowering activity, it can treat pancreatitis and it can be used for treating pancreatitis alone or in combination with a known therapy. For treatment of this disease, the compound (I) can be administered orally or topically and it can be used alone or in combination with a known active compound. In this case, ingredients which can be combined include, for example, aprotinin (Trasylol), gabexate mesilate (FOY), nafamostat mesilate (Fusan), citicoline (Nicoline), urinastatin (Miraclid) and the like for anti-enzymatic therapy. For the purpose of alleviation of pain, an anticholinergic agent, a non-narcotic analgesic or a narcotic is also used.

A further notable example of application of the compound (I) is application for secondary hyperlipemia. The disease includes diabetes, hypothyroidism, nephrotic syndrome or chronic renal failure and the like, and hyperlipemia onsets subsequent to these diseases. In many cases, hyperlipemia aggravates these diseases, namely, forms a vicious circle. In view of lipid lowering activity, the compound (I) is also suitable for treatment or prevention of development of these diseases, and in this case, it can be administered alone or in combination with the medicaments listed below.

Diabetes treating drugs: Kinedak, Penfil, Humalin, Euglucon, Glimicron, Daonil, Novolin, Monotard, insulins, Glucobay, Dimelin, Rastinon, bacilcon, Deamelin S, iszilins; insulin sensitizers (thiazolidine and non-thiazolidine PPAR agonists: pioglitazone, Avandia, KRP-297, TAK-559, MCC-555 etc., isoxazolidine drugs: JTT-501 etc.), biguanide drugs (e.g. mitiglinide etc.), insulin secretion promoting drugs (e.g. sulfonylurea agents etc.), α-glucocidase inhibitors (e.g. voglibose etc.), β₃-agonists (e.g. TAK-677 etc.), insulin preparations etc.; Hypothyroidism treating drugs: dried thyroid (Thireoid), levothyroxine sodium (Thyradin S), liothyronine sodium (Thyronine, Thyronamin); Nephrotic syndrome terating drugs: prednisolone (Predonine), prednisolone sodium succinate (Predonine), methylprednisolone sodium succinate (Solu-Medrol), betamethasone (Lynderon); Anticoagulant therapy agents: dipyridamole (Persantin), dilazep hydrochloride (Comelian) and the like;

Chronic renal failure terating drugs: diuretic [e.g., furosemide (Lasix), bumetanide (Lunetoron), azosemide (Diart) etc.], ACE inhibitors [e.g. enalapril maleate (Renivace) etc.], angiotensin II receptor antagonists [e.g. candesartan cilexetil (Blopress), losartan potassium (Nu-lotan), valsartan (Diovan), irbesartan etc.], Ca antagonists (maninhiron), α receptor blockers and the like. In combination with these drugs, the compound (I) can be preferably orally administered.

Since hyperlipemia aggravates arterial sclerosis and causes hypertension, the compound (I) is suitable for treatment or prevention of hypertension, and in this case, the compound (I) can be administered alone or in combination with the medicaments listed below. In this case, the possible combination includes combinations with, for example, angiotensin-II antagonists [e.g., losartan potassium (Nu-Lotan), candesartan cilexetil (Blopress) and the like], ACE inhibitors [e.g., enalapril maleate (Renivace), lisinopril (Zestril, Longes), delapril hydrochloride (Adecut), captopril and the like], calcium antagonists [e.g., amlodipine besylate (Amlodin, Norvasc), manidipine hydrochloride (Calslot) and the like], hypotensive diuretic, α receptor blockers, β receptor blockers and the like.

A further notable indication of the compound (I) is osteoporosis accompanied by increase of blood cholesterol. Because of the superior lipid lowering activity, the compound (I) can be used for treatment or prevention of osteoporosis accompanied by increase of blood cholesterol, and in this case, the compound (I) can be administered alone or in combination with the medicaments listed below. The possible combination includes combinations with a sex hormone and associated drugs [e.g., an estrogen preparation, ipriflavone (Osten), raloxifene, osaterone, tibolone and the like], a bone resorption inhibitor such as calcitonins, vitamin D preparations [e.g., alfacalcidol, calcitriol and the like], bisphosphonates (e.g., etidronate, clodronate and the like) and the like, a bone formation-accelerating agent such as fluorine compound, PTH and the like.

Further possible application of the compound (I) is use for suppression of thrombus formation. Since blood triglyceride level and Factor VII that involves in blood clotting have a positive correlation, and triglyceride is decreased and clotting is suppressed by intake of ω-3 fatty acid, hypertriglyceridemia accelerates thrombus formation. Furthermore, since the VLDL of a hyperlipemia patient increased secretion of a plasminogen activator inhibitor from a vascular endothelial cell more strongly than that of a person having normal blood lipid, it is also thought that triglyceride (hereinafter referred as TG) decreases the fibrinolysis ability. Therefore, in view of the TG lowering activity, the compound (I) is suitable for prevention or treatment of thrombus formation. In this case, the compound can be used alone or in combination with the known therapeutic agents listed below, preferably for oral administration.

Thrombus formation preventing or treating drugs: blood coagulation inhibitors [e.g., heparin sodium, heparin calcium, warfarin calcium (Warfarin) etc.], thrombolytic drugs [e.g., urokinase], antiplatelet drugs [e.g., aspirin, sulfinpyrazone (Anturan), dipyridamole (Persantin), ticlopidine (Panaldine), cilostazol (Pletal), clopidogrel etc.].

In addition, the compound (I) has superior high-density lipoprotein-cholesterol level elevating activity and is low toxic. Therefore, these compounds and salts thereof can be used safely as, for example, an agent for prevention or treatment of primary low or high-density lipoprotein-cholesterolemia, Tangier disease or the like, as well as an agent for prevention or treatment of cardiac infarction, atherosclerotic disease, hyperlipemia, mixed-type lipid abnormality, diabetes, diabetic complication or the like and can be used for preventing or treating arteriosclerosis, hyperlipemia, hypertension, diabetes, diabetic complication, diabetic nephropathy, diabetic neuropathy, diabetic retinopathy, arrhythmia, peripheral vascular disease, thrombosis, pancreatic disorder, ischemic cardiac disease, brain ischemia, sequela of cardiac infarction, heart failure, cardiac valvular disease or Alzheimer's disease, in a mammal (e.g., mouse, rat, hamster, rabbit, cat, dog, cattle, horse, sheep, monkey, human and the like). In addition, the compound (I) is suitable for treating and preventing hypoHDLemia (including primary hypoHDLemia), Tangier disease, and ischemic heart disease that is developed frequently in a diabetic patient after menopause. In addition, the compound (I) is particularly suitable for treating and preventing hyperlipemia, in particular, hypertriglyceridemia, hyperlipoproteinemia and hypercholesterolemia, as well as atherosclerosis vascular lesion arising therefrom and the secondary diseases thereof such as coronary artery disease, cerebral ischemia, aneurysm, cerebral arteriosclerosis, peripheral arteriosclerosis, intermittent claudication, gangrene and the like

As a further notable example for application of the compound (I), prevention or treatment of Alzheimer's disease is exemplified. It has been known that increase of blood choresterol is a risk factor of Alzheimer's disease. The compound (I) can be used for prevention or treatment of Alzheimer's disease due to its superior high density lipoprotein-cholesterol level elevating activity and lipid lowering activity, and when the compound (I) is used for such purpose, it can be administered alone or in combination with the following drugs. In this case, the possible combination includes combinations with an acetylcholinesterase inhibitor (e.g., Aricept, Excelon and the like), an amyloid β-production or secretion inhibitor (e.g., a γ or β secretase inhibitor such as JT-52, LY-374973 and the like, or SIB-1848 and the like), an amyloid β coagulation inhibitor (e.g., PTI-00703, BETABLOC (AN-1792) and the like) and the like.

In addition, since the compound (I) has blood glucose lowering activity and shows blood glucose lowering activity in a rat suffering from endomorphic diabetes, it improves insulin resistance. It is, in view of its biological characteristics, especially suitable for prevention or treatment of hyperglycemia and the secondary disease arising therefrom such as complications observed in the diabetic nephropathy and renal failure stage, circulatory disease such as anaemia, bone metabolism abnormality, vomiting, nausea, anorexia and diarrhea, nervous symptoms such as neuropathy, diabetic neuropathy, diabetic retinopathy, diabetic vascular disorder, as well as insulin resistance and diseases arising therefrom such as hypertension and impaired glucose tolerance and the secondary disease such as cardiac disease, cardiac failure, ischemic cardiac disease, cerebral ischemia, intermittent claudication and gangrene.

In treatment of these diseases, the high-density lipoprotein-cholesterol level elevating agent of the present invention may be used alone for prevention or treatment, or may be used in combination with other blood glucose lowering drugs or hypotensive drugs. In this case, these compounds are preferably administered as oral preparations (including intraoral disintegrating tablets). Alternatively, if necessary, the compounds may be administered as rectal preparations in the form of suppository. The ingredients that can be combined in this case include (1) an insulin preparation (e.g., human insulin and the like), (2) a sulfonylurea agent (e.g., glibenclamide, gliclazide, glimepiride and the like), (3) an α-glucosidase inhibitor (e.g., voglibose, acarbose and the like), (4) an insulin sensitizer (e.g., pioglitazone, rosiglitazone and the like), (5) an aldose reductase inhibitor (e.g., Epalrestat, Torestat and the like), and a glycation inhibitor (e.g., aminoguanidine and the like).

A combination with a gynecologic disease treating drug (a climacteric disorder treating drug (conjugated estrogen, estradiol, testosterone enanthate, estradiol valerate and the like), a breast cancer treating drug (tamoxifen citrate and the like), a endometriosis and hysteromyoma treating drug (leuprorelin acetate, danazol and the like) or the like, or a combination with such a drug and a diabetes treating drug is also possible.

Furthermore, a combination with a hypotensive agent is possible, and the agent includes, for example, (1) a diuretic (e.g., furosemide, spironolactone and the like), (2) a sympathetic nerve inhibitor (e.g., atenolol and the like), (3) an angiotensin II receptor antagonist (e.g., losartan, candesartan cilexetil, valsartan, irbesartan, olmesartan and the like), (4) an angiotensin I converting enzyme inhibitor (e.g., enalapril maleate, delapril hydrochloride and the like), (5) a calcium antagonist (e.g., nifedipine, manidipine hydrochloride, amlodipine besylate and the like) and the like.

In addition, a combination with a chronic cardiac failure treating drug is possible, and the drug includes, for example, (1) a β receptor blocker, (2) an ACE inhibitor [e.g. enalapril maleate (renivace), lisinopril (longes, zestril etc.)], (3) an angiotensin II receptor antagonist [e.g. candesartan cilexetil (blopress), valsartan, losartan etc.], (4) an aldosterone antagonist (spironolactone, eplerenone), (5) an endothelin antagonist, (6) a Na-H exchange inhibitor, (7) a Na-Ca exchange inhibitor, (8) a MCC-135 and the like.

The compound (I) has superior skeletal muscle protecting activity. That is, the compound (I) exhibits treating of preventing effect against condition in which skeletal muscle is necrosed or colliquated, caused by various factors such as ischemia, effort, excessive exercise, trauma (bruise, skeletal muscle bleeding, electric shock), burn, malignant hyperthermia, malignant syndrome, metabolic myopathy, inflammatory myopathy, muscular dystrophy, infection, intoxication, metabolism abnormality and hyperthermia. More specifically, the compound (I) protects skeletal muscle from cell disorder resulting from the above factors. In addition, the compound (I) has treating or preventing effect against myalgia resulting from cytotoxicity of other drugs (e.g. HMG-CoA reductase inhibitors, cyclosporin, fibrate drugs etc.) and further, rhabdomyolysis in a serious case, etc..

In particular, the compound (I) exhibits superior treating or preventing effect against myalgia induced by a HMG-CoA reductase inhibitor and further, serious rhabdomyolysis, and inhibits decrease of geranylgeranylpyrophosphate in a muscular cell induced by a HMG-CoA reductase inhibitor (e.g. atorvastatin, lovastatin, simvastatin, pravastatin, rosuvastatin, itavastatin, fluvastatin, cerivastatin, pitavastatin etc.).

The compound (I) has superior skeletal muscle protecting activity and is low toxic. Therefore, the agent of the present invention can be safely used, for example, as an agent for preventing or treating rhabdomyolysis, myoglobinuria associated with rhabdomyolysis, or myalgia, in a mammal (e.g. mouse, rat, hamster, rabbit, cat, dog, cow, horse, sheep, monkey, human etc.).

The compound (I) has superior ubiquinone increasing activity, and preventing or treating effect against and inhibiting effect of progression of organ dysfunction and organ failure, and thus, is useful as an agent for preventing or treating and for inhibiting progression of diseases based on this pharmacological activity. Herein, organ dysfunction includes hypofunction of various organs and complications thereof and, inter alia, preferred is ischemic organ dysfunction. That is, the compound of the present invention can prevent cell death caused by ischemia or the like and can protect the function of a cell and an organ, and thus, it can be also used as an agent for maintaining the function of an organ, an agent for protecting an organ, an inhibitor of organ cell death, or the like. Specifically, the compound of the present invention can prevent or treat heart hypofunction (including damage of cardiac muscle), brain hypofunction and pancreatic hypofunction resulting from various factors (in particular, based on ischemia) and the failure of each organ and can inhibit progress to death.

In addition, organ dysfunction may be organ dysfunction resulting from diseases such as arteriosclerotic diseases (cardiac infarction, angina pectoris, brain infarction, cerebral hemorrhage etc.) including ischemic cardiac disease, or may be organ dysfunction occurring during or after an operation or transplantation of an organ. Further, the agent of the present invention is also useful for inhibiting progression, improving prognosis, and preventing secondary development and recurrence of diseases (in particular, ischemic disease such as ischemic cardiac disease or brain infarction) which are causes of the aforementioned organ dysfunctions.

Herein, the organ includes, for example, brain, liver, kidney, heart, spleen, pancreas, eye (retina), and nervous tissue (central nervous tissue, peripheral nervous tissue etc.); preferably, peripheral nervous tissue and, inter alia, preferred are heart and nervous tissue.

Having both of squalene synthase inhibitory activity and ubiquinone increasing activity allows application of the agent to the prognosis of ischemic disease or the like in brain, heart, kidney, nervous tissue, pancreas or the like. For example, in the case of a brain (nervous tissue), inhibiting the progress to a coma, maintaining consciousness or life-prolonging becomes possible, and Parkinson's disease or Alzheimer's disease resulting from central neurodegenerative disease can be treated. In addition, treatment of numbness of hands and feet, imperception, pain, thermal sensitivity abnormality, ulcer, or nervous hyporeflexia resulting from peripheral neuropathy becomes possible. In the case of a heart, treatment of cardiac failure or arrhythmia, or life-prolonging becomes possible. In the case of kidneys, inhibiting the progress to dialysis or renal transplantation, or life-prolonging becomes possible.

The compound of the present invention has superior ubiquinone increasing activity and preventing or treating effect against organ dysfunction or organ failure, and is low toxic. Therefore, the compound of the present invention can be safely used as an agent for preventing or treating cardiac infarction, arteriosclerotic diseases, hyperlipemia, brain infarction, cerebral hemorrhage, neurodegenerative disease or diabetes, or diabetic complications, in addition to an agent for preventing or treating and an agent for inhibiting progression of organ dysfunction or organ failure.

The compound (I) can be used for treating or preventing arteriosclerosis, hyperlipemia, mixed-type lipid abnormality, diabetes, diabetic complications, diabetic nephropathy, diabetic neuropathy, diabetic retinopathy, arrhythmia, peripheral vascular disease, thrombosis, pancreatic disorder, ischemic heart disease, CHD (coronary heart diseases), cerebral ischemia, cardiac infarction, the sequelae of cardiac infarction, heart valvular disease, or Alzheimer's disease. In addition, the compound (I) can be used for preventing or treating cerebral infarction; cerebral hemorrhage; pollakiuria, urinary incontinence or deteriorated excretion of urinary bladder based on neuropathy; Parkinson's disease based on neurodegenerative disease; and cerebral vascular dementia, in addition to cardiac failure and renal failure. The compound (I) is also useful for inhibiting death caused by these diseases or life-prolonging.

Furthermore, ubiquinone has been reported to activate the UCP (uncoupling protein) function. Therefore, the compound (I) is useful for preventing or treating obesity and is suitable for preventing or treating impaired glucose tolerance, diabetes, insulin resistance, hypertension, hyperlipemia or the like associated with obesity.

Since the compound (I) has both of squalene synthase inhibiting activity and ubiquinone increasing activity in addition to lipid metabolism improving activity such as cholesterol lowering activity, triglyceride lowering activity and high-density lipoprotein-cholesterol (HDL) level elevating activity, it can be extremely advantageously used for preventing or treating organ dysfunction or organ failure, inter alia, organ dysfunction or organ failure resulting from diseases such as arteriosclerotic disease (cardiac infarction, angina pectoris, cerebral infarction, cerebral hemorrhage etc.) including ischemic cardiac disease. In addition, since the compound (I) has ubiquinone increasing activity in addition to lipid lowering activity, for example, it can treat ischemic cardiac disease, prevent or treat more serious cardiac failure, and inhibit progress from cardiac failure to death, and thus it is extremely useful as compared with a lipid lowering agent having no ubiquinone increasing activity. In addition, regarding other organs such as brain, kidney and nervous tissue, the compound (I) having ubiquinone increasing activity in addition to lipid lowering activity is excellent in terms of being capable of preventing or treating organ or tissue failure in addition to organ dysfunction, and also has life-prolonging effect.

When the compound (I) is applied to the aforementioned various diseases, it can be used in combination with biologics (e.g. antibodies, vaccine preparations, etc.). Alternatively, the compound (I) can be applied as a combination therapy in combination with genetic therapy. The antibodies and vaccine preparations include vaccine preparations directed to angiotensin II, vaccine preparations directed to CETP, CETP antibodies, TNF α antibodies and antibodies directed to other cytokine, amyloid β vaccine preparations, and 1-type diabetes vaccines (DIAPEP-277 of Peptor Company, etc.), as well as antibodies or vaccine preparations directed to cytokine, renin-angiotensin enzymes and the products thereof, antibodies or vaccine preparations directed to enzymes or proteins involved in blood lipid metabolism, antibodies or vaccines relating to enzymes or proteins involved in coagulation or fibrinogenolysis system in blood, and antibodies or vaccine preparations directed to proteins involved in glucose metabolism or insulin resistance. The genetic therapy includes therapies using genes associated with cytokine, renin-angiotensin enzymes and the products thereof, therapies using DNA decoy such as NFκB decoy, therapies using antisense, therapies using genes associated with enzymes or a proteins involved in blood lipid metabolism (e.g. genes associated with metabolism, excretion or absorption of cholesterol, triglyceride, HDL-cholesterol or blood phospholipid), therapies using genes associated with enzymes or proteins (e.g. growth factors such as HGF, VEGF etc.) involved in vascularization therapy for peripheral vascular obstruction, therapies using genes associated with proteins involved in glucose metabolism or insulin resistance, and antisense for cytokine such as TNF. Alternatively, the compound (I) can be used in combination with various organs regeneration method such as heart regeneration, kidney regeneration, pancreas regeneration or revascularization, or vascularization therapy utilizing transplantation of marrow cells (marrow mononuclear cell, marrow stem cell etc.).

When the compound (I) is combined with other drugs upon application to the aforementioned respective diseases, these active ingredients may be administered as a combined agent obtained by formulating them in one preparation.

The compound (I) can be used orally or parenterally via injection, infusion, inhalation, rectal administration or local administration, and can be used as it is or as a pharmaceutical composition (e.g. powders, granules, tablets, pills, capsules, injections, syrups, emulsions, elixirs, suspensions, solutions etc.). That is, at least one compound (I) can be used alone or as a mixture of a pharmaceutically acceptable carrier (adjuvant, excipient, additive and/or diluent).

For the medicine of the present invention, the compound (I) which is the active ingredient may be administered as bulk powder, but usually, is administered in a form prepared by a conventional method appropriately using an appropriate amount of a conventional pharmaceutical carrier, for example, an excipient (e.g. calcium carbonate, kaolin, sodium hydrogen carbonate, lactose, starch, crystalline cellulose, talc, granulated sugar, porous substances etc.), a binder (e.g. dextrin, gums, alcoholated starch, gelatin, hydroxypropylcellulose, hydroxypropylmethylcellulose, pullulan etc.), a disintegrant (e.g. carboxymethylcellulose potassium, croscarmerose sodium, crospovidone, low-substituted hydroxypropylcellulose, partially α-starch etc.), a lubricant (e.g. magnesium stearate, calcium stearate, talc, starch, sodium benzoate etc.), a coloring agent (e.g. tar dye, caramel, iron sesquioxide, titanium dioxide, riboflavins), a corrigent (e.g. sweetener, flavor etc.), a stabilizer (e.g. sodium sulfite etc.) and a preservative (e.g. parabens, sorbic acid etc.). The medicine of the invention including the aforementioned agent usually contains the compound (I) in an effective amount for treating and preventing disease. The content of the compound of the formula (I) in the medicine of the present invention is usually 0.1 to 100% by weight of the total preparation. The medicine used in the present invention may contain drug ingredients other than the compound (I) as an active ingredient, said ingredients are not particularly limited as long as the object of the invention is achieved, and can be used at an appropriate combination ratio. Specific examples of the dosage form include tablets (including sugar-coated tablets, film-coated tablets), pills, capsules, granules, fine granules, powders, syrups, emulsions, suspensions, injections, sustained-release injections, inhalants, and ointments. Such preparations are prepared according to a conventional method (e.g. a method described in Japanese Pharmacopoeia).

Specifically, a tablet can be produced by granulating the compound (I) alone or a uniform mixture of the compound (I) and an excipient, a binder, a disintegrant or another suitable additive by a suitable method, followed by addition of a lubricant or the like, and then compressing the resulting granule; by compressing directly the compound (I) alone or a uniform mixture of the compound (I) and an excipient, a binder, a disintegrant or another suitable additive; or by compressing a granule prepared in advance alone or a uniform mixture of the granule and a suitable additive. The present tablet may contain a coloring agent, a corrigent or the like if necessary. The present tablet may be also coated with a suitable coating agent. An injection can be prepared by dissolving, suspending or emulsifying a certain amount of the compound (I) in water for injection, physiological saline, Ringer's solution or the like for an aqueous solvent, or in a conventional vegetable oil or the like for a non-aqueous solvent to obtain a certain amount of the resulting injection, or by putting a certain amount of the compound (I) into a container for injection and then sealing it.

A carrier for an oral preparation includes substances used conventionally in the drug preparation art such as starch, mannitol, crystalline cellulose,carboxymethylcellulose sodium, etc.. A carrier for injection includes distilled water, a physiological saline, a glucose solution and an infusion solution. Additionally, other additives used for general preparation may be suitably added.

The medicine of the present invention can be also used as a sustained-release preparation. The sustained-release preparation may be a microcapsule (e.g. microsphere microcapsule, microparticle etc.) as it is prepared by a drying-in-water method (o/w method, w/o/w method, etc.), a phase separation method, spray drying or a similar manner thereto, or a preparation obtained by formulating the microcapsule or a pharmaceutical composition in sphere form, needle form, pellet form, film form or cream form, as raw material, into various dosage forms. The dosage form includes a parenteral preparation (e.g., an injection or an implant for intramuscular, subcutaneous, organ, etc.; an intramucosal agent for nasal cavity, rectum, uterus, etc., etc.), and an oral preparation (e.g., hard capsule, soft capsule, granule, powder, suspension, etc.).

When the sustained-release preparation is an injection, the sustained-release injection is prepared by dispersing the microcapsule in a dispersing agent (e.g., surfactants such as Tween 80, HCO-60, etc.; polysaccharides such as carboxymethylcellulose, sodium arginate, sodium hyaluronate, etc.; protamine sulfate, polyethyleneglycol, etc.), preservatives (e.g., methylparaben, propylparaben, etc.), isotonizing agents (e.g., sodium chloride, mannitol, sorbitol, glucose, etc.), local anesthetics (e.g., xylocaine hydrochloride, chlorobutanol, etc.), etc. to give an aqueous suspension, or by dispersing the microcapsule in vegetable oil (e.g., sesame oil, corn oil, etc.) or a mixture thereof with phospholipids (e.g., lecithin, etc.) or middle chain fatty acid triglycerides (e.g., Miglyol 812, etc.) to give an oily suspension.

When the sustained-release preparation is a microcapsule, its average particle diameter is about 0.1 to about 300 µm, preferably about 1 to about 150 µm, further preferably about 2 to about 100 µm.

When the microcapsule is formulated into a sterile preparation, there are a method comprising sterilizing the whole steps of preparation, a method comprising sterilizing using gamma ray, a method comprising adding an antiseptic, etc., but the method is not specifically limited.

The medicine of the present invention can be formulated into a preparation according to a conventional method. Such preparation can be usually prepared by mixing/kneading an active ingredient with an additive such as an excipient, a diluent or a carrier. The "parenteral", when used herein, includes subcutaneous injection, intravenous injection, intramuscular injection, intraperitoneal injection and infusion. An injectable preparation, for example, a sterile aqueous or oily suspension for injection may be prepared using a suitable dispersing agent or wetting agent and a suitable suspending agent by a method known in the art. The sterile injectable preparation may be a sterile injectable solution or suspension in a diluent or a solvent, which is non-toxic and can be administered parenterally, for example, an aqueous solution. An acceptable vehicle or solvent which can be used includes water, Ringer's solution, and an isotonic sodium chloride solution. A sterile involatile oil may be also usually used as a solvent or a suspending solvent. For this, any involatile oil or fatty acid can be used, including synthetic or semisynthetic fatty oil or fatty acid, and natural or synthetic or semisynthetic mono- or di- or triglycerides.

A suppository for rectal administration can be prepared by mixing the drug with an appropriate non-stimulative additive, for example, an additive which is solid at the normal temperature but is liquid at the temperature of an intestine tract, is melted in a rectum and releases a drug, such as cocoa butter or polyethylene glycol.

Examples of a solid dosage form for oral administration include the aforementioned powders, granules, tablets, pills and capsules, etc.. A preparation in such dosage form can be prepared by mixing and/or kneading an active ingredient compound with at least one additive, for example, sucrose, lactose, cellulose sugar, mannitol (D-mannitol), maltitol, dextran, starches (e.g. corn starch), microcrystalline cellulose, agar, alginates, chitins, chitosans, pectins, tragacanths gums, gums arabic, gelatins, collagens, caseins, albumin, and synthetic or semisynthetic polymers or glycerides. Such dosage forms can contain further additives as usual, and said additives include an inert diluent, a lubricant such as stearic acid and magnesium stearate, a preservative such as parabens and sorbic acid, an antioxidant such as ascorbic acid, α-tocopherol and cysteine, a disintegrant (e.g. croscarmerose sodium), a binder (e.g. hydroxypropylcellulose), a thickener, a buffer, a sweetener, a flavor, and a perfume. Tablets and pills may be prepared by further enteric coating. Solutions for oral administration include pharmaceutically acceptable emulsions, syrups, elixirs, suspensions and solutions. They may contain an inert diluent which is conventionally used in the art, for example, water and, if necessary, additives. These oral solutions can be prepared according to a conventional method, for example, by mixing an active ingredient compound and an inert diluent and, if necessary, other additives.

An oral preparation may contain, depending on the dosage form, usually about 0.01 to 99 W%, preferably about 0.1 to 90 W%, usually about 0.5 to 50% of the active ingredient compound of the present invention.

The dose for a particular patient is determined depending on the age, weight, general health condition, sex, diet, administration time, administration method, secretion rate, combination of drugs, and extent of symptom of a patient to be treated, or considering them and other factors.

The compound of the present invention has squalene synthase inhibitory activity, cholesterol lowering activity and triglyceride lowering activity, and has high selectivity for transition to a target organ and a wide safety margin. Thus, the compound of the present invention is useful for preventing or treating hyperlipemia as a lipid lowering agent and is also useful for preventing or treating arteriosclerosis.

A medicine containing the compound (I) of the present invention is low toxic and can be safely used. The daily dose of the medicine varies depending on the condition and weight of a patient, the kind of a compound and the administration route, etc. For example, when the medicine is used as an agent for preventing or treating hyperlipemia, the daily dose for an adult (body weight about 60 kg) is about 1 to 500 mg, preferably about 10 to 200 mg of the active ingredient [the compound (I)] in the case of an oral preparation, and is about 0.1 to 100 mg, preferably about 1 to 50 mg, usually about 1 to 20 mg of the active ingredient in the case of a parenteral preparation. No toxicity is found within such a range.

The following Examples, Preparation Examples and Experimental Examples will further explain the present invention in detail, but the present invention is not limited to them.

¹H-NMR spectrum was measured by Varian Gemini 200(200MHz)-type or 300(300MHz)-type spectrometer using tetramethylsilane as an internal standard. All δ values are shown in ppm. The numerical value denoted for a mixed solvent is the mixing ratio of respective solvents based on volume unless otherwise indicated. The % means % by weight unless otherwise indicated. The ratio of elution solvents for silica gel chromatography is based on volume unless otherwise indicated. The room temperature (normal temperature), as used herein, is from about 20°C to about 30°C.

Respective symbols in Examples have the following meanings:
Ac: acetyl, Pr: n-propyl, Me: methyl, Bu: n-butyl, Et: ethyl, ⁱPr: isopropyl, Et₂O: diethyl ether, DMF: N,N-dimethylformamide, DMSO: dimethyl sulfoxide, THF: tetrahydrofuran, s: singlet, d: doublet, t: triplet, q: quartet, dd: double doublet, dt: double triplet, m: multiplet, br: broad, J: coupling constant

### Example 1

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid

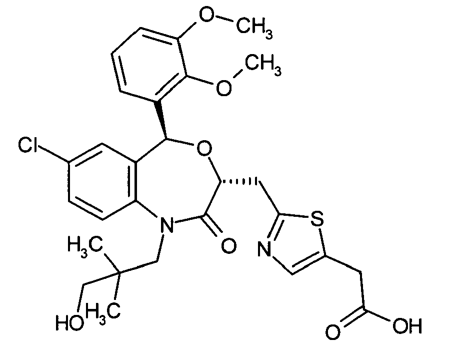

(1) Carbonyldiimidazole (10.2 g, 63.1 mmol) was added to a suspension of 2-(tert-butoxycarbonylamino)acetic acid (10 g, 57.1 mmol) in THF (100 ml) and the mixture was stirred at room temperature for 2 hours. To the reaction solution were added magnesium chloride (5.4 g, 57.1 mmol) and potassium monoethylmalonate (9.7 g, 57.1 mmol) and the mixture was stirred at 60°C for 1 hour. The mixture was filtered to remove insoluble substances. The filtrate was diluted with ethyl acetate (200 ml), washed with 1N hydrochloric acid, an aqueous saturated sodium hydrogen carbonate solution and an aqueous saturated sodium chloride solution, dried over sodium sulfate, and then concentrated under reduced pressure. The residue was purified by silica gel column chromatography [developing solvent: hexane-ethyl acetate (2:1)] to obtain ethyl 4-(tert-butoxycarbonylamino)-3-oxobutanoate (11.9 g, 48.5 mmol, 85%) as a colorless oil.
   ¹H-NMR (CDCl₃) δ: 1.29 (3H, t, J = 7.4 Hz), 1.45 (2H, s), 3.87 (2H, s), 3.96 (3H, s), 4.21 (2H, q, J = 7.2 Hz), 7.08 (1H, d, J = 8.7 Hz), 7.39 (9H, s), 3.49 (2H, s), 4.14 (2H, d, J = 5.8 Hz), 4.21 (2H, q, J = 7.4 Hz), 5.15-5.24 (1H, br).
(2) 4N HCl solution in ethyl acetate (50 ml) was added to a solution of ethyl 4-(tert-butoxycarbonylamino)-3-oxobutanoate (11 g, 44.8 mmol) obtained in Example 1-(1) in acetic acid (6 ml) and the mixture was stirred at room temperature for 30 minutes. The reaction solution was concentrated under reduced pressure and the residue was crystallized from ethyl acetate to obtain ethyl 4-amino-3-oxobutanoate hydrochloride (7.9 g, 43.5 mmol, 97%) as colorless powder.
   ¹H-NMR (CD₃OD) δ: 1.27 (3H, t, J = 7.2 Hz), 3.69 (2H, s), 4.124 (2H, s), 4.20 (2H, q, J = 7.2 Hz).
(3) Pivaloyl chloride (0.51 g, 4.23 mmol) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (2 g, 3.85 mmol) and triethylamine (0.41 g, 4.04 mmol) in acetonitrile (15 ml) at 0°C and the mixture was stirred at 0°C for 30 minutes. To the reaction solution was added ethyl 4-amino-3-oxobutanoate hydrochloride (0.84 g, 4.62 mmol) obtained in Examples 1-(2) at 0°C and then added dropwise a solution of triethylamine (0.58 g, 5.77 mmol) in acetonitrile (2 ml).
   After stirring at room temperature for 30 minutes, the reaction solution was diluted with ethyl acetate (50 ml), washed with 0.1N hydrochloric acid, a 5% aqueous potassium hydrogen sulfate solution, an aqueous saturated sodium hydrogen carbonate solution and an aqueous saturated sodium chloride solution, dried over sodium sulfate, and then concentrated under reduced pressure. The residue was purified by silica gel column chromatography [developing solvent: hexane-ethyl acetate (1:3)] to obtain ethyl 4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-3-oxobutanoate (1.8 g, 2.75 mmol, 71%) as colorless powder.
   ¹H-NMR (CDCl₃) δ: 0.94 (3H, s), 1.01 (3H, s), 1.28 (3H, t, J = 7.0 Hz), 2.03 (3H, s), 2.71 (1H, dd, J = 5.6, 14.8 Hz), 2.79 (1H, dd, J = 7.4, 14.8 Hz), 3.48 (2H, s), 3.54 (1H, d, J = 14.2 Hz), 3.62 (3H, s), 3.71 (1H, d, J = 11.4 Hz), 3.86 (1H, d, J = 11.4 Hz), 3.89 (3H, s), 4.14-4.25 (4H, m), 4.37 (1H, dd, J = 5.6, 7.4 Hz), 4.55 (1H, d, J = 14.2 Hz), 6.26 (1H, s), 6.58-6.62 (1H, br), 6.63 (1H, s), 6.96-7.33 (5H, m).
(4) A solution of ethyl 4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-3-oxobutanoate (0.5 g, 0.773 mmol) obtained in Example 1-(3) and a Lawesson's reagent (0.46 g, 1.16 mmol) in THF (5 ml) was stirred at 70°C for 1 hour. The reaction solution was concentrated under reduced pressure and the residue was purified by silica gel column chromatography [developing solvent: hexane-ethyl acetate (1:1)] to obtain ethyl (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acetate (0.41 g, 0.635 mmol, 82%) as colorless powder.
   ¹H-NMR (CDCl₃) δ: 0.95 (3H, s), 1.03 (3H, s), 1.28 (3H, t, J = 7.2 Hz), 2.03 (3H, s), 3.44 (1H, dd, J = 7.2, 15.0 Hz), 3.55 (1H, dd, J = 6.9, 15.0 Hz), 3.58 (1H, d, J = 14.4 Hz), 3.62 (3H, s), 3.73 (1H, d, J = 10.8 Hz), 3.80 (2H, s), 3.85 (1H, d, J = 10.8 Hz), 3.89 (3H, s), 4.20 (2H, q, J = 7.2 Hz), 4.33 (1H, dd, J = 6.9, 7.2 Hz), 4.57 (1H, d, J = 14.4 Hz), 6.31 (1H, s), 6.60 (1H, t, J = 2.1 Hz), 6.96-7.34 (5H, m), 7.43 (1H, s).
(5) A mixture of ethyl (2-{[(3R, 5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acetate (0.3 g, 0.465 mmol) obtained in Example 1-(4), a 1N aqueous sodium hydroxide solution (1 ml) and ethanol (3 ml) was stirred at 60°C for 30 minutes. This mixture was diluted with water (50 ml), acidified and then extracted with ethyl acetate (50 ml) twice. These extracts was washed with an aqueous saturated sodium chloride solution, dried over sodium sulfate, and then concentrated under reduced pressure. The residue was recrystallized from ethanol-hexane (1:5) to obtain (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid (0.23 g, 0.400 mmol, 86%) as a colorless prismatic crystal.
   m.p.: 219-222°C (dec.) (EtOH-hexane).
   [α] _{D}²² -209.2° (c = 0.34, MeOH).
   IR νₘₐₓ(KBr) cm⁻¹: 3500-2400, 1724, 1658.
   ¹H-NMR (CDCl₃) δ: 0.64 (3H, s), 1.05 (3H, s), 3.19 (1H, d, J = 12.3 Hz), 3.38 (1H, d, J = 14.4 Hz), 3.46 (1H, dd, J = 7.2, 15.0 Hz), 3.59 (1H, dd, J = 6.0, 15.0 Hz), 3.61 (3H, s), 3.64 (1H, d, J = 12.3 Hz), 3.82 (2H, s), 3.88 (3H, s), 4.35 (1H, dd, J = 6.0, 7.2 Hz), 4.47 (1H, d, J = 14.4 Hz), 6.20 (1H, s), 6.58 (1H, d, J = 1.8 Hz), 6.96-7.36 (5H, m), 7.48 (1H, s).
   nal. Calcd for C₂₈H₃₁N₂O₇ClS: C, 58.48; H, 5.43; N, 4.87. Found: C, 58.32; H, 5.30; N, 4.68.

### Example 2

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-methyl-1,3-thiazol-5-yl)acetic acid

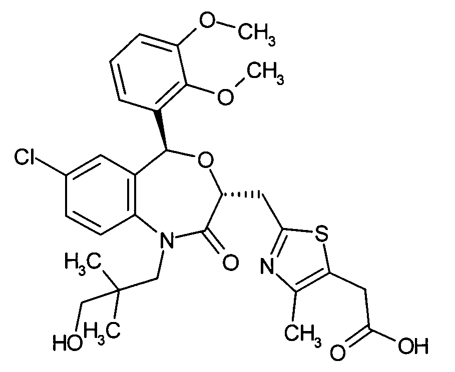

According to a similar manner to that of Example 1, the title compound was obtained.

¹H-NMR (CDCl₃) δ: 0.641 (3H, s), 1.05 (3H, s), 2.29 (3H, s), 3.18 (1H, d, J = 12.3 Hz), 3.37 (1H, d, J = 13.8 Hz), 3.42 (1H, dd, J = 6.9, 15.0 Hz), 3.54 (1H, dd, J = 5.4, 15.0 Hz), 3.61 (3H, s), 3.64 (1H, d, J = 12.3 Hz), 3.73 (2H, s), 3.88 (3H, s), 4.29 (1H, dd, J = 5.4, 6.9 Hz), 4.47 (1H, d, J = 13.8 Hz), 6.19 (1H, s), 6.57 (1H, d, J = 2.1 Hz), 6.96-7.35 (5H, m).

### Example 3

### 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-2-methylpropionic acid

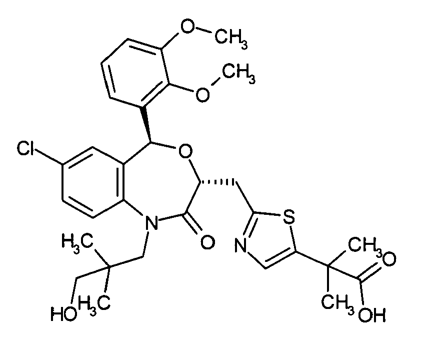

According to a similar manner to that of Example 1, the title compound was obtained.

¹H-NMR (CDCl₃) δ: 0.67 (3H, s), 1.04 (3H, s), 1.65 (6H, s), 3.15 (1H, d, J = 11.6 Hz), 3.41 (1H, dd, J = 7.2, 14.8 Hz), 3.41 (1H, dd, J = 14.4 Hz), 3.54 (1H, dd, J = 6.2, 14.8 Hz), 3.61 (3H, s), 3.62 (1H, d, J = 11.6 Hz), 3.89 (3H, s), 4.34 (1H, dd, J = 6.2, 7.2Hz), 4.48 (1H, d, J = 14.4 Hz), 6.20 (1H, s), 6.55 (1H, s), 6.96-7.36 (5H, m), 7.46 (1H, s).

### Example 4

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-ethyl-1,3-thiazol-5-yl)acetic acid

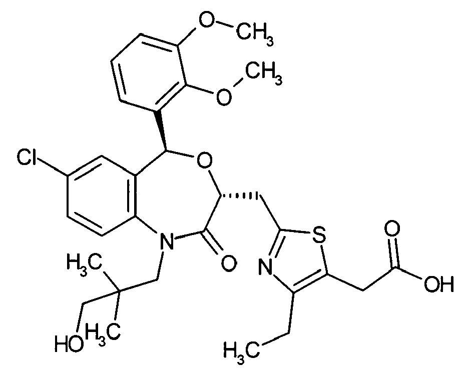

According to a similar manner to that of Example 1, the title compound was obtained.

¹H-NMR (CDCl₃) δ: 0.64 (3H, s), 1. 05 (3H, s), 1. 18 (3H, t, J = 7.5 Hz), 2.63 (2H, q, J = 7.5 Hz), 3.18 (1H, d, J = 12.0 Hz), 3.37 (1H, d, J = 14.4 Hz), 3.43 (1H, dd, J = 7.2, 15.0 Hz), 3.54 (1H, dd, J = 6.0, 15.0 Hz), 3.61 (3H, s), 3.64 (1H, d, J = 12.0 Hz), 3.74 (2H, s), 3.88 (3H, s), 4.31 (1H, dd, J = 6.0, 7.2 Hz), 4.47 (1H, d, J = 14.4 Hz), 6.19 (1H, s), 6.57 (1H, d, J = 1.8Hz), 6.95-7.36 (5H, m).

### Example 5

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)propionic acid

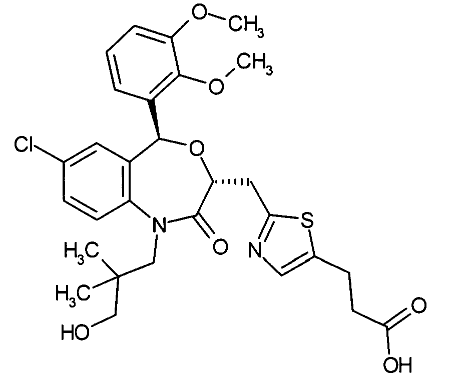

(1) Carbonyldiimidazole (9.7 g, 60 mmol) was added to a solution of monoethylsuccinic acid (7.3 g, 50 mmol) in THF (70 ml) and the mixture was stirred at room temperature for 1 hour. To the reaction solution were added magnesium chloride (4.8 g, 50 mmol) and potassium monoethylmalonate (8.5 g, 50 mmol) and the mixture was stirred at 60°C for 1 hour. The mixture was filtered to remove insoluble substances and the filtrate was concentrated under reduced pressure. The residue was diluted with water, acidified and then extracted with ethyl acetate (70 ml) twice. The extracts were washed with an aqueous saturated sodium chloride solution, dried over sodium sulfate, and then concentrated under reduced pressure. The residue was purified by silica gel column chromatography [developing solvent: hexane-ethyl acetate (3:1)] to obtain diethyl 3-oxohexanedioate (8.9 g, 41.2 mmol, 82%) as a colorless oil.
   ¹H-NMR (CDCl₃) δ: 1.26 (3H, t, J = 7.2 Hz), 1.29 (3H, t, J = 7.2 Hz), 2.61 (2H, t, J = 6.6 Hz), 2.87 (2H, t, J = 6.6 Hz), 3.50 (97/100 x 2H, s), 4.14 (2H, q, J = 7.2 Hz), 4.21 (2H, q, J = 7.2 Hz), 5.02 (3/100 x 1H, s).
(2) A solution of sodium nitrite (3.3 g, 48 mmol) in water (40 ml) was added dropwise to a solution of diethyl 3-oxohexanedioate (8.7 g, 40.2 mmol) obtained in Example 5-(1) in acetic acid (20 ml) under ice-cooling. Further, water (60 ml) was added and the mixture was stirred overnight. The reaction solution was extracted with ethyl acetate (50 ml) twice. The extracts were washed with an aqueous saturated sodium hydrogen carbonate solution and an aqueous saturated sodium chloride solution, dried over sodium sulfate, and then concentrated under reduced pressure. The residue was purified by silica gel column chromatography [developing solution: hexane-ethyl acetate (2:1)] to obtain diethyl 2-hydroxyimino-3-oxohexanedioate (9.3 g, 37.9 mmol, 94%) as a colorless oil.
   ¹H-NMR (CDCl₃) δ: 1.27 (3H, t, J = 7.4 Hz), 1.35 (3H, t, J = 7.4 Hz), 2.68 (2H, t, J = 6.6 Hz), 3.17 (2H, t, J = 6.6 Hz), 4.17 (2H, q, J = 7.4 Hz), 4.38 (2H, q, J = 7.4 Hz).
(3) Zinc powder (18.7 g, 0.286 mol) was added to a solution of diethyl 2-hydroxyimino-3-oxohexanedioate (9 g, 36.7 mmol) obtained in Example 5-(2) and acetic anhydride (26 g, 0.220 mol) in acetic acid (120 ml) at room temperature and the mixture was stirred for 24 hours. The mixture was filtered to remove insoluble substances and the insoluble substances were washed with ethyl acetate (50 ml). The filtrate and the washing solution were combined and then concentrated under reduced pressure to obtain diethyl 2-acetylamino-3-oxohexanedioate (9.0 g, 32.9 mmol, 90%) as a colorless oil.
   ¹H-NMR (CDCl₃) δ: 1.25 (3H, t, J = 7.2 Hz), 1.33 (3H, t, J = 7.2 Hz), 2.08 (3H, s), 2.62-2.67 (2H, m), 2.95 (1H, dt, J = 18.6, 6.6 Hz), 3.11 (1H, dt, J = 18.6, 6.6 Hz), 4.12 (2H, q, J = 7.2 Hz), 4.29 (2H, q, J = 7.2 Hz), 5.30 (1H, d, J = 6.6 Hz), 6.68 (1H, d, J = 6.6 Hz).
(4) Diethyl 2-acetylamino-3-oxohexanedioate (8.8 g, 32.2 mmol) obtained in Example 5-(3) was added to a 4N HCl solution in ethyl acetate (90 ml) and the mixture was heated to reflux for 4 hours. The reaction solution was concentrated under reduced pressure and the residue was crystallized from ethyl acetate-ether to obtain 5-amino-4-oxopentanoic acid hydrochloride (4.4 g, 26.3 mmol, 82%) as a colorless needle crystal.
   ¹H-NMR (CD₃OD) δ: 2.65 (2H, t, J = 7.0 Hz), 2.81 (2H, t, J = 7.0 Hz), 4.02 (2H, s).
(5) Pivaloyl chloride (0.51 g, 4.23 mmol) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (2 g, 3.85 mmol) and triethylamine (0.41 g, 4.04 mmol) in acetonitrile (15 ml) at 0°C and the mixture was stirred at 0°C for 30 minutes. To the reaction solution at 0°C, 5-amino-4-oxopentanoic acid hydrochloride (0.77 g, 4.62 mmol) obtained in Example 5-(4) was added and a solution of triethylamine (0.58 g, 5.77 mmol) in acetonitrile (2 ml) was then added dropwise.
   After stirring at room temperature for 30 minutes, the reaction solution was diluted with ethyl acetate (50 ml), washed with 0.1N hydrochloric acid and an aqueous saturated sodium chloride solution, dried over sodium sulfate, and then concentrated under reduced pressure. The residue was dissolved in DMF (30 ml), potassium carbonate (0.65 g, 4.69 mmol) and methyl iodide (0.67 g, 4.69 mmol) were added, and the mixture was stirred at room temperature for 1 hour. The reaction solution was diluted with ethyl acetate (100 ml), washed with water, a 5% aqueous potassium hydrogen sulfate solution, an aqueous saturated sodium hydrogen carbonate solution and an aqueous saturated sodium chloride solution, dried over sodium sulfate, and then concentrated under reduced pressure. The residue was purified by silica gel column chromatography [developing solvent: hexane-ethyl acetate (1:3)] to obtain methyl 5-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-4-oxopentanoate (1.2 g, 1.85 mmol, 44%) as colorless powder.
   ¹H-NMR (CDCl₃) δ: 0.94 (3H, s), 1. 02 (3H, s), 2.03 (3H, s), 2.60-2.76 (5H, m), 2.93 (1H, dd, J = 7.0, 14.6 Hz), 3.53 (1H, d, J = 14.4 Hz), 3.61 (3H, s), 3.68 (3H, s), 3.71 (1H, d, J = 12.0 Hz), 3.86 (1H, d, J = 12.0 Hz), 3.89 (3H, s), 4.17 (2H, d, J = 4.6 Hz), 4.38 (1H, dd, J = 6.8, 7.0 Hz), 4.55 (1H, d, J = 14.4 Hz), 6.26 (1H, s), 6.58 (1H, t, J = 4.6 Hz), 6.63 (1H, s), 6.96-7.32 (5H, m).
(6) According to the similar manners to those of Examples 1-(4) to (5), 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)propionic acid was obtained.
   ¹H-NMR (CDCl₃) δ: 0.64 (3H, s), 1.05 (3H, s), 2.68 (2H, t, J = 7.0 Hz), 3.11 (2H, t, J = 7.0 Hz), 3.17 (1H, d, J = 11.8 Hz), 3.38 (1H, d, J = 14.2 Hz), 3.42 (1H, dd, J = 7.0, 15.4 Hz), 3.57 (1H, dd, J = 5.8, 15.4 Hz), 3.62 (3H, s), 3.64 (1H, d, J = 11.8 Hz), 3.89 (3H, s), 4.34 (1H, dd, J = 5.8, 7.0 Hz), 4.48 (1H, d, J = 14.2 Hz), 6.20 (1H, s), 6.58 (1H, s), 6.97-7.37 (6H, m).

### Example 6

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)butanoic acid

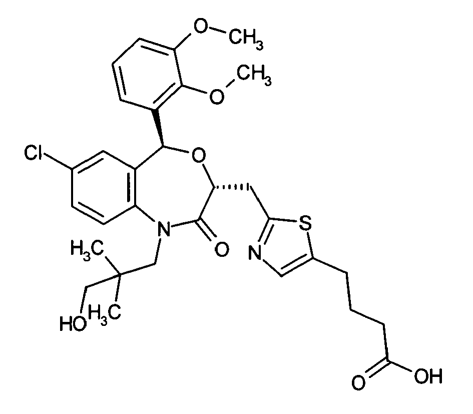

According to a similar manner to that of Example 5, the title compound was obtained.

¹H-NMR (CDCl₃) δ: 0. 64 (3H, s), 1.05 (3H, s), 1.96 (2H, quintet, J = 7.0 Hz), 2.41 (2H, t, J = 7.0 Hz), 2.86 (2H, t, J = 7.0 Hz), 3.17 (1H, d, J = 11.8 Hz), 3.38 (1H, d, J = 14.4 Hz), 3.43 (1H, dd, J = 7.0, 15.0 Hz), 3.57 (1H, dd, J = 5.8, 15.0 Hz), 3.62 (3H, s), 3.64 (1H, d, J = 11.8 Hz), 3.89 (3H, s), 4.35 (1H, dd, J = 5.8, 7.0 Hz), 4.48 (1H, d, J = 14.4 Hz), 6.20 (1H, s), 6.58 (1H, d, J = 1.8 Hz), 6.96-7.38 (6H, m).

### Example 7

### 2-{[(3R,5S)-7-chloro-5-(2,3-dimethoxyphenyl)-1-neopentyl-2-oxo-1,2,3,5-tetrahydro-4,1-benzoxazepin-3-yl]methyl}-1,3-thiazol-5-yl)acetic acid

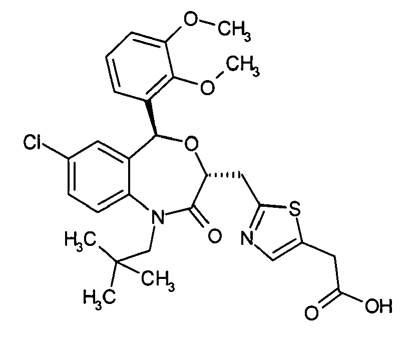

According to a similar manner to that of Example 1, the title compound was obtained.

¹H-NMR (CDCl₃) δ: 0.95 (9H, s), 3.34 (1H, d, J = 13.5 Hz), 3.46 (1H, dd, J = 7.2, 15.0 Hz), 3.57 (1H, dd, J = 6.0, 15.0 Hz), 3.62 (3H, s), 3.84 (2H, s), 3.88 (3H, s), 4.33 (1H, dd, J = 6.0, 7.2 Hz), 4.50 (1H, d, J = 13.5 Hz), 6.31 (1H, s), 6.56 (1H, d, J = 2.1 Hz), 6.90-7.00 (1H, m), 7.10-7.20 (2H, m), 7.26 (1H, d, J = 8.7 Hz), 7.30 (1H, d, J = 2.1, 8.7 Hz),7.47 (1H, s).

### Example 8

### {2-[((3R,5S)-7-chloro-5-(2,3-dimethoxyphenyl)-1-([1-(hydroxymethyl)cyclobutyl]methyl}-2-oxo-1,2,3,5-tetrahydro-4,1-benzoxazepin-3-yl)methyl]-1,3-thiazol-5-yl}acetic acid

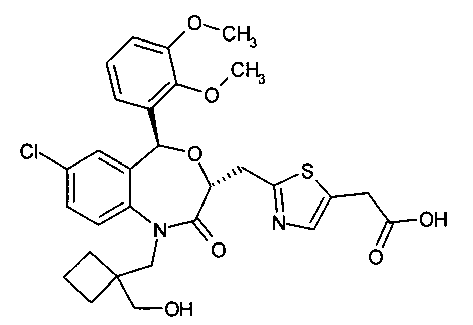

According to a similar manner to that of Example 1, the title compound was obtained.

¹H-NMR (CDCl₃) δ: 1.33-1.55 (2H, m), 1.76-1.94 (3H, m), 1.96-2.12 (1H, m), 3.41-3.78 (9H, m), 3.83 (2H, s), 3.87 (3H, s), 4.34 (1H, dd, J = 6.0, 6.9 Hz), 4.58 (1H, d, J = 14.4 Hz), 6.08 (1H, s), 6.57 (1H, d, J = 2.1 Hz), 6.95 (1H, dd, J = 1.8, 7.8 Hz), 7.07-7.18 (2H, m), 7.33-7.42 (2H, m), 7.48 (1H, s).

### Example 9

### 3-{2-[((3R,5S)-7-chloro-5-(2,3-dimethoxyphenyl)-1-{[1-(hydroxymethyl)cyclobutyl]methyl}-2-oxo-1,2,3,5-tetrahydro-4,1-benzoxazepin-3-yl)methyl]-1,3-thiazol-5-yl}propionic acid

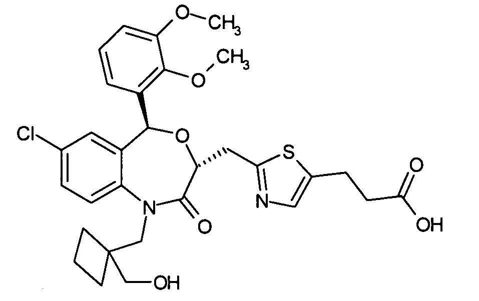

According to a similar manner to that of Example 5, the title compound was obtained.

¹H-NMR (CDCl₃) δ: 1.34-1.55 (2H, m), 1.77-1.95 (3H, m), 1.98-2.10 (1H, m), 2.68 (2H, t, J = 7.5 Hz), 3.11 (2H, t, J = 7.5 Hz), 3.42 (1H, dd, J = 6.9, 15.0 Hz), 3.50-3.82 (7H, m), 3.89 (3H, s), 4.34 (1H, t, J = 6.41 Hz), 4.58 (1H, d, J = 14.32 Hz), 6.09 (1H, s), 6.58 (1H, d, J = 2.1 Hz), 6.98 (1H, dd, J = 1.8, 8.1 Hz), 7.09 (1H, dd, J = 1.5, 7.8 Hz), 7.17 (1H, t, J = 8.1 Hz), 7.35-7.43 (3H, m).

### Example 10

### (2-{[(3R,5S)-7-chloro-5-(2,3-dimethoxyphenyl)-2-oxo-1-propyl-1,2,3,5-tetrahydro-4,1-benzoxazepin-3-yl]methyl}-1,3-thiazol-5-yl)acetic acid

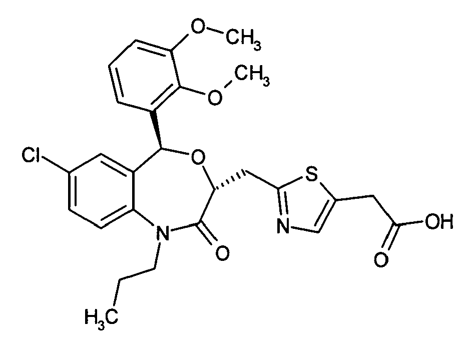

According to a similar manner to that of Example 1, the title compound was obtained.

¹H-NMR (CDCl₃) δ: 0.96 (3H, t, J = 7.5 Hz), 1.57-1.81 (2H, m), 3.43-3.63 (6H, m), 3.84 (2H, s), 3.87 (3H, s), 4.30-4.52 (2H, m), 6.11 (1H, s), 6.60 (1H, d, J = 1.8 Hz), 6.91-7.36 (5H, m), 7.49 (1H, s)

### Example 11

### (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-thiazol-5-yl)acetic acid

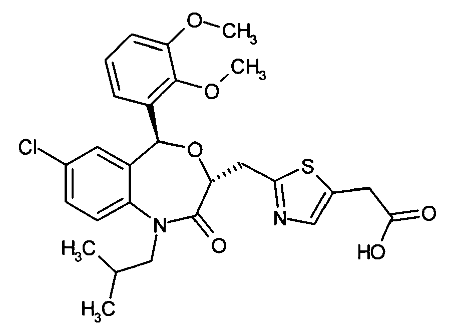

According to a similar manner to that of Example 1, the title compound was obtained.

¹H-NMR (CDCl₃) δ: 0.92 (3H, d, J = 6.4 Hz), 1.01 (3H, d, J = 6.8 Hz), 1.95-2.08 (1H, m), 3.32-3.62 (6H, m), 3.82 (2H, s), 3.87 (3H, s), 4.31-4.40 (2H, m), 6.21 (1H, s), 6.59 (1H, d, J = 2.3 Hz), 6.92-7.35 (5H, m), 7.49 (1H, s).

### Example 12

### (2-{[(3R,5S)-7-chloro-5-(2,3-dimethoxyphenyl)-1-ethyl-2-oxo-1,2,3,5-tetrahydro-4,1-benzoxazepin-3-yl]methyl}-1,3-thiazol-5-yl)acetic acid

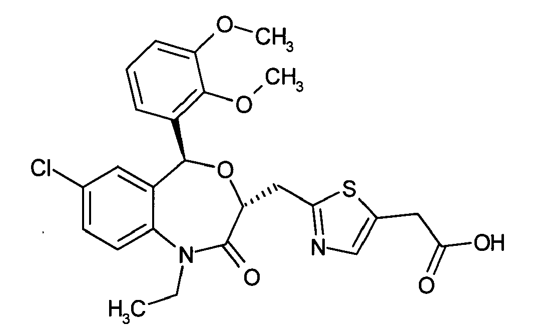

According to a similar manner to that of Example 1, the title compound was obtained.

¹H-NMR (CDCl₃) δ: 1.26 (3H, t, J = 7.2 Hz), 3.43-3.64 (6H, m), 3.84 (2H, s), 3.87 (3H, s), 4.33 (1H, t, J = 6.0 Hz), 4.49-4.62 (1H, m), 6.08 (1H, s), 6.60 (1H, d, J = 1.8 Hz), 6.91-7.36 (5H, m), 7.48 (1H, s).

### Example 13

### (4-Benzyl-2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid

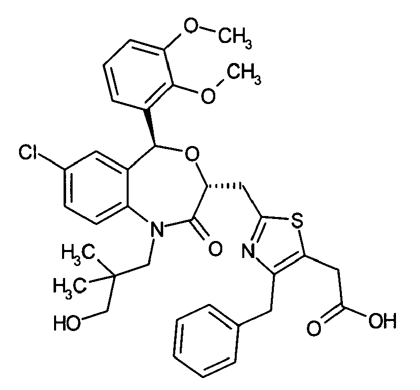

According to a similar manner to that of Example 1, the title compound was obtained.

¹H-NMR (CDCl₃) δ: 0.63 (3H, s), 1.04 (3H, s), 3.15 (1H, d, J = 11.7 Hz), 3.32 (1H, d, J = 14.4 Hz), 3.41 (1H, dd, J = 7.5, 14.7 Hz), 3.53 (1H, dd, J = 6.3, 14.7 Hz), 3.60 (3H, s), 3.62 (1H, d, J = 11.7 Hz), 3.76 (2H, s), 3.87 (3H, s), 4.00 (2H, s), 4.32-4.38 (1H, m), 4.42 (1H, d, J = 14.4 Hz), 6.17 (1H, s), 6.55 (1H, d, J = 2.4 Hz), 6.95 (1H, dd, J = 2.1, 7.2 Hz), 7.02-7.30 (8H, m), 7.32 (1H, dd, J = 2.4, 8.7 Hz).

### Example 14

### 3-(2-([(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)propionic acid

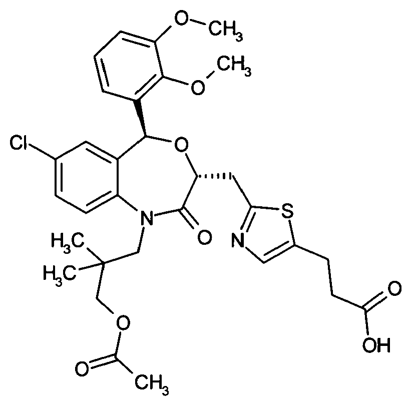

To a solution of 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)propionic acid (0.5 g, 0.849 mmol) obtained in Example 5 in THF (10 ml), pyridine (0.34 ml, 4.24 mol) was added at room temperature and acetyl chloride (0.18 ml, 2.55 mmol) was then added dropwise. The mixture was stirred for 2.5 hours. After water (5 ml) was added, the mixture was stirred at room temperature for 19 hours and the reaction solution was extracted with ethyl acetate. The organic layer was washed with 1N hydrochloric acid, water and an aqueous saturated sodium chloride solution, dried over magnesium sulfate, and then concentrated under reduced pressure to obtain 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)propionic acid (0.5 g, 0.792 mmol, 93%) as colorless powder.

¹H-NMR (CDCl₃) δ: 0.95 (3H, s), 1.23 (3H, s), 2.02 (3H, s), 2.68 (2H, t, J = 7.5 Hz), 3.10 (2H, t, J = 7.5 Hz), 3.42 (1H, dd, J = 7.2, 15.0 Hz), 3.53 (1H, d, J = 14.1 Hz), 3.54 (1H, dd, J = 6.3, 15.0 Hz), 3.61 (3H, s), 3.73 (1H, d, J = 11.1 Hz), 3.85 (1H, d, J = 11.1 Hz), 3.88 (3H, s), 4.32 (1H, dd, J = 6.3, 7.2 Hz), 4.56 (1H, d, J = 14.1 Hz), 6.29 (1H, s), 6.59 (1H, d, J = 2.1 Hz), 6.96 (1H, dd, J = 2.1, 7.8 Hz), 7.06-7.26 (2H, m), 7.27 (1H, d, J = 8.7 Hz), 7.31 (1H, dd, J = 2.1, 8.7 Hz), 7.35 (1H, s).

### Example 15

### 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-methyl-1,3-thiazol-5-carboxylic acid

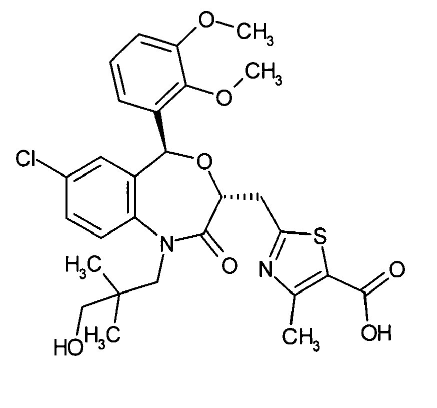

(1) DMF(0.01 ml) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (3 g, 6.49 mmol) and thionyl chloride (2.3 g, 19.5 mmol) in THF (10 ml). The reaction solution was stirred at room temperature for 1 hour and then concentrated under reduced pressure. The residue was dissolved in THF (10 ml) and added dropwise to a solution of hydrogen sulfide in pyridine (30 ml). The reaction solution was stirred at 0°C for 1 hour and then diluted with 2.5M sulfuric acid. The product was extracted with ethyl acetate (50 ml), washed with an aqueous saturated sodium chloride solution, dried over sodium sulfate, and then concentrated under reduced pressure to obtain [(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]thio-S-acetic acid (6.53 g) as colorless powder.
   ¹H-NMR (CDCl₃) δ: 0.94 (3H, s), 1.02 (3H, s), 2.03 (3H, s), 3.11 (1H, dd, J = 6.2, 16.4 Hz), 3.33 (1H, dd, J = 7.0, 16.4 Hz), 3.54 (1H, d, J = 14.0 Hz), 3.61 (3H, s), 3.72 (1H, d, J = 11.4 Hz), 3.85 (1H, d, J = 11.4 Hz), 3.89 (3H, s), 4.37 (1H, dd, J = 6.2, 7.0 Hz), 4.56 (1H, d, J = 14.0 Hz), 6.25 (1H, s), 6.64 (1H, d, J = 1.8 Hz), 6.96-7.35 (5H, m).
(2) A solution of [(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]thio-S-acetic acid (1 g, 1.87 mmol) obtained in Example 15-(1), triethylamine (0.41 g, 4.04 mmol) and methyl 2-chloroacetoacetate (0.31 g, 2.06 mmol) in ether (10 ml) was heated to reflux for 2 hours. After water was added to the reaction solution, the mixture was extracted with ethyl acetate (50 ml), washed with an aqueous saturated sodium chloride solution, dried over sodium sulfate, and then concentrated under reduced pressure. The residue was purified by silica gel column chromatography [developing solvent: hexane-ethyl acetate (2:1)] to obtain methyl 2-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}thio)-3-oxobutanoate (0.9 g, 1.38 mmol, 74%) as colorless powder.
   ¹H-NMR (CDCl₃) δ: 0.94 (3H, s), 1.02 (3H, s), 2.02 (3H, s), 2.18 (3H, s), 3.14-3.26 (2H, m), 3.53 (1H, d, J = 14.4 Hz), 3.61 (3H, s), 3.72 (1H, d, J = 12.0 Hz), 3.74 (9/10 x 3H, s), 3.78 (1/10 x 3H, s), 3.85 (1H, d, J = 12.0 Hz), 3.89 (3H, s), 4.43 (1H, t, J = 6.4 Hz), 4.54 (1H, d, J = 14.4 Hz), 5.50 (1/10 x 3H, s), 6.26 (1H, s), 6.62 (1H, d, J = 1.8 Hz), 6.96-7.35 (5H, m).
(3) A solution of methyl 2-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}thio)-3-oxobutanoate (0.8 g, 1.23 mmol) obtained in Example 15-(2) and ammonium acetate (0.33 g, 4.32 mmol) in acetic acid (5 ml) was heated to reflux for 1 hour. The reaction solution was diluted with ethyl acetate (50 ml), washed with an aqueous saturated sodium hydrogen carbonate solution and an aqueous saturated sodium chloride solution, dried over sodium sulfate, and then concentrated under reduced pressure. The residue was purified by silica gel column chromatography [developing solvent: hexane-ethyl acetate (3:2)]to obtain methyl 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-4-methyl-1,3-thiazol-5-carboxylate (0.62 g, 0.982 mmol, 80%) as colorless powder.
   ¹H-NMR (CDCl₃) δ: 0.95 (3H, s), 1.03 (3H, s), 2.03 (3H, s), 2.65 (3H, s), 3.421 (1H, dd, J = 7.0, 14.8 Hz), 3.53 (1H, d, J = 14.0 Hz), 3.56 (1H, dd, J = 6.0, 14.8 Hz), 3.62 (3H, s), 3.72 (1H, d, J = 11.4 Hz), 3.86 (3H, s),3.86 (1H, d, J = 11.4 Hz), 3.89 (3H, s), 4.31 (1H, dd, J = 6.0, 7.0 Hz), 4.56 (1H, d, J = 14.0 Hz), 6.30 (1H, s), 6.61 (1H, d, J = 2.2 Hz), 6.96-7.37 (5H, m).
(4) A mixture of methyl 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-4-methyl-1,3-thiazol-5-carboxylate (0.4 g, 0.634 mmol) obtained in Example 15-(3), a 1N aqueous sodium hydroxide solution (2 ml) and ethanol (4 ml) was stirred at 60°C for 30 minutes. This mixture was diluted with water (50 ml), acidified and then extracted with ethyl acetate (50 ml) twice. The extracts were washed with an aqueous saturated sodium chloride solution, dried over sodium sulfate, and then concentrated under reduced pressure. The residue was recrystallized from ethanol-hexane (1:1) to obtain 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-methyl-1,3-thiazol-5-carboxylic acid (0.15 g, 0.261 mmol, 41%) as a colorless prismatic crystal.
   ¹H-NMR (CDCl₃) δ: 0.66 (3H, s), 1.04 (3H, s), 2.65 (3H, s), 3.16 (1H, d, J = 12.0 Hz), 3.40 (1H, d, J = 14.1 Hz), 3.43 (1H, dd, J = 6.9, 14.4 Hz), 3.55 (1H, dd, J = 5.7, 14.1 Hz), 3.62 (1H, d, J = 12.0 Hz), 3.62 (3H, s), 3.90 (3H, s), 4.32 (1H, dd, J = 5.7, 6.9 Hz), 4.47 (1H, d, J = 14.1 Hz), 6.21 (1H, s), 6.59 (1H, s), 6.99-7.38 (5H, m).

### Example 16

### 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-carboxylic acid

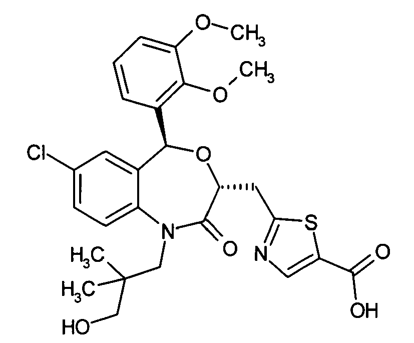

(1) To a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (10 g, 19.2 mmol) in DMF (100 ml), 1-hydroxy-1H-benzotriazole ammonium salt (4.1 g, 26.9 mmol) and 1-ethyl-3-(3-dimethylaminopropyl)carbodiimide hydrochloride (5.16 g, 26.9 mmol) were added. After the mixture was stirred at room temperature for 13 hours, water (50 ml) was added and the mixture was diluted with ethyl acetate (350 ml). The organic layer was washed with 1N hydrochloric acid, aqueous saturated sodium hydrogen carbonate solution, water and an aqueous saturated sodium chloride solution, dried over magnesium sulfate, and then concentrated under reduced pressure to obtain 3-[(3R,5S)-3-(2-amino-2-oxoethyl)-7-chloro-5-(2,3-dimethoxyphenyl)-2-oxo-2,3-dihydro-4,1-benzoxazepin-1(5H)-yl]-2,2-dimethylpropyl acetate (10.0 g) as colorless powder.
   ¹H-NMR (CDCl₃) δ: 0. 95 (3H, s), 1.01 (3H, s), 2.03 (3H, s), 2.68 (1H, dd, J = 5.7, 14.7 Hz), 2.87 (1H, dd, J = 7.5, 14.7 Hz), 3.54 (1H, d, J = 13.8 Hz), 3.61 (3H, s), 3.73 (1H, d, J = 11.1 Hz), 3.86 (1H, d, J = 11.1 Hz), 3.89 (3H, s), 4.39 (1H, dd, J = 5.7, 7.5 Hz), 4.56 (1H, d, J = 13.8 Hz), 5.46 (1H, br), 5.95 (1H, br), 6.26 (1H, s), 6.64 (1H, d, J = 2.4 Hz), 6.91-7.01 (1H, m), 7.15-7.24 (2H, m), 7.31 (1H, d, J = 8.4 Hz), 7.35 (1H, dd, J = 2.4, 8.4 Hz).
(2) A solution of 3-[(3R,5S)-3-(2-amino-2-oxoethyl)-7-chloro-5-(2,3-dimethoxyphenyl)-2-oxo-2,3-dihydro-4,1-benzoxazepin-1(5H)-yl]-2,2-dimethylpropyl acetate (10.8 g, 20.8 mmol) obtained in Example 16-(1) and a Lawesson's reagent (10.1 g, 24.8 mmol) in THF (100 ml) was heated to reflux for 1 hour with stirring. After standing to cool to room temperature, an aqueous saturated sodium hydrogen carbonate solution (20 ml) was added and the mixture was stirred for 30 minutes. The reaction solution was extracted with ethyl acetate (800 ml), washed with water and an aqueous saturated sodium chloride solution, dried over magnesium sulfate, and then concentrated under reduced pressure. The resulting residue was purified by silica gel column chromatography [developing solvent: hexane-ethyl acetate (1:1)] to obtain 3-[(3R,5S)-3-(2-amino-2-thioxoethyl)-7-chloro-5-(2,3-dimethoxyphenyl)-2-oxo-2,3-dihydro-4,1-benzoxazepin-1(5H)-yl]-2,2-dimethylpropyl acetate (5.14 g) as a white crystal.
   ¹H-NMR (CDCl₃) δ: 0.95 (3H, s), 1.01 (3H, s), 2.03 (3H, s), 3.08 (1H, dd, J = 5.4, 13.5 Hz), 3.31 (1H, dd, J = 7.5, 13.5 Hz), 3.55 (1H, d, J = 14.1 Hz), 3.55 (1H, d, J = 14.1 Hz), 3.61 (3H, s), 3.72 (1H, d, J = 11.4 Hz), 3.86 (1H, d, J = 11.4 Hz), 3.89 (3H, s), 4.46 (1H, dd, J = 5.4, 7.5 Hz), 4.53 (1H, d, J = 14.1 Hz), 6.26 (1H, s), 6.65 (1H, d, J = 2.4 Hz), 6.91-7.01 (1H, m), 7.15-7.24 (2H, m), 7.31 (1H, d, J = 8.7 Hz), 7.36 (1H, dd, J = 2.4, 8.7 Hz), 7.49 (1H, br), 7.64 (1H, br)
(3) Acetic acid (0.13 ml, 2.34 mmol) was added to a suspension of ethyl 2-chloro-3-oxopropionate potassium salt (0.42 g, 2.24 mmol) in isopropanol (10 ml) and the mixture was stirred for 30 minutes. Thereto 3-[(3R,5S)-3-(2-amino-2-thioxoethyl)-7-chloro-5-(2,3-dimethoxyphenyl)-2-oxo-2,3-dihydro-4,1-benzoxazepin-1(5H)-yl]-2,2-dimethylpropyl acetate (1.0 g, 1.87 mmol) obtained in Example 16-(2) was added and the mixture was heated to reflux for 30 hours with stirring. After standing to cool, water (5 ml) was added to the reaction solution, followed by extraction with ethyl acetate (200 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (7:3-65:35)] to obtain ethyl 2-{[(3R, 5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]methyl}-1,3-thiazol-5-caboxylate (0.6 g) as colorless noncrystalline powder.
   ¹H-NMR (CDCl₃) δ: 0.95 (3H, s), 1. 02 (3H, s), 1. 31 (3H, t, J = 7.2 Hz), 2.03 (3H, s), 2.82 (1H, dd, J = 5.7, 16.8 Hz), 2.92 (1H, dd, J = 7.5, 16.8 Hz), 3.54 (1H, d, J = 14.1 Hz), 3.62 (3H, s), 3.72 (1H, d, J = 11.1 Hz), 3.85 (1H, d, J = 11.1 Hz), 3.87 (3H, s), 4.12 (2H, q, J = 7.2 Hz), 4.19 (1H, dd, J = 5.7, 7.5 Hz), 4.57 (1H, d, J = 14.1 Hz), 6.28 (1H, s), 6.53-6.55 (1H, m), 6.89-7.40 (5H, m), 8.70 (1H, s).
(4) Potassium carbonate (0.26 g, 1.91 mmol) was added to a solution of ethyl 2-{[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]methyl}-1,3-thiazol-5-caboxylate (0.6 g, 0.95 mmol) obtained in Example 16-(3) in methanol (6 ml) and the mixture was stirred at room temperature for 2 hours. Water (3 ml) was added to the reaction solution, followed by extraction with ethyl acetate (80 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (67:33-3:2)] to obtain methyl 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl-1,3-thiazol-5-carboxylate (0.3 g) as colorless noncrystalline powder.
   ¹H-NMR (CDCl₃) δ: 0.64 (3H, s), 1.05 (3H, s), 3.07-3.21 (1H, m), 3.38 (1H, d, J = 14.1 Hz), 3.58-3.70 (1H, m), 3.61 (3H, s), 3.73 (2H, d, J = 6.6 Hz), 3.85 (3H, s), 3.88 (3H, s), 4.48 (1H, d, J = 14.1 Hz), 4.53 (1H, t, J = 6.6 Hz), 6.17 (1H, s), 6.52-6.55 (1H, m), 6.90-7.10 (2H, m), 7.07-7.20 (1H, m), 7.26-7.40 (2H, m), 8.71 (1H, s).
(5) A mixture of methyl 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl-1,3-thiazol-5-carboxylate (0.3 g, 0.52 mmol) obtained in Example 16-(4), 1N aqueous sodium hydroxide solution (1 ml) and ethanol (5 ml) was stirred at room temperature for 8 hours. After this mixture was acidified with 1N hydrochloric acid (1.2 ml), ethyl acetate (70 ml) was added thereto. The organic layer was washed with an aqueous saturated sodium chloride solution, dried over magnesium sulfate, and then concentrated under reduced pressure. The resulting crystal was washed with hexane to obtain 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-carboxylic acid (0.19 g) as a white crystal.
   ¹H-NMR (CDCl₃) δ: 0.66 (3H, s), 1.05 (3H, s), 3.19 (1H, d, J = 12.3 Hz), 3.40 (1H, d, J = 14.1 Hz), 3.58-3.78 (3H, m), 3.61 (3H, s), 3.87 (3H, s), 4.47 (1H, d, J = 14.1 Hz), 4.57 (1H, t, J = 6.0 Hz), 6.18 (1H, s), 6.56 (1H, s), 6.90-7.01 (2H, m), 7.12 (1H, t, J = 7.8 Hz), 7.30-7.40 (2H, m), 8.76 (1H, s).

### Example 17

### 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-carboxylic acid

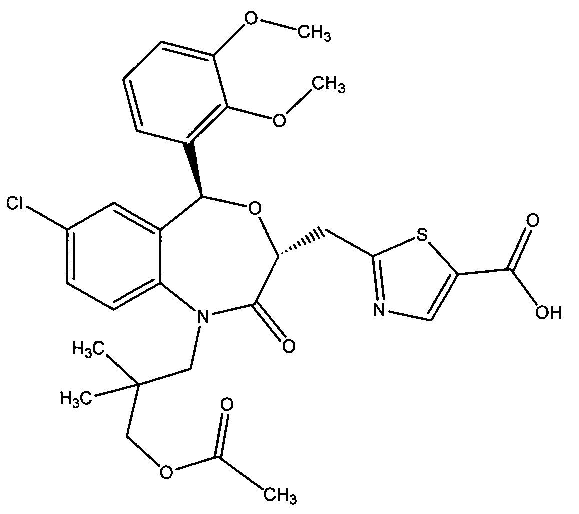

After pyridine (0.9 ml, 8.91 mol) was added to a solution of 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-acetic acid (1.0 g, 1.78 mmol) obtained in Example 16 in THF(10 ml) at room temperature, acetyl chloride (0.38 ml, 5.35 mmol) was added dropwise and the mixture was stirred for 1 hour. Thereto pyridine (0.3 ml, 3.71 mmol) and then acetyl chloride (0.1 ml, 1.41 mmol) were added and the mixture was further stirred for 30 minutes. After water (5 ml) was added, the mixture was stirred at room temperature for 19 hours and the reaction solution was extracted with ethyl acetate. The organic layer was washed with 1N hydrochloric acid, water and an aqueous saturated sodium chloride solution, dried over magnesium sulfate, and then concentrated under reduced pressure to obtain 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-carboxylic acid (0.76 g) as pale brown noncrystalline powder.

¹H-NMR (CDCl₃) δ: 0.95 (3H, s), 1.02 (3H, s), 2.03 (3H, s), 3.42-3.63 (3H, m), 3.63 (3H, s), 3.73 (1H, d, J = 11.1 Hz), 3.70-3.90 (1H, m), 3.85 (1H, d, J = 11.1 Hz), 3.89 (3H, s), 4.55 (1H, d, J = 14.1 Hz), 4.50-4.60 (1H, m), 6.29 (1H, s), 6.63 (1H, d, J = 2.7 Hz), 6.98 (1H, dd, J = 1.5, 8.1 Hz), 7.08 (1H, dd, J = 1.5, 8.1 Hz), 7.17 (1H, t, J = 8.1 Hz), 7.29 (1H, d, J = 9.0 Hz), 7.34 (1H, dd, J = 2.7, 9.0 Hz), 8.76 (1H, s).

### Example 18

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-hydroxypropionic acid

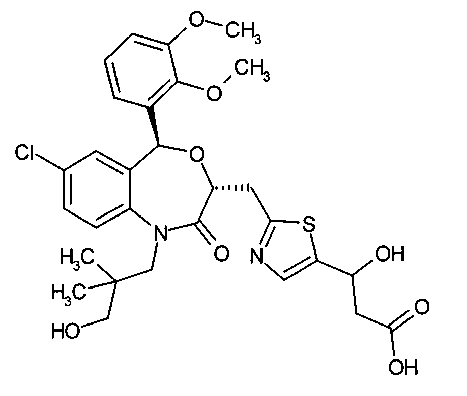

(1) 1,1'-Carbonyldiimidazole (0.24 g, 1.49 mol) was added to a solution of 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-carboxylic acid (0.75 g, 1.24 mmol) obtained in Example 17 in THF (10 ml) at room temperature and the mixture was stirred for 1 hour. Monoethyl malonate potassium (0.23 g, 1.37 mmol) and magnesium chloride (0.13 g, 1.37 mmol) were added and the mixture was stirred at 55°C for 1 hour. After standing to cool, 1N hydrochloric acid (3 ml) was added and the mixture was extracted with ethyl acetate (150 ml). The organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (67:33-1:1)] to obtain ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-oxopropionate (0.59 g, 0.88 mmol, 71%) as colorless noncrystalline powder.
   ¹H-NMR (CDCl₃) δ: 0.94 (3H, s), 1.03 (3H, s), 1.26 (3H, t, J = 7.5 Hz), 2.03 (3H, s), 3.54 (1H, d, J = 14. 1 Hz), 3.58-3.80 (2H, m), 3.60 (3H, s), 3.73 (1H, d, J = 10.8 Hz), 3.84 (1H, d, J = 10.8 Hz), 3.87 (3H, s), 3.93 (1H, d, J = 15.9 Hz), 4.00 (1H, d, J = 15.9 Hz), 4.12 (2H, q, J = 7.5 Hz), 4.50 (1H, t, J = 6.6 Hz), 4.54 (1H, d, J = 14.1 Hz), 6.28 (1H, s), 6.56 (1H, s), 6.89-7.00 (2H, m), 7.10 (1H, t, J = 7.8 Hz), 7.30-7.34 (2H, m), 8.74 (1H, s).
(2) A suspension of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-oxopropionate (0.59 g, 0.88 mmol) obtained in Example 18-(1) in ethanol (20 ml) was cooled to -20°C and sodium borohydride (36 mg, 0.96 mmol) was added thereto. After stirring for 2 hours, an aqueous saturated ammonium chloride solution (5 ml) was added and the mixture was extracted with ethyl acetate (150 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (55:45-35:65)] to obtain ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-hydroxypropionate (0.49 g) as colorless powder.
   ¹H-NMR (CDCl₃) δ: 0.92 (3H, s), 0.97 (3H, s), 1.26 (3H, t, J = 7.2 Hz), 2.04 (3H, s), 2.77 (1H, dd, J = 5.1, 15.9 Hz), 2.94 (1H, dd, J = 9.0, 15.9 Hz), 3.05-3.21 (2H, m), 3.42 (1H, d, J = 14.1 Hz), 3.62 (3H, s), 3.66 (1H, d, J = 11.7 Hz), 3.82 (1H, d, J = 11.7 Hz), 3.89 (3H, s), 4.12 (2H, q, J = 7.2 Hz), 4.42 (1H, d, J = 14.1 Hz), 4.30-4.52 (1H, m), 4.62-4.71 (1H, m), 5.62-5.80 (1H, m), 6.26 (1H, s), 6.62 (1H, d, J = 2.1 Hz), 6.98 (1H, dd, J = 2.4, 7.2 Hz), 7.10-7.26 (3H, m), 7.30 (1H, dd, J = 2.1, 8.7 Hz), 8.55 (1H, s) .
(3) A mixture of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-hydroxypropionate (0.06 g, 0.09 mmol) obtained in Example 18-(2), a 1N aqueous sodium hydroxide solution (0.5 ml) and ethanol (1 ml) was stirred at room temperature for 2 hours. This mixture was acidified with 1N hydrochloric acid (0.6 ml) and ethyl acetate (40 ml) was added thereto. The organic layer was washed with an aqueous saturated sodium chloride solution, dried over magnesium sulfate, and then concentrated under reduced pressure. The resulting crystal was washed with hexane to obtain 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-hydroxypropionic acid (0.05 g) as a white crystal.
   ¹H-NMR (CDCl₃) δ: 0.66 (3H, s), 1.00 (3H, s), 2.73 (1H, dd, J = 5.1, 16.2 Hz), 2.81 (1H, dd, J = 8.4, 16.2 Hz), 3.14 (1H, d, J = 12.0 Hz), 3.19 (1H, dd, J = 5.4, 14.1 Hz), 3.36 (1H, d, J = 14.1 Hz), 3.48 (1H, dd, J = 7.8, 14.1 Hz), 3.60 (1H, d, J = 12.0 Hz), 3.61 (3H, s), 3.89 (3H, s), 4.41 (1H, d, J = 14.1 Hz), 4.50 (1H, dd, J = 5.4, 7.8 Hz), 5.64 (1H, dd, J = 5.1, 8.4 Hz), 6.18 (1H, s), 6.56 (1H, d, J = 2.1 Hz), 6.95-7.03 (2H, m), 7.16 (1H, t, J = 7.8 Hz), 7.29 (1H, d, J = 9.0 Hz), 7.35 (1H, dd, J = 2.1, 9.0 Hz), 8.59 (1H, s).

### Example 19

### (2E)-3-(2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazo1-5-yl)acrylic acid

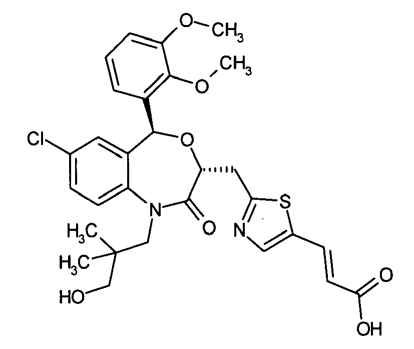

(1) Triethylamine (0.11 ml, 0.77 mmol) and methanesulfonyl chloride (0.05 ml, 0.68 mmol) were added to a solution of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-hydroxypropionate (0.4 g, 0.59 mmol) obtained in Example 18-(2) in THF (5 ml) under ice-cooling and the mixture was stirred for 1 hour. Triethylamine (0.11 ml, 0.77 mmol) and methanesulfonyl chloride (0.05 ml, 0.68 mmol) were additionally added thereto and the mixture was stirred for 1 hour. Then, DBU (0.12 ml, 0.77 mmol) was added thereto and the mixture was stirred for 1 hour. Then, water (10 ml) was added thereto and the mixture was extracted with ethyl acetate (150 ml). The organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (63:37-52:48)] to obtain ethyl (2E)-3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acrylate (0.05 g) as colorless noncrystalline powder.
   ¹H-NMR (CDCl₃) δ: 0.96 (3H, s), 1.05 (3H, s), 1.25 (3H, t, J = 7.2 Hz), 2.04 (3H, s), 3.40 (2H, d, J = 6.0 Hz), 3.54 (1H, d, J = 14.0 Hz), 3.61 (3H, s), 3.73 (1H, d, J = 11.0 Hz), 3.86 (1H, d, J = 11.0 Hz), 3.87 (3H, s), 4.19 (2H, q, J = 7.2 Hz), 4.37 (1H, t, J = 6.6 Hz), 4.57 (1H, d, J = 14.0 Hz), 6.18 (1H, d, J = 15.4 Hz), 6.28 (1H, s), 6.54 (1H, s), 6.88 (1H, dd, J = 1.4, 7.6 Hz), 6.92 (1H, dd, J = 1.4, 7.6 Hz), 7.05 (1H, t, J = 7.6 Hz), 7.23-7.38 (2H, m), 7.92 (1H, d, J = 15.4 Hz), 8.63 (1H, s).
(2) A mixture of ethyl (2E)-3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acrylate (0.34 g, 0.52 mmol) obtained in Example 19-(1), a 1N aqueous sodium hydroxide solution (1.5 ml) and ethanol (7 ml) was stirred at 40°C for 1 hour. After standing to cool, this mixture was acidified with 1N hydrochloric acid (1.7 ml) and ethyl acetate (160 ml) was added. The organic layer was washed with an aqueous saturated sodium chloride solution, dried over magnesium sulfate, and then concentrated under reduced pressure. The resulting crystal was washed with hexane to obtain (2E)-3-(2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acrylic acid (0.28 g) as a pale yellow crystal.
   ¹H-NMR (CDCl₃) δ: 0.64 (3H, s), 1.06 (3H, s), 3.19 (1H, d, J = 11.7 Hz), 3.37 (1H, d, J = 13.8 Hz), 3.39-3.45 (2H, m), 3.61 (3H, s), 3.67 (1H, d, J = 11.7 Hz), 3.86 (3H, s), 4.38 (1H, t, J = 7.2 Hz), 4.46 (1H, t, J = 13.8 Hz), 6.16 (1H, d, J = 15.6 Hz), 6.16 (1H, s), 6.53 (1H, d, J = 2.1 Hz), 6.88 (1H, dd, J = 1.5, 8.1 Hz), 6.93 (1H, dd, J = 1.5, 8.1 Hz), 7.08 (1H, t, J = 8.1 Hz), 7.29 (1H, d, J = 8.7 Hz), 7.33 (1H, dd, J = 2.1, 8.7 Hz), 8.02 (1H, d, J = 15.6 Hz), 8.66 (1H, s).

### Example 20

### 3- (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-methyl-1,3-thiazol-4-yl)propionic acid

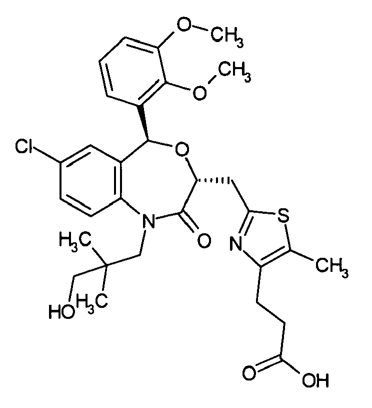

(1) Pivaloyl chloride (0.51 g, 4.23 mmol) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (2 g, 3.85 mmol) and triethylamine (0.41 g, 4.04 mmol) in acetonitrile (15 ml) at 0°C and the mixture was stirred at 0°C for 30 minutes. To the reaction solution at 0°C, methyl (4S)-4-amino-5-oxohexanoate hydrochloride (0.83 g, 4.24 mmol) was added and a solution of triethylamine (0.58 g, 5.77 mmol) in acetonitrile (2 ml) was then added dropwise.
   After stirring at room temperature for 30 minutes, the reaction solution was diluted with ethyl acetate (50 ml), washed with 0.1N hydrochloric acid, a 5% aqueous potassium hydrogen sulfate solution, an aqueous saturated sodium hydrogen carbonate solution and an aqueous saturated sodium chloride solution, dried over sodium sulfate, and then concentrated under reduced pressure. The residue was purified by silica gel column chromatography [developing solvent: hexane-ethyl acetate (1:2)] to obtain methyl (4S)-4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-5-oxohexanoate (1.85 g, 2.80 mmol, 73%) as colorless powder.
   ¹H-NMR (CDCl₃) δ: 0.94 (3H, s), 1.02 (3H, s), 1.78-1.90 (1H, m), 2.03 (3H, s), 2.23 (3H, s), 2.25-2.42 (3H, m), 2.70 (1H, dd, J = 5.4, 14.4 Hz), 2.89 (1H, dd, J = 7.8, 14.4 Hz), 3.54 (1H, d, J = 14.1 Hz), 3.61 (3H, s), 3.67 (3H, s), 3.72 (1H, d, J = 11.1 Hz), 3.85 (1H, d, J = 11.1 Hz), 3.89 (3H, s), 4.36 (1H, dd, J = 5.4, 7.8 Hz), 4.54 (1H, d, J = 14.1 Hz), 4.56-4.65 (1H, m), 6.25 (1H, s), 6.60-6.64 (1H, br), 6.64 (1H, s), 6.97-7.32 (5H, m).
(2) A solution of methyl (4S)-4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-5-oxohexanoate (0.51 g, 0.773 mol) obtained in Example 20-(1) and Lawesson's reagent (0.40 g, 1.00 mmol) in THF (5 ml) was stirred at 70°C for 1 hour. The reaction solution was concentrated under reduced pressure and the residue was purified by silica gel column chromatography [developing solvent: hexane-ethyl acetate (1:1)] to obtain methyl 3-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-5-methyl-1,3-thiazol-4-yl)propionate (0.31 g, 0.470 mmol, 61%) as colorless powder.
   ¹H-NMR (CDCl₃) δ: 0.95 (3H, s), 1.03 (3H, s), 2.03 (3H, s), 2.34 (3H, s), 2.64 (2H, t, J = 7.4 Hz), 2.87(2H, t, J = 7.4 Hz), 3.34 (1H, dd, J = 6.8, 15.0 Hz), 3.50 (1H, dd, J = 6.2, 15.0 Hz), 3.53 (1H, d, J = 14.2 Hz), 3.62 (3H, s), 3.63 (3H, s), 3.72 (1H, d, J = 11.0 Hz), 3.85 (1H, d, J = 11.0 Hz), 3.89 (3H, s), 4.27 (1H, dd, J = 6.2, 6.8 Hz), 4.56 (1H, d, J = 14.2 Hz), 6.29 (1H, s), 6.60 (1H, d, J = 1.8 Hz), 6.95-7.36 (5H, m).
(3) A mixture of methyl 3-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-5-methyl-1,3-thiazol-4-yl)propionate (0.2 g, 0.303 mmol) obtained in Example 20-(2), a 1N aqueous sodium hydroxide solution (1 ml) and ethanol (2 ml) was stirred at 60°C for 30 minutes. This mixture was diluted with water (50 ml), acidified, and extracted with ethyl acetate (50 ml) twice. This extract was washed with an aqueous saturated sodium chloride solution, dried over sodium sulfate, and then concentrated under reduced pressure to obtain 3-((2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-methyl-1,3-thiazol-4-yl)propionic acid (0.18 g, 0.298 mmol, 98%) as colorless powder.
   ¹H-NMR (CDCl₃) δ: 0.64 (3H, s), 1.05 (3H, s), 2.33 (3H, s), 2.66-2.72 (2H, m), 2.83-2.89 (2H, m), 3.16 (1H, d, J = 11.8 Hz), 3.37 (1H, dd, J = 6.2, 15.4 Hz), 3.42 (1H, d, J = 14.6 Hz), 3.59 (1H, dd, J = 6.6, 15.4 Hz), 3.62 (3H, s), 3.63 (1H, d, J = 11.8 Hz), 3.90 (3H, s), 4.30 (1H, dd, J = 6.2, 6.6 Hz), 4.46 (1H, d, J = 14.6 Hz), 6.20 (1H, s), 6.60 (1H, d, J = 1.4 Hz), 6.97-7.39 (5H, m).

### Example 21

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-yl)acetic acid

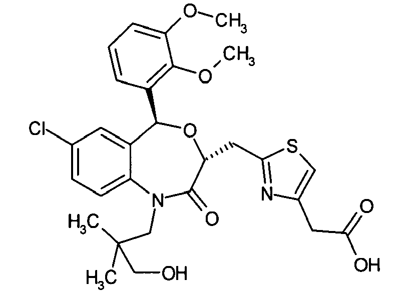

According to a similar manner to that of Example 16, the title compound was obtained.

¹H-NMR (CDCl₃) δ: 0.65 (3H, s), 1.05 (3H, s), 3.17 (1H, d, J = 11.9 Hz), 3.41 (1H, d, J = 14.5 Hz), 3.48 (1H, dd, J = 6.5, 15.4 Hz), 3.60-3.71 (5H, m), 3.80 (2H, s), 3.90 (3H, s), 4.35 (1H, t, J = 6.5 Hz), 4.46 (1H, d, J = 14.5 Hz), 6.21 (1H, s), 6.60 (1H, s), 7.00 (1H, dd, J = 2.1, 7.7 Hz), 7.05 (1H, s), 7.11-7.22 (2H, m), 7.33-7.40 (2H, m).

### Example 22

### 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-carboxylic acid

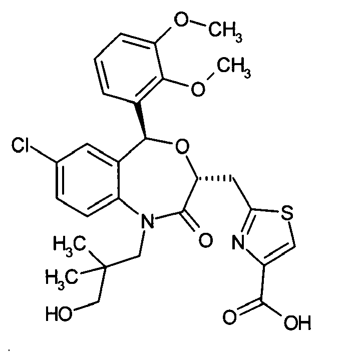

(1) A solution of 3-[(3R,5S)-3-(2-amino-2-oxoethyl)-7-chloro-5-(2,3-dimethoxyphenyl)-2-oxo-2,3-dihydro-4,1-benzoxazepin-1(5H)-yl]-2,2-dimethylpropyl acetate (3.0 g, 5.61 mmol) obtained in Example 16-(2) and ethyl 3-bromo-2-oxopropionate (0.79 ml, 6.28 mmol) in ethanol (30 ml) was heated to reflux for 2 hours with stirring. After standing to stand, water (15 ml) was added to the reaction solution, followed by extraction with ethyl acetate (300 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (3:2-1:1)] to obtain ethyl 2-{[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]methyl}-1,3-thiazol-4-carboxylate (1.1 g) as colorless noncrystalline powder from a first eluted fraction, and ethyl 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-carboxylate (2.0 g) as colorless powder from a second eluted fraction.
   First eluted fraction
      ¹H-NMR (CDCl₃) δ: 0.95 (3H, s), 1.03 (3H, s), 1.37 (3H, t, J = 7.0 Hz), 2.03 (3H, s), 3.41-3.62 (3H, m), 3.61 (3H, s), 3.72 (1H, d, J = 11.0 Hz), 3.96 (1H, d, J = 11.0 Hz), 3.89 (3H, s), 4.27-4.40 (1H, m), 4.39 (2H, q, J = 7.0 Hz), 4.55 (1H, d, J = 14.0 Hz), 6.31 (1H, s), 6.59 (1H, d, J = 1.8 Hz), 6.98 (1H, dd, J = 2.0, 7.8 Hz), 7.08 (1H, dd, J = 2.0, 7.8 Hz), 7.17 (1H, t, J = 7.8 Hz), 7.23-7.40 (2H, m), 8.09 (1H, s).
   Second eluted fraction
      ¹H-NMR (CDCl₃) δ: 0.65 (3H, s), 1.05 (3H, s), 1.38 (3H, t, J = 7.0 Hz), 3.16 (1H, t, J = 11.8 Hz), 3.37 (1H, d, J = 14.4 Hz), 3.45-3.72 (3H, m), 3.62 (3H, s), 3.89 (3H, s), 4.05-4.15 (1H, m), 4.35 (1H, t, J = 7.0 Hz), 4.39 (2H, q, J = 7.0 Hz), 4.47 (1H, d, J = 14.4 Hz), 6.21 (1H, s), 6.56 (1H, d, J = 2.2 Hz), 6.99 (1H, dd, J = 1.8, 8.0 Hz), 7.07 (1H, dd, J = 1.8, 8.0 Hz), 7.18 (1H, t, J = 8.0 Hz), 7.29 (1H, d, J = 8.4 Hz), 7.35 (1H, dd, J = 2.2, 8.4 Hz), 8.09 (1H, s).
(2) A mixture of ethyl 2-{[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]methyl}-1,3-thiazol-4-carboxylate (0.34 g, 0.52 mmol) and ethyl 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-carboxylate (2.0 g, 3.40 mmol) obtained in Example 22-(1), a 1N aqueous sodium hydroxide solution (14.0 ml) and ethanol (60 ml) was stirred at room temperature for 5 hours. This mixture was acidified with 1N hydrochloric acid (14.5 ml) and ethyl acetate (380 ml) was added thereto. The organic layer was washed with an aqueous saturated sodium chloride solution, dried over magnesium sulfate, and then concentrated under reduced pressure. To the resulting residue were added diethyl ether (30 ml), a 1N aqueous sodium hydroxide solution (14 ml) and water (100m). The aqueous layer was washed with diethyl ether (50 ml) and then acidified with 1N hydrochloric acid (14.5 ml) and ethyl acetate (380 ml) was added thereto. The organic layer was washed with an aqueous saturated sodium chloride solution, dried over magnesium sulfate, and then concentrated under reduced pressure to obtain 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-carboxylic acid (2.9 g) as pale yellow noncrystalline powder.
   ¹H-NMR (CDCl₃) δ: 0.65 (3H, s), 1.05 (3H, s), 3.18 (1H, d, J = 12.0 Hz), 3.38 (1H, d, J = 13.8 Hz), 3.54 (1H, dd, J = 7.2, 14.7 Hz), 3.56-3.68 (1H, m), 3.62 (3H, s), 3.89 (3H, s), 4.34 (1H, dd, J = 5.7, 7.2 Hz), 4.46 (1H, d, J = 13.8 Hz), 6.21 (1H, s), 6.57 (1H, d, J = 2.1 Hz), 6.98 (1H, dd, J = 1.5, 8.1 Hz), 7.06 (1H, dd, J = 1.5, 8.1 Hz), 7.17 (1H, t, J = 8.1 Hz), 7.29 (1H, d, J = 8.7 Hz), 7.35 (1H, dd, J = 2.1, 8.7 Hz), 8.18 (1H, s).

### Example 23

### 2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-4-carboxylic acid

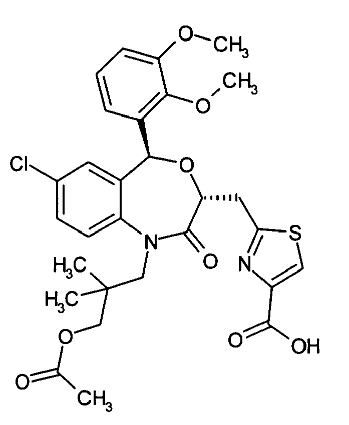

According to a similar manner to that of Example 17, the title was obtained.

¹H-NMR (CDCl₃) δ: 0.95 (3H, s), 1.03 (3H, s), 2.03 (3H, s), 3.45-3.66 (3H, m), 3.61 (3H, s), 3.72 (1H, d, J = 10.8 Hz), 3.85 (1H, d, J = 10.8 Hz), 3.89 (3H, s), 4.32 (1H, dd, J = 5.7, 7.2 Hz), 4.55 (1H, d, J = 13.8 Hz), 6.31 (1H, s), 6.60 (1H, d, J = 2.4 Hz), 6.98 (1H, dd, J = 1.5, 7.8 Hz), 7.06 (1H, dd, J = 1.5, 7.8 Hz), 7.10-7.20 (1H, m), 7.27 (1H, d, J = 9.0 Hz), 7.32 (1H, dd, J = 2.4, 9.0 Hz), 8.18 (1H, s).

### Example 24

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-yl)-3-hydroxypropionic acid

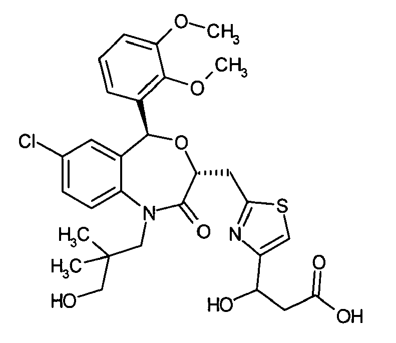

According to a similar manner to that of Example 18, the title compound was obtained.

¹H-NMR (CDCl₃) δ: 0.65 (3H, s), 1.05 (3H, s), 2.78-3.04 (2H, m), 3.16 (1H, d, J = 11.4 Hz), 3.39 (1H, d, J = 14.1 Hz), 3.40-3.70 (3H, m), 3.61 (3H, s), 3.89 (3H, s), 4.31 (1H, t, J = 6.6 Hz), 4.46 (1H, d, J = 14.1 Hz), 5.11-5.21 (1H, m), 6.20 (1H, s), 6.59 (1H, s), 6.96-7.02 (1H, m), 7.08-7.20 (3H, m), 7.29-7.41 (2H, m)

### Example 25

### (2E)-3-{2-[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl]-1,3-thiazol-4-yl}acrylic acid

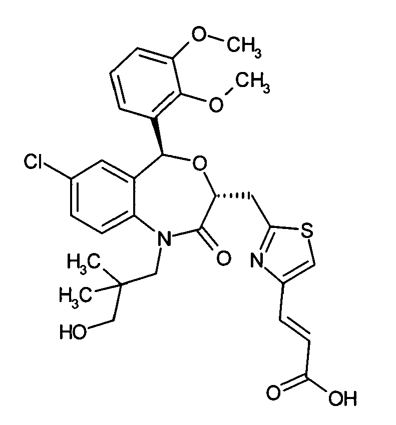

According to a similar manner to that of Example 19, the title compound was obtained.

¹H-NMR (CDCl₃) δ: 0.66 (3H, s), 1.06 (3H, s), 3.18 (1H, d, J = 12.0 Hz), 3.41 (1H, d, J = 13.8 Hz), 3.49 (1H, dd, J = 6.6, 15.3 Hz), 3.58-3.70 (2H, m), 3.63 (3H, s), 3.90 (3H, s), 4.40 (1H, t, J = 6.6 Hz), 4.48 (1H, d, J = 13.8 Hz), 6.23 (1H, s), 6.60 (1H, d, J = 2.4 Hz), 6.62 (1H, d, J = 15.3 Hz), 7.00 (1H, dd, J = 2.4, 7.2 Hz), 7.13-7.21 (2H, m), 7.30-7.41 (3H, m), 7.62 (1H, d, J = 15.3 Hz).

### Example 26

### 4-[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl]-2-methyl-1,3-thiazol-5-carboxylic acid

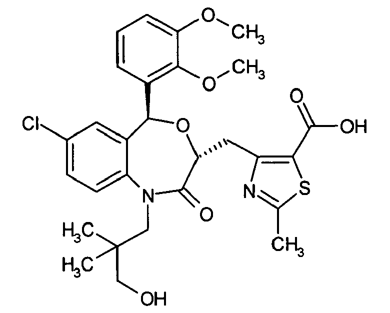

(1) Carbonyldiimizazole (3.7 g) was added to a suspension of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (10 g) in THF (100 ml) and the mixture was stirred at room temperature for 2 hours. To the reaction solution were added magnesium chloride (2.2 g) and potassium monoethylmalonate (4.0 g) and the mixture was stirred at 60°C for 1 hour. The reaction solution was diluted with ethyl acetate (200 ml), washed with 1N hydrochloric acid, an aqueous saturated sodium hydrogen carbonate solution and an aqueous saturated sodium chloride solution, dried over sodium sulfate, and then concentrated under reduced pressure. The residue was purified by silica gel column chromatography to obtain ethyl 4-{[(3R,5S)-1-(3-acetoxy-2,2-dimethylpropyl)-7-chloro-5-(2,3-dimethoxyhphenyl)-2-oxo-1,2,3,5-tetrahydro-4,1-benzoxazepin-3-yl]}-3-oxobutanoate (10.2 g) as a white crystal.
   ¹H-NMR (CDCl₃) δ: 0.94 (3H, s), 1.01 (3H, s), 1.27 (3H, s), 2.02 (3H, s), 2.96 (1H, dd, J = 5.0, 17.2 Hz), 3.27 (1H, dd, J = 7.8, 17.0 Hz), 3.42-3.57 (3H, m), 3.61 (3H, s), 3.69-3.82 (2H, m), 3.89 (3H, s), 4.18 (2H, q, J = 7.4 Hz), 4.41-4.57 (2H, m), 6.24 (1H, s), 6.63 (1H, s), 6.95-7.02 (1H, m), 7.15-7.20 (2H, m), 7.33-7.34 (4H, m)
(2) Sulfuryl chloride (0.23 ml) was added to a solution of ethyl 4-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]}-3-oxobutanoate (1.5 g) obtained in Example 26-(1) in dichloromethane (30 ml) and the mixture was stirred at room temperature for 2 hours. The reaction solution was poured into water, which was extracted with chloroform. The organic layer was concentrated under reduced pressure. Thioacetamide (0.21 g) and ethanol (30 ml) were added to the residue and the mixture was refluxed overnight. The reaction solution was concentrated and the residue was purified by silica gel column chromatography to obtain ethyl 4-[((3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl)methyl]-2-methyl-1,3-thiazol-5-carboxylate (0.80 g) as a colorless amorphous crystal.
   ¹H-NMR (CD₃OD) δ: 0.93 (3H, s), 1.23 (3H, s), 1.27 (3H, t, J = 7.2 Hz), 2.03 (3H, s), 2.64 (3H, s), 3.51-3.57 (1H, m), 3.60 (3H, s), 3.67-3.85 (4H, m), 3.87 (3H, s), 4.23 (2H, q, J = 7.2 Hz), 4.37-4.44 (2H, m), 6.26 (1H, s), 6.53 (1H, s), 6.87-6.99 (2H, m), 7.05-7.10 (1H, m), 7.16-7.18 (1H, m), 7.32-7.37(2H, m).
(3) A mixture of ethyl 4-[((3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl)methyl]-2-methyl-1,3-thiazol-5-carboxylate (0.7 g) obtained in Example 26-(2), a 2N aqueous sodium hydroxide solution (2 ml) and ethanol (10 ml) was stirred at room temperature for 30 minutes. This mixture was diluted with water (50 ml), acidified, and extracted with chloroform (50 ml) twice. These extracts were washed with an aqueous saturated sodium chloride solution, dried over magnesium sulfate, and then concentrated under reduced pressure. The residue was purified by silica gel column chromatography to obtain 4-[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl]-2-methyl-1,3-thiazol-5-carboxylic acid (0.19 g) as a white crystal.
   ¹H-NMR (CDCl₃) δ: 0.64 (3H, s), 1.04 (3H, s), 1.12 (3H, s), 1.14 (3H, s), 2.62 (3H, s), 3.12-3.16 (1H, m), 3.35-3.40 (1H, m), 3.53 (3H, s), 3.62-3.68 (3H, m), 3.87 (3H, s), 4.41-4.50 (2H, m), 6.16 (1H, s), 6.53 (1H, s), 6.93-6.97 (2H, m), 7.09-7.14 (1H, m), 7.26-7.33(2H, m)

### Example 27

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)acetic acid calcium salt

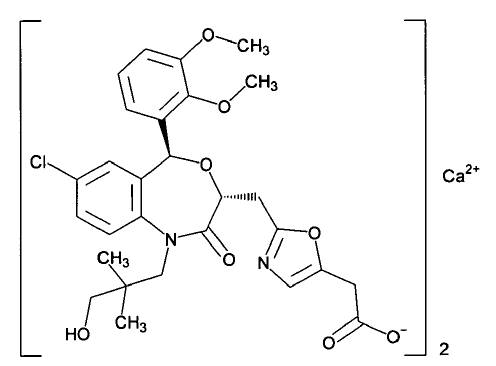

(1) Phosphorus oxychloride (0.35 g, 2.33 mmol) was added to a solution of ethyl 4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-3-oxobutanoate (1 g, 1.55 mmol) obtained in Example 1-(3) in DMF (3 ml) and the mixture was stirred at 70°C for 1 hour. The reaction solution was cooled in an ice bath and an aqueous saturated sodium hydrogen carbonate solution (50 ml) was added thereto. The product was extracted with ethyl acetate (50 ml) twice, washed with water and an aqueous saturated sodium chloride solution, dried over sodium sulfate, and then concentrated under reduced pressure. The residue was purified by silica gel column chromatography [developing solvent: hexane-ethyl acetate (3:2)] to obtain ethyl (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)acetate (0.57 g, 0.906 mmol, 58%) as colorless powder.
   ¹H-NMR (CDCl₃) δ: 0. 94 (3H, s), 1.03 (3H, s), 1.26 (3H, t, J = 7.4 Hz), 2.03 (3H, s), 3.24 (1H, dd, J = 7.4, 15.8 Hz), 3.36 (1H, dd, J = 6.6, 15.8 Hz), 3.54 (1H, d, J = 14.0 Hz), 3.61 (3H, s), 3.68 (2H, s), 3.73 (1H, d, J = 11.4 Hz), 3.85 (1H, d, J = 11.4 Hz), 3.89 (3H, s), 4.18 (2H, q, J = 7.4 Hz), 4.44 (1H, dd, J = 6.6, 7.4 Hz), 4.58 (1H, d, J = 14.0 Hz), 6.30 (1H, s), 6.60 (1H, s), 6.85 (1H, s), 6.94-7.33 (5H, m).
(2) A mixture of ethyl (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)acetate (0.47 g, 0.747 mmol) obtained in Example 27-(1), a 1N aqueous sodium hydroxide solution (2 mmol) and ethanol (5 ml) was stirred at 60°C for 30 minutes. This mixture was washed with water (5 ml), acidified, and extracted with ethyl acetate (50 ml) twice. These extracts were washed with an aqueous saturated sodium chloride solution, dried over sodium sulfate, and then concentrated under reduced pressure to obtain (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)acetic acid (0.42 g). This was dissolved in ethanol (6 ml) and a 1N aqueous sodium hydroxide solution (0.75 ml) was added thereto. The mixture was then concentrated under reduced pressure. The residue was dissolved in water (2 ml) and a solution of calcium chloride (42 mg, 0.378 mmol) in water (0.5 ml) was added. The produced precipitates were filtered to obtain (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)acetic acid calcium salt (0.29 g, 0.247 mmol, 66%) as colorless powder.
   ¹H-NMR (CDCl₃-D₃OD (1:2)) δ: 0.81 (3H, s), 0.97 (3H, s), 3.19 (1H, d, J = 11.8 Hz), 3.22-3.29 (2H, m), 3.50 (1H, d, J = 11.8 Hz), 3.55 (2H, s), 3.60 (3H, s), 3.62 (1H, d, J = 13.4 Hz), 3.90 (3H, s), 4.40-4.47 (2H, m), 6.21 (1H, s), 6.55 (1H, d, J = 2.6 Hz), 6.78 (1H, s), 7.02-7.37 (5H, m).

### Example 28

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-methyl-1,3-oxazol-5-yl)acetic acid calcium salt

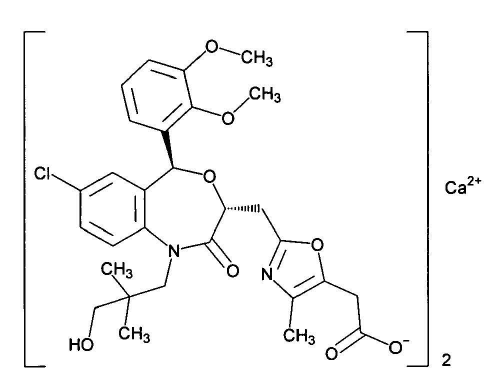

According to a similar manner to that of Example 27, the title compound was obtained.

¹H-NMR (CDCl₃-CD₃OD (1:2)) δ: 0.83 (3H, s), 0.95 (3H, s), 2.05 (3H, s), 3.18-3.26 (3H, m), 3.44-3.68 (4H, m), 3.59 (3H, s), 3.89 (3H, s), 4.38-4.46 (2H, m), 6.20 (1H, s), 6.53 (1H, d, J = 2.2 Hz), 7.02-7.59 (5H, m).

### Example 29

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid

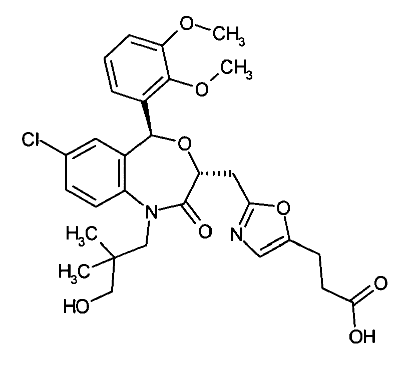

(1) According to a similar manner to that of Example 27-(1), methyl (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)propionate was obtained.
   ¹H-NMR (CDCl₃) δ: 0. 94 (3H, s), 1.03 (3H, s), 2.03 (3H, s), 2.64 (2H, t, J = 7.4 Hz), 2.96 (2H, t, J = 7.4 Hz), 3.21 (1H, dd, J = 7.0, 15.4 Hz), 3.33 (1H, dd, J = 6.6, 15.4 Hz), 3.54 (1H, d, J = 14.2 Hz), 3.61 (3H, s), 3.69 (3H, s), 3.73 (1H, d, J = 11.0 Hz), 3.85 (1H, d, J = 11.0 Hz), 3.89 (3H, s), 4.41 (1H, dd, J = 6.6, 7.0 Hz), 4.58 (1H, d, J = 14.2 Hz), 6.30 (1H, s), 6.60 (1H, s), 6.65 (1H, s), 6.95-7.33 (5H, m).
(2) A mixture of methyl (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-oxazol-5-yl)propionate (0.11 g, 0.175 mmol) obtained in Example 29-(1), a 1N aqueous sodium hydroxide solution (0.5 ml) and ethanol (2 ml) was stirred at 60°C for 30 minutes. This was diluted with water (50 ml), acidified, and then extracted with ethyl acetate (50 ml) twice. This was washed with an aqueous saturated sodium chloride solution, dried over sodium sulfate, and then concentrated under reduced pressure. The residue was purified by recrystallization from ethanol-hexane (1:1) to obtain (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid (50 mg, 0.0873 mmol, 50%) as colorless powder.
   ¹H-NMR (CDCl₃) δ: 0.65 (3H, s), 1.05 (3H, s), 2.67 (2H, t, J = 7.0 Hz), 2.95 (2H, t, J = 7.0 Hz), 3.16 (1H, d, J = 12.8 Hz), 3.22 (1H, dd, J = 7.0, 15.8 Hz), 3.37 (1H, dd, J = 6.6, 15.8 Hz), 3.39 (1H, d, J = 15.4 Hz), 3.62 (3H, s), 3.63 (1H, d, J = 12.8 Hz), 3.89 (3H, s), 4.44 (1H, dd, J = 6.6, 7.0 Hz), 4.48 (1H, d, J = 15.4 Hz), 6.19 (1H, s), 6.59 (1H, s), 6.68 (1H, s), 6.96-7.36 (5H, m)

### Example 30

### 4-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)butanoic acid

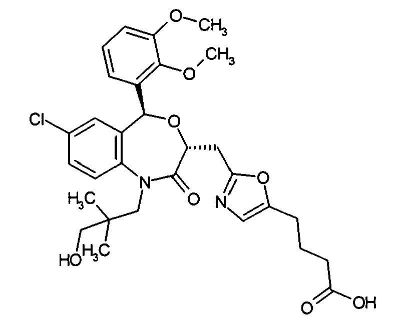

According to a similar manner to that of Example 29, the title compound was obtained.

¹H-NMR (CDCl₃) δ: 0.64 (3H, s), 1.04 (3H, s), 1.95 (2H, quintet, J = 7.4 Hz), 2.38 (2H, t, J = 7.4 Hz), 2.69 (2H, t, J = 7.4 Hz), 3.16 (1H, d, J = 12.2 Hz), 3.21 (1H, dd, J = 6.6, 15.6 Hz), 3.38 (1H, dd, J = 7.0, 15.6 Hz), 3.40 (1H, d, J = 15.0 Hz), 3.61 (3H, s), 3.63 (1H, d, J = 12.2 Hz), 3.89 (3H, s), 4.42-4.52 (2H, m), 6.19 (1H, s), 6.58 (1H, s), 6.66 (1H, s), 6.95-7.36 (5H, m).

### Example 31

### 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)-2-methylpropionic acid

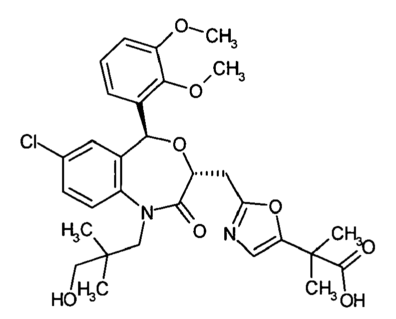

According to a similar manner to that of Example 29, the title compound was obtained.

¹H-NMR (CDCl₃) δ: 0.64 (3H, s), 1.037 (3H, s), 1.56 (6H, s), 3.17 (1H, d, J = 11.6 Hz), 3.25 (1H, dd, J = 7.0, 15.8 Hz), 3.38 (3H, dd, J = 6.6, 15.8 Hz), 3.39 (1H, d, J = 14.0 Hz), 3.61 (3H, s), 3.62 (1H, d, J = 11.6 Hz), 3.88 (3H, s), 4.41 (1H, dd, J = 6.6, 7.0 Hz), 4.47 (1H, d, J = 14.0 Hz), 6.18 (1H, s), 6.57 (1H, s), 6.80 (1H, s), 6.95-7.53 (5H, m) .

### Example 32

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-ethyl-1,3-oxazol-5-yl)acetic acid

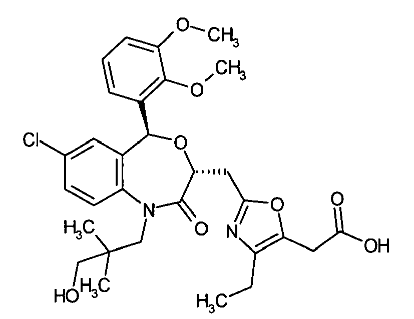

According to a similar manner to that of Example 29, the title compound was obtained.

¹H-NMR (CDCl₃) δ: 0.64 (3H, s), 1.04 (3H, s), 1.16 (3H, t, J = 7.6 Hz), 2.43 (2H, q, J = 7.6 Hz), 3.17 (1H, d, J = 12.0 Hz), 3.26-3.30 (2H, m), 3.38 (1H, d, J = 14.4 Hz), 3.60 (3H, s), 3.62 (2H, s), 3.62 (1H, d, J = 12.0 Hz), 3.87 (3H, s), 4.39-4.50 (2H, m), 6.17 (1H, s), 6.57 (1H, d, J = 1.4 Hz), 6.93-7.39 (5H, m).

### Example 33

### (2-{[(3R,5S)-7-chloro-5-(2,3-dimethoxyphenyl)-1-neopentyl-2-oxo-1,2,3,5-tetrahydro-4,1-benzoxazepin-3-yl]methyl}-1,3-oxazol-5-yl)acetic acid

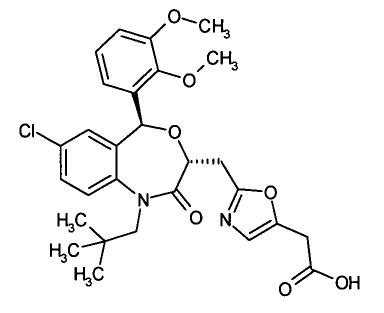

According to a similar manner to that of Example 29, the title compound was obtained.

¹H-NMR (CDCl₃) δ: 0.94 (9H, s), 3.27 (1H, dd, J = 7.2, 15.6 Hz), 3.35 (1H, dd, J = 6.0, 15.6 Hz), 3.36 (1H, d, J = 13.8 Hz), 3.61 (3H, s), 3.71 (2H, s), 3.87 (3H, s), 4.44 (1H, dd, J = 6.0, 7.2 Hz), 4.50 (1H, d, J = 13.8 Hz), 6.29 (1H, s), 6.56 (1H, s), 6.88 (1H, s), 6.94 (1H, dd, J = 1.5, 7.8 Hz), 7.03 (1H, dd, J = 0.9, 7.8 Hz), 7.13 (1H, t, J = 7.8 Hz), 7.26-7.34 (2H, m).

### Example 34

### (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid

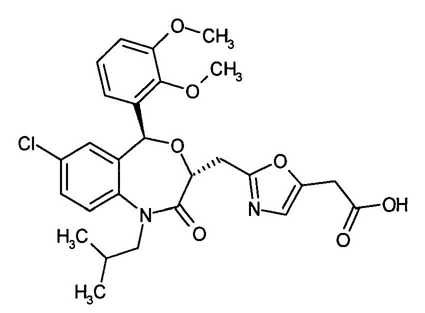

According to a similar manner to that of Example 29, the title compound was obtained.

¹H-NMR (CDCl₃) δ: 0.92 (3H, d, J = 6.6 Hz), 1.00 (3H, d, J = 6.9 Hz), 1.95-2.09 (1H, m), 3.32-3.41 (3H, m), 3.57 (3H, s), 3.69 (2H, s), 3.87 (3H, s), 4.37 (1H, t, J = 7.8, 13.8 Hz), 4.46 (1H, t, J = 6.3 Hz), 6.19 (1H, s), 6.60 (1H, d, J = 1.8 Hz), 6.90 (1H, s), 6.92-7.36 (5H, m).

### Example 35

### (2-{[(3R,5S)-7-chloro-5-(2,3-dimethoxyphenyl)-2-oxo-1-propyl-1,2,3,5-tetrahydro-4,1-benzoxazepin-3-yl]methyl}-1,3-oxazol-5-yl)acetic acid

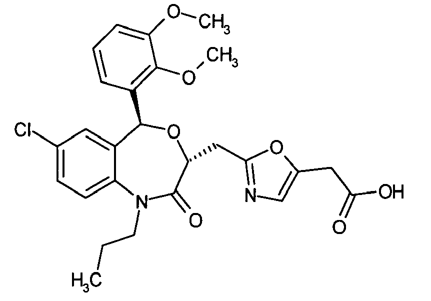

According to a similar manner to that of Example 29, the title compound was obtained.

¹H-NMR (CDCl₃) δ: 0.95 (3H, t, J = 7.5 Hz), 1.56-1.84 (2H, m), 3.23-3.54 (3H, m), 3.56 (3H, s), 3.69 (2H, s), 3.86 (3H, s), 4.40-4.52 (2H, m), 6.09 (1H, s), 6.60 (1H, d, J = 2.1 Hz), 6.90 (1H, s), 6.93-7.37 (5H, m).

### Example 36

### (2-{[(3R,5S)-7-chloro-5-(2,3-dimethoxyphenyl)-1-ethyl-2-oxo-1,2,3,5-tetrahydro-4,1-benzoxazepin-3-yl]methyl}-1,3-oxazol-5-yl)acetic acid

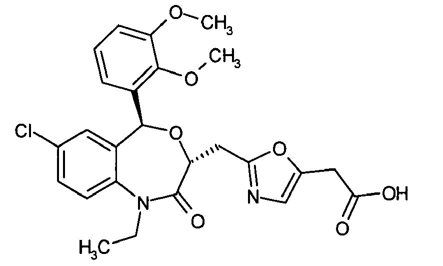

According to a similar manner to that of Example 29, the title compound was obtained.

¹H-NMR (CDCl₃) δ: 1.25 (3H, t, J = 7.2 Hz), 3.28 (1H, dd, J = 7.2, 15.6 Hz), 3.39 (1H, dd, J = 7.2, 15.6 Hz), 3.50-3.66 (4H, m), 3.69 (2H, s), 3.86 (3H, s), 4.45 (1H, t, J = 6.3 Hz), 4.48-4.62 (1H, m), 6.06 (1H, s), 6.61 (1H, d, J = 1.8 Hz), 6.90 (1H, s), 6.92-7.37 (5H, m).

### Example 37

### (4-Benzyl-2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)acetic acid

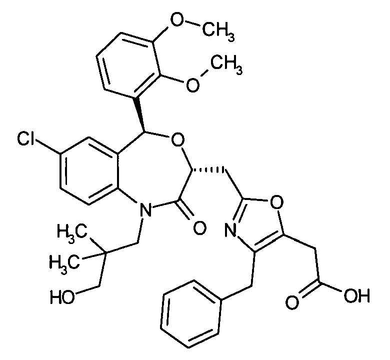

According to a similar manner to that of Example 29, the title compound was obtained.

¹H-NMR (CDCl₃) δ: 0.63 (3H, s), 1.04 (3H, s), 3.15 (1H, d, J = 12.6 Hz), 3.26 (2H, dd, J = 4.5, 7.2 Hz), 3.35 (1H, d, J = 14.4 Hz), 3.51 (2H, d, J = 1.8 Hz), 3.59 (3H, s), 3.60 (1H, d, J = 12.6 Hz), 3.79 (2H, s), 3.86 (3H, s), 4.41 (1H, t, J = 7.2 Hz), 4.43 (1H, d, J = 14.4 Hz), 6.15 (1H, s), 6.55 (1H, d, J = 2.1 Hz), 6.93 (1H, dd, J = 1.5, 8.1 Hz), 6.98 (1H, dd, J = 1.5, 8.1 Hz), 7.08 (1H, t, J = 8.1 Hz), 7.13-7.26 (5H, m), 7.28 (1H, d, J = 9.0 Hz), 7.34 (1H, dd, J = 2.1, 9.0 Hz).

### Example 38

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-methyl-1,3-oxazol-4-yl)propionic acid

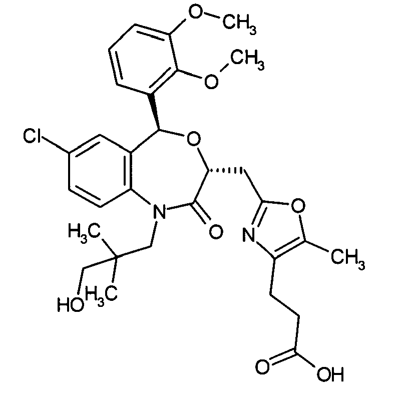

(1) Phosphorus oxychloride (0.35 g, 2.33 mmol) was added to a solution of methyl (4S)-4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}amino)-5-oxohexanoate (1 g, 1.55 mmol) obtained in Example 20-(1) in DMF (3 ml) and the mixture was stirred at 80°C for 1 hour. The reaction solution was cooled in an ice bath and an aqueous saturated sodium hydrogen carbonate solution (50 ml) was added. The product was extracted with ethyl acetate (50 ml) twice, washed with water and an aqueous saturated sodium chloride solution, dried over sodium sulfate, and then concentrated under reduced pressure. The residue was purified by silica gel column chromatography [developing solvent : hexane-ethyl acetate (1:1)] to obtain methyl 3-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-5-methyl-1,3-oxazol-4-yl)propionate (0.83 g, 1.29 mmol, 83%) as colorless powder.
   ¹H-NMR (CDCl₃) δ: 0.94 (3H, s), 1.03 (3H, s), 2.03 (3H, s), 2.22 (3H, s), 2.57-2.62 (2H, m), 2.66-2.71 (2H, m), 3.19 (1H, dd, J = 7.2, 15.6 Hz), 3.25 (1H, dd, J = 6.0, 15.6 Hz), 3.54 (1H, d, J = 13.8 Hz), 3.61 (3H, s), 3.64 (3H, s), 3.74 (1H, d, J = 11.1 Hz), 3.84 (1H, d, J = 11.1 Hz), 3.89 (3H, s), 4.36 (1H, dd, J = 6.0, 7.2 Hz), 4.57 (1H, d, J = 13.8 Hz), 6.29 (1H, s), 6.59 (1H, d, J = 1.8 Hz), 6.95-7.34 (5H, m).
(2) A mixture of methyl 3-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-5-methyl-1,3-oxazol-4-yl)propionate (0.67 g, 1.04 mmol) obtained in Example 38-(1), a 1N aqueous sodium hydroxide solution (2.5 ml) and ethanol (7 ml) was stirred at 60°C for 30 minutes. This mixture was diluted with water (50 ml), acidified, and then extracted with ethyl acetate (50 ml) twice. The extract was washed with an aqueous saturated sodium chloride solution, dried over sodium sulfate, and then concentrated under reduced pressure to obtain 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-methyl-1,3-oxazol-4-yl)propionic acid (0.47 g, 0.801 mmol, 77%) as colorless powder.
   ¹H-NMR (CDCl₃) δ: 0.64 (3H, s), 1.05 (3H, s), 2.22 (3H, s), 2.62-2.72 (4H, m), 3.12-3.19 (2H, m), 3.67-3.46 (2H, m), 3.62 (3H, s), 3.63 (1H, d, J = 12.3 Hz), 3.89 (3H, s), 4.39-4.48 (2H, m), 6.19 (1H, s), 6.60 (1H, d, J = 2.1 Hz), 6.98-7.47 (5H, m).

### Example 39

### 3-[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl]pyrazol-4-carboxylic acid

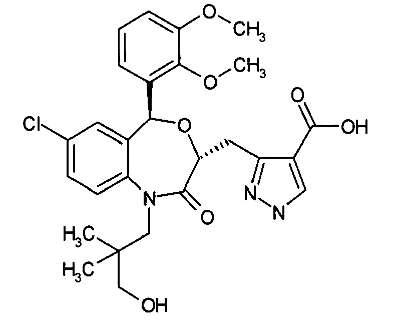

(1) N,N-dimethylformaldehydedimethylacetal (0.41 ml) was added to a solution of ethyl 4-[((3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl)]-3-oxobutanoate (1.5 g) obtained in Example 26-(1) in toluene (30 ml) and the mixture was refluxed overnight. After the reaction solution was concentrated, ethanol (30 ml) and hydrazine hydrate (0.14 g) were added to the residue and the mixture was refluxed for 3 hours. The reaction solution was concentrated and the residue was purified by silica gel column chromatography to obtain ethyl 3-[((3R,5S)-7-chloro-5-(2,3-dimethoxyphenyl)-1-(3-acetoxy-2,2-dimethylpropyl)-2-oxo-1,2,3,5-tetrahydro-4,1-benzoxazepin-3-yl)methyl]pyrazol-4-carboxylate (0.90 g) as a brown oil.
   ¹H-NMR (CD₃OD) δ: 0.95 (3H, s), 1.02 (3H, s), 1.26 (3H, t, J = 7.4 Hz), 2.03 (3H, s), 3.47-3.55 (2H, m), 3.62 (3H, s), 3.71-3.88 (2H, m), 3.89 (3H, s), 4.08-4.15 (2H, m), 4.21 (2H, q, J = 7.4 Hz), 4.54 (1H, d, J = 14.1 Hz), 6.27 (1H, s), 6.61 (1H, s), 6.96-6.99 (1H, m), 7.10-7.33 (4H, m), 7.87 (1H, s).
(2) A mixture of ethyl 3-[((3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl)] methyl]pyrazol-4-carboxylate (1.0 g) obtained in Example 39-(1), a 2N aqueous saturated sodium hydroxide solution (3.3 ml) and ethanol (20 ml) was stirred at 80°C overnight. This mixture was diluted with water (50 ml), acidified, and then extracted with chloroform (50 ml) twice. This extract was washed with an aqueous saturated sodium chloride solution, dried over magnesium sulfate, and then concentrated under reduced pressure. The residue was purified by silica gel column chromatography to obtain 3-[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl]pyrazol-4-carboxylic acid (0.18 g) as a white crystal.
   ¹H-NMR (CDCl₃) δ: 0.65 (3H, s), 1.06 (3H, s), 3.18 (1H, d, J = 11.7 Hz), 3.37 (1H, d, J = 15.0 Hz), 3.56-3.61 (3H, m), 3.63 (3H, s), 3.89 (3H, s), 4.14 (1H, t, J = 6.9 Hz), 4.49 (1H, d, J = 14.4 Hz), 6.19 (1H, s), 6.60 (1H, d, J = 2.4 Hz), 6.97-7.00 (1H, m), 7.08-7.10 (1H, m), 7.16-7.36 (5H, m), 7.94 (1H, s).

### Example 40

### 1-(3-carboxypropyl)-3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

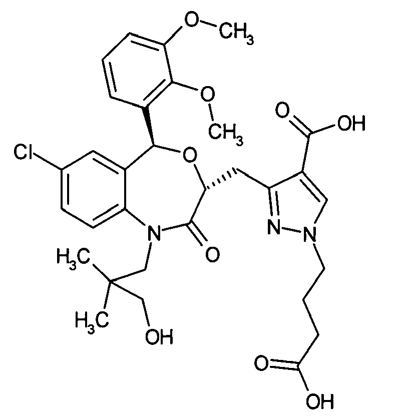

(1) Potassium carbonate (0.741 g) was added to a solution of ethyl-5-{[(3R,5S)-1-(3-(acetoxy)-2,2-dimethylpropyl)-7-chloro-5-(2,3-dimethoxyphenyl)-2-oxo-1,2,3,5-tetrahydro-4,1-benzoxazepin-3-yl]methyl}-1H-pyrazol-4-carboxylate (3.00 g) obtained in Example 39-(1) and ethyl 4-bromobutanoate (0.794 ml) in DMF (30 ml) and the mixture was stirred overnight. The reaction solution was diluted with ethyl acetate, washed with water and an aqueous saturated sodium chloride solution, dried over magnesium sulfate, and then concentrated under reduced pressure. The residue was purified by silica gel column chromatography to obtain ethyl 3-{[(3R,5S)-1-(3-(acetoxy)-2,2-dimethylpropyl)-7-chloro-5-(2,3-dimethoxyphenyl)-2-oxo-1,2,3,5-tetrahydro-4,1-benzoxazepin-3-yl]methyl}-1-(4-ethoxy-4-oxobutyl)-1H-pyrazol-4-carboxylate (0.98 g) from a first eluted fraction and ethyl 5-({(3R,5S)-1-[3-(acetoxy)-2,2-dimethylpropyl]-7-chloro-5-(2,3-dimethoxyphenyl)-2-oxo-1,2,3,5-tetrahydro-4,1-benzoxazepin-3-yl}methyl)-1-(4-ethoxy-4-oxobutyl)-1H-pyrazol-4-carboxylate (1.78 g) from a second eluted fraction.
(2) To a solution of ethyl 3-{[(3R,5S)-1-(3-(acetoxy)-2,2-dimethylpropyl)-7-chloro-5-(2,3-dimethoxyphenyl)-2-oxo-1,2,3,5-tetrahydro-4,1-benzoxazepin-3-yl]methyl}-1-(4-ethoxy-4-oxobutyl)-1H-pyrazol-4-carboxylate (0.8 g) obtained in Example 40-(1) in ethanol (8 ml) was added a 1N aqueous sodium hydroxide solution (4 ml) and the mixture was stirred overnight. The reaction solution was neutralized with 1N hydrochloric acid and extracted with ethyl acetate. This extract was washed with an aqueous saturated sodium chloride solution, dried over magnesium sulfate, and then concentrated under reduced pressure. The residue was purified by silica gel column chromatography to obtain the title compound (157 mg).
   ¹H-NMR (CDCl₃) δ: 0.64 (3H, s), 1.03 (3H, s), 2.10-2.21 (2H, m), 2.28-2.38 (2H, m), 3.16 (1H, d, J = 12.1 Hz), 3.28-3.45 (2H, m), 3.52-3.69 (5H, m), 3.86 (3H, s), 4.13 (2H, t, J = 6.22 Hz), 4.41-4.52 (2H, m), 6.17 (1H, s), 6.56 (1H, d, J = 2.07 Hz), 6.94 (1H, dd, J = 1.9, 7.9 Hz), 7.05-7.17 (2H, m), 7.30-7.40 (2H, m), 7.85 (1H, s).

### Example 41

### 1-(3-carboxypropyl)-5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

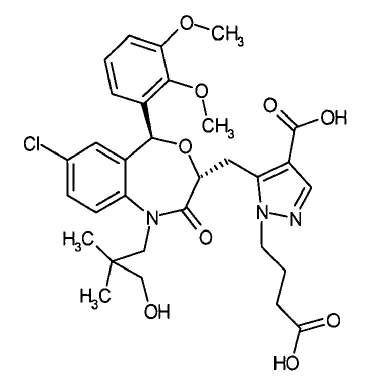

A 1N aqueous sodium hydroxide solution (4 ml) was added to a solution of ethyl 5-({(3R,5S)-1-[3-(acetyloxy)-2,2-dimethylpropyl]-7-chloro-5-(2,3-dimethoxyphenyl)-2-oxo-1,2,3,5-tetrahydro-4,1-benzoxazepin-3-yl}methyl)-1-(4-ethoxy-4-oxobutyl)-1H-pyrazol-4-carboxylate (0.75 g) obtained in Example 40-(1) in ethanol (20 ml) and the mixture was stirred for 2 days. The reaction solution was neutralized with 1N hydrochloric acid and extracted with ethyl acetate. This extract was washed with an aqueous saturated sodium chloride solution, dried over magnesium sulfate, and then concentrated under reduced pressure. The residue was purified by silica gel column chromatography to obtain the title compound (590 mg).

¹H-NMR (CDCl₃) δ: 0.76 (3H, s), 0.87 (3H, s), 1.83-1.96 (2H, m), 2.14 (2H, t, J = 7.3 Hz), 3.03-3.20 (2H, m), 3.23-3.43 (2H, m), 3.50 (3H, s), 3.68 (1H, d, J = 14.1 Hz), 3.83 (3H, s), 4.07-4.25 (3H, m), 4.35 (1H, d, J = 14.1 Hz), 4.57 (1H, s), 6.10 (1H, s), 6.26 (1H, d, J = 2.6 Hz), 6.59 (1H, d, J = 3.3, 5.9 Hz), 7.09-7.18 (2H, m), 7.50 (1H, dd, J = 2.5, 8.7 Hz), 7.70 (1H, d, J = 8.9Hz), 7.77 (1H, s).

### Example 42

1-(carboxymethyl)-3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

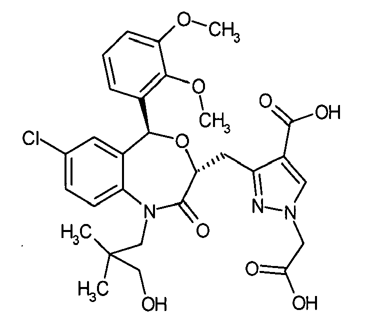

According to a similar manner to that of Example 40, the title compound was obtained.

¹H-NMR (DMSO-d₆) δ: 0.76 (3H, s), 0.84 (3H, s), 3.00-3.21 (2H, m), 3.22-3.57 (5H, m), 3.65 (1H, d, J = 14.0 Hz), 3.82 (3H, s), 4.23-4.40 (2H, m), 4.50 (2H, s), 6.06 (1H, s), 6.31 (1H, d, J = 2.5 Hz), 6.96 (1H, d, J = 7.0 Hz), 7.05-7.22 (2H, m), 7.49 (1H, dd, J = 2.1, 8.7 Hz), 7.68 (1H, d, J = 8.9 Hz), 7.99 (1H, s).

### Example 43

### 1-(carboxymethyl)-5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

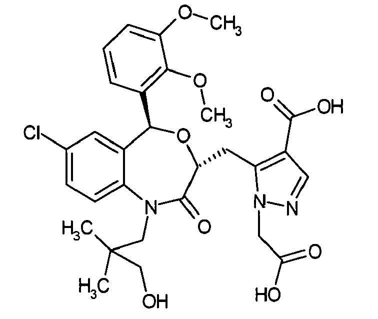

According to a similar manner to that of Example 41, the title compound was obtained.

¹H-NMR (DMSO-d₆) δ: 0.75 (3H, s), 0.87 (3H, s), 3.03-3.20 (2H, m), 3.24-3.41 (2H, m), 3.51 (3H, s), 3.66 (1H, d, J = 14.1 Hz), 3.84 (3H, s), 4.10-4.18 (1H, m), 4.33 (1H, d, J = 14.1 Hz), 4.51-4.60 (1H, m), 5.00 (2H, s), 6.11 (1H, s), 6.28 (1H, d, J = 2.6 Hz), 6.72 (1H, dd, J = 2.0, 7.3 Hz), 7.11-7.22 (2H, m), 7.50 (1H, dd, J = 2.5, 8.7 Hz), 7.69 (1H, d, J = 8.9 Hz), 7.77 (1H, s).

### Example 44

### 2-[5-({(3R,5S)-1-[3-(acetyloxy)-2,2-dimethylpropyl]-7-chloro-5-(2,3-dimethoxyphenyl)-2-oxo-1,2,3,5-tetrahydro-4,1-benzoxazepin-3-yl}methyl)-4-(ethoxycarbonyl)-1H-pyrazol-1-yl]benzoic acid

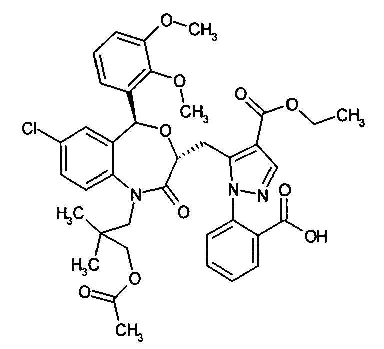

1,1-Dimethoxy-N,N-dimethylmethaneamine (0.598 ml) was added to a solution of ethyl 4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-3-oxobutanoate (2 g) in toluene (20 ml) and the mixture was refluxed overnight. After the mixture was concentrated under reduced pressure, ethanol (20 ml) and 2-hydrazinobenzoic acid (0.567 g) were added to the residue and the mixture was refluxed for 3 hours. After concentration under reduced pressure, the residue was purified by silica gel column chromatography to obtain the title compound (0.83 g).

¹H-NMR (DMSO-d₆) δ: 0.83-0.87 (6H, m), 1.11 (3H, t, J = 7.1 Hz), 1.89 (3H, s), 3.18-3.31 (2H, m), 3.45 (3H, s), 3.56-3.71 (3H, m), 3.83 (3H, s), 3.99-4.16 (3H, m), 4.26 (1H, d, J = 14.3 Hz), 6.00 (1H, s), 6.25 (1H, d, J = 2.3 Hz), 6.67 (1H, dd, J = 1.4, 7.3 Hz), 7.09-7.21 (2H, m), 7.38-7.45 (1H, m), 7.46-7.64 (4H, m), 7.83 (1H, d, J = 8.1 Hz), 7.97 (1H, s).

### Example 45

### 3-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-4-(ethoxycarbonyl)-1H-pyrazol-1-yl)benzoic acid

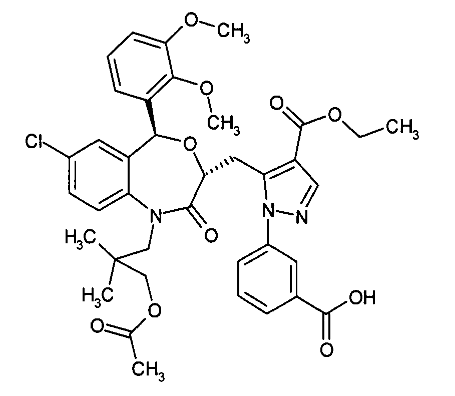

According to a similar manner to that of Example 44, the title compound was obtained.

¹H-NMR (DMSO-d₆) δ: 0.85 (3H, s), 0.87 (3H, s), 1.04-1.11 (3H, m), 1.87 (3H, s), 3.28-3.38 (1H, m), 3.45 (3H, s), 3.46-3.75 (4H, m), 3.82 (3H, s), 4.10 (3H, m), 4.32 (1H, d, J = 14.3 Hz), 6.01 (1H, s), 6.20 (1H, d, J = 2.5 Hz), 6.46 (1H, dd, J = 2.6, 6.6 Hz), 7.07-7.15 (2H, m), 7.47-7.59 (2H, m), 7.68 (1H, d, J = 8.9 Hz), 7.73-7.79 (1H, m), 7.93-7.98 (1H, m), 8.02 (1H, t, J = 1.8 Hz), 8.10 (1H, s).

### Example 46

### 4-(5-([(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-4-(ethoxycarbonyl)-1H-pyrazol-1-yl)benzoic acid

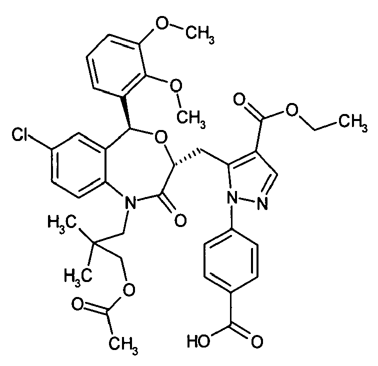

According to a similar manner to that of Example 44, the title compound was obtained.

¹H-NMR (DMSO-d₆) δ: 0.86 (s, 6H), 1.10 (3H, t, J = 7.1 Hz), 1.87 (3H, s), 3.38 (1H, d, J = 4.0 Hz), 3.44 (3H, s), 3.53-3.74 (4H, m), 3.82 (3H, s), 4.03-4.15 (3H, m), 4.32 (1H, d, J = 14.3 Hz), 5.99 (1H, s), 6.22 (1H, d, J = 2.5 Hz), 6.46 (1H, dd, J = 2.1, 7.0 Hz), 7.09-7.19 (2H, m), 7.46 (1H, dd, J = 8.7, 2.5 Hz), 7.58-7.69 (3H, m), 7.94 (2H, d, J = 8.5 Hz), 8.11 (1H, s).

### Example 47

### 1-(2-carboxyphenyl)-5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

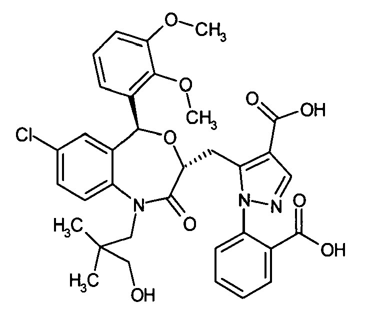

A 1N aqueous sodium hydroxide solution (3.4 ml) was added to a solution of 2-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-4-(ethoxycarbonyl)-1H-pyrazol-1-yl)benzoic acid (0.5 g) obtained in Example 44 in ethanol (5 ml) and the mixture was stirred overnight. The reaction solution was neutralized with 1N hydrochloric acid and extracted with ethyl acetate. This extract was washed with an aqueous saturated sodium chloride solution, dried over magnesium sulfate, and then concentrated under reduced pressure. The residue was purified by silica gel column chromatography to obtain the title compound (0.28 g).

¹H-NMR (DMSO-d₆) δ: 0.70 (3H, s), 0.79 (3H, s), 2.96-3.12 (2H, m), 3.22-3.37 (2H, m), 3.46 (3H, s), 3.56 (1H, d, J = 14.0 Hz), 3.83 (3H, s), 4.21 (1H, d, J = 14.0 Hz), 4.50 (1H, t, J = 5.2 Hz), 5.95 (1H, s), 6.24 (1H, d, J = 2.5 Hz), 6.69 (1H, dd, J = 1.6, 7.4 Hz), 7.08-7.23 (2H, m), 7.36-7.42 (1H, m), 7.44-7.58 (4H, m), 7.76-7.85 (1H, m), 7.91 (1H, s).

### Example 48

### 1-(3-carboxyphenyl)-5-([(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

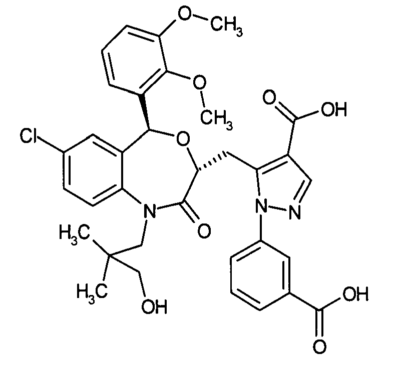

According to a similar manner to that of Example 47, the title compound was obtained.

¹H-NMR (DMSO-d₆) δ: 0.70 (3H, s), 0.81 (3H, s), 2.98-3.14 (2H, m), 3.46 (3H, s), 3.48-3.66 (2H, m), 3.82 (3H, s), 4.20 (1H, dd, J = 3.9, 9.6 Hz), 4.28 (1H, d, J = 14.1 Hz), 5.96 (1H, s), 6.19 (1H, d, J = 2.5 Hz), 6.46 (1H, dd, J = 2.5, 6.7 Hz), 7.10 (2H, m), 7.43-7.65 (4H, m), 7.71-7.78 (1H, m), 7.94 (1H, d, J = 7.9 Hz), 8.02-8.06 (2H, m).

### Example 49

### 1-(4-carboxyphenyl)-5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-4-carboxylic acid

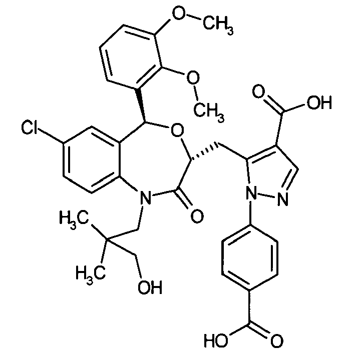

According to a similar manner to that of Example 47, the title compound was obtained.

¹H-NMR (DMSO-d₆) δ: 0.70 (3H, s), 0.80 (3H, s), 2.97-3.13 (2H, m), 3.45 (3H, s), 3.56-3.70 (2H, m), 3.82 (3H, s), 4.07 (1H, dd, J = 3.9, 9.8 Hz), 4.23-4.32 (1H, m), 4.51 (1H, t, J = 4.9 Hz), 5.93 (1H, s), 6.20 (1H, d, J = 2.5 Hz), 6.47 (1H, dd, J = 1.8, 7.1 Hz), 7.07-7.20 (2H, m), 7.41 (1H, dd, J = 2.5, 8.7 Hz), 7.54-7.64 (3H, m), 7.92 (2H, d, J = 8.7 Hz), 8.06 (1H, s).

### Example 50

### 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-5-carboxylic acid

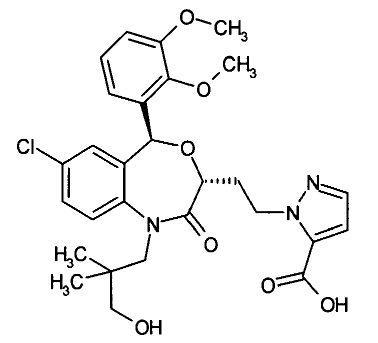

(1) [(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (10 g, 19.2 mmol) was dissolved in THF (80 ml), and N-methylmorpholine (2.2 ml, 23.1 mmol) and ethyl chlorocarbonate (2.54 ml, 23.1 mmol) were added dropwide under ice-cooling thereto. After stirring at the same temperature for 1 hour, the mixture was cooled to -30°C and sodium borohydrate (2.18 g, 58.0 mmol) was added thereto. Further, MeOH (80 ml) was added dropwise gradually and the mixture was stirred for 1 hour. At the same temperature, an aqueous saturated ammonium chloride solution was added thereto and the mixture was extracted with AcOEt (480 ml). The organic layer was washed with water and an aqueous saturated sodium chloride solution, dried over anhydrous sodium sulfate, and then concentrated under reduced pressure. The resulting residue was purified by silica gel column chromatography [developing solvent: hexane-ethyl acetate (2:3)] to obtain (3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-ethanol (9.3 g) as a colorless crystal.
   ¹H-NMR (CDCl₃) δ: 0.95 (3H, s), 1.03 (3H, s), 2.03 (3H, s), 2.05-2.30 (2H, m), 3.57 (1H, d, J = 13.8 Hz), 3.63 (3H, s), 3.70-3.93 (7H, m), 4.10 (1H, t, J = 6.2 Hz), 4.55 (1H, d, J = 13.8 Hz), 6.28 (1H, s), 6.64 (1H, d, J = 2.2 Hz), 6.93-7.05 (1H, m), 7.15-7.23 (2H, m), 7.31 (1H, s), 7.31 (1H, dd, J = 2.2, 8.8 Hz).
(2) Under nitrogen atmosphere, n-tributylphosphine (0.74 ml, 2.96 mmol) and 1,1'-azodicarbonyldipiperidine (0.75 g, 2.96 mmol) were added to a solution of (3R,5S)-1-(3-acetoxyl-2,2-dimethylpropyl)-7-chloro-5-(2,3-dimethoxyphenyl)-1,2,3,5-tetrahydro-2-oxo-4,1-benzoxazepin-3-ethanol (1.0 g, 1.98 mmol) obtained in Example 50-(1), methyl 1H-pyrazol-5-carboxylate (0.25 g, 1.98 mmol) and toluene (20 ml) and the mixture was stirred at room temperature for 8 hours. After hexane (10 ml) was added thereto, the mixture was stirred for 30 minutes and then filtered to remove insoluble substances. The filtrate was concentrated under reduced pressure and the resulting residue was purified by silica gel column chromatography [developing solvent: hexane-ethyl acetate (70:30-65:35)] to obtain methyl 1-{2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1H-pyrazol-5-carboxylate (0.81 g) as colorless noncrystalline powder from a first eluted fraction and methyl 1-{2-[(3R,5S)-(1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1H-pyrazol-3-carboxylate (0.17 g) as colorless powder from a second eluted fraction.
   First eluted fraction
      ¹H-NMR (CDCl₃) δ: 0.94 (3H, s), 1. 02 (3H, s), 2. 02 (3H, s), 2.30-2.41 (2H, m), 3.50 (1H, d, J = 14.1 Hz), 3.60 (3H, s), 3.73 (1H, d, J = 11.4 Hz), 3.77 (1H, t, J = 6.6 Hz), 3.82 (3H, s), 3.84 (1H, d, J = 11.4 Hz), 3.88 (3H, s), 4.55 (1H, d, J = 14.1 Hz), 4.65-4.75 (2H, m), 6.24 (1H, s), 6.60 (1H, d, J = 2.4 Hz), 6.74 (1H, d, J = 2.1 Hz), 6.97 (1H, dd, J = 1.5, 8.1 Hz), 7.10-7.27 (3H, m), 7.30 (1H, dd, J = 2.4, 8.4 Hz), 7.35 (1H, d, J = 2.1 Hz).
   Second eluted fraction
      ¹H-NMR (CDCl₃) δ: 0.94 (3H, s), 1.01 (3H, s), 2.02 (3H, s), 2.30-2.45 (2H, m), 3.50 (1H, d, J = 14.1 Hz), 3.61 (3H, s), 3.71 (1H, d, J = 11.1 Hz), 3.78 (1H, t, J = 6.9 Hz), 3.84 (1H, d, J = 11.1 Hz), 3.89 (6H, s), 4.28-4.50 (2H, m), 4.51 (1H, d, J = 14.1 Hz), 6.24 (1H, s), 6.62 (1H, d, J = 2.4 Hz), 6.71 (1H, d, J = 2.4 Hz), 6.95-7.02 (1H, m), 7.18-7.22 (2H, m), 7.26 (1H, d, J = 8.7 Hz), 7.32 (1H, dd, J = 2.4, 8.7 Hz), 7.37 (1H, d, J = 2.4 Hz)
(3) A mixture of methyl 1-{2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1H-pyrazol-5-carboxylate (0.72 g, 1.17 mmol) obtained in Example 50-(2), a 1N aqueous sodium hydroxide solution (4.0 ml) and ethanol (14 ml) was stirred at room temperature for 3 hours. This mixture was acidified with 1N hydrochloric acid (5.0 ml) and ethyl acetate (150 ml) was added. The organic layer was washed with an aqueous saturated sodium chloride solution, dried over magnesium sulfate, and then concentrated under reduced pressure. The resulting crystal was recrystallized with ethyl acetate-hexane to obtain 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-5-carboxylic acid (0.63 g) as a colorless crystal.
   ¹H-NMR (CDCl₃) δ: 0.63 (3H, s), 1.04 (3H, s), 2.28-2.50 (2H, m), 3.16 (1H, d, J = 12.3 Hz), 3.35 (1H, d, J = 14.4 Hz), 3.60 (3H, s), 3.62 (1H, d, J = 12.3 Hz), 3.79 (1H, t, J = 6.6 Hz), 3.88 (3H, s), 4.43 (1H, d, J = 14.4 Hz), 4.59-4.81 (2H, m), 6.12 (1H, s), 6.58 (1H, d, J = 2.1 Hz), 6.84 (1H, d, J = 2.1 Hz), 6.97 (1H, dd, J = 1.5, 7.8 Hz), 7.20 (1H, t, J = 7.8 Hz), 7.21-7.29 (2H, m), 7.32 (1H, dd, J = 2.1, 8.7 Hz), 7.40 (1H, d, J = 2.1 Hz).

### Example 51

### 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-3-carboxylic acid

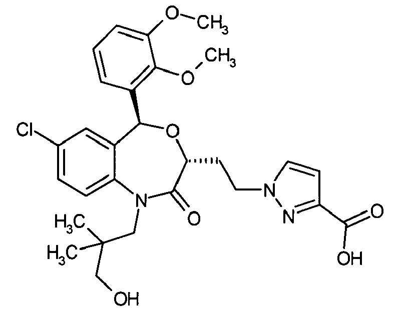

A mixture of methyl 1-{2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1H-pyrazol-3-carboxylate (0.17 g, 0.28 mmol) obtained in Example 50-(2), a 1N aqueous sodium hydroxide solution (1.0 ml) and ethanol (5 ml) was stirred at room temperature for 3 hours. Further, a 1N aqueous sodium hydroxide solution (1.5 ml) was added and the mixture was stirred for 3 hours. This mixture was acidified with 1N hydrochloric acid (2.8 ml) and ethyl acetate (80 ml) was then added. The organic layer was washed with an aqueous saturated sodium chloride solution, dried over magnesium sulfate, and then concentrated under reduced pressure. The resulting crystal was recrystallized with ethyl acetate-hexane to obtain 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-3-carboxylic acid (0.12 g) as a colorless crystal.

¹H-NMR (CDCl₃) δ: 0.63 (3H, s), 1.05 (3H, s), 2.30-2.50 (2H, m), 3.15 (1H, d, J = 11.1 Hz), 3.36 (1H, d, J = 14.4 Hz), 3.60 (1H, d, J = 11.1 Hz), 3.61 (3H, s), 3.82 (1H, t, J = 6.6 Hz), 3.90 (3H, s), 4.30-4.50 (3H, m), 6.15 (1H, s), 6.61 (1H, s), 6.75-6.87 (1H, m), 7.15-7.50 (5H, m).

### Example 52

### (2E)-3-(1-{2-[(3R,5S)-7-chloro-5-(2,3-dimethoxyphenyl-1-(3-hydroxy-2,2-dimethylpropyl)-2-oxo-1,2,3,5-tetrahydro-4,1-benzoxazepin-3-yl)ethyl]-1H-pyrazol-5-yl}acrylic acid

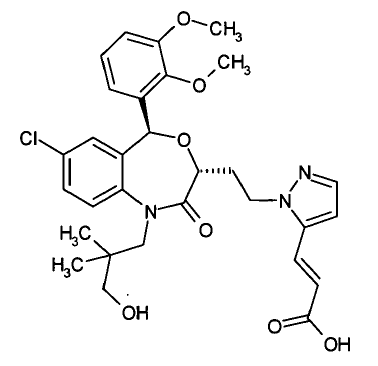

According to a similar manner to that of Example 19, the title compound was obtained.

¹H-NMR (CDCl₃) δ: 0. 64 (3H, s), 1.05 (3H, s), 1.26 (3H, t, J = 7.0 Hz), 2.29-2.45 (2H, m), 3.15 (1H, d, J = 11.8 Hz), 3.36 (1H, d, J = 14.2 Hz), 3.64 (1H, d, J = 11.8 Hz), 3.36 (1H, d, J = 14.2 Hz), 3.64 (1H, d, J = 11.8 Hz), 3.65 (3H, s), 3.85 (1H, t, J = 6.2 Hz), 3.90 (3H, s), 4.21-4.40 (2H, m), 4.44 (1H, d, J = 14.2 Hz), 6.15 (1H, s), 6.32 (1H, d, J = 16.0 Hz), 6.41 (1H, d, J = 2.2 Hz), 6.60 (1H, d, J = 1.8 Hz), 6.95-7.10 (1H, m), 7.16-7.30 (2H, m), 7.26-7.41 (3H, m), 7.64 (1H, d, J = 16.0 Hz).

### Example 53

### 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-imidazol-5-carboxylic acid

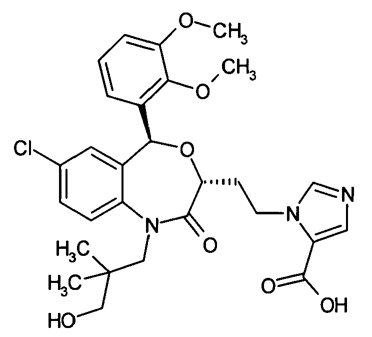

According to a similar manner to that of Example 50, the title compound was obtained.

¹H-NMR (CDCl₃) δ: 0.63 (3H, s), 1.04 (3H, s), 2.29-2.40 (2H, m), 3.15 (1H, d, J = 12.0 Hz), 3.36 (1H, d, J = 14.1 Hz), 3.60 (1H, d, J = 12.0 Hz), 3.61 (3H, s), 3.85 (1H, t, J = 7.5 Hz), 3.89 (3H, s), 4.44 (1H, d, J = 14.1 Hz), 6.14 (1H, s), 6.61 (1H, d, J = 2 . 1 Hz), 6.98-7.02 (1H, m), 7.13-7.26 (2H, m), 7.28 (1H, d, J = 8.7 Hz), 7.34 (1H, dd, J = 2.1, 8.7 Hz), 7.64 (1H, s), 7.77 (1H, s).

### Example 54

### 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrrol-2-carboxylic acid

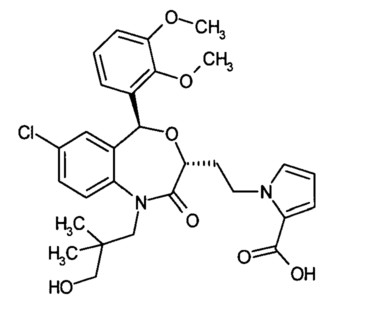

According to a similar manner to that of Example 50, the title compound was obtained.

¹H-NMR (CDCl₃) δ: 0.63 (3H, s), 1.04 (3H, s), 2.21-2.39 (2H, m), 3.14 (1H, d, J = 11.4 Hz), 3.35 (1H, d, J = 14.4 Hz), 3.61 (1H, d, J = 11.4 Hz), 3.61 (3H, s), 3.75 (1H, t, J = 6.9 Hz), 3.89 (3H, s), 4.30-4.61 (2H, m), 4.44 (1H, d, J = 14.4 Hz), 6.05-6.10 (1H, m), 6.13 (1H, s), 6.60 (1H, d, J = 2.1 Hz), 6.81-6.90 (1H, m), 6.94-7.03 (2H, m), 7.15-7.30 (3H, m), 7.32 (1H, dd, J = 2.1, 8.7 Hz).

### Example 55

### (5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-oxadiazol-2-yl)acetic acid

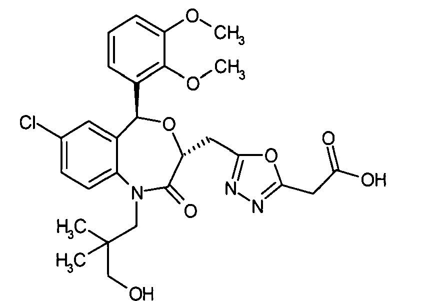

(1) Pivaloyl chloride (2.8 ml) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (10 g) and triethylamine (3.2 ml) in THF (100 ml) at 0°C and the mixture was stirred at 0°C for 30 minutes. To the reaction solution at 0°C, ethyl 3-hydrazino-3-oxopropionate (3.5 g) was added and triethylamine (3.2 ml) was then added dropwise. The mixture was stirred at room temperature for 2 hours and the reaction solution was diluted with ethyl acetate (50 ml). This extract was washed with 0.1N hydrochloric acid, a 5% aqueous potassium hydrogen sulfate solution, an aqueous saturated sodium hydrogen carbonate solution and an aqueous saturated sodium chloride solution, dried over magnesium sulfate, and then concentrated under reduced pressure. The residue was purified by silica gel column chromatography to obtain ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}hydrazino)-3-oxopropionate (2.5 g) as a colorless oil.
   ¹H-NMR (CDCl₃) δ: 0. 94 (3H, s), 1.00 (3H, s), 1.25 (3H, t, J = 7.2 Hz), 2.02 (3H, s), 2.70-2.77 (1H, m), 2.92-3.00 (1H, m), 3.39 (2H, s), 3.53 (1H, d, J = 14.2 Hz), 3.60 (3H, s), 3.69-3.87 (2H, m), 3.88 (3H, s), 4.19 (2H, q, J = 7.2 Hz), 4.22-4.39 (1H, m), 4.54 (1H, d, J = 14.4 Hz), 6.25 (1H, s), 6.63 (1H, d, J = 1.8 Hz), 6.96-6.99 (1H, s), 7.15-7.24 (2H, m), 7.28-7.35(2H, m).
(2) Phosphorus oxychloride (0.8 ml) was added to a solution of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}hydrazino)-3-oxopropionate (3.7 g) obtained in Example 55-(1) in DMF (50 ml) and the mixture was refluxed overnight. The reaction solution was concentrated under reduced pressure, poured into an aqueous potassium carbonate solution, and extracted with ethyl acetate. This extract was washed with an aqueous saturated sodium chloride solution, dried over magnesium sulfate, and then concentrated under reduced pressure. The residue was purified by silica gel column chromatography to obtain ethyl 5-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-1,3,4-oxadiazol-2-yl)acetate (1.2 g) as a yellow oil.
   ¹H-NMR (CDCl₃) δ: 0.95 (3H, s), 1.02 (3H, s), 1.26 (3H, t, J = 6.9 Hz), 2.03 (3H, s), 3.31-3.57 (3H, m), 3.61 (3H, s), 3.75-3.87 (2H, m), 3.88 (3H, s), 3.91 (2H, s), 4.17 (2H, q, J = 6.9 Hz), 4.46-4.57 (2H, m), 6.30 (1H, s), 6.61 (1H, d, J = 2.1 Hz), 6.95-6.98 (1H, m), 7.05-7.19 (2H, m), 7.33-7.34(2H, m).
(3) A mixture of ethyl 5-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-1,3,4-oxadiazol-2-yl)acetate (1.1 g) obtained in Example 55-(2), a 1N aqueous sodium hydroxide solution (9.35 ml) and methanol (20 ml) was stirred at room temperature for 30 minutes. This mixture was diluted with water (50 ml), acidified, and extracted with chloroform (50 ml) twice. This extract was washed with an aqueous saturated sodium chloride solution, dried over magnesium sulfate, and then concentrated under reduced pressure. The residue was purified by silica gel column chromatography to obtain (5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-oxadiazol-2-yl)acetic acid (0.19 g) as a white crystal.
   ¹H-NMR (DMSO-d₆) δ: 0.63 (3H, s), 0.92 (3H, s), 3.12-3.16(2H, m), 3.38-3.44 (2H, m), 3.56 (3H, s), 3.61-3.79 (2H, m), 3.85 (3H, s), 4.24-4.50 (3H, m), 6.14 (1H, s), 6.55 (1H, s), 6.92-6.95 (1H, m), 7.12-7.17 (2H, m), 7.31-7.37(2H, m).

### Example 56

### (5-([(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-thiadiazol-2-yl)acetic acid

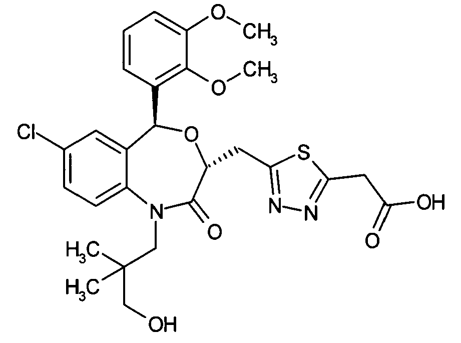

(1) A solution of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}hydrazino))-3-oxopropionate (1.0 g) obtained in Example 55-(1) and Lawesson's reagent (0.96 g) in THF (50 ml) was stirred at 80°C for 1 hour. The reaction solution was poured into water, followed by extraction with ethyl acetate. This extract was washed with an aqueous saturated sodium chloride solution, dried over magnesium sulfate, and then concentrated under reduced pressure. The residue was purified by silica gel column chromatography to obtain ethyl 5-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-1,3,4-thiadiazol-2-yl)acetate (0.8 g) as a white amorphous crystal.
   ¹H-NMR (CDCl₃) δ: 0. 95 (3H, s), 1.02 (3H, s), 1.30 (3H, t, J = 7.0 Hz), 2.03 (3H, s), 3.51-3.58 (1H, m), 3.62 (3H, s), 3.66-3.87 (4H, m), 3.89 (3H, s), 4.11-4.16 (2H, m), 4.24 (2H, q, J = 7.0 Hz), 4.35 (1H, t, J = 6.4 Hz), 4.55 (1H, d, J = 14.3 Hz), 6.32 (1H, s), 6.61 (1H, d, J = 1.9 Hz), 6.99 (1H, dd, J = 2.3, 7.5 Hz), 7.17-7.20 (2H, m), 7.30-7.32(2H, m).
(2) A mixture of ethyl 5-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-1,3,4-thiadiazol-2-yl)acetate (0.9 g) obtained in Example 56-(1), a 1N aqueous saturated sodium hydroxide solution (8.0 ml) and methanol (20 ml) was stirred at room temperature for 30 minutes. This mixture was diluted with water (50 ml), acidified, and extracted with chloroform (50 ml) twice. This extract was washed with an aqueous saturated sodium chloride solution, dried over magnesium sulfate, and then concentrated under reduced pressure. The residue was purified by silica gel column chromatography to obtain ethyl 5-(2-{[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]methyl}-1,3,4-thiadiazol-2-yl)acetate (0.19 g) as a white crystal.
   ¹H-NMR (DMSO-d₆) δ: 0.76 (3H, s), 0.84 (3H, s), 3.06-3.18 (2H, m), 3.35-3.49 (2H, m), 3.52 (3H, s), 3.66-3.71 (1H, m), 3.78 (2H, s), 3.84 (3H, s), 4.26-4.35 (2H, m), 6.14 (1H, s), 6.38 (1H, d, J = 2.3 Hz), 7.06-7.22 (3H, m), 7.51-7.55 (1H, m), 7.71(1H, d, J = 9.0 Hz).

### Example 57

### 3-(5-{[(3R,5S)-7-chloro-5-(2,3-dimethoxyphenyl)-1-(3-hydroxyl-2,2-dimethylpropyl)-2-oxo-1,2,3,5-tetrahydro-4,1-benzoxazepin-3-yl]methyl}-1,3,4-thiadiazol-2-yl)propionic acid

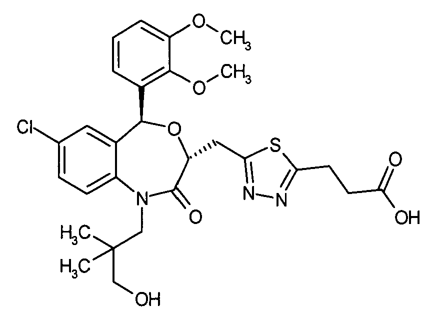

(1) Pivaloyl chloride (2.8 ml) was added to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (10 g) and triethylamine (3.2 ml) in THF (100 ml) at 0°C and the mixture was stirred at 0°C for 30 minutes. to the reaction solution at 0°C, ethyl 4-hydrazino-4-oxobutanoate (3.7 g) was added and triethylamine (3.2 ml) was then added dropwise. After stirring at room temperature for 2 hours, the reaction solution was diluted with ethyl acetate (50 ml). This diluted solution was washed with 0.1N hydrochloric acid, a 5% aqueous potassium hydrogen sulfate solution, an aqueous saturated sodium hydrogen carbonate solution and an aqueous saturated sodium chloride solution, dried over magnesium sulfate, and then concentrated under reduced pressure. The residue was purified by silica gel column chromatography to obtain ethyl 4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}hydrazino)-4-oxobutanoate (3.6 g) as a colorless oil.
   ¹H-NMR (CDCl₃) δ: 0.95 (3H, s), 1.00 (3H, s), 1.25 (3H, t, J = 7.2 Hz), 2.03 (3H, s), 2.54 (3H, t, J = 6.4 Hz), 2.66-2.75 (3H, m), 2.89-2.97 (1H, m), 3.54 (1H, d, J = 14.3 Hz), 3.61 (3H, s), 3.69-3.88 (2H, m), 3.89 (3H, s), 4.15 (2H, q, J = 7.2 Hz), 4.33-4.38 (1H, m), 4.54 (1H, d, J = 13.9 Hz), 6.26 (1H, s), 6.64 (1H, d, J = 1.9 Hz), 6.97-7.00 (1H, s), 7.16-7.23 (2H, m), 7.32-7.36(2H, m), 8.11 (1H, brd), 8.31 (1H, brd).
(2) A solution of ethyl 4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}hydrazino)-4-oxobutanoate (1.0 g) obtained in Example 57-(1) and Lawesson's reagent (0.92 g) in THF (50 ml) was stirred at 80°C for 1 hour. The reaction solution was poured into water and then extracted with ethyl acetate. This extract was washed with an aqueous saturated sodium chloride solution, dried over magnesium sulfate, and then concentrated under reduced pressure. The residue was purified by silica gel column chromatography to obtain ethyl 3-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3,4-thiadiazol-2-yl)propionate (0.7 g) as white powder.
   ¹H-NMR (CDCl₃) δ: 0.95 (3H, s), 1.02 (3H, s), 1.29 (3H, t, J = 7.2 Hz), 2.03 (3H, s), 2.85 (2H, t, J = 7.4 Hz), 3.37 (3H, t, J = 7.4 Hz), 3.48-3.55 (2H, m), 3.62 (3H, s), 3.65-3.87 (2H, m), 3.90 (3H, s), 4.15 (2H, q, J = 7.2 Hz), 4.35 (1H, t, J = 6.4 Hz), 4.54 (1H, d, J = 14.3 Hz), 6.31 (1H, s), 6.61 (1H, d, J = 2.3 Hz), 6.98-7.01 (1H, m), 7.13-7.23 (2H, m), 7.28-7.35(2H, m).
(3) A mixture of ethyl 3-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3,4-thiadiazol-2-yl)propionate (0.7 g) obtained in Example 57-(2), a 1N aqueous sodium hydroxide solution (8.0 ml) and methanol (20 ml) was stirred at room temperature for 30 minutes. This mixture was diluted with water (50 ml), acidified, and extracted with chloroform (50 ml) twice. This extract was washed with an aqueous saturated sodium chloride solution, dried over magnesium sulfate, and then concentrated under reduced pressure. The residue was purified by silica gel column chromatography to obtain 3-(5-{[(3R,5S)-7-chloro-5-(2,3-dimethoxyphenyl)-1-(3-hydroxyl-2,2-dimethylpropyl)-2-oxo-1,2,3,5-tetrahydro-4,1-benzoxazepin-3-yl]methyl}-1,3,4-thiadiazol-2-yl)propionic acid (0.19 g) as a white crystal.
   ¹H-NMR (DMSO-d₆) δ: 0.78 (3H, s), 0.88 (3H, s), 2.75 (3H, t, J = 7.0 Hz), 3.08-3.20 (2H, m) 3.26 (3H, t, J = 7.0 Hz), 3.49-3.52 (1H, m), 3.53 (3H, s), 3.67-3.81 (1H, m), 3.85 (3H, s), 4.27-4.37 (2H, m) 4.58 (1H, brs), 6.16 (1H, s), 6.40 (1H, d, J = 2.6 Hz), 7.02-7.05 (1H, m), 7.14-7.26 (2H, m), 7.54 (1H, dd, J = 2.6, 8.7 Hz), 7.72(1H, d, J = 8.7 Hz).

### Example 58

### (3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-5-oxo-1,2,4-oxadiazol-4(5H)-yl)acetic acid

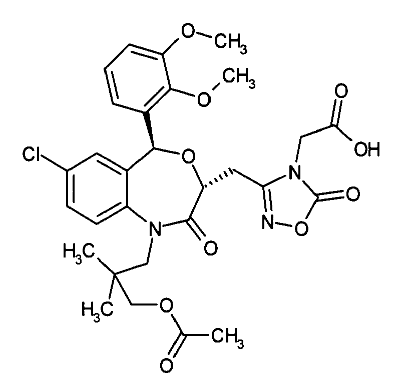

(1) Triethylamine (10.0 ml, 0.0717 mol) was added to a suspension of hydroxylamine hydrochloride (4.98 g, 0.0717 mol) in DMSO and the mixture was stirred for 30 minutes. The reaction solution was filtered and to the filtrate was added 3-[(3R,5S)-7-chloro-3-(cyanomethyl)-5-(2,3-dimethoxyphenyl)-2-oxo-2,3-dihydro-4,1-benzoxazepin-1(5H)-yl]-2,2-dimethylpropyl acetate (7.18 g, 0.0143 mol). The mixture was stirred at 80°C for 18 hours. The reaction solution was extracted with ethyl acetate, washed successively with a 1N aqueous sodium hydroxide solution and an aqueous saturated sodium chloride solution, dried over magnesium sulfate, and then concentrated under reduced pressure to obtain 3-[(3R,5S)-3-[(2Z)-2-amino-2-(hydroxyimino)ethyl]-7-chloro-5-(2,3-dimethoxyphenyl)-2-oxo-2,3-dihydro-4,1-benzoxazepin-1(5H)-yl]-2,2-dimethylpropyl acetate (7.9 g) as an acetone-containing amorphous substance.
   ¹H-NMR (CDCl₃) δ: 0.94 (3H, s), 1.02 (3H, s), 2.03 (3H, s), 2.59 (1H, dd, J = 6.1, 14.4 Hz), 2.72-2.80 (1H, m), 3.50-3.66 (4H, m), 3.72 (1H, d, J = 11.0 Hz), 3.82-3.88 (1H, m), 3.89 (3H, s), 4.05-4.16 (1H, m), 4.54 (1H, d, J = 14.2 Hz), 4.94 (2H, s), 6.25 (1H, s), 6.62 (1H, d, J = 2.2 Hz), 6.95-7.01 (1H, m), 7.15-7.23 (2H, m), 7.28-7.35 (2H, m).
(2) CDI (1.34 g, 8.24 mmol) and DBU (1.23 ml, 8.24 mmol) were added to a solution of 3-[(3R,5S)-3-[(2Z)-2-amino-2-(hydroxyimino)ethyl]-7-chloro-5-(2,3-dimethoxyphenyl)-2-oxo-2,3-dihydro-4,1-benzoxazepin-1(5H)-yl]-2,2-dimethylpropyl acetate (4.0 g, 7.49 mmol) obtained in Example 58-(1) in THF (40 ml) and the mixture was stirred at room temperature for 20 hours. The reaction solution was extracted with ethyl acetate, washed successively with a 1N aqueous hydrochloric acid solution and an aqueous saturated sodium chloride solution, dried over magnesium sulfate, and then concentrated under reduced pressure. The residue was purified by silica gel column chromatography [developing solvent: hexane-ethyl acetate (3:2-2:3)] to obtain 3-[(3R,5S)-7-chloro-5-(2,3-dimethoxyphenyl)-2-oxo-3-[(5-oxo-4,5-dihydro-1,2,4-oxadiazol-3-yl)methyl]-2,3-dihydro-4,1-benzoxazepin-1(5H)-yl]-2,2-dimethylpropyl acetate (2.53 g) as a colorless amorphous substance.
   ¹H-NMR (CDCl₃) δ: 0.97 (3H, s), 1.00 (3H, s), 2.05 (3H, s), 3.01 (1H, dd, J = 4.0, 15.0 Hz), 3.11 (1H, dd, J = 6.9, 15.0 Hz), 3.53 (1H, d, J = 14.2 Hz), 3.65 (3H, s), 3.68 (1H, d, J = 11.2 Hz), 3.89 (3H, s), 3.95 (1H, d, J = 11.2 Hz), 4.20 (1H, dd, J = 4.2, 6.8 Hz), 4.58 (1H, d, J = 14.2 Hz), 6.24 (1H, s), 6.67 (1H, d, J = 2.4 Hz), 7.00 (1H, dd, J = 1.7, 8.1 Hz), 7.10-7.22 (2H, m), 7.29-7.40 (2H, m).
(3) Sodium hydride (46 mg, 1.16 mmol) was added to a solution of 3-[(3R,5S)-7-chloro-5-(2,3-dimethoxyphenyl)-2-oxo-3-[(5-oxo-4,5-dihydro-1,2,4-oxadiazol-3-yl)methyl]-2,3-dihydro-4,1-benzoxazepin-1(5H)-yl]-2,2-dimethylpropyl acetate (0.6 g, 0.893 mmol) obtained in Example 58-(2) in DMF under ice-cooling and the mixture was stirred for 30 minutes. To the reaction solution was added tert-butyl bromoacetate and the mixture was stirred for 1.5 hours under ice-cooling and then at room temperature for 30 minutes. After 1N hydrochloric acid was added to the reaction solution, the mixture was extracted with ethyl acetate, washed successively water and an aqueous saturated sodium chloride solution, and then dried over magnesium sulfate. After concentration under reduced pressure, the residue was purified by silica gel column chromatography (developing solvent: hexane-ethyl acetate (3:1-2:1)) to obtain tert-butyl (3-{[(3R,5S)-1-(3-acetoxy)-2,2-dimethylpropyl]-7-chloro-5-(2,3-dimethoxyphenyl)-2-oxo-1,2,3,5-tetrahydro-4,1-benzoxazepin-3-yl}methyl)-5-oxo-1,2,4-oxadiazol-4(5H)-ylacetate (0.43 g).
   ¹H-NMR (CDCl₃) δ: 0.95 (3H, s), 1.01 (3H, s), 1.47 (9H, s), 2.03 (3H, s), 2.94-3.09 (2H, m), 3.55 (1H, d, J = 14.2 Hz), 3.61 (3H, s), 3.71 (1H, d, J = 11.2 Hz), 3.85 (1H, d, J = 11.0 Hz), 3.89 (3H, s), 4.34-4.39 (3H, m), 4.51 (1H, d, J = 14.2 Hz), 6.28 (1H, s), 6.63 (1H, d, J = 2.2 Hz), 6.97-7.06 (2H, m), 7.19 (1H, t, J = 7.9 Hz), 7.29-7.39 (2H, m).
(4) Trifluoroacetic acid (5 ml) was added to tert-butyl (3-{[(3R,5S)-1-(3-acetyloxy)-2,2-dimethylpropyl]-7-chloro-5-(2,3-dimethoxyphenyl)-2-oxo-1,2,3,5-tetrahydro-4,1-benzoxazepin-3-yl}methyl)-5-oxo-1,2,4-oxadiazol-4(5H)-ylacetate (0.42 g, 0.616 mmol) obtained in Example 58-(3) under ice-cooling and the mixture was stirred for 30 minutes and then at room temperature for 50 minutes. The reaction solution was concentrated under reduced pressure to obtain [3-{[(3R,5S)-1-(3-acetyloxy-2,2-dimethylpropyl)-7-chloro-5-(2,3-dimethoxyphenyl)-2-oxo-1,2,3,5-tetrahydro-4,1-benzoxazepin-3-yl]methyl}-5-oxo-1,2,4-oxadiazol-4(5H)-yl]acetic acid (0.40 g).
   ¹H-NMR (CDCl₃) δ: 0.96 (3H, s), 0.98 (3H, s), 2.05 (3H, s), 3.05-3.08 (2H, m), 3.54 (1H, d, J = 14.2 Hz), 3.62 (3H, s), 3.73 (1H, d, J = 11.0 Hz), 3.85-3.95 (4H, m), 4.34 (1H, t, J = 11.0 Hz), 4.47-4.54 (3H, m), 6.27 (1H, s), 6.62 (1H, d, J = 2.2 Hz), 6.98-7.39 (6H, m).

### Example 59

### [3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-oxo-1,2,4-oxadiazol-4(5H)-yl]acetic acid

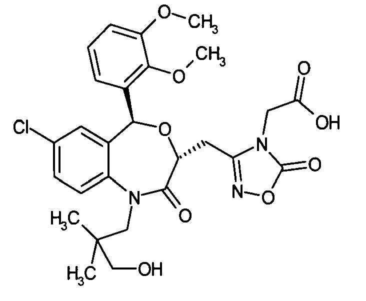

A 1N aqueous sodium hydroxide solution (1.21 ml, 1.21 mmol) was added to a mixture of (3-{[(3R,5S)-1-(3-acetyloxy)-2,2-dimethylpropyl)-7-chloro-5-(2,3-dimethoxyphenyl)-2-oxo-1,2,3,5-tetrahydro-4,1-benzoxazepin-3-yl]methyl}-5-oxo-1,2,4-oxadiazol-4(5H)-yl)acetic acid (0.25 g, 0.405 mmol) obtained in Example 58 in ethanol-THF (1:1) (3 ml) and the mixture was stirred for 2 hours. The reaction solution was acidified with 1N hydrochloric acid (1.5 ml), extracted with ethyl acetate, washed with water and an aqueous saturated sodium chloride solution, and then dried over magnesium sulfate. After concentration under reduced pressure, the residue was purified by preparative HPLC to obtain [3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-oxo-1,2,4-oxodiazol-4(5H)-yl]acetic acid (0.16 g).

¹H-NMR (CDCl₃) δ: 0.65 (3H, s), 1.05 (3H, s), 3.09 (2H, d, J = 7.4 Hz), 3.24 (1H, d, J = 12.1 Hz), 3.42 (1H, d, J = 14.6 Hz), 3.58-3.65 (4H, m), 3.87-3.90 (4H, m), 4.34 (1H, t, J = 6.7 Hz), 4.41 (1H, d, J = 14.6 Hz), 4.46 (2H, s), 6.16 (1H, s), 6.61 (1H, d, J = 2.2 Hz), 7.00 (2H, d, J = 8.3 Hz), 7.16-7.43 (3H, m).

### Example 60

### 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)propionic acid

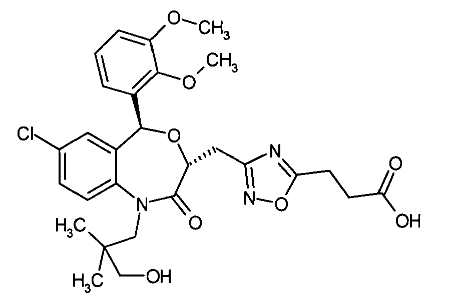

Triethylamine (0.44 ml, 3.18 mmol) and ethyl 4-chloro-4-oxobutyrate (0.29 ml, 2.06 mmol) were added dropwise to a solution of 3-[(3R,5S)-3-[(2Z)-2-amino-2-(hydroxyimino)ethyl]-7-chloro-5-(2,3-dimethoxyphenyl)-2-oxo-2,3-dihydro-4,1-benzoxazepin-1(5H)-yl]-2,2-dimethylpropyl acetate (1.0 g, 1.87 mmol) obtained in Example 58-(1) in THF (10 ml) under ice-cooling and the mixture was stirred at room temperature for 24 hours. After the reaction solution was concentrated under reduced pressure, water (10 ml) was added and the mixture was heated to reflux for 22 hours. The reaction solution was extracted with ethyl acetate, washed with water and an aqueous saturated sodium chloride solution, and dried over magnesium sulfate. After concentration under reduced pressure, the residue was dissolved in ethanol (10 ml) and a 2N aqueous sodium hydroxide solution (3.74 ml, 7.48 mmol) was added thereto. The mixture was then stirred for 2 hours. The reaction solutions was diluted with water and then extracted with diethyl ether. The extract was acidified with 6N hydrochloric acid, extracted with ethyl acetate, washed with an aqueous saturated sodium chloride solution, dried over magnesium sulfate, and then concentrated under reduced pressure. After the residue was dissolved in ethanol, active carbon was added thereto and the mixture was stirred for 10 minutes. Insoluble substances were filtered off and the filtrate was concentrated under reduced pressure to obtain 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)propionic acid (0.66 g).

¹H-NMR (CDCl₃) δ: 0.65 (3H, s), 1.04 (3H, s), 2.89 (2H, d, J = 7.2 Hz), 3.12-3.27 (4H, m), 3.34-3.43 (2H, m), 3.58-3.67 (4H, m), 3.89 (3H, s), 4.39-4.52 (2H, m), 6.19 (1H, s), 6.60 (1H, d, J = 1.5 Hz), 6.96-7.40 (5H, m).

### Example 61

### 2-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)acetic acid

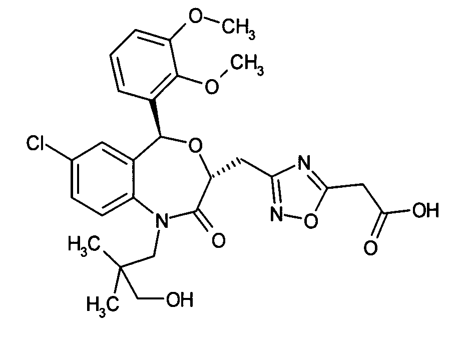

According to a similar manner to that of Example 60, the title compound was obtained.

¹H-NMR (CDCl₃) δ: 0. 65 (3H, s), 1. 04 (3H, s), 3. 17-3.44 (4H, m), 3.60-3.67 (4H, m), 3.88 (3H, s), 3.99 (2H, s), 4.43-4.50 (2H, m), 6.18 (1H, s), 6.60 (1H, d, J = 1.5 Hz), 6.96-7.41 (4H, m).

### Example 62

### 3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-1,2,4-triazol-5-carboxylic acid

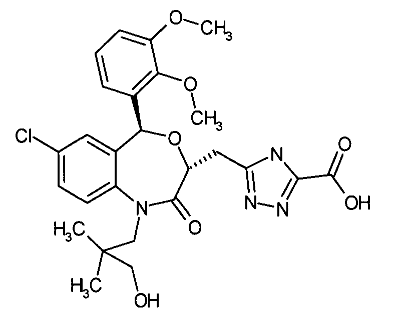

Iodomethane (0.2 ml) was added to a solution of 3-[(3R,5S)-3-(2-amino-2-thioxoethyl)-7-chloro-5-(2,3-dimethoxyphenyl)-2-oxo-2,3-dihydro-4,1-benzoxazepin-1(5H)-yl]-2,2-dimethylpropyl acetate (2 g) obtained in Example 16-(2) in acetone (20 ml) and the mixture was stirred overnight. After concentration under reduced pressure, butanol (20 ml) and ethyl hydrazino(oxo)acetate (0.317 g) were added to the residue and the mixture was refluxed for 2 hours. After concentration under reduced pressure, ethanol (20 ml) and a 1N aqueous sodium hydroxide solution (4 ml) were added to the residue and the mixture was stirred overnight. The reaction solution was neutralized with 1N hydrochloric acid and extracted with ethyl acetate. This extract was washed with an aqueous saturated sodium chloride solution, dried over magnesium sulfate, and then concentrated under reduced pressure. The residue was purified by silica gel column chromatography to obtain the title compound (0.32 g).

¹H-NMR (CDCl₃) δ: 0.76 (3H, s), 0.86 (3H, s), 3.05-3.22 (3H, m), 3.51 (3H, s), 3.67 (1H, d, J = 14.1 Hz), 3.83 (3H, s), 4.25-4.42 (2H, m), 4.56 (1H, s), 6.11 (1H, s), 6.35 (1H, d, J = 2.5Hz), 6.78 (1H, s), 7.10-7.18 (2H, m), 7.55 (1H, dd, J = 2.5, 8.9 Hz), 7.72 (1H, d, J = 8.9 Hz).

### Example 63

### (3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-1,2,4-triazol-5-yl)acetic acid

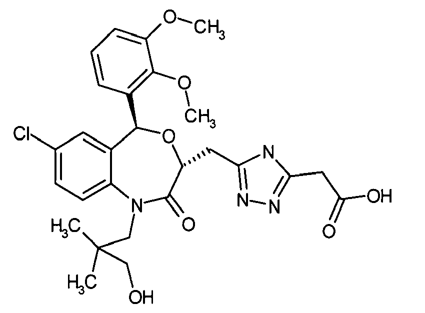

According to a similar manner to that of Example 62, the title compound was obtained.

¹H-NMR (CDCl₃) δ: 0.75 (3H, s), 0.86 (3H, s), 3.04-3.19 (4H, m), 3.50 (3H, s), 3.65-3.72 (3H, m), 3.83 (3H, s), 4.28-4.40 (2H, m), 6.10 (1H, s), 6.34 (1H, d, J = 2.4Hz), 6.86 (1H, dd, J = 2.3, 7.0 Hz), 7.09-7.20 (1H, m), 7.55 (1H, dd, J = 2.4, 8.9 Hz), 7.71 (1H, d, J = 8.9 Hz).

### Example 64

### 3-[3-({(3R,5S)-1-[3-(acetyloxy)-2,2-dimethylpropyl]-7-chloro-5-(2,3-dimethoxyphenyl)-2-oxo-1,2,3-5-tetrahydro-4,1-benzoxazepin-3-yl}methyl)-5-hydroxy-1H-pyrazol-1-yl]benzoic acid

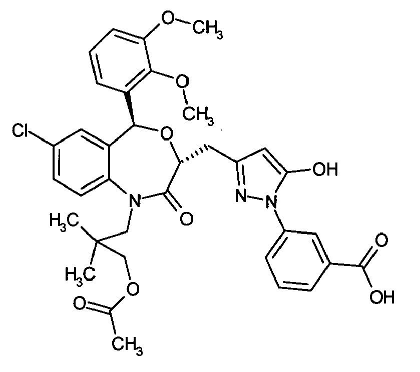

To a solution of ethyl 4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]}-3-oxobutanoate (2 g) obtained in Example 26-(1) in ethanol (20 ml), 3-hydrazinobenzoic acid (0.567 g) was added and the mixture was refluxed overnight. After concentration under reduced pressure, the residue was purified by silica gel column chromatography to obtain the title compound (1.9 g).

¹H-NMR (DMSO-d₆) δ: 0.92 (6H, s), 1.99 (3H, s), 2.79-3.07 (2H, m), 3.52 (3H, s), 3.61-3.80 (3H, m), 3.85 (3H, s), 4.19 (1H, t, J = 6.5 Hz), 4.36 (1H, d, J = 14.3 Hz), 5.42 (1H, s), 6.13 (1H, s), 6.39 (1H, d, J = 2.4 Hz), 7.10-7.24 (3H, m), 7.48-7.59 (2H, m), 7.75 (2H, t, J = 9.0 Hz), 7.95 (1H, d, J = 7.9 Hz), 8.28 (1H, s).

### Example 65

### 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-hydroxy-1H-pyrazol-1-yl)benzoic acid

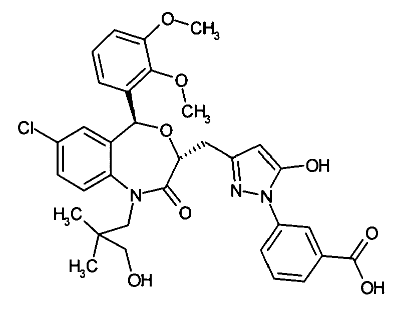

A 1N aqueous sodium hydroxide solution (3.4 ml) was added to a solution of 3-[3-({(3R,5S)-1-(3-acetoxy-2,2-dimethylpropyl)-7-chloro-5-(2,3-dimethoxyphenyl)-2-oxo-1,2,3-5-tetrahdyro-4,1-benzoxazepin-3-yl]methyl}-5-hydroxy-1H-pyrazol-1-yl)benzoic acid (0.9 g) obtained in Example 64 in ethanol (4.5 ml) and the mixture was stirred overnight. The reaction solution was neutralized with 1N hydrochloric acid and extracted with ethyl acetate. This extract was washed with an aqueous saturated sodium chloride solution, dried over magnesium sulfate, and then concentrated under reduced pressure. The residue was purified by silica gel column chromatography to obtain the title compound (0.586 g).

¹H-NMR (DMSO-d₆) δ: 0.78 (3H, s), 0.86 (3H, s), 2.78-3.24 (4H, m), 3.53 (3H, s), 3.69 (1H, d, J = 14.1 Hz), 3.85 (3H, s), 4.18 (1H, t, J = 6.5 Hz), 4.32 (1H, d, J = 14.1 Hz), 4.56 (1H, t, J = 4.9 Hz), 5.42 (1H, s), 6.12 (1H, s), 6.39 (1H, d, J = 2.4 Hz), 7.07-7.26 (3H, m), 7.47-7.60 (2H, m), 7.67-7.81 (2H, m), 7.95 (1H, d, J = 8.1 Hz), 8.29 (1H, s).

### Example 66

### 4-[3-({(3R,5S)-1-[3-(acetyloxy)-2,2-dimethylpropyl]-7-chloro-5-(2,3-dimethoxyphenyl)-2-oxo-1,2,3,5-tetrahydro-4,1-benzoxazepin-3-yl}methyl)-5-hydroxy-1H-pyrazol-1-yl]benzoic acid

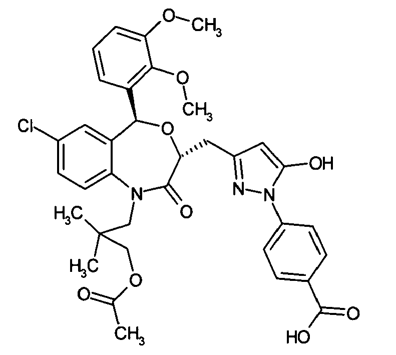

According to a similar manner to that of Example 64, the title compound was obtained.

¹H-NMR (DMSO-d₆) δ: 0.92 (3H, s), 0.93 (3H, s), 1.99 (3H, s), 2.80-3.05 (2H, m), 3.52 (3H, s), 3.61-3.81 (3H, m), 3.85 (3H, s), 4.17 (1H, t, J = 6.4 Hz), 4.36 (1H, d, J = 14.1Hz), 5.43 (1H, s), 6.13 (1H, s), 7.10-7.23 (3H, m), 7.54 (1H, dd, J = 2.2, 8.6 Hz), 7.75 (d, J = 8.8 Hz), 7.84 (2H, d, J = 8.8Hz), 7.99 (d, J = 8.6 Hz), 8.32 (1H, s).

### Example 67

### 4-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-hydroxy-1H-pyrazol-1-yl)benzoic acid

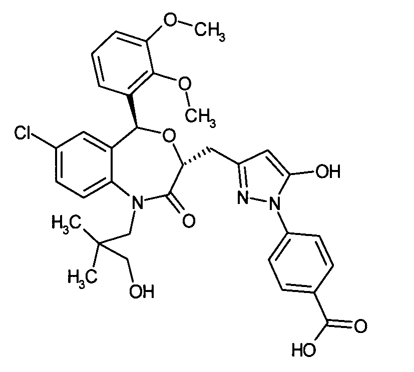

According to a similar manner to that of Example 65, the title compound was obtained.

¹H-NMR (DMSO-d₆) δ: 0.78 (3H, s), 0.87 (3H, s), 2.80-3.25 (4H, m), 3.53 (3H, s), 3.69 (1H, d, J = 14.1 Hz), 3.85 (3H, s), 4.16 (1H, t, J = 6.5 Hz), 4.32 (1H, d, J = 14.1 Hz), 4.56 (1H, t, J = 4.8 Hz), 5.43 (1H, s), 6.12 (1H, s), 6.39 (1H, d, J = 2.4 Hz), 7.09-7.24 (3H, m) 7.53 (1H, dd, J = 2.4, 8.6 Hz), 7.72 (1H, d, J = 8.8 Hz), 7.85 (2H, d, J = 8.8 Hz), 7.94-8.04 (2H, m).

### Example 68

### (3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-ethoxy-1H-pirazol-1-yl)acetic acid

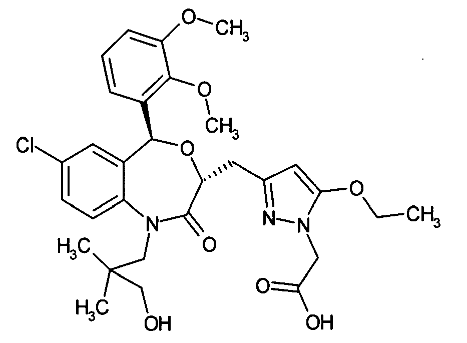

(1) Pyridine (0.3 ml) was added to a solution of ethyl 4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-3-oxobutanoate (2 g) obtained in Example 26-(1) and ethyl hydrazinoacetate hydrochloride (0.577 g) in ethanol (20 ml) and the mixture was refluxed for 3 hours. After concentration under reduced pressure, the residue was purified by silica gel column chromatography to obtain ethyl (3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-5-ethoxy-1H-pyrazol-1-yl)acetate (0.62 g) from a first eluted fraction and ethyl [3-({(3R,5S)-1-[3-(acetoxy)-2,2-dimethylpropyl]-7-chloro-5-(2,3-dimethoxyphenyl)-2-oxo-1,2,3,5-tetrahydro-4,1-benzoxazepin-3-yl}methyl)-5-hydroxy-1H-pyrazol-1-yl]acetate (1.2 g) from a second eluted fraction.
(2) A 1N aqueous sodium hydroxide solution (3.3 ml) was added to a solution of ethyl (3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-5-ethoxy-1H-pyrazol-1-yl)acetate (0.45 g) obtained in Example 68-(1) in ethanol (4.5 ml) and the mixture was stirred overnight. The reaction solution was neutralized with 1N hydrochloric acid and extracted with chloroform. This extract was washed with an aqueous saturated sodium chloride solution, dried over magnesium sulfate, and then concentrated under reduced pressure. The residue was purified by silica gel column chromatography to obtain the title compound (0.38 g).
   ¹H-NMR (CDCl₃) δ: 0.63 (3H, s), 1.04 (3H, s), 1.40 (3H, t, J = 7.0 Hz), 3.02 (1H, dd, J = 6.3, 14.8 Hz), 3.11-3.26 (2H, m), 3.37 (1H, d, J = 14.3 Hz), 3.57-3.70 (4H, m), 3.89 (3H, s), 4.05-4.23 (3H, m), 4.45 (1H, d, J = 14.3 Hz), 4.63 (2H, s), 5.49 (1H, s), 6.17 (1H, s), 6.58 (1H, d, J = 1.8 Hz), 6.98 (1H, dd, J = 2.4, 7.1 Hz), 7.11-7.23 (2H, m), 7.25-7.39 (3H, m).

### Example 69

### (3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-5-hydroxy-1H-pyrazol-1-yl)acetic acid

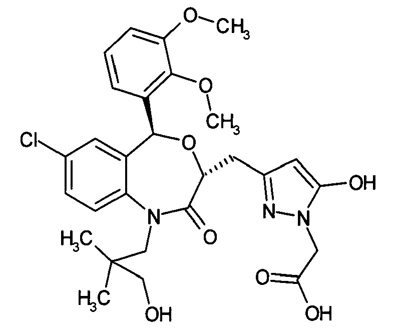

A 1N aqueous sodium hydroxide solution (0.5 ml) was added to a solution of ethyl [3-({(3R,5S)-1-[3-(acetyloxy)-2,2-dimethylpropyl]-7-chloro-5-(2,3-dimethoxyphenyl)-2-oxo-1,2,3,5-tetrahydro-4,1-benzoxazepin-3-yl}methyl)-5-hydroxy-1H-pyrazol-1-yl]acetate (0.1 g) obtained in Example 68-(1) in ethanol (1 ml) and the mixture was stirred overnight. The reaction solution was neutralized with 1N hydrochloric acid and extracted with chloroform-methanol. This extract was washed with an aqueous saturated sodium chloride solution, dried over magnesium sulfate, and then concentrated under reduced pressure. The residue was purified by silica gel column chromatography to obtain the title compound (0.042 g).

¹H-NMR (CDCl₃) δ: 0.64 (3H, s), 1.03 (3H, s), 2.84-3.05 (2H, m), 3.11-3.23 (2H, m), 3.32-3.44 (2H, m), 3.62 (3H, s), 3.89 (3H, s), 4.26 (1H, t, J = 6.5 Hz), 4.34-4.49 (2H, m), 4.53-4.63 (1H, m), 6.16 (1H, s), 6.62 (1H, d, J = 2.0 Hz), 6.96-7.04 (1H, m), 7.08-7.25 (3H, m), 7.30-7.43 (3H, m).

### Example 70

### [5-(carboxymethoxy)-3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1H-pyrazol-1-yl]acetic acid

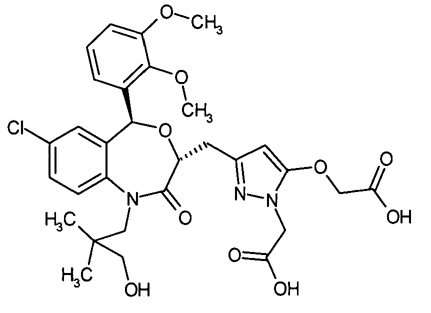

Methyl bromoacetate (0.459 g) and potassium carbonate (0.441 g) were added to a solution of ethyl [3-({(3R,5S)-1-[3-(acetoxy)-2,2-dimethylpropyl]-7-chloro-5-(2,3-dimethoxyphenyl)-2-oxo-1,2,3,5-tetrahydro-4,1-benzoxazepin-3-yl}methyl)-5-hydroxy-1H-pyrazol-1-yl]acetate (0.5 g) obtained in Example 68-(1) in acetonitrile (5 ml) and the mixture was stirred overnight. The reaction solution was diluted with ethyl acetate, washed with water and an aqueous saturated sodium chloride solution, dried over magnesium sulfate, and then concentrated under reduced pressure. To this residue were added ethanol (5 ml) and a 1N aqueous sodium hydroxide solution (2.78 ml) and the mixture was stirred overnight. The reaction solution was neutralized with 1N hydrochloric acid and extracted with chloroform. This extract was washed with an aqueous saturated sodium chloride solution, dried over magnesium sulfate, and then concentrated under reduced pressure. The residue was purified by silica gel column chromatography to obtain the title compound (0.116 g).

¹H-NMR (DMSO-d₆) δ: 0.76 (3H, s), 0.85 (3H, s), 2.83 (2H, m), 3.12 (2H, m), 3.51 (3H, s), 3.65 (1H, m), 3.84 (3H, s), 4.01 (1H, m), 4.32 (1H, d, J = 14.1 Hz), 4.58 (2H, s), 4.66 (2H, s), 5.47 (1H, s), 6.09 (1H, s), 6.35 (1H, d, J = 2.4 Hz), 7.12 (3H, m), 7.51 (1H, dd, J = 2.5, 8.8 Hz), 7.67 (1H, d, J = 8.8 Hz).

### Example 71

### (2E)-3-(4-([(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acrylic acid

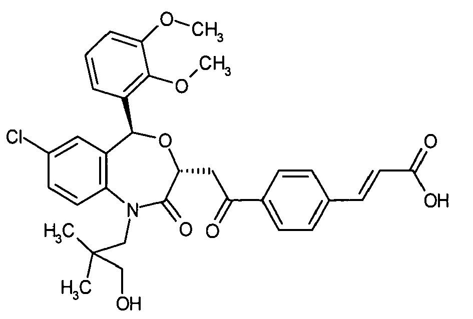

(1) Oxalyl chloride (10 ml) was added to a solution of (2E)-4-(bromophenyl)-4-oxo-2-butenoic acid (24.5 g) in a mixture of THF (200 ml) and DMF (3 drops) and the mixture was stirred at room temperature for 1 hour. The reaction solution was concentrated under reduced pressure and the residue was dissolved in ethyl acetate (120 ml). The resulting solution was added dropwise to a mixed solvent of a solution of 3-({4-chloro-2-[(S)-(2,3-dimethoxyphenyl)(hydroxyl)methyl]phenyl}amino)-2,2-dimethyl-1-propanol (24.5 g) in ethyl acetate (300 ml) and a 1N aqueous sodium hydroxide solution (130 ml) under ice-cooling, and the mixture was stirred at 0°C for 1 hour. The organic layer was washed successively with an aqueous saturated sodium hydrogen carbonate solution and an aqueous saturated sodium chloride solution, dried over sodium sulfate, and then concentrated under reduced pressure. The resulting residue was dissolved in ethanol (240 ml), DBU (11 ml) was added, and the mixture was stirred at room temperature overnight. Water (60 ml) was added to the reaction solution gradually and the mixture was stirred for 1 hour. Precipitated crystals were filtered and washed with a mixed solution of ethanol/water = 2/1 to obtain (3R,5S)-3-[2-(4-bromophenyl)-2-oxoethyl]-7-chloro-5-(2,3-dimethoxyphenyl)-1-(3-hydroxy-2,2-dimethylpropyl)-1,5-dihydro-4,1-benzoxazepin-2(3H)-one (24.5 g).
   ¹H-NMR (CDCl₃) δ: 0.65 (3H, s), 1.05 (3H, s), 3.14 (1H, d, J = 12.0 Hz), 3.32 (1H, dd, J = 4.5 Hz, 17.4 Hz), 3.42 (1H, d, J = 14.4 Hz), 3.62 (3H, s), 3.64 (1H, m), 3.81(1H, m), 3.90 (3H, s), 4.48 (1H, d, J = 12.0 Hz), 4.61 (1H, q, J = 4.2 Hz), 6.19 (1H, s), 6.63 (1H, s), 6.99 (1H, dd, J = 2.4 Hz, 7.5 Hz), 7.15 (2H, m), 7.41 (2H, m), 7.61 (2H, d, J = 8.4 Hz), 7.84 (2H, d, J = 8.4 Hz).
(2) Palladium acetate (338 mg), triphenylphosphine (788 mg), ethyl acrylate (2.2 ml) and triethylamine (3.3 ml) were added to a solution of (3R,5S)-3-[2-(4-bromophenyl)-2-oxoethyl]-7-chloro-5-(2,3-dimethoxyphenyl)-1-(3-hydroxy-2,2-dimethylpropyl)-1,5-dihydro-4,1-benzoxazepin-2(3H)-one (6.0 g) obtained in Example 71-(1) in DMF (60 ml) and the mixture was heated at 100°C for 20 hours under nitrogen atmosphere. 1N hydrochloric acid was added to the reaction solution, followed by extraction with ethyl acetate. The extract was washed with water, dried over magnesium sulfate, and then concentrated under reduced pressure. The residue was purified by silica gel column chromatography to obtain ethyl (2E)-3-(4-{[(3R,5S)-7-chloro-5-(2,3-dimethoxyphenyl)-1-(3-hydroxy-2,2,-dimethylpropyl)-2-oxo-1,2,3,5-tetrahydro-4,1-benzoxazepin-3-yl]acetyl}phenyl)acrylate (3.1 g).
   ¹H-NMR (CDCl₃) δ: 0.65 (3H, s), 1.05 (3H, s), 1.35 (3H, t, J = 7.2 Hz), 3.14 (1H, m), 3.36 (1H, m), 3.43 (1H, m), 3.63 (3H, s), 3.64 (1H, m), 3.81(1H, m), 3.90 (3H, s), 4.48 (1H, d, J = 13.2 Hz), 4.61 (1H, q, J = 3.6 Hz), 6.20 (1H, s), 6.53 (1H, d, J = 16.5 Hz), 6.64 (1H, m), 6.99 (1H, m), 7.16 (2H, m), 7.41 (2H, m), 7.60 (2H, d, J = 8.4 Hz), 7.68 (1H, d, J = 16.5 Hz), 7.98 (2H, d, J = 8.4 Hz).
(3) A 2N aqueous sodium hydroxide solution (25 ml) was added to a solution of ethyl (2E)-3-(4-{[(3R,5S)-7-chloro-5-(2,3-dimethoxyphenyl)-1-(3-hydroxy-2,2,-dimethylpropyl)-2-oxo-1,2,3,5-tetrahydro-4,1-benzoxazepin-3-yl]acetyl}phenyl)acrylate (3.1 g) obtained in Example 71-(2) in a mixture of THF (100 ml) and ethanol (100 ml), and the mixture was stirred at room temperature for 10 hours. After the reaction solution was concentrated under reduced pressure, the residue was dissolved in water and washed with ether. The aqueous layer was acidified with 1N hydrochloric acid and then extracted with ethyl acetate. The organic layer was washed with water, dried over magnesium sulfate, and then concentrated under reduced pressure to obtain (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)-acrylic acid (1.1 g).
   ¹H-NMR (CDCl₃) δ: 0.66 (3H, s), 1.05 (3H, s), 3.15 (1H, d, J = 12.0 Hz), 3.36 (1H, dd, J = 3.9 Hz, 17.1 Hz), 3.43 (1H, d, J = 15.0 Hz), 3.62 (3H, s), 3.66 (1H, m), 3.82 (1H, t, J = 9.3 Hz), 3.89 (3H, s), 4.48 (1H, d, J = 14.4 Hz), 4.62 (1H, q, J = 3.9 Hz), 6.19 (1H, s), 6.52 (1H, d, J = 15.9 Hz), 6.63 (1H, s), 6.98 (1H,m), 7.15 (2H, m), 7.40 (2H, m), 7.62 (2H, d, J = 8.4 Hz), 7.77 (1H, d, J = 15.9 Hz), 7.99 (2H, d, J = 8.4 Hz).

### Example 72

### 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid

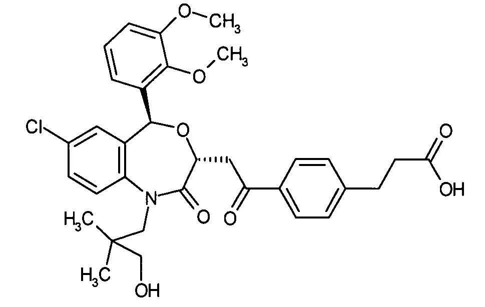

(1) Raney nickel was added to a solution of ethyl (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acrylate (6.2 g) obtained in Example 71-(2) in a mixture of THF (100 ml) and ethanol (50 ml), and the mixture was stirred at room temperature overnight under hydrogen atmosphere. The catalyst was filtered off with Celite and the filtrate was concentrated under reduced pressure to obtain ethyl 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionate (6.6 g) as a crude crystal, which was used in the next step without purification.
   ¹H-NMR (CDCl₃) δ: 0.65 (3H, s), 1.04 (3H, s), 1.24 (3H, t, J = 7.4 Hz), 2.63 (2H, t, J = 8.4 Hz), 3.00 (2H, t, J = 8.4 Hz), 3.13 (1H, m), 3.33 (1H, m), 3.42 (1H, d, J = 14.6 Hz), 3.62 (3H, s), 3.75 (2H, m), 3.89 (3H, s), 4.21 (1H, m), 4.48 (1H, d, J = 14.6 Hz), 4.62 (1H, q, J = 4.2 Hz), 6.19 (1H, s), 6.63 (1H, m), 6.98 (1H, t, J = 5.0 Hz), 7.18 (2H, m), 7.29 (2H, d, J = 8.0 Hz), 7.40 (2H, m), 7.90 (2H, d, J = 8.0 Hz).
(2) A 2N aqueous sodium hydroxide solution (15 ml) was added to a solution of ethyl 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionate (3.5 g) obtained in Example 72-(1) in a mixture of THF (50 ml) and ethanol (50 ml), and the mixture was stirred at room temperature for 2 hours. The reaction solution was concentrated under reduced pressure and the residue was dissolved in water and then washed with ether. The aqueous layer was acidified with 1N hydrochloric acid and then extracted with ethyl acetate. The organic layer was washed with water, dried over magnesium sulfate, and then concentrated under reduced pressure to obtain 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid(2.2 g).
   ¹H-NMR (CDCl₃) δ: 0.65 (3H, s), 1.04 (3H, s), 2.70 (2H, t, J = 7.8 Hz), 3.01 (2H, t, J = 7.8 Hz), 3.15 (1H, d, J = 12.0 Hz), 3.33 (1H, dd, J = 4.5 Hz, 17.4 Hz), 3.42 (1H, d, J = 17.4 Hz), 3.62 (3H, s), 3.66 (1H, m), 3.82 (1H, t, J = 8.7 Hz), 3.89 (3H, s), 4.48 (1H, d, J = 14.4 Hz), 4.62 (1H, q, J = 4.2 Hz), 6.19 (1H, s), 6.63 (1H, m), 6.99 (1H, t, J = 5.1 Hz), 7.18 (2H, d, J = 5.1 Hz), 7.30 (2H, d, J = 8.1 Hz), 7.40 (2H, m), 7.91 (2H, d, J = 8.1 Hz).

### Example 73

### (2E)-3-(4-{[(3R,5S)-7-chloro-5-(2,3-dimethoxyphenyl)-1-neopentyl-2-oxo-1,2,3,5-tetrahydro-4,1-benzoxazepin-3-yl]acetyl}phenyl)acrylic acid

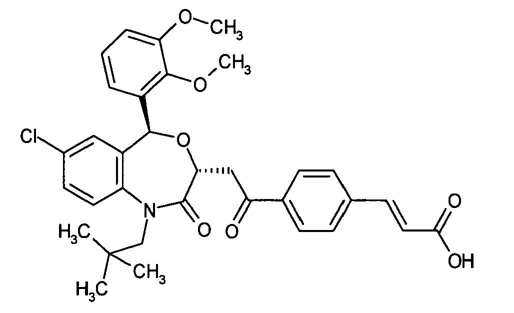

According to a similar manner to that of Example 71, the title compound was obtained.

¹H-NMR (CDCl₃) δ: 0.95 (9H, s), 3.31 (1H, dd, J = 17.1, 4.8 Hz), 3.41 (1H, d, J = 14.1 Hz), 3.63 (3H, s), 3.80 (1H, t, J = 9.0 Hz), 3.90 (3H, s), 4.51 (1H, d, J = 14.1 Hz), 4.67 (1H, dd, J = 8.4, 4.8 Hz), 6.31 (1H, s), 6.52 (1H, d, J = 16.2 Hz), 6.63 (1H, m), 6.98 (1H, t, J = 4.8 Hz), 7.16 (2H, d, J = 4.5 Hz), 7.37 (2H, m), 7.61 (2H, d, J = 8.1 Hz), 7.76 (1H, d, J = 16.2 Hz), 8.01 (2H, d, J = 8.1 Hz).

### Example 74

### (2E)-3-(3-{[(3R,5S)-7-chloro-5-(2,3-dimethoxyphenyl)-1-neopentyl-2-oxo-1,2,3,5-tetrahydro-4,1-benzoxazepin-3-yl]acetyl}phenyl)acrylic acid

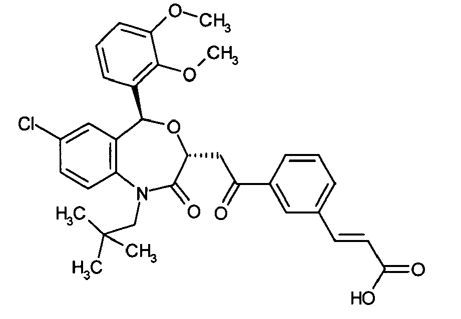

According to a similar manner to that of Example 71, the title compound was obtained.

¹H-NMR (CDCl₃) δ: 0.96 (9H, s), 3.37 (1H, m), 3.42 (1H, d, J = 14.1 Hz), 3.64 (3H, s), 3.82 (1H, t, J = 8.1 Hz), 3.89 (3H, s), 4.52 (1H, d, J = 14.1 Hz), 4.67 (1H, dd, J = 7.8, 4.5 Hz), 6.32 (1H, s), 6.50 (1H, d, J = 16.2 Hz), 6.64 (1H, m), 6.98 (1H, m), 7.13 (2H, m), 7.36 (2H, m), 7.49 (1H, t, J = 7.5 Hz), 7.72 (1H, d, J = 7.8 Hz), 7.79 (1H, d, J = 16.2 Hz), 8.01 (1H, d, J = 7.5 Hz), 8.17 (1H, s).

### Example 75

### (2E)-3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acrylic acid

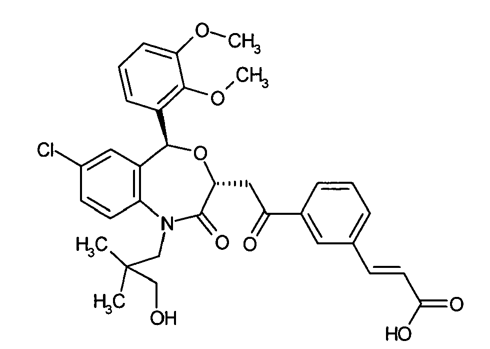

According to a similar manner to that of Example 71, the title compound was obtained.

¹H-NMR (CDCl₃) δ: 0.66 (3H, s), 1.05 (3H, s), 3.16 (1H, d, J = 12.0 Hz), 3.39 (1H, m), 3.44 (1H, d, J = 14.4 Hz), 3.63 (3H, s), 3.66 (1H, m), 3.82(1H, m), 3.90 (3H, s), 4.49 (1H, d, J = 14.4 Hz), 4.64 (1H, q, J = 4.5 Hz), 6.20 (1H, s), 6.52 (1H, d, J = 15.9 Hz), 6.65 (1H, s), 7.00 (1H,m), 7.19 (2H, m), 7.42 (2H, m), 7.51 (1H, t, J = 7.5 Hz), 7.76 (1H, m), 7.79 (1H, d, J = 15.9 Hz), 8.01 (1H, d, J = 7.5 Hz), 8.14 (1H, s).

### Example 76

### 3-(4-{[(3R,5S)-7-chloro-5-(2,3-dimethoxyphenyl)-1-neopentyl-2-oxo-1,2,3,5-tetrahydro-4,1-benzoxazepin-3-yl]acetyl}phenyl)propionic acid

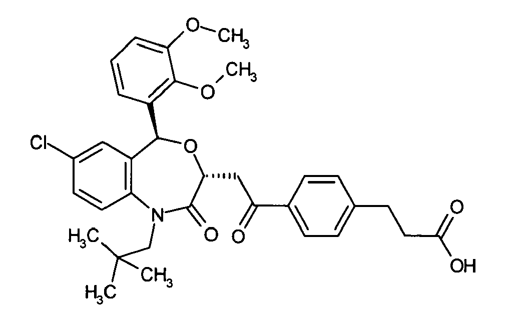

According to a similar manner to that of Example 72, the title compound was obtained.

¹H-NMR (CDCl₃) δ: 0.94 (9H, s), 2.67 (2H, t, J = 7.5 Hz), 2.99 (2H, t, J = 7.5 Hz), 3.31 (1H, dd, J = 17.4 Hz, 4.5 Hz), 3.40 (1H, d, J = 13.8 Hz), 3.81 (1H, m), 3.89 (3H, s), 4.50 (1H, d, J = 13.8 Hz), 4.65 (1H, q, J = 4.8 Hz), 6.31 (1H, s), 6.63 (1H, m), 6.97 (1H, m), 7.15 (2H, m), 7.28 (2H, d, J = 8.1 Hz), 7.37 (2H, m), 7.92 (2H, d, J = 8.1 Hz).

### Example 77

### 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid

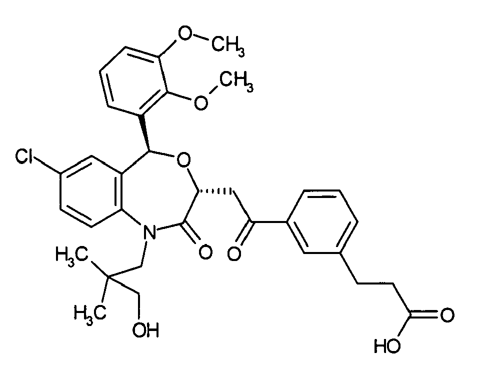

According to a similar manner to that of Example 72, the title compound was obtained.

¹H-NMR (CDCl₃) δ: 0. 66 (3H, s), 1.03 (3H, s), 2. 65 (2H, t, J = 7.5 Hz), 2.98 (2H, t, J = 7.5 Hz), 3.19 (1H, d, J = 12.0 Hz), 3.37 (1H, dd, J = 4.2 Hz, 16.8 Hz), 3.44 (1H, d, J = 17.4 Hz), 3.62 (3H, s), 3.66 (1H, m), 3.86 (1H, t, J = 9.6 Hz), 3.88 (3H, s), 4.47 (1H, d, J = 12.0 Hz), 4.63 (1H, q, J = 4.5 Hz), 6.19 (1H, s), 6.64 (1H, m), 6.98 (1H, m), 7.18 (2H, m), 7.41 (4H, m), 7.78 (2H, m).

### Example 78

### 3-(3-{[(3R,5S)-7-chloro-5-(2,3-dimethoxyphenyl)-1-neopentyl-2-oxo-1,2,3,5-tetrahydro-4,1-benzoxazepin-3-yl]acetyl}phenyl)propionic acid

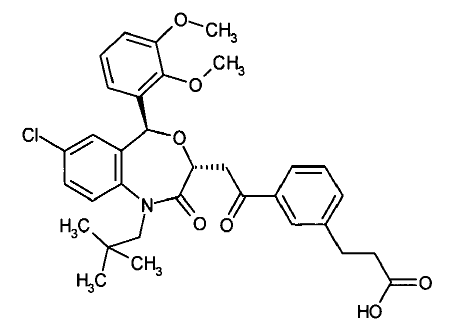

According to a similar manner to that of Example 72, the title compound was obtained.

¹H-NMR (CDCl₃) δ: 0.95 (9H, s), 2.66 (2H, t, J = 7.5 Hz), 2.98 (2H, t, J = 7.5 Hz), 3.34 (1H, dd, J = 17.1 Hz, 4.8 Hz), 3.40 (1H, d, J = 14.1 Hz), 3.63 (3H, s), 3.65 (1H, m), 3.78 (1H, t, J = 7.8 Hz), 3.88 (3H, s), 4.52 (1H, d, J = 14.1 Hz), 4.65 (1H, q, J = 3.9 Hz), 6.31 (1H, s), 6.63 (1H, m), 6.98 (1H, m), 7.17 (2H, m), 7.38 (4H, m), 7.83 (2H, m).

### Example 79

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid

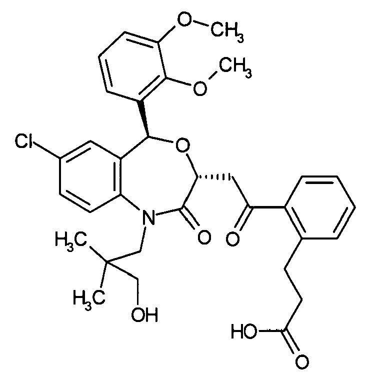

According to a similar manner to that of Example 72, the title compound was obtained.

¹H-NMR (CDCl₃) δ: 0.66 (3H, s), 1.04 (3H, s), 2.66 (2H, m), 3.08 (2H, m), 3.20 (1H, d, J = 12.0 Hz), 3.31 (1H, dd, J = 4.5 Hz, 17.7 Hz), 3.45 (1H, d, J = 17.4 Hz), 3.61 (3H, s), 3.63 (1H, m), 3.77 (1H, m), 3.87 (3H, s), 4.47 (1H, d, J = 12.0 Hz), 4.63 (1H, q, J = 4.2 Hz), 6.18 (1H, s), 6.65 (1H, m), 6.98 (1H, m), 7.18 (2H, m), 7.29 (2H, m), 7.38 (3H, m), 7.80 (1H, d, J = 8.1 Hz).

### Example 80

### 4-(4-{[(3R,5S)-7-chloro-5-(2,3-dimethoxyphenyl)-1-(3-hydroxy-2,2-dimethylpropyl)-2-oxo-1,2,3,5-tetrahdyro-4,1-benzoxazepin-3-yl]acetyl}phenyl)butanoic acid

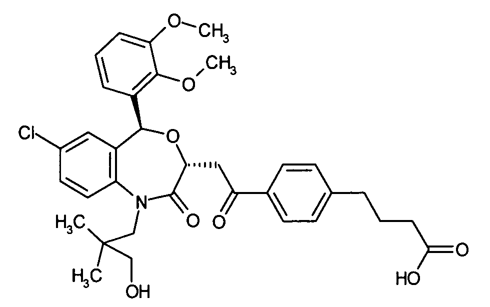

According to a similar manner to that of Example 72, the title compound was obtained.

¹H-NMR (CDCl₃) δ: 0.65 (3H, s), 1.04 (3H, s), 1.96 (2H, m), 2.35 (2H, t, J = 6.9 Hz), 2.71 (2H, t, J = 7.8 Hz), 3.16 (1H, d, J = 12.0 Hz), 3.34 (1H, dd, J = 4.5 Hz, 17.4 Hz), 3.42 (1H, d, J = 14.1 Hz), 3.61 (3H, s), 3.63 (1H, m), 3.81 (1H, t, J = 8.7 Hz), 3.88 (3H, s), 4.48 (1H, d, J = 14.1 Hz), 4.62 (1H, q, J = 3.9 Hz), 6.18 (1H, s), 6.62 (1H, m), 6.97 (1H, m), 7.17 (2H, m), 7.29 (2H, m), 7.39 (2H, m), 7.89 (2H, d, J = 8.1 Hz).

### Example 81

### 3-(4-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}phenyl)propionic acid

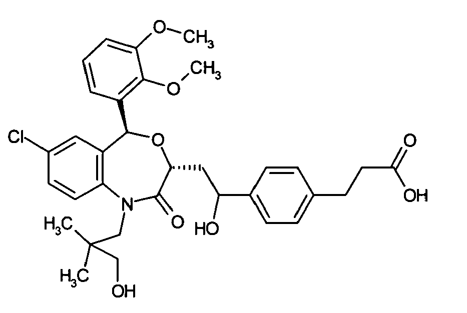

(1) Acetyl chloride (0.4 ml) was added to a solution of ethyl 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionate (3.0 g) obtained in Example 72-(1) in a mixture of THF (50 ml) and pyridine (0.8 ml), and the mixture was stirred at room temperature for 2 hours. Water was added to the reaction solution, followed by extraction with ethyl acetate. The resulting organic layer was washed successively with diluted hydrochloric acid, an aqueous saturated NaHCO₃ solution and water, dried over magnesium sulfate, and then concentrated under reduced pressure to obtain ethyl 3-(4-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}phenyl)propionate (2.7 g) as an oil.
   ¹H-NMR (CDCl₃) δ: 0.94 (3H, s), 1.02 (3H, s), 1.26 (3H, t, J = 7.2 Hz), 2.04 (3H, s), 2.62 (2H, t, J = 7.8 Hz), 2.99 (2H, t, J = 7.8 Hz), 3.30 (1H, dd, J = 4.8, 17.4 Hz), 3.60 (1H, m), 3.61 (3H, s), 3.76 (2H, m), 3.89 (3H, s), 4.11 (2H, q, J = 7.2 Hz), 4.56 (1H, d, J = 14.4 Hz), 4.62 (1H, q, J = 4.8 Hz), 6.28 (1H, s), 6.65 (1H, m), 6.97 (1H, m), 7.16 (2H, m), 7.26 (2H, m), 7.36 (2H, m), 7.89 (2H, d, J = 8.4 Hz).
(2) Sodium borohydride (40 mg) and methanol (2 ml) were successively added to a solution of ethyl 3-(4-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}phenyl)propionate (0.2 g) obtained in Example 81-(1) in THF (5 ml) and the mixture was stirred at room temperature for 30 minutes. After 1N hydrochloric acid was added, the reaction solution was extracted with ethyl acetate. The resulting organic layer was washed successively with diluted hydrochloric acid and water, dried over magnesium sulfate, and then concentrated under reduced pressure. After the resulting residue was dissolved in THF (5 ml) and ethanol (5 ml), a 2N aqueous sodium hydroxide solution (2.5 ml) was added thereto and the mixture was stirred at room temperature for 2 hours. The reaction solution was acidified with diluted hydrochloric acid and then extracted with ethyl acetate. The organic layer was washed with water, dried over magnesium sulfate, and then concentrated under reduced pressure to obtain 3-(4-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}phenyl)propionic acid (83 mg) as powder.
   ¹H-NMR (CDCl₃) δ: 0.64 (3H, s), 1.05 (3H, s), 2.27 (2H, m), 2.64 (2H, m), 2.93 (2H, m), 3.16 (1H, d, J = 12.0 Hz), 3.37 (1H, d, J = 14.1 Hz), 3.61 (2H, m), 3.63 (3H, s), 3.90 (3H, s), 4.14 (1H, m), 4.47 (1H, d, J = 14.1 Hz), 4.83 (1H, m), 6.18 (1H, s), 6.64 (1H, m), 7.00 (1H, m), 7.1-7.4 (8H, m).

### Example 82

### 3-(4-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}phenyl)propionic acid

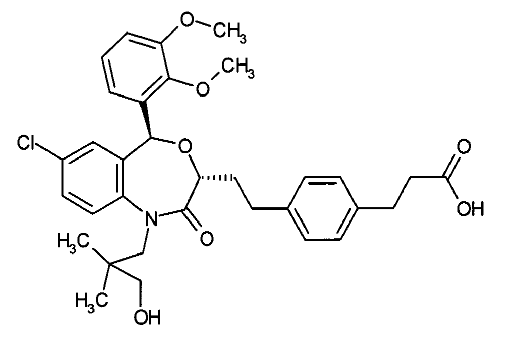

Thionyl chloride (0.2 ml) was added to a solution of ethyl 3-(4-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}phenyl)propionate (1.2 g) obtained in Example 81-(1) in a mixture of dichloromethane (30 ml) and pyridine (0.5 ml), and the mixture was stirred at room temperature for 1 hour. After diluted hydrochloric acid was added, the reaction solution was extracted with dichloromethane. The organic layer was washed successively with diluted hydrochloric acid, an aqueous saturated NaHCO₃ solution and water, dried over magnesium sulfate, and then concentrated under reduced pressure. The resulting residue was dissolved in acetic acid (10 ml), a zinc powder (2.5 g) was added thereto, and the mixture was stirred at room temperature for 2 hours. The reaction mixture was filtered with Celite and the filtrate was concentrated under reduced pressure. An aqueous saturated NaHCO₃ solution was added to the resulting residue and the mixture was extracted with ethyl acetate. The organic layer was washed with water, dried over magnesium sulfate, and then concentrated under reduced pressure. The resulting residue was purified by silica gel column chromatography. The resulting oil was dissolved in THF (10 ml) and ethanol (10 ml), a 2N aqueous NaOH solution (5.0 ml) was added thereto, and the mixture was stirred at room temperature for 2 hours. The reaction solution was acidified with diluted hydrochloric acid and then extracted with ethyl acetate. The organic layer was washed with water, dried over magnesium sulfate, and then concentrated under reduced pressure to obtain 3-(4-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}phenyl)propionic acid (115 mg) as powder.

¹H-NMR (CDCl₃) δ: 0.64 (3H, s), 1.05 (3H, s), 2.19 (2H, m), 2.63 (2H, m), 2.90 (2H, m), 3.15 (1H, d, J = 11.7 Hz), 3.37 (1H, d, J = 14.1 Hz), 3.62 (4H, m), 3.63 (3H, s), 3.81 (2H, m), 3.90 (3H, s), 4.45 (1H, d, J = 14.1 Hz), 6.18 (1H, s), 6.64 (1H, m), 7.00 (1H, m), 7.1-7.4 (8H, m).

### Example 83

### 4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}benzoic acid

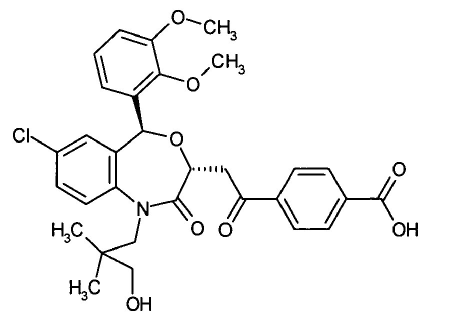

Palladium acetate (655 mg), 1,1'-bis(diphenylphosphino)ferrocene (1.6 g) and triethylamine (4.0 ml) were added to a solution of (3R,5S)-3-[2-(4-bromophenyl)-2-oxoethyl]-7-chloro-5-(2,3-dimethoxyphenyl)-1-(3-hydroxy-2,2-dimethylpropyl)-1,5-dihydro-4,1-benzoxazepin-2-(3H)-one (6.0 g) obtained in Example 71-(1) in a mixture of DMF (60 ml) and methanol (30 ml), and the mixture was heated at 60°C for 20 hours under carbon monoxide atmosphere. After 1N hydrochloric acid was added, the reaction solution was extracted with ethyl acetate. The extract was washed with water, dried over magnesium sulfate, and then concentrated under reduced pressure. The residue was purified by silica gel column chromatography and the resulting oil was dissolved in a mixture of THF (100 ml) and ethanol (100 ml). Thereto a 2N aqueous NaOH solution (25 ml) was added and the mixture was stirred at room temperature for 2 hours. The reaction solution was acidified with diluted hydrochloric acid and then extracted with ethyl acetate. The organic layer was washed with water, dried over magnesium sulfate, and then concentrated under reduced pressure to obtain 4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}benzoic acid (1.7 g).

¹H-NMR (CDCl₃) δ: 0.66 (3H, s), 1.05 (3H, s), 3.16 (1H, d, J = 11.7 Hz), 3.39 (1H, dd, J = 3.9 Hz, 17.7 Hz), 3.43 (1H, d, J = 13.8 Hz), 3.62 (3H, s), 3.65 (1H, m), 3.85 (1H, t, J = 7.8 Hz), 3.89 (3H, s), 4.49 (1H, d, J = 14.4 Hz), 4.63 (1H, q, J = 4.5 Hz), 6.19 (1H, s), 6.63 (1H, m), 6.98 (1H, m), 7.16 (2H, m), 7.41 (2H, m), 7.04 (2H, d, J=8.4 Hz), 8.17 (2H, d, J = 8.4 Hz).

### Example 84

### 3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}benzoic acid

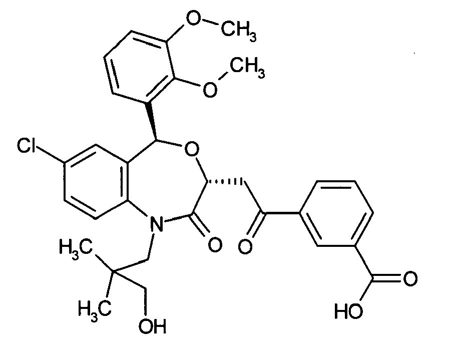

According to a similar manner to that of Example 83, the title compound was obtained.

¹H-NMR (CDCl₃) δ: 0.66 (3H, s), 1.06 (3H, s), 3.17 (1H, d, J = 11.7 Hz), 3.42 (1H, m), 3.63 (3H, s), 3.67 (1H, m), 3.90 (3H, s), 3.93 (1H, m), 4.50 (1H, d, J = 14.7 Hz), 4.66 (1H, q, J = 4.5 Hz), 6.21 (1H, s), 6.65 (1H, m), 7.00 (1H, m), 7.19 (2H, m), 7.42 (2H, m), 7.59 (1H, t, J = 7.8 Hz), 8.20 (1H, m), 8.30 (1H, m), 8.71 (1H, m).

### Example 85

### (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenoxy)acetic acid

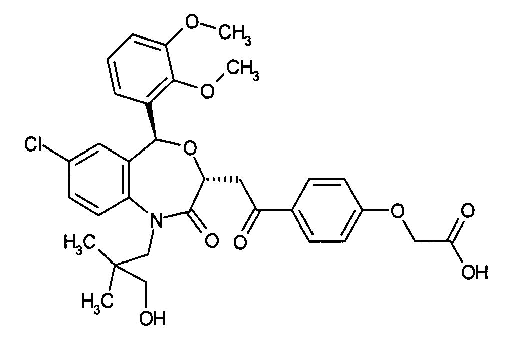

(1) According to a similar manner to that of Example 71-(1), (3R,5S)-7-chloro-5-(2,3-dimethoxyphenyl)-1-(3-hydroxy-2,2-dimethylpropyl)-3-[2-(4-hydroxyphenyl)-2-oxoethyl]-1,5-dihydro-4,1-benzoxazepin-2(3H)-one was synthesized using (2E)-4-(acetoxyphenyl)-4-oxo-2-butenoic acid as the starting material.
   ¹H-NMR (CDCl₃) δ: 0.66 (3H, s), 1.05 (3H, s), 3.17 (1H, m), 3.28 (1H, dd, J = 4.5 Hz, 16.8 Hz), 3.43 (1H, d, J = 14.1 Hz), 3.61 (3H, s), 3.65 (1H, m), 3.79 (1H, m), 3.89 (3H, s), 4.47 (1H, d, J = 14.1 Hz), 4.62 (1H, m), 6.18 (1H, s), 6.62 (1H, m), 6.84 (2H, d, J = 8.7 Hz), 6.97 (1H, t, J = 4.5 Hz), 7.17 (2H, m), 7.39 (2H, m), 7.87 (2H, d, J = 8.7 Hz) .
(2) K₂CO₃(0.5 g) and ethyl bromoacetate (0.22 ml) were added to a solution of (3R,5S)-7-chloro-5-(2,3-dimethoxyphenyl)-1-(3-hydroxy-2,2-dimethylpropyl)-3-[2-(4-hydroxyphenyl)-2-oxoethyl]-1,5-dihydro-4,1-benzoxazepin-2(3H)-one (1.0 g) obtained in Example 85-(1) in DMF (20 ml) and the mixture was stirred at room temperature for 2 hours. After 1N hydrochloric acid was added, the reaction solution was extracted with ethyl acetate. The extract was washed with water, dried over magnesium sulfate, and then concentrated under reduced pressure. After the resulting residue was dissolved in a mixture of THF (10 ml) and ethanol (10 ml), a 2N aqueous NaOH solution (5 ml) was added thereto and the mixture was stirred at room temperature for 2 hours. The reaction solution was acidified with diluted hydrochloric acid and then extracted with ethyl acetate. The organic layer was washed with water, dried over magnesium sulfate, and then concentrated under reduced pressure to obtain (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenoxy)acetic acid (0.82 g) as powder.
   ¹H-NMR (CDCl₃) δ: 0.65 (3H, s), 1.04 (3H, s), 3.20 (1H, d, J = 12.0 Hz), 3.31 (1H, dd, J = 3.9 Hz, 17.1 Hz), 3.42 (1H, d, J = 15.0 Hz), 3.62 (3H, s), 3.66 (1H, m), 3.81 (1H, m), 3.90 (3H, s), 4.46 (1H, d, J = 14.7 Hz), 4.61 (1H, m), 4.71 (2H, s), 6.17 (1H, s), 6.63 (1H, m), 6.94 (2H, d, J = 9.0 Hz), 7.00 (1H, m), 7.17 (2H, m), 7.40 (2H, m), 7.95 (2H, d, J = 9.0 Hz).

### Example 86

### (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetic acid

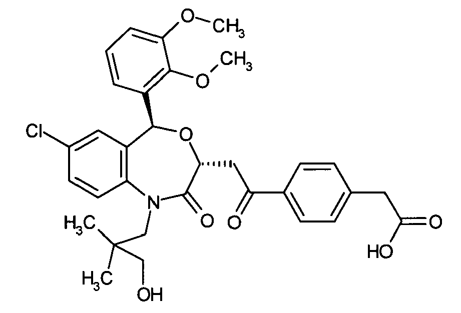

(1) According to a similar manner to that of Example 71-(1), ethyl (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetate was synthesized using (2E)-4-[4-(2-ethoxy-2-oxoethyl)phenyl]-4-oxo-2-butenoic acid as the starting material.
   ¹H-NMR (CDCl₃) δ: 0.65 (3H, s), 1.05 (3H, s), 1.25 (3H, t, J = 7.2 Hz), 3.14 (1H, m), 3.35 (1H, m), 3.43 (1H, d, J = 15.3 Hz), 3.65 (3H, s), 3.65 (3H, m), 3.81 (1H, m), 3.90 (3H, s), 4.15 (2H, q, J = 7.2 Hz), 4.49 (1H, d, J = 15.3 Hz), 4.63 (1H, m), 6.20 (1H, s), 6.64 (1H, m), 6.99 (1H, m), 7.18 (2H, m), 7.40 (4H, m), 7.90 (2H, m).
(2) A 2N aqueous NaOH solution (30 ml) was added to a solution of ethyl (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetate (7.1 g) obtained in Example 86-(1) in a mixture of THF (50 ml) and ethanol (100 ml), and the mixture was stirred at room temperature for 2 hours. The reaction solution was acidified with diluted hydrochloric acid and then extracted with ethyl acetate. The organic layer was washed with water, dried over magnesium sulfate, and then concentrated under reduced pressure to obtain (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetic acid (4.8 g).
   ¹H-NMR (CDCl₃) δ: 0.65 (3H, s), 1.04 (3H, s), 3.16 (1H, d, J = 11.7 Hz), 3.34 (1H, dd, J = 4.2 Hz, 17.4 Hz), 3.42 (1H, d, J = 14.1 Hz), 3.62 (3H, s), 3.65 (1H, m), 3.69 (2H, s), 3.81 (1H, m), 3.89 (3H, s), 4.47 (1H, d, J = 14.1 Hz), 4.62 (1H, m), 6.18 (1H, s), 6.64 (1H, m), 6.99 (1H, m), 7.17 (2H, m), 7.45 (4H, m), 7.89 (2H, m).

### Example 87

### 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-3-furancarboxylic acid

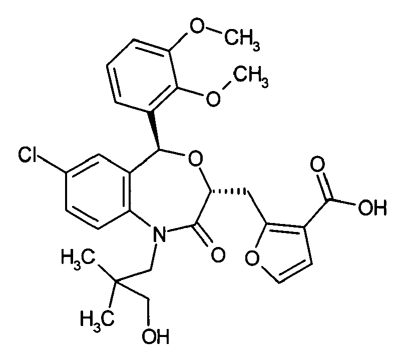

Ethyl 4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-3-oxobutanoate (1.2 g) was dissolved in toluene (5 ml), and DBU (0.3 ml) was then added thereto. After a 40% aqueous chloroacetoaldehyde solution (0.4 ml) was then added under ice-cooling, the mixture was stirred at room temperature for 1 hour. After water was added, the reaction solution was extracted with toluene. The resulting organic layer was concentrated under reduced pressure. The resulting residue was dissolved in toluene (20 ml), a catalytic amount of p-toluenesulfonic acid was added, and the mixture was heated at 100°C for 2 hours. The reaction solution was concentrated and then purified by silica gel column. The resulting residue was dissolved in a mixture of THF (5 ml) and methanol (20 ml), a 2N aqueous NaOH solution (5 ml) was added, and the mixture was stirred at room temperature overnight. The reaction solution was acidified with diluted hydrochloric acid and then extracted with ethyl acetate. The organic layer was washed with water, dried over magnesium sulfate, and then concentrated under reduced pressure to obtain 2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-3-furancarboxylic acid (26 mg).

¹H-NMR (CDCl₃) δ: 0.65 (3H, s), 1.06 (3H, s), 3.16 (1H, d, J = 11.7 Hz), 3.40 (1H, d, J = 14.7 Hz), 3.57 (3H, m), 3.60 (3H, s), 3.87 (3H, s), 4.29 (1H, m), 4.50 (1H, d, J = 14.7 Hz), 6.17 (1H, s), 6.53 (1H, s), 6.70 (1H, m), 6.99 (1H, d, J = 8.4 Hz), 6.95 (1H, d, J = 9.3 Hz), 7.12 (1H, t, J = 8.1 Hz), 7.31 (3H, m).

### Example 88

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-yl)propionic acid

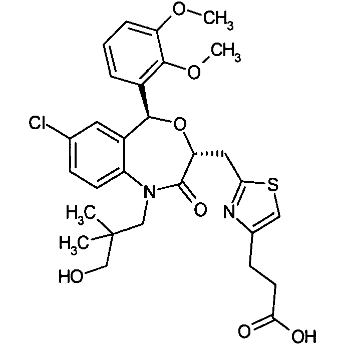

(1) 10%Pd carbon (47 mg) was added to a solution of ethyl (2E)-3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-4-yl)acrylate (0.47 g, 0.72 mmol) obtained as in Example 19-(1) in THF (5 ml) under nitrogen atmosphere, and hydrogen was then introduced. The mixture was stirred at room temperature for 48 hours and then at 40°C for 12 hours. The catalyst was filtered off using Celite and the filtrate was concentrated under reduced pressure. The residue was purified by silica gel column chromatography [developing solvent: hexane-ethyl acetate (73:27-65:35)] to obtain ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-4-yl)propionate (0.38 g, 0.58 mmol, 81%) as a colorless oil.
   ¹H-NMR (CDCl₃) δ: 0.95 (3H, s), 1.03 (3H, s), 1.21 (3H, t, J = 7.2 Hz), 2.03 (3H, s), 2.67 (2H, t, J = 7.5 Hz), 3.02 (2H, t, J = 7.5 Hz), 3.45 (1H, dd, J = 7.2, 15.0 Hz), 3.54 (1H, d, J = 14.4 Hz), 3.56 (1H, dd, J = 5.7, 15.0 Hz), 3.62 (3H, s), 3.73 (1H, d, J = 11.1 Hz), 3.85 (1H, d, J = 11.1 Hz), 3.89 (3H, s), 4.11 (2H, q, J = 7.2 Hz), 4.29 (1H, dd, J = 5.7, 7.2 Hz), 4.56 (1H, d, J = 14.4 Hz), 6.31 (1H, s), 6.60 (1H, d, J = 2.4 Hz), 6.82 (1H, s), 6.98 (1H, dd, J = 2.4, 7.2 Hz), 7.10-7.20 (2H, m), 7.28 (1H, d, J = 8.4 Hz), 7.33 (1H, dd, J = 2.4, 8.4 Hz).
(2) A mixture of ethyl 3-(2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-4-yl)propionate (0.35 g, 0.53 mmol) obtained in Example 88-(1), a 1N aqueous sodium hydroxide solution (3.0 ml) and ethanol (10 ml) was stirred at room temperature for 2 hours. This mixture was acidified with 1N aqueous hydrochloric acid (3.5 ml) and ethyl acetate (80 ml) was added thereto. The organic layer was washed with an aqueous saturated sodium chloride solution, dried over magnesium sulfate, and then concentrated under reduced pressure to obtain 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-yl)propionic acid (0.3 g, 0.51 mmol, 96%) as colorless noncrystalline powder.
   ¹H-NMR (CDCl₃) δ: 0.65 (3H, s), 1.05 (3H, s), 2.69-2.80 (2H, m), 3.02 (2H, t, J = 7.2 Hz), 3.16 (1H, d, J = 12.0 Hz), 3.41 (1H, d, J = 14.7 Hz), 3.48 (1H, dd, J = 6.6, 15.3 Hz), 3.59-3.70 (2H, m), 3.62 (3H, s), 3.90 (3H, s), 4.33 (1H, t, J = 6.6 Hz), 4.47 (1H, d, J = 14.7 Hz), 6.21 (1H, s), 6.59 (1H, d, J = 2.7 Hz), 6.89 (1H, s), 7.00 (1H, dd, J = 2.4, 7.8 Hz), 7.10-7.22 (2H, m), 7.38-8.01 (2H, m).

### Example 89

### [2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-(2-phenylethyl)-1,3-thiazol-5-yl]acetic acid

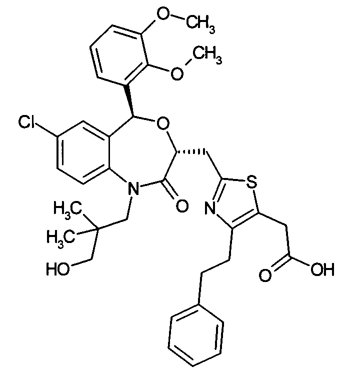

According to a similar manner to that of Example 1, the title compound was obtained.

¹H-NMR (CDCl₃) δ: 0.64 (3H, s), 1.05 (3H, s), 2.81-2.92 (4H, m), 3.17 (1H, d, J = 11.4 Hz), 3.36 (1H, d, J = 14.4 Hz), 3.40-3.70 (3H, m), 3.46 (2H, s), 3.61 (1H, d, J = 11.7 Hz), 3.87 (3H, s), 4.34 (1H, t, J = 6.9 Hz), 4.47 (1H, d, J = 14.4 Hz), 6.20 (1H, s), 6.58 (1H, d, J = 2.4 Hz), 6.90-7.06 (3H, m), 7.09-7.38 (7H, m).

### Example 90

### [2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-4-(2-phenylethyl)-1,3-oxazol-5-yl]acetic acid

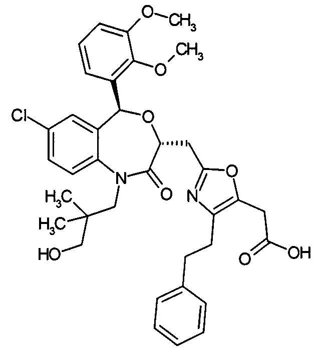

According to a similar manner to that of Example 27, the title compound was obtained.

¹H-NMR (CDCl₃) δ: 0.64 (3H, s), 1.04 (3H, s), 2.68 (2H, t, J = 7.5 Hz), 2.87 (2H, t, J = 7.5 Hz), 3.16 (1H, d, J = 12.0 Hz), 3.20-3.42 (3H, m), 3.36 (2H, s), 3.61 (3H, s), 3.62 (1H, d, J = 12.0 Hz), 3.87 (3H, s), 4.40-4.51 (2H, m), 6.18 (1H, s), 6.59 (1H, d, J = 2.1 Hz), 6.95 (1H, dd, J = 1.8, 8.1 Hz), 7.02-7.29 (8H, m), 7.37 (1H, dd, J = 2.1, 8.4 Hz).

### Example 91

### 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazole-4-carboxylic acid

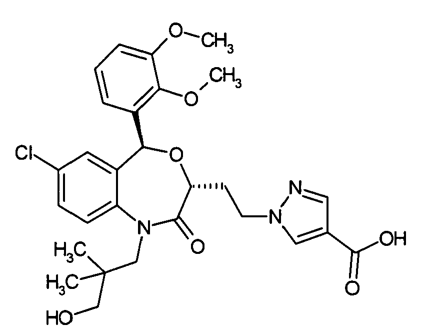

According to a similar manner to that of Example 50, the title compound was obtained.

¹H-NMR (CDCl₃) δ: 0. 64 (3H, s), 1.05 (3H, s), 2.29-2.50 (2H, m), 3.15 (1H, d, J = 12.3 Hz), 3.36 (1H, d, J = 13.8 Hz), 3.607 (1H, d, J = 12.3 Hz), 3.61 (3H, s), 3.78 (1H, t, J = 7.5 Hz), 3.89 (3H, s), 4.25-4.43 (2H, m), 4.45 (1H, d, J = 13.8 Hz), 6.15 (1H, s), 6.61 (1H, d, J = 2.4 Hz), 7.00 (1H, dd, J = 2.4, 7.2 Hz), 7.15-7.26 (2H, m), 7.28 (1H, d, J = 8.4 Hz), 7.34 (1H, dd, J = 2.4, 8.4 Hz), 7.85 (1H, s), 7.88 (1H, s).

### Example 92

### 3-(1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-5-yl)propionic acid

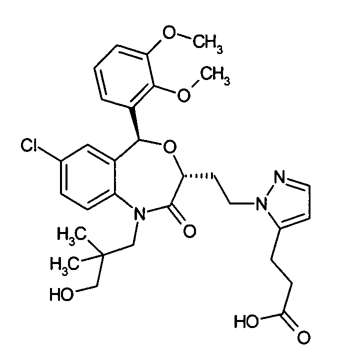

According to a similar manner to that of Example 88 using 1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-5-carboxylic acid obtained in Example 50, the title compound was obtained.

¹H-NMR (CDCl₃) δ: 0.63 (3H, s), 1.04 (3H, s), 2.20-2.43 (2H, m), 2.69 (2H, t, J = 8.1 Hz), 2.87-2.98 (2H, m), 3.15 (1H, d, J = 12.3 Hz), 3.35 (1H, d, J = 14.1 Hz), 3.605 (3H, s), 3.613 (1H, d, J = 12.3 Hz), 3.88 (1H, t, J = 6.3 Hz), 3.89 (3H, s), 4.20-4.30 (2H, m), 4.41 (1H, d, J = 14.1 Hz), 5.97 (1H, d, J = 1.8 Hz), 6.13 (1H, s), 6.61 (1H, d, J = 2.1 Hz), 6.99 (1H, dd, J = 2.1, 7.8 Hz), 7.17-7.40 (5H, m) .

### Example 93

### 3-(1-(2-[(3R,5S)-7-chloro-5-(2,3-dimethoxyphenyl)-1-(3-hydroxy-2,2-dimethylpropy)-2-oxo-1,2,3,5-tetrahydro-4,1-benzoxazepin-3-yl]ethyl}-1H-pyrazol-4-yl)propionic acid

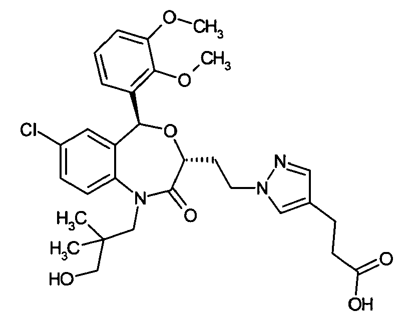

According to a similar manner to that of Example 92, the title compound was obtained.

¹H-NMR (CDCl₃) δ: 0.63 (3H, s), 1.04 (3H, s), 2.23-2.40 (2H, m), 2.50 (2H, t, J = 7.2 Hz), 2.71 (2H, t, J = 7.2 Hz), 3.15 (1H, d, J = 11.7 Hz), 3.35 (1H, d, J = 14.4 Hz), 3.607 (1H, d, J = 11.7 Hz), 3.61 (3H, s), 3.72 (1H, t, J = 6.6 Hz), 3.90 (3H, s), 4.18-4.37 (2H, m), 4.42 (1H, d, J = 14.4 Hz), 6.14 (1H, s), 6.61 (1H, d, J = 2.1 Hz), 6.96-7.03 (1H, m), 7.15-7.30 (5H, m), 7.35 (1H, dd, J = 2.1, 8.7 Hz).

### Example 94

### 1-{2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1H-pyrazole-4-carboxylic acid

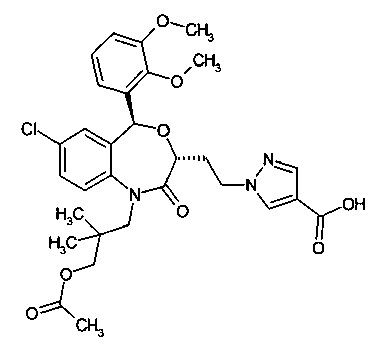

According to a similar manner to that of Example 14, the title compound was obtained.

¹H-NMR (CDCl₃) δ: 0.95 (3H, s), 1.02 (3H, s), 2.03 (3H, s), 2.30-2.50 (2H, m), 3.52 (1H, d, J = 14.1 Hz), 3.62 (3H, s), 3.72 (1H, d, J = 10.8 Hz), 3.77 (1H, dd, J = 5.4, 7.2 Hz), 3.86 (1H, d, J = 10.8 Hz), 3.90 (3H, s), 4.25-4.43 (2H, m), 4.56 (1H, d, J = 14.1 Hz), 6.26 (1H, s), 6.64 (1H, d, J = 2.7 Hz), 6.97-7.05 (1H, m), 7.17-7.25 (2H, m), 7.26 (1H, d, J = 9.0 Hz), 7.33 (1H, dd, J = 2.7, 9.0 Hz), 7.86 (1H, s), 7.90 (1H, s).

### Example 95

### 3-(1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-4-yl)-3-hydroxypropionic acid

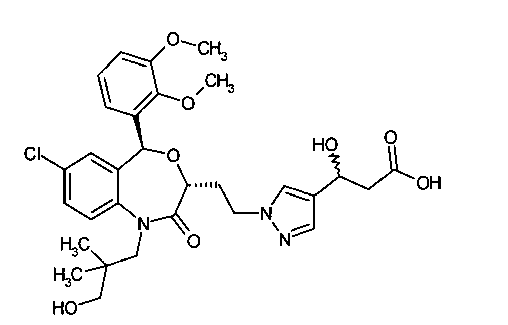

According to a similar manner to that of Example 18, the title compound was obtained.

¹H-NMR (CDCl₃) δ: 0.63 (3H, s), 1.04 (3H, s), 2.28-2.40 (2H, m), 2.63-2.80 (2H, m), 3.15 (1H, d, J = 12.0 Hz), 3.35 (1H, d, J = 14.1 Hz), 3.55-3.62 (1H, m), 3.61 (3H, s), 3.76-3.82 (1H, m), 3.90 (3H, s), 4.19-4.44 (3H, m), 5.00-5.09 (1H, m), 6.13 (1H, s), 6.62 (1H, d, J = 2.1 Hz), 6.98-7.02 (1H, m), 7.19-7.22 (6H, m).

### Example 96

### 3-(1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-5-yl)-3-hydroxypropionic acid

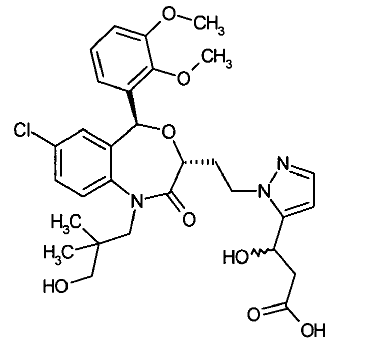

According to a similar manner to that of Example 18, the title compound was obtained.

¹H-NMR (CDCl₃) δ: 0.62 (3/2H, s), 0.63 (3/2H, s), 1.02 (3/2H, s), 1.03 (3/2H, s), 2.20-2.40 (1H, m), 2.21-2.43 (1H, m), 2.79-3.03 (2H, m), 3.148 (1/2H, d, J = 12.0 Hz), 3.158 (1/2H, d, J = 12.0 Hz), 3.31 (1/2H, d, J = 14.1 Hz), 3.35 (1/2H, d, J = 14.1 Hz), 3.50-3.63 (1H, m), 3.59 (3/2H, s), 3.60 (3/2H, s), 3.88 (3H, s), 3.91-4.03 (1H, m), 4.20-4.51 (3H, m), 5.18-5.28 (1H, m), 6.09 (1/2H, s), 6.11 (1/2H, s), 6.139 (1/2H, s), 6.144 (1/sH, s), 6.61 (1H, d, J = 1.8 Hz), 6.96-7.01 (1H, m), 7.15-7.40 (5H, m).

### Example 97

### (2E)-3-(1-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1H-pyrazol-4-yl)acrylic acid

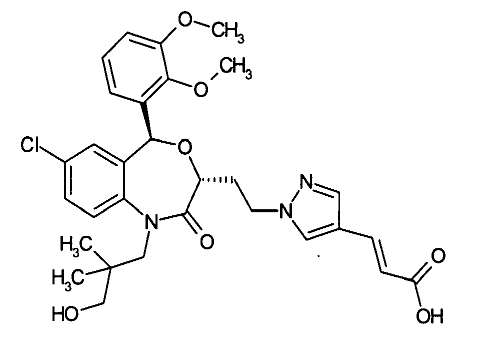

According to a similar manner to that of Example 19, the title compound was obtained.

¹H-NMR (CDCl₃) δ: 0.64 (3H, s), 1.05 (3H, s), 2.29-2.49 (2H, m), 3.15 (1H, d, J = 12.3 Hz), 3.36 (1H, d, J = 13.5 Hz), 3.61 (1H, d, J = 12.3 Hz), 3.62 (3H, s), 3.80 (1H, dd, J = 5.4, 7.2 Hz), 3.90 (3H, s), 4.28-4.40 (2H, m), 4.46 (1H, d, J = 13.5 Hz), 6.09 (1H, d, J = 15.9 Hz), 6.16 (1H, s), 6.62 (1H, d, J = 2.4 Hz), 7.03 (1H, dd, J = 2.1, 7.2 Hz), 7.18-7.24 (2H, m), 7.29 (1H, d, J = 8.4 Hz), 7.36 (1H, dd, J = 2.4, 8.4 Hz), 7.55 (1H, s), 7.57 (1H, d, J = 15.9 Hz), 7.62 (1H, s).

### Example 98

### 4-(5-{[(3R,5S)-7-chloro-5-(2,3-dimethoxyphenyl-1-(3-hydroxy-2,2-dimethylpropyl)-2-oxo-1,2,3,5-tetrahydro-4,1-benzoxazepin-3-yl)-1,3,4-thiadiazol-2-yl]butanoic acid

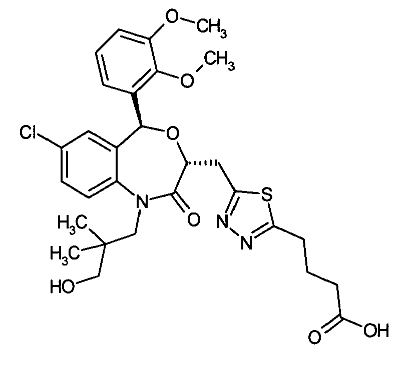

According to a similar manner to that of Example 56, the title compound was obtained.

¹H-NMR (CDCl₃) δ: 0. 65 (3H, s), 1.05 (3H, s), 2.15 (2H, quintet, J = 7.2 Hz), 2.49 (2H, t, J = 7.2 Hz), 3.16 (2H, t, J = 7.2 Hz), 3.10-3.20 (1H, m), 3.38 (1H, d, J = 13.8 Hz), 3.53 (1H, dd, J = 6.6, 15.3 Hz), 3.58-3.70 (2H, m), 3.62 (3H, s), 4.39 (1H, t, J = 6.6 Hz), 4.45 (1H, d, J = 13.8 Hz), 6.20 (1H, s), 6.59 (1H, d, J = 2.1 Hz), 6.99 (1H, dd, J = 1.8, 8.1 Hz), 7.12 (1H, dd, J = 1.8, 8.2 Hz), 7.19 (1H, t, J = 8.1 Hz), 7.32 (1H, d, J = 8.4 Hz), 7.36 (1H, dd, J = 2.1, 8.4 Hz).

### Example 99

### 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiadol-5-yl)-3-phenylpropionic acid

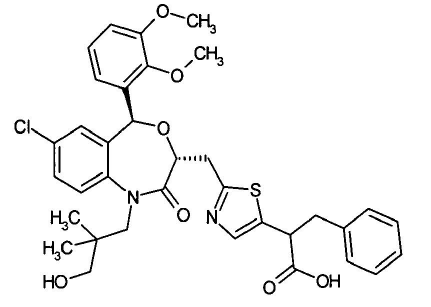

(1) Concentrated sulfuric acid (0.1 ml) was added to a solution of (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid (7.5 g, 13.0 mmol) obtained in Example 1 in ethanol (75 ml) and the mixture was heated to reflux for 24 hours with stirring. After standing to cool to room temperature, the mixture was concentrated under reduced pressure and ethyl acetate (400 ml) was added to the residue. The organic layer was washed with an aqueous saturated sodium carbonate solution, water and an aqueous saturated sodium chloride solution, dried over magnesium sulfate and then concentrated under reduced pressure. The resulting residue was dissolved in DMF (70 ml). Imidazole (1.06 g, 15.7 mmol) and tert-butyldimethylchlorosilane (2.36 g, 15.7 mmol) were added thereto and the mixture was stirred for 14 hours. After water (50 ml) was added, the reaction solution was extracted with ethyl acetate (350 ml) and the organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (1:1)] to obtain ethyl (2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acetate (7.97 g, 11.1 mmol, 85%) as pale yellow noncrystalline powder.
   ¹H-NMR (CDCl₃) δ: 0.09 (3H, s), 0.11 (3H, s), 0.96 (3H, s), 0.98 (9H, s), 1.10 (3H, s), 1.39 (3H, t, J = 6.9 Hz), 3.29 (1H, d, J = 9.9 Hz), 3.34 (1H, d, J = 9.9 Hz), 3.55 (1H, dd, J = 7.2, 15.0 Hz), 3.67 (1H, dd, J = 6.0, 15.0 Hz), 3.72 (3H, s), 3.78 (1H, d, J = 13.8 Hz), 3.89 (2H, s), 3.99 (3H, s), 4.30 (2H, q, J = 6.9 Hz), 4.43 (1H, dd, J = 6.0, 7.2 Hz), 4.50 (1H, d, J = 13.8 Hz), 6.38 (1H, s), 6.65-6.68 (1H, m), 7.07 (1H, dd, J = 2.4, 6.9 Hz), 7.20-7.31 (2H, m), 7.36-7.40 (2H, m), 7.52-7.54 (1H, m).
(2) Lithium bistrimethylsilylamide (0.63 ml, 0.7 mmol) was added dropwise to a solution of ethyl (2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl) acetate (0.5 g, 0.70 mmol) obtained in Example 99-(1) in THF (5 ml) at -78°C under nitrogen atmosphere. After stirring at the same temperature for 1 hour, benzyl bromide (0.08 ml, 0.7 mmol) was added dropwise thereto and the mixture was stirred at - 50°C for 5 hours. After an aqueous saturated ammonium chloride solution (3 ml) was added, the mixture was extracted with ethyl acetate (60 ml), and the organic layer was washed with an aqueous saturated sodium chloride solution, dried over magnesium sulfate and then concentrated under reduced pressure. The resulting residue was purified by silica gel column chromatography [developing solvent: hexane-ethyl acetate (4:1-73:27)] to obtain ethyl 2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-phenylpropionate (0.33 g, 0.41 mmol, 59%) as a colorless oil.
   ¹H-NMR (CDCl₃) δ: -0.11 (3H, s), -0.02 (3H, s), 0.85 (3H, s), 0.88 (9H, s), 1.00 (3H, s), 1.15 (3/2H, t, J = 6.9 Hz), 1.16 (3/2H, t, J = 6.9 Hz), 3.05 (1/2H, dd, J = 4.8, 6.9 Hz), 3.10 (1/2H, dd, J = 4.8, 6.9 Hz), 3.19 (1H, d, J = 9.6 Hz), 3.24 (1H, d, J = 9.6 Hz), 3.32 (1/2H, dd, J = 3.3, 8.4 Hz), 3.37 (1/2H, dd, J = 3.3, 8.4 Hz), 3.44 (1H, dd, J = 7.5, 15.0 Hz), 3.53 (1H, dd, J = 5.7, 15.0 Hz), 3.62 (3H, s), 3.68 (1H, d, J = 14.1 Hz), 3.89 (3H, s), 4.02-4.16 (3H, m), 4.25-4.34 (1H, m), 4.41 (1H, d, J = 14.1 Hz), 6.28 (1H, s), 6.57 (1H, s), 6.92-7.00 (1H, m), 7.08-7.33 (9H, m), 7.37 (1H, s).
(3) A boron trifluoride diethyl ether complex (0.16 ml, 1.22 mmol) was added dropwise to a solution of ethyl 2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-phenylpropionate (0.33 g, 0.41 mmol) obtained in Example 99-(2) in acetonitrile (5 ml). After stirring at the same temperature for 1.5 hours, water (2 ml) was added thereto and the mixture was extracted with ethyl acetate (60 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (1:1-2:3)] to obtain ethyl 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-phenylpropionate (0.24 g, 0.35 mmol, 85%) as colorless noncrystalline powder.
   ¹H-NMR (CDCl₃) δ: 0.65 (3H, s), 1.06 (3H, s), 1.16 (3H, t, J = 7.2 Hz), 3.00-3.21 (2H, m), 3.29-3.50 (3H, m), 3.55 (1H, dd, J = 5.7, 15.3 Hz), 3.59-3.70 (1H, m), 3.62 (3H, s), 3.89 (3H, s), 4.00-4.21 (4H, m), 4.27-4.39 (1H, m), 4.49 (1H, d, J = 14.4 Hz), 6.20 (1H, s), 6.57 (1H, d, J = 1.5 Hz), 6.94-7.01 (1H, m), 7.03-7.35 (9H, m), 7.37 (1H, s).
(4) A mixture of ethyl 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-phenylpropionate (0.2 g, 0.29 mmol) obtained in Example 99-(3), a 1N aqueous sodium hydroxide solution (1 ml) and ethanol (5 ml) was stirred at room temperature for 1 hour. The mixture was acidified with 1N aqueous hydrochloric acid (2.3 ml) and then extracted with ethyl acetate (35 ml). The organic layer was washed with an aqueous saturated sodium chloride solution, dried over magnesium sulfate, and then concentrated under reduced pressure to obtain 2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-phenylpropionic acid (0.19 g, 0.29 mmol, quant.) as a colorless noncrystalline powder.
   ¹H-NMR (CDCl₃) δ: 0.64 (3H, s), 1.04 (3/2H, s), 1.05 (3/2H, s), 3.06 (1H, dd, J = 7.5, 13.8 Hz), 3.18 (1H, d, J = 12.0 Hz), 3.37 (1H, d, J = 14.7 Hz), 3.30-3.63 (3H, m), 3.61 (3H, s), 3.63 (1H, d, J = 12.0 Hz), 3.87 (3/2H, s), 3.88 (3/2H, s), 4.05-4.17 (1H, m), 4.27-4.37 (1H, m), 4.46 (1H, d, J = 14.7 Hz), 6.19 (1H, s), 6.55-6.60 (1H, m), 6.92-7.00 (1H, m), 7.05-7.41 (10H, m).

### Example 100

### (2-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acetic acid

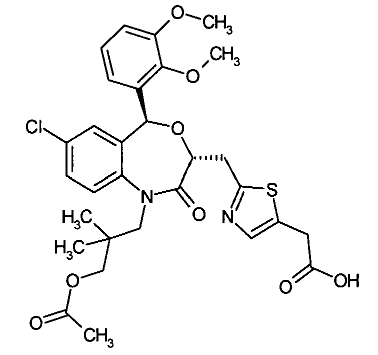

According to a similar manner to that of Example 14, the title compound was obtained.

¹H-NMR (CDCl₃) δ: 0.95 (3H, s), 1.02 (3H, s), 2.02 (3H, s), 3.39-3.62 (3H, m), 3.61 (3H, s), 3.73 (1H, d, J = 10.8 Hz), 3.82 (2H, s), 3.85 (1H, d, J = 10.8 Hz), 3.88 (3H, s), 4.33 (1H, t, J = 6.9 Hz), 4.56 (1H, d, J = 13.8 Hz), 6.30 (1H, s), 6.59 (1H, d, J = 2.4 Hz), 6.96 (1H, dd, J = 3.3, 6.6 Hz), 7.10-7.20 (2H, m), 7.26-7.34 (2H, m), 7.47 (1H, s).

### Example 101

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid calcium salt

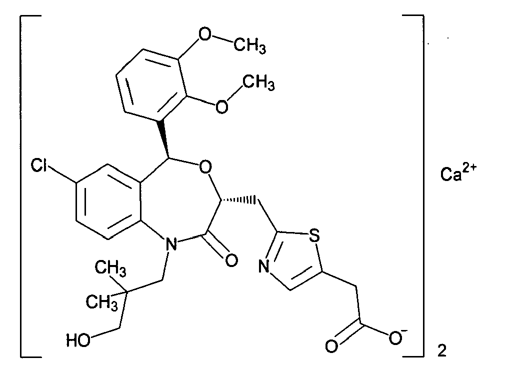

According to a similar manner to that of Example 27, the title compound was obtained.

¹H-NMR (CDCl₃) δ: 0.61 (3H, s), 0.87 (3H, s), 3.04-3.30 (3H, m), 3.40-3.65 (7H, m), 3.84 (3H, s), 4.23-4.50 (2H, m), 6.13 (1H, s), 6.55 (1H, s), 6.93 (1H, d, J = 8.1 Hz), 7.09-7.38 (5H, m).

### Example 102

### (2Z)-2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-phenylacrylic acid

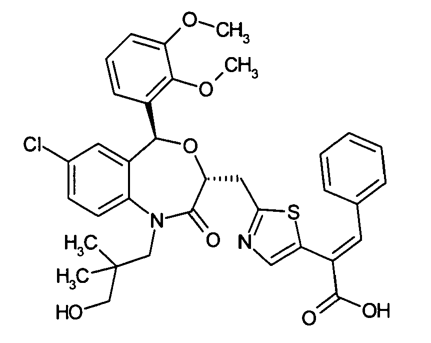

(1) Lithium bistrimethylsilylamide (1.33 ml, 1.46 mmol) was added dropwise to a solution of ethyl (2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)acetate (1.0 g, 1.39 mmol) obtained in Example 99-(1) in THF (10 ml) at -78°C under nitrogen atmosphere. After stirring at the same temperature for 1 hour, benzaldehyde (0.15 g, 1.39 mmol) was added dropwise thereto and the mixture was stirred at -20°C for 5 hours. After an aqueous saturated ammonium chloride solution (5 ml) was added, the mixture was extracted with ethyl acetate (120 ml) and the organic layer was washed with an aqueous saturated sodium chloride solution, dried over magnesium sulfate and then concentrated under reduced pressure. The resulting residue was purified by silica gel column chromatography [developing solvent: hexane-ethyl acetate (73:27-62:38)] to obtain ethyl 2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-hydroxy-3-phenylpropionate (0.23 g, 0.28 mmol, 20%) as a colorless oil.
   ¹H-NMR (CDCl₃) δ: -0.11 (3H, s), -0.02 (3H, s), 0.80-1.35 (9H, m), 0.87 (9H, s), 2.90-3.28 (3H, m), 3.30-3.74 (6H, m), 3.88 (3H, s), 4.00-4.43 (4H, m), 5.03-5.12 (1/2H, m), 5.17 (1/2H, m), 6.25 (1/2H, s), 6.27 (1/2H, s), 6.56 (1H, br), 6.90-7.40 (11H, m).
(2) Triethylamine (0.06 ml, 0.43 mmol) and methanesulfonyl chloride (0.03 ml, 0.43 mmol) were added to a solution of ethyl 2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-hydroxy-3-phenylpropionate (0.22 g, 0.27 mmol) obtained in Example 102-(1) in THF (5 ml) under ice-cooling. After stirring at the same temperature for 1.5 hours, DBU (0.08 ml, 0.53 mmol) was added dropwise thereto and the mixture was stirred for 2 hours. After water (5 ml) was added, the reaction solution was extracted with ethyl acetate (60 ml) and the organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (67:33-1:1)] to obtain ethyl (2Z)-2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-phenylacrylate (0.09 g, 0.12 mmol, 44%) as colorless noncrystalline powder. ¹H-NMR (CDCl₃) δ: -0.10 (3H, s), -0.01 (3H, s), 0.85 (3H, s), 0.88 (9H, s), 1.00 (3H, s), 1.33 (3H, t, J = 7.2 Hz), 3.20 (1H, d, J = 9.9 Hz), 3.24 (1H, d, J = 9.9 Hz), 3.50-3.59 (2H, m), 3.62 (3H, s), 3.69 (1H, d, J = 13.8 Hz), 3.88 (3H, s), 4.20-4.35 (3H, m), 4.42 (1H, d, J = 13.8 Hz), 6.29 (1H, s), 6.57 (1H, s), 6.95 (1H, dd, J = 2.4, 7.5 Hz), 6.99-7.11 (2H, m), 7.14-7.37 (4H, m), 7.44 (1H, s), 7.93 (1H, s) .
(3) A boron trifluoride diethyl ether complex (22 µl, 0.18 mmol) was added dropwise to a solution of ethyl (2Z)-2-(2-{[(3R,5S)-1-(3-{[tert-butyl(dimethyl)silyl]oxy}-2,2-dimethylpropyl)-7-chloro-5-(2,3-dimethoxyphenyl)-2-oxo-1,2,3,5-tetrahydro-4,1-benzoxazepin-3-yl]methyl}-1,3-thiazol-5-yl)-3-phenylacrylate (0.09 g, 0.12 mmol) obtained in Example 102-(2) in acetonitrile (5 ml) under ice-cooling. After stirring at the same temperature for 1.5 hours, water (2 ml) was added thereto and the mixture was extracted with ethyl acetate (60 ml). The organic layer was washed with an aqueous saturated sodium chloride solution, dried over magnesium sulfate, and then concentrated under reduced pressure. The resulting residue was dissolved in ethanol (3 ml), a 1N aqueous sodium hydroxide solution (0.6 ml) was added, and the mixture was stirred at room temperature for 5 hours. The reaction solution was acidified with 1N aqueous hydrochloric acid (1 ml) and then extracted with ethyl acetate (35 ml). The organic layer was washed with an aqueous saturated sodium chloride solution, dried over magnesium sulfate, and then concentrated under reduced pressure to obtain (2Z)-2-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-phenylacrylic acid (0.07 g, 0.11 mmol, 85%) as pale yellow noncrystalline powder.
   ¹H-NMR (CDCl₃) δ: 0.65 (3H, s), 1.06 (3H, s), 3.18 (1H, d, J = 12.0 Hz), 3.39 (1H, d, J = 14.4 Hz), 3.53-3.69 (3H, m), 3.61 (3H, s), 3.87 (3H, s), 4.37 (1H, dd, J = 5.7, 7.2 Hz), 4.49 (1H, d, J = 14.4 Hz), 6.21 (1H, s), 6.58 (1H, d, J = 1.8 Hz), 6.90-7.00 (1H, m), 7.02-7.10 (2H, m), 7.17-7.40 (7H, m), 7.51 (1H, s), 8.06 (1H, s).

### Example 103

### (2R)-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-l,3-thiazol-5-yl)(hydroxy)acetic acid

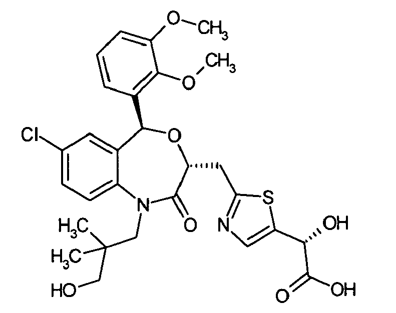

According to a similar manner to that of Example 99, the title compound was obtained.

¹H-NMR (CDCl₃) δ: 0.64 (3H, s), 1.03 (3H, s), 3.20 (1H, d, J = 12.3 Hz), 3.38 (1H, d, J = 14.1 Hz), 3.48 (1H, dd, J = 6.6, 15.3 Hz), 3.53-3.67 (2H, m), 3.60 (3H, s), 3.88 (3H, s), 4.30-4.40 (1H, m), 4.44 (1H, d, J = 14.1 Hz), 5.33 (1H, s), 6.18 (1H, s), 6.59 (1H, d, J = 1.8 Hz), 6.97 (1H, dd, J = 1.8, 7.8 Hz), 7.09-7.21 (2H, m), 7.26-7.40 (2H, m), 7.66 (1H, s).

### Example 104

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-4-yl)-3-methylbutanoic acid

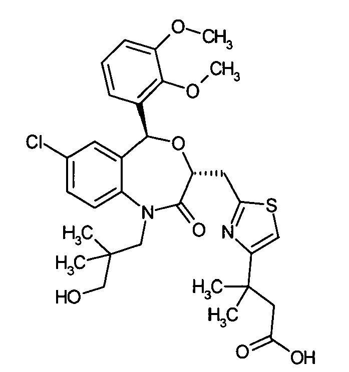

According to a similar manner to that of Example 16, the title compound was obtained.

¹H-NMR (CDCl₃) δ: 0.65 (3H, s), 1.05 (3H, s), 1.39 (3H, s), 1.41 (3H, s), 2.71 (2H, s), 3.10-3.21 (1H, brs), 3.43 (1H, d, J = 14.4 Hz), 3.51 (1H, dd, J = 6.3, 15.6 Hz), 3.59-3.71 (2H, m), 3.62 (3H, s), 3.89 (3H, s), 4.32 (1H, t, J = 6.3 Hz), 4.46 (1H, d, J = 14.4 Hz), 6.21 (1H, s), 6.60 (1H, d, J = 1.8 Hz), 6.92 (1H, s), 6.99 (1H, dd, J = 2.7, 7.5 Hz), 7.10-7.20 (2H, m), 7.35-7.4.5 (2H, m).

### Example 105

### (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-5-yl)acetic acid

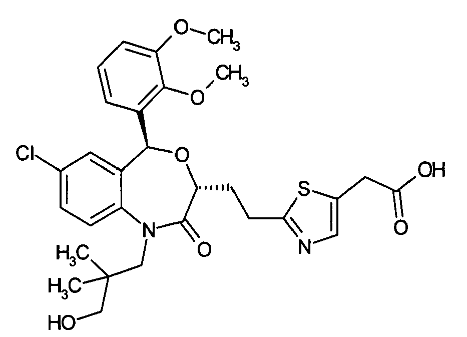

(1) To a solution of (3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-ethanol (1.0 g, 1.98 mmol) obtained in Example 50-(1), acetone cyanohydrin (0.23 ml, 2.57 mmol) and toluene (20 ml) were added n-tributylphosphine (0.74 ml, 2.96 mmol) and 1,1'-azodicarbonyldipiperidine (0.75 g, 2.96 mmol) under nitrogen atmosphere, and the mixture was stirred at room temperature for 3 hours. After hexane (10 ml) was added, the mixture was stirred for 30 minutes and then filtered to remove insoluble substances. The filtrate was concentrated under reduced pressure and the resulting residue was purified by silica gel column chromatography [developing solvent: hexane-ethyl acetate (7:3-3:2)] to obtain 3-[(3R,5S)-7-chloro-3-(2-cyanoethyl)-5-(2,3-dimethoxyphenyl)-2-oxo-2,3-dihydro-4,1-benzoxazepin-1(5H)-yl]-2,2-dimethylpropyl acetate (0.75 g, 1.46 mmol, 74%) as colorless noncrystalline powder.
   ¹H-NMR (CDCl₃) δ: 0.94 (3H, s), 2.03 (3H, s), 2.00-2.35 (2H, m), 2.57 (2H, t, J = 7.5 Hz), 3.53 (1H, d, J = 14.4 Hz), 3.62 (3H, s), 3.72 (1H, d, J = 11.1 Hz), 3.86 (1H, d, J = 11.1 Hz), 3.90 (3H, s), 3.99 (1H, dd, J = 4.8, 7.5 Hz), 4.57 (1H, d, J = 14.4 Hz), 6.27 (1H, s), 6.66 (1H, d, J = 2.1 Hz), 7.00 (1H, dd, J = 3.0, 7.2 Hz), 7.16-7.25 (2H, m), 7.30 (1H, d, J = 8.7 Hz), 7.36 (1H, dd, J = 2.1, 8.7 Hz).
(2) O,O'-diethyl dithiophosphate (0.20 ml, 1.17 mmol) was added to a solution of 3-[(3R,5S)-7-chloro-3-(2-cyanoethyl)-5-(2,3-dimethoxyphenyl)-2-oxo-2,3-dihydro-4,1-benzoxazepin-1(5H)-yl]-2,2-dimethylpropyl acetate (0.5 g, 0.97 mmol) obtained in Example 105-(1) and 4N HCl (2.5 ml), and the mixture was stirred at room temperature for 16 hours. After water (5 ml) was added, the mixture was extracted with ethyl acetate and the organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (3:2-1:1)] to obtain 3-[(3R,5S)-3-(3-amino-3-thioxopropyl)-7-chloro-5-(2,3-dimethoxyphenyl)-2-oxo-2,3-dihydro-4,1-benzoxazepin-1(5H)-yl]-2,2-dimethylpropyl acetate (0.38 g, 0.69 mmol, 71%) as a pale yellow crystal.
   ¹H-NMR (CDCl₃) δ: 0.95 (3H, s), 1.01 (3H, s), 2.03 (3H, s), 2.20-2.34 (2H, m), 2.71-2.94 (2H, m), 3.52 (1H, d, J = 14.1 Hz), 3.62 (3H, s), 3.72 (1H, d, J = 11.1 Hz), 3.88 (1H, d, J = 11.1 Hz), 3.90 (3H, s), 3.96 (1H, t, J = 6.3 Hz), 4.56 (1H, d, J = 14.1 Hz), 6.26 (1H, s), 6.64 (1H, d, J = 2.4 Hz), 6.95-7.04 (1H, m), 7.15-7.24 (2H, m), 7.29 (1H, d, J = 9.0 Hz), 7.35 (1H, dd, J = 2.4, 9.0 Hz), 7.30-7.40 (1H, m), 7.48 (1H, br).
(3) According to the similar manner as that of Example 16 using 3-[(3R,5S)-3-(3-amino-3-thioxopropyl)-7-chloro-5-(2,3-dimethoxyphenyl)-2-oxo-2,3-dihydro-4,1-benzoxazepin-1(5H)-yl]-2,2-dimethylpropyl acetate obtained in Example 105-(2), the title compound was obtained.
   ¹H-NMR (CDCl₃) δ: 0.64 (3H, s), 1.04 (3H, s), 2.18-2.42 (2H, m), 2.95-3.20 (3H, m), 3.37 (1H, d, J = 14.4 Hz), 3.57-3.70 (1H, m), 3.61 (3H, s), 3.74 (2H, s), 3.82-3.99 (1H, m), 3.89 (3H, s), 4.46 (1H, d, J = 14.4 Hz), 6.15 (1H, s), 6.59 (1H, s), 6.92-7.02 (1H, m), 7.13-7.23 (2H, m), 7.25-7.40 (3H, m).

### Example 106

### 3-(2-(2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-tihazol-5-yl)propionic acid

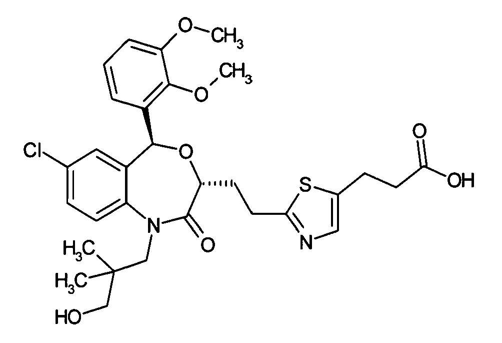

According to a similar manner to that of Example 105, the title compound was obtained.

¹H-NMR (CDCl₃) δ: 0.64 (3H, s), 1.04 (3H, s), 2.17-2.41 (2H, m), 2.62 (2H, t, J = 7.2 Hz), 2.95-3.21 (5H, m), 3.36 (1H, d, J = 14.4 Hz), 3.607 (3H, s), 3.612 (1H, d, J = 11.7 Hz), 3.88 (3H, s), 3.84-3.94 (1H, m), 4.44 (1H, d, J = 14.4 Hz), 6.14 (1H, s), 6.60 (1H, d, J = 1.8 Hz), 6.98 (1H, dd, J = 2.4, 6.9 Hz), 7.13-7.22 (2H, m), 7.26 -7.40 (3H, m).

### Example 107

### 2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-5-carboxylic acid

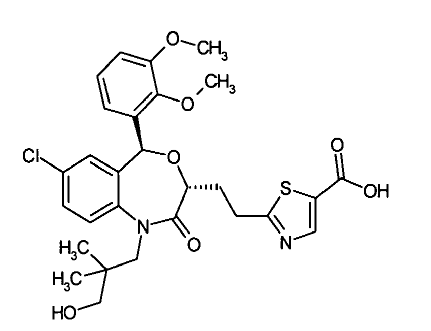

According to a similar manner to that of Example 105, the title compound was obtained.

¹H-NMR (CDCl₃) δ: 0.64 (3H, s), 1.06 (3H, s), 2.20-2.50 (2H, m), 3.08-3.32 (2H, m), 3.17 (1H, d, J = 11.4 Hz), 3.37 (1H, d, J = 14.4 Hz), 3.62 (3H, s), 3.63 (1H, d, J = 11.4 Hz), 3.89 (3H, s), 3.95 (1H, dd, J = 5.1, 7.5 Hz), 4.48 (1H, d, J = 14.4 Hz), 6.16 (1H, s), 6.61 (1H, d, J = 2.1 Hz), 6.99 (1H, dd, J = 3.0, 6.6 Hz), 7.13-7.23 (2H, m), 7.29 (1H, d, J = 8.7 Hz), 7.35 (1H, dd, J = 2.1, 8.7 Hz), 8.27 (1H, s).

### Example 108

### 2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-tihazol-4-carboxylic acid

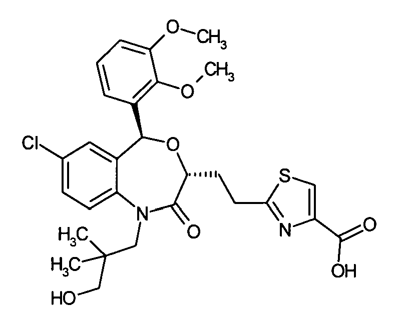

According to a similar manner to that of Example 105, the title compound was obtained.

¹H-NMR (CDCl₃) δ: 0.64 (3H, s), 1.05 (3H, s), 2.22-2.50 (2H, m), 3.06-3.38 (2H, m), 3.17 (1H, d, J = 11.4 Hz), 3.37 (1H, d, J = 14.1 Hz), 3.61 (3H, s), 3.62 (1H, d, J = 11.4 Hz), 3.89 (3H, s), 3.95 (1H, dd, J = 5.4, 7.5 Hz), 4.46 (1H, d, J = 14.1 Hz), 6.15 (1H, s), 6.60 (1H, d, J = 2.1 Hz), 6.99 (1H, dd, J = 2.4, 7.5 Hz), 7.10-7.23 (2H, m), 7.31 (1H, d, J = 8.4 Hz), 7.36 (1H, dd, J = 2.1, 8.4 Hz), 8.10 (1H, s) .

### Example 109

### (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-4-yl)acetic acid

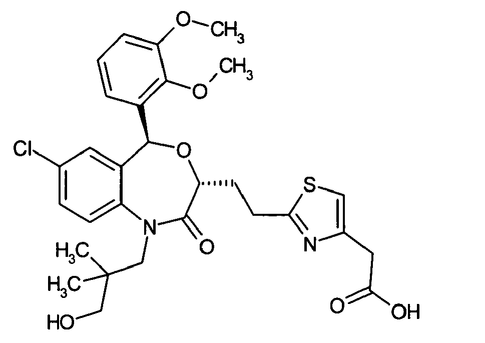

According to a similar manner to that of Example 105, the title compound was obtained.

¹H-NMR (CDCl₃) δ: 0.64 (3H, s), 1.05 (3H, s), 2.18-2.45 (2H, m), 3.03-3.30 (3H, m), 3.38 (1H, d, J = 14.4 Hz), 3.617 (3H, s), 3.618 (1H, d, J = 11.7 Hz), 3.77 (2H, d, J = 1.2 Hz), 3.89 (3H, s), 3.93 (1H, dd, J = 5.4, 7.5 Hz), 4.47 (1H, d, J = 14.4 Hz), 6.16 (1H, s), 6.61 (1H, d, J = 2.1 Hz), 6.95 (1H, t, J = 1.2 Hz), 6.95-7.01 (1H, m), 7.16-7.23 (2H, m), 7.31 (1H, d, J = 8.4 Hz), 7.36 (1H, dd, J = 2.1, 8.4 Hz).

### Example 110

### 2-{2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]ethyl}-1,3-thiazol-5-carboxylic acid

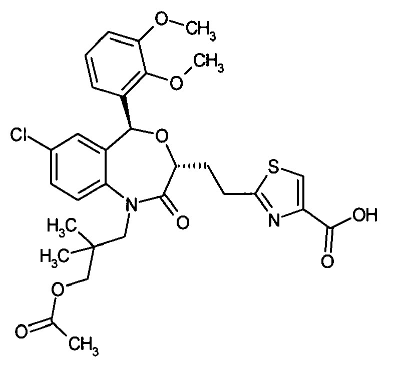

According to a similar manner to that of Example 14, the title compound was obtained.

¹H-NMR (CD₃OD) δ: 0.98 (3H, s), 0.99 (3H, s), 1.98 (3H, s), 1.90-2.23 (2H, m), 2.90-3.20 (2H, m), 3.58 (3H, s), 3.60-3.92 (3H, m), 3.87 (3H, s), 4.45 (1H, d, J = 14.1 Hz), 4.75-4.90 (1H, m), 6.20 (1H, s), 6.51 (1H, d, J = 2.1 Hz), 6.99-7.21 (3H, m), 7.35-7.45 (1H, m), 7.54 (1H, d, J = 9.0 Hz), 7.90 (1H, s).

### Example 111

### 3-(2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazole-4-yl)-3-hydroxypropionic acid

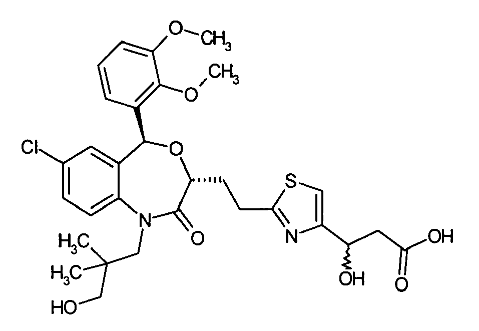

According to a similar manner to that of Example 18, the title compound was obtained.

¹H-NMR (CDCl₃) δ: 0.64 (3H, s), 1.05 (3H, s), 2.18-2.42 (2H, m), 2.73-2.82 (1H, m), 2.82-2.96 (2H, m), 3.00-3.22 (2H, m), 3.15 (1H, d, J = 12.0 Hz), 3.37 (1H, d, J = 13.8 Hz), 3.61 (3H, s), 3.62 (1H, d, J = 12.0 Hz), 3.89 (3H, s), 3.90-4.01 (1H, m), 4.47 (1H, d, J = 13.8 Hz), 5.10-5.17 (1H, m), 6.15 (1H, s), 6.60 (1H, d, J = 2.1 Hz), 6.95-7.01 (1H, m), 7.07 (1H, s), 7.15-7.21 (2H, m), 7.31 (1H, d, J = 8.7 Hz), 7.36 (1H, dd, J = 2.1, 8.7 Hz).

### Example 112

### (2E)-3-(2-(2-[3R,5S]-7-chloro-5-(2,3-dimethoxyphenyl)-1-(3-hydroxy-2,2-dimethylpropyl)-2-oxo-1,2,3,5-tetrahydro-4,1-benzoxazepin-3-yl}ethyl)-1,3-thiazol-4-yl)acrylic acid

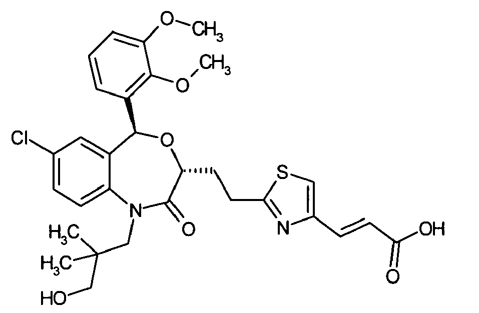

According to a similar manner to that of Example 19, the title compound was obtained.

¹H-NMR (CDCl₃) δ: 0.65 (3H, s), 1.06 (3H, s), 2.20-2.43 (2H, m), 3.05-3.24 (3H, m), 3.38 (1H, d, J = 14.4 Hz), 3.62 (3H, s), 3.60-3.69 (1H, m), 4.05 (1H, dd, J = 5.7, 7.2 Hz), 4.47 (1H, d, J = 14.4 Hz), 6.16 (1H, s), 6.59 (1H, d, J = 15.3 Hz), 6.60 (1H, d, J = 2.4 Hz), 6.98 (1H, dd, J = 2.7, 6.6 Hz), 7.14-7.29 (3H, m), 7.32 (1H, d, J = 8.7 Hz), 7.37 (1H, dd, J = 2.4, 8.4 Hz), 7.58 (1H, d, J = 15.3 Hz) .

### Example 113

### 3-(2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-4-yl)propionic acid

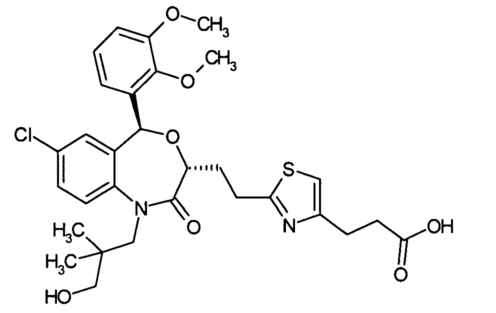

According to a similar manner to that of Example 88, the title compound was obtained.

¹H-NMR (CDCl₃) δ: 0.64 (3H, s), 1.05 (3H, s), 2.20-2.42 (2H, m), 2.60-2.75 (2H, m), 2.90-3.25 (3H, m), 3.38 (1H, d, J = 14.7 Hz), 3.619 (3H, s), 3.624 (1H, d, J = 11.7 Hz), 3.89 (3H, s), 3.96 (1H, dd, J = 5.4, 6.9 Hz), 4.48 (1H, d, J = 14.7 Hz), 6.16 (1H, s), 6.61 (1H, d, J = 1.8 Hz), 6.80 (1H, s), 6.96-7.03 (1H, m), 7.17-7.23 (2H, m), 7.27-7.40 (2H, m).

### Example 114

### 3-(2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-4-yl)-3-methylbutanoic acid

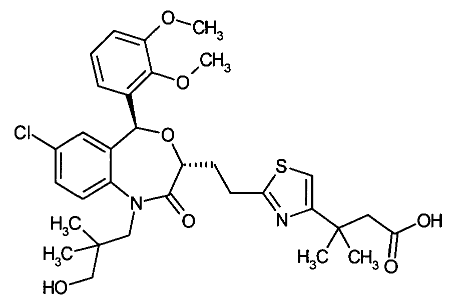

According to a similar manner to that of Example 16, the title compound was obtained.

¹H-NMR (CDCl₃) δ: 0.65 (3H, s), 1.06 (3H, s), 1.38 (3H, s), 1.39 (3H, s), 2.20-2.42 (2H, m), 2.67 (1H, d, J = 15.0 Hz), 3.10-3.35 (3H, m), 3.41 (1H, d, J = 14.7 Hz), 3.62 (3H, s), 3.63 (1H, d, J = 11.7 Hz), 3.90 (3H, s), 3.96 (1H, t, J = 5.7Hz), 4.48 (1H, d, J = 14.7 Hz), 6.16 (1H, s), 6.61 (1H, s), 6.90 (1H, s), 7.00 (1H, dd, J = 2.1, 7.8 Hz), 7.14-7.22 (2H, m), 7.30-7.40 (2H, m).

### Example 115

### (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,3-thiazol-4-yl)acetic acid

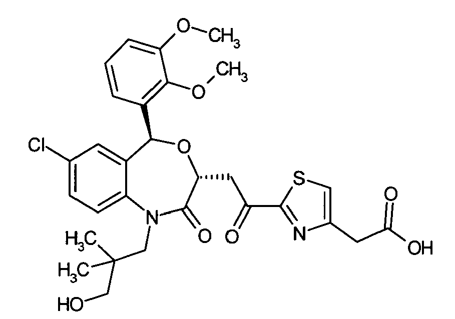

(1) A solution of a sulfur trioxide pyridine complex (5.76 g, 36.2 mmol) in DMSO (18 ml) was added dropwise to a solution of (3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-ethanol (3.66 g, 7.23 mmol) obtained in Example 50-(1) in dichloromethane (70 ml). After stirring at room temperature for 1.5 hours, water (15 ml) was added. After extraction with dichloromethane (150 ml), the organic layer was washed with a 5% aqueous potassium hydrogen sulfate solution, an aqueous saturated sodium hydrogen carbonate solution, water and an aqueous saturated sodium chloride solution, and then dried over magnesium sulfate. After concentration under reduced pressure, the resulting residue was purified by silica gel column chromatography [developing solvent: hexane-ethyl acetate (2:1)] and then washed with 2-propanol-hexane to obtain 3-[(3R,5S)-7-chloro-5-(2,3-dimethoxyphenyl)-2-oxo-3-(2-oxoethyl)-2,3-dihydro-4,1-benzoxazepin-1(5H)-yl]-2,2-dimethylpropyl acetate (2.39 g, 4.74 mmol, 66%) as a white crystal.
   ¹H-NMR (CDCl₃) δ: 0.95 (3H, s), 1.02 (3H, s), 2.03 (3H, s), 2.86 (1H, ddd, J = 1.2, 5.4, 17.7 Hz), 3.09 (1H, ddd, J = 1.2, 6.9, 17.7 Hz), 3.55 (1H, d, J = 14.4 Hz), 3.62 (3H, s), 3.73 (1H, d, J = 11.1 Hz), 3.86 (1H, d, J = 11.1 Hz), 3.89 (3H, s), 4.42 (1H, dd, J = 5.4, 6.9 Hz), 4.56 (1H, d, J = 14.1 Hz), 6.28 (1H, s), 6.66 (1H, d, J = 1.8 Hz), 6.96-7.01 (1H, m), 7.15-7.23 (2H, m), 7.28-7.40 (2H, m), 9.81 (1H, br) .
(2) A solution of 3-[(3R,5S)-7-chloro-5-(2,3-dimethoxyphenyl)-2-oxo-3-(2-oxoethyl)-2,3-dihydro-4,1-benzoxazepin-1(5H)-yl]-2,2-dimethylpropyl acetate (2.0 g, 3.97 mmol) obtained in Example 115-(1) in dichloromethane (25 ml) was added dropwise to a solution of tertbutyldimethylsilyl cyanide (0.59 g, 4.17 mmol), potassium cyanide (44 mg, 0.68 mmol) and 18-crown-6-ether (0.42 g, 1.59 mmol) in dichloromethane (25 ml) under nitrogen atmosphere. The mixture was stirred at room temperature for 18 hours and then concentrated under reduced pressure. The resulting residue was purified by silica gel column chromatography [developing solvent: hexane-ethyl acetate (41:9-3:1)] to obtain 3-[(3R,5S)-3-(2-{[tert-butyl(dimethyl)silyl]oxy}-2-cyanoethyl)-7-chloro-5-(2,3-dimethoxyphenyl)-2-oxo-2,3-dihydro-4,1-benzoxazepin-1(5H)-yl]-2,2-dimethylpropyl acetate (1.96 g, 3.04 mmol, 77%) as a white crystal.
   ¹H-NMR (CDCl₃) δ: 0.04 (3H. s), 0.14 (3/2H, s), 0.15 (3/2H, s), 0.68 (9/2H, s), 0.79 (9/2H, s), 0.95 (3/2H, s), 0.96 (3/2H, s), 2.03 (3H, s), 2.18-2.45 (2H, m), 3.52 (1H, d, J = 14.1 Hz), 3.62 (3H, s), 3.73 (1/2H, d, J = 11.1 Hz), 3.74 (1/2H, d, J = 11.1 Hz), 3.98-4.16 (1H, m), 4.55 (1/2H, d, J = 14.1 Hz), 4.56 (1/2H, d, J = 14.1 Hz), 4.61-4.76 (1H, m), 6.26 (1H, s), 6.99 (1/2H, d, J = 2.7 Hz), 7.01 (1/2H, d, J = 2.7 Hz), 6.95-7.02 (1H, m), 7.10-7.39 (4H, m).
(3) Diphenyldithiophosphoric acid (0.39 g, 1.55 mmol) was added to a solution of 3-[(3R,5S)-3-(2-{[tert-butyl(dimethyl)silyl]oxy}-2-cyanoethyl)-7-chloro-5-(2,3-dimethoxyphenyl)-2-oxo-2,3-dihydro-4,1-benzoxazepin-1(5H)-yl]-2,2-dimethylpropyl acetate (0.5 g, 0.78 mmol) obtained in Example 115-(2) in isopropyl alcohol (10 ml), and the mixture was stirred at 60°C for 14 hours. The mixture was cooled to room temperature and then concentrated under reduced pressure. The resulting residue was purified by silica gel column chromatography [developing solvent: hexane-ethyl acetate (41:9-3:1)] to obtain 3-[(3R,5S)-3-(3-amino-2-{[tert-butyl(dimethyl)silyl]oxy}-3-thioxopropyl)-7-chloro-5-(2,3-dimethoxyphenyl)-2-oxo-2,3-dihydro-4,1-benzoxazepin-1(5H)-yl]-2,2-dimethylpropyl acetate (0.48 g, 0.71 mmol, 91%) as a white crystal.
   ¹H-NMR (CDCl₃) δ: -0.06 (3/2H. s), -0.02 (3/2H, s), 0.03 (3/2H, s), 0.04 (3/2H, s), 0.76 (9/2H, s), 0.81 (9/2H, s), 0.94 (3/2H, s), 0.95 (3/2H, s), 1.03 (3H, s), 2.03 (3H, s), 2.01-2.20 (1H, m), 2.39-2.60 (1H, m), 3.51 (1/2H, d, J = 14.1 Hz), 3.52 (1/2H, d, J = 14.1 Hz), 3.60 (3H, s), 3.69-3.78 (1H, m), 3.84 (1/2H, d, J = 10.8 Hz), 3.85 (1/2H, d, J = 10.8 Hz), 3.89 (3H, s), 4.08 (1/2H, dd, J = 6.0, 10.5 Hz), 4.29 (1/2H, t, J = 6.6 Hz), 4.54 (1/2H, d, J = 14.1 Hz), 4.56 (1/2H, d, J = 14.1 Hz), 4.63 (1/2H, t, J = 6.6 Hz), 4.72 (1/2H, dd, J = 4.5, 9.0 Hz), 6.25 (1H, s), 6.60-6.67 (1H, m), 6.95-7.01 (1H, m), 7.14-7.43 (5H, m), 7.72-7.82 (1H, m).
(4) A solution of 3-[(3R,5S)-3-(3-amino-2-{[tert-butyl(dimethyl)silyl]oxy}-3-thioxopropyl)-7-chloro-5-(2,3-dimethoxyphenyl)-2-oxo-2,3-dihydro-4,1-benzoxazepin-1(5H)-yl]-2,2-dimethylpropyl acetate (0.42 g, 0.62 mmol) obtained in Example 115-(3) and ethyl 4-chloro-3-oxobutanoate (84 µl, 0.62 mmol) in ethanol (10 ml) was stirred at 80°C for 32 hours. After standing to cool, water (5 ml) was added thereto and the mixture was extracted with ethyl acetate (130 ml). The organic layer was washed with an aqueous saturated sodium chloride solution, dried over magnesium sulfate, and then concentrated under reduced pressure. The resulting residue was purified by silica gel column chromatography [developing solvent: hexane-ethyl acetate (41:9-3:1)] to obtain ethyl (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-4-yl)acetate (0.18 g, 0.28 mmol, 45%) as a white crystal.
   ¹H-NMR (CDCl₃) δ: 0.65 (3H, s), 1.05 (3H, s), 1.26 (3H, t, J = 7.2 Hz), 2.25-2.48 (1H, m), 2.50-2.70 (1H, m), 3.15 (1H, t, J = 11.1 Hz), 3.38 (1H, d, J = 14.4 Hz), 3.54-3.70 (1H, m), 3.62 (3/2H, s), 3.64 (3/2H, s), 3.74 (1H, s), 3.75 (1H, s), 3.89 (3H, s), 3.99 (1/2H, dd, J = 4.2, 11.1 Hz), 4.07 (1/2H, dd, J = 4.2, 11.1 Hz), 4.10-4.30 (3H, m), 4.47 (1/2H, d, J = 14.4 Hz), 4.50 (1/2H, d, J = 14.4 Hz), 5.11-5.23 (1H, m), 6.18 (1/2H, s), 6.19 (1/2H, s), 6.61 (1/2H, d, J = 2.1 Hz), 6.63 (1/2H, d, J = 2.1 Hz), 6.94-7.02 (1H, m), 7.095 (1/2H, s), 7.103 (1/2H, s), 7.14-7.40 (4H, m).
(5) Manganese dioxide (0.2 g, 2.38 mmol) was added to a solution of ethyl (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-4-yl)acetate (0.15 g, 0.24 mmol) obtained in Example 115-(4) in THF (5 ml), and the mixture was stirred at 70°C for 4 days. After standing to cool, insoluble substances were filtered off using Celite and the filtrate was concentrated under reduced pressure. The resulting residue was purified by silica gel column chromatography [developing solvent: hexane-ethyl acetate (3:1-2:1)] to obtain ethyl (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,3-thiazol-4-yl)acetate (71 mg, 0.11 mmol, 48%) as a white crystal.
   ¹H-NMR (CDCl₃) δ: 0.65 (3H, s), 1.05 (3H, s), 1.29 (3H, t, J = 6.9 Hz), 3.14 (1H, t, J = 10.5 Hz), 3.41 (1H, d, J = 14.4 Hz), 3.58 (1H, dd, J = 4.8, 18.3 Hz), 3.56-3.69 (1H, m), 3.62 (3H, s), 3.89 (3H, s), 3.98 (1H, dd, J = 8.1, 18.3 Hz), 4.15 (1H, dd, J = 3.9, 10.8 Hz), 4.20 (2H, q, J = 6.9 Hz), 4.49 (1H, d, J = 14.4 Hz), 4.60 (1H, dd, J = 4.8, 8.1 Hz), 6.18 (1H, s), 6.63 (1H, s), 6.95-7.01 (1H, m), 7.15-7.22 (2H, m), 7.36-7.43 (2H, m), 7.57 (1H, s).
(6) A solution of ethyl (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,3-thiazol-4-yl)acetate (60 mg, 0.10 mmol) obtained in Example 115-(5) and potassium carbonate (26 mg, 0.20 mmol) in ethanol (3 ml) and water (0.5 ml) was heated to reflux for 5 hours with stirring. After standing to cool, the mixture was acidified with 1N aqueous hydrochloric acid (1 ml) and extracted with ethyl acetate (70 ml). The organic layer was washed with an aqueous saturated sodium chloride solution, dried over magnesium sulfate, and then concentrated under reduced pressure. The resulting crystal was washed with ethyl acetate-hexane and then dried under reduced pressure to obtain (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,3-thiazol-4-yl)acetic acid (40 mg, 0.07 mmol, 70%) as a pale yellow crystal.
   ¹H-NMR (CDCl₃) δ: 0.65 (3H, s), 1.04 (3H, s), 3.16 (1H, d, J = 12.0 Hz), 3.41 (1H, d, J = 14.1 Hz), 3.54 (1H, dd, J = 4.8, 18.3 Hz), 3.61 (3H, s), 3.62 (1H, d, J = 12.0 Hz), 3.88 (3H, s), 3.99 (1H, dd, J = 8.4, 18.3 Hz), 4.47 (1H, d, J = 14.1 Hz), 4.60 (1H, dd, J = 4.8, 8.4 Hz), 6.16 (1H, s), 6.62 (1H, d, J = 2.7 Hz), 6.93-7.00 (1H, m), 7.10-7.20 (2H, m), 7.33-7.42 (2H, m), 7.55 (1H, s).

### Example 116

### 3-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-l,3-thiazol-5-yl)propionic acid

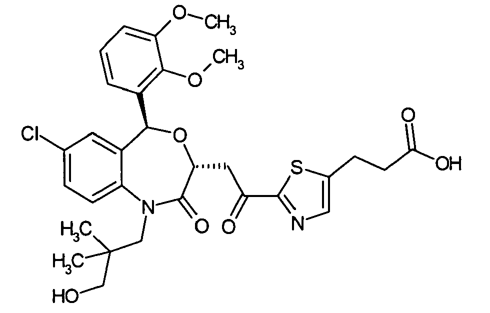

According to a similar manner to that of Example 115, the title compound was obtained.

¹H-NMR (CDCl₃) δ: 0.65 (3H, s), 1.04 (3H, s), 2.76 (2H, t, J = 7.2 Hz), 3.14 (1H, d, J = 12.0 Hz), 3.23 (1H, t, J = 7.2 Hz), 3.41 (1H, d, J = 15.0 Hz), 3.54 (1H, dd, J = 4.8, 18.0 Hz), 3.61 (3H, s), 3.62 (1H, d, J = 12.0 Hz), 3.89 (3H, s), 3.95 (1H, dd, J = 8.4, 18.0 Hz), 4.48 (1H, d, J = 15.0 Hz), 4.58 (1H, dd, J = 4.8, 8.4 Hz), 6.17 (1H, s), 6.62 (1H, s), 6.95-7.01 (1H, m), 7.13-7.20 (2H, m), 7.38-7.42 (2H, m), 7.74 (1H, s).

### Example 117

### (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-4-yl)acetic acid

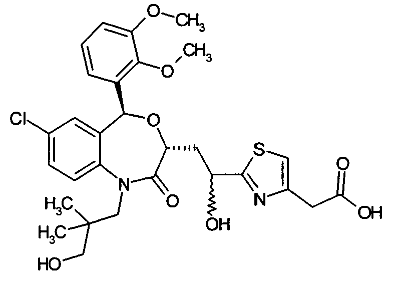

A mixture of ethyl (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-4-yl)acetate (0.25 g, 0.40 mmol) obtained in Example 115-(4), a 1N aqueous sodium hydroxide solution (1 ml) and ethanol (5 ml) was stirred at room temperature for 2 hours. This mixture was acidified with 1N aqueous hydrochloric acid (1.2 ml) and then extracted with ethyl acetate (35 ml). This extract was washed with an aqueous saturated sodium chloride solution, dried over magnesium sulfate, and then concentrated under reduced pressure to obtain (2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-4-yl)acetic acid (0.24 g, 0.40 mmol, quant.) as colorless noncrystalline powder.

¹H-NMR (CDCl₃) δ: 0.66 (3H, s), 1.05 (3H, s), 2.30-2.70 (2H, m), 3.17 (1H, t, J = 11.7 Hz), 3.40 (1H, d, J = 14.4 Hz), 3.61 (1H, d, J = 11.7 Hz), 3.63 (3H, s), 3.79 (2H, s), 3.90 (3H, s), 4.19-4.32 (1H, m), 4.48 (1/3H, d, J = 14.4 Hz), 4.50 (2/3H, d, J = 14.4 Hz), 5.20-5.30 (1H, m), 6.18 (2/3H, s), 6.21 (1/3H, s), 6.62 (2/3H, d, J = 2.4 4 Hz), 6.67 (1/3H, d, J = 2.4 Hz), 6.98-7.03 (1H, m), 7.07 (1H, s), 7.15-7.23 (2H, m), 7.30-7.42 (2H, m).

### Example 118

### 3-(2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]-1-hydroxyethyl}-1,3-thiazol-5-yl)propionic acid

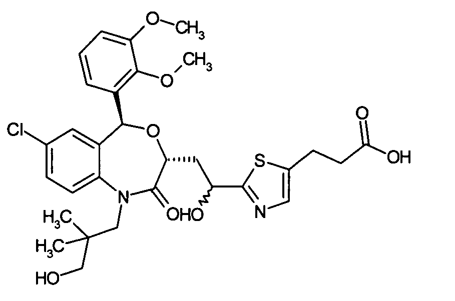

According to a similar manner to that of Example 117, the title compound was obtained.

¹H-NMR (CDCl₃) δ: 0.66 (3H, s), 1.04 (3H, s), 2.13-2.41 (2H, m), 2.50-2.60 (1H, m), 2.61 (2H, t, J = 7.5 Hz), 3.10 (2H, t, J = 7.5 Hz), 3.10-3.10 (1H, m), 3.39 (1/2H, d, J = 13.8 Hz), 3.40 (1/2H, d, J = 13.8 Hz), 3.57-3.64 (1H, m), 3.62 (3H, s), 3.90 (3H, s), 4.19-4.30 (1H, m), 4.466 (1/2H, d, J = 13.8 Hz), 4.474 (1/2H, d, J = 13.8 Hz), 5.01-5.20 (1H, m), 6.17 (1/2H, s), 6.19 (1/2H, s), 6.58-6.63 (1H, m), 6.95-7.02 (1H, m), 7.15-7.30 (2H, m), 7.31-7.43 (3H, m).

### Example 119

### (5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-2-thienyl)acetic acid

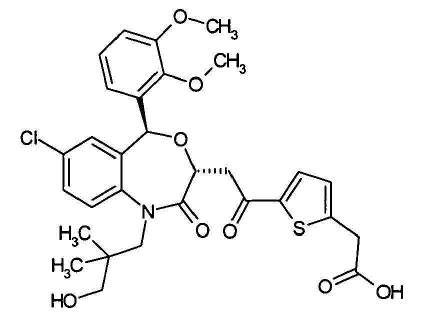

DMF (1 drop) and thionyl chloride (0.42 ml, 5.77 mmol) were added dropwise to a solution of 2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetic acid (2.5 g, 4.81 mmol) in THF (25 ml) at room temperature. The mixture was stirred for 4 hours and then concentrated under reduced pressure. The resulting residue was dissolved in dichloromethane (30 ml). Ethyl 2-thiopheneacetate (0.69 ml, 4.57 mmol) and tin chloride (1.24 ml, 10.6 mmol) were added dropwise under ice-cooling thereto. The mixture was warmed to room temperature and stirred for 13 hours. The reaction solution was poured into ice-water and extracted with dichloromethane (170 ml). The organic layer was washed with 1N aqueous hydrochloric acid, water and an aqueous saturated sodium chloride solution, dried over magnesium sulfate, and then concentrated under reduced pressure. The resulting residue was subjected to silica gel column chromatography [developing solvent: hexane-ethyl acetate (1:1-3:7)] to obtain ethyl (5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}-2-thienyl)acetate as a mixture with impurities. This mixture was dissolved in ethanol (7 ml) and water (1 ml), potassium carbonate (86 mg, 0.63 mmol) was added thereto, and the mixture was then heated to reflux for 2 hours with stirring. After standing to cool, the mixture was acidified with 1N aqueous hydrochloric acid (1.5 ml) and then extracted with ethyl acetate (35 ml). The organic layer was washed with an aqueous saturated sodium chloride solution, dried over magnesium sulfate, and then concentrated under reduced pressure. The resulting residue was purified by preparative liquid chromatography [developing solvent: water-acetonitrile (9:1-5:95)] to obtain (5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-2-thienyl)acetic acid (52 mg, 0.09 mmol, 2% (2 step)) as pale yellow noncrystalline powder.

¹H-NMR (CDCl₃) δ: 0.65 (3H, s), 1.04(3H, s), 3.19 (1H, d, J = 11.7 Hz), 3.28 (1H, dd, J = 4.5, 16.8 Hz), 3.41 (1H, d, J = 14.1 Hz), 3.61 (3H, s), 3.64 (1H, d, J = 11.7 Hz), 3.70 (1H, dd, J = 8.1, 16.8 Hz), 3.89 (5H, s), 4.45 (1H, d, J = 14.1 Hz), 4.58 (1H, dd, J = 4.5, 8.1 Hz), 6.17 (1H, s), 6.62 (1H, s), 6.95-7.10 (2H, m), 7.11-7.20 (2H, m), 7.35-7.41 (2H, m), 7.66 (1H, d, J = 3.6 Hz).

### Example 120

### 4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-tihazol-5-carboxylic acid

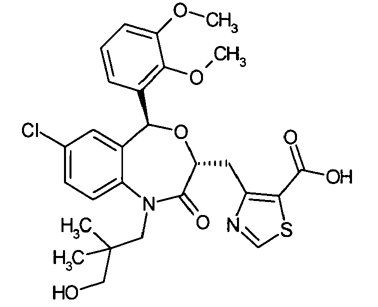

According to a similar manner to that of Example 26, the title compound was obtained.

¹H-NMR (CDCl₃) δ: 0.65 (3H, s), 1.05 (3H, s), 3.15 (1H, d, J = 12.0 Hz), 3.40 (1H, d, J = 14.4 Hz), 3.60 (3H, s), 3.65 (1H, d, J = 12.0 Hz), 3.75 (2H, dd, J = 1.5, 6.6 Hz), 3.88 (3H, s), 4.48 (1H, d, J = 14.4 Hz), 4.55 (1H, t, J = 6.6 Hz), 6.18 (1H, s), 6.55 (1H, s), 6.96 (2H, dd, J = 2.7, 7.8 Hz), 7.13 (1H, t, J = 7.8 Hz), 7.32-7.40 (2H, m), 8.71 (1H, s).

### Example 121

### (2-{3-[(3R,5S)-7-chloro-5-(2,3-diemthoxyphenyl)-1-(3-hydroxy-2,2-dimethylpropyl)-2-oxo-1,2,3,5-tetrahydro-4,1-benzoxazepine-3-yl]propyl}-1,3-thiazol-5-yl)acetic acid

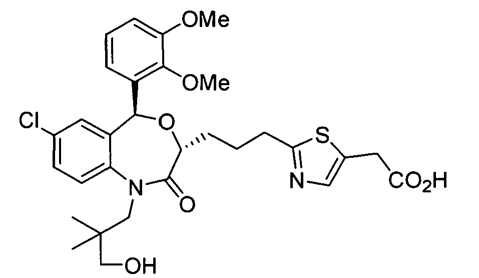

(1) Ethyl (diethoxyphosphoryl)acetate (4.76 ml, 24 mmol) was added dropwise to a suspension of sodium hydride (0,88 g, 22 mmol) in THF (108 ml) at 0°C. After stirring for 30 minutes, 3-[(3R,5S)-7-chloro-5-(2,3-dimethoxyphenyl)-2-oxo-3-(2-oxoethyl)-2,3-dihydro-4,1-benzoxazepin-1(5H)-yl]-2,2-dimethylpropyl acetate (10.8 g, 20 mmol) was added. After stirring for 1 hour, the mixture was diluted with ethyl acetate, washed with an aqueous saturated ammonium chloride solution and an aqueous saturated sodium chloride solution, dried over magnesium sulfate, and then concentrated under reduced pressure. The residue was purified by silica gel column chromatography to obtain ethyl (2E)-4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-2-butenoate (11.5 g, 20 mmol, 100%).
   ¹H-NMR (CDCl₃) δ: 0.95 (3 H, s) 1.03 (3 H, s) 1.28 (3 H, t, J = 7.06 Hz) 2.03 (3 H, s) 2.74 (2 H, m) 3.53 (1 H, d, J = 14.13 Hz) 3.62 (3 H, s) 3.86 (6 H, m) 4.18 (2 H, q, J = 7.16 Hz) 4.56 (1 H, d, J = 14.13 Hz) 5.90 (1 H, d, J = 15.64 Hz) 6.26 (1 H, s) 6.64 (1 H, m, J = 2.26 Hz) 6.95 (2 H, m) 7.27 (4 H, m).
(2) Ethyl (2E)-4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-2-butenoate (15 g, 26.1 mmol) obtained in Example 121-(1) was dissolved in methanol (150 ml), magnesium (3.17 g, 131 mmol) was added thereto, and the mixture was stirred for 3 hours. The reaction solution was diluted with ethyl acetate, washed with 1N aqueous hydrochloric acid, an aqueous saturated sodium chloride solution, dried over anhydrous magnesium sulfate, and then concentrated under reduced pressure. The residue was dissolved in methanol (150 ml), a 1N aqueous sodium hydroxide solution (52 ml) was added, and the mixture was stirred for 2 hours. The reaction solution was concentrated under reduced pressure, and the residue was dissolved in water and washed with ether. The aqueous layer was acidified with 1N aqueous hydrochloric acid and then extracted with ethyl acetate. The organic layer was washed with water, dried over magnesium sulfate, and then concentrated under reduced pressure. The residue was dissolved in methylene chloride (100 ml), pyridine (6.46 ml, 80 mmol) and acetyl chloride (4.98 ml, 70 mmol) were added thereto, and the mixture was stirred for 1 hour. After water (50 ml) was added, the reaction solution was stirred for 4 hours and then extracted with ethyl acetate. The organic layer was washed with an aqueous saturated sodium chloride solution, dried over magnesium sulfate, and then concentrated under reduced pressure. The residue was purified by silica gel column chromatography to obtain 4-[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]butanoic acid (10.9 g, 19.8 mmol, 76%).
   ¹H-NMR (CDCl₃) δ: 0.94 (3 H, s) 1.02 (3 H, s) 1.56-2.00 (4 H, m) 2.03 (3 H, s) 2.37 (2 H, t, J = 7.16 Hz) 3.51 (1 H, d, J = 14.13 Hz) 3.61 (3 H, s) 3.70-3.92 (6 H, m) 4.56 (1 H, d, J = 14.13 Hz) 6.24 (1 H, s) 6.63 (1 H, d, J = 2.26 Hz) 6.98 (1 H, s dd, J = 7.54, 2.26 Hz) 7.15-7.37 (4 H, m) .
(3) Isobutyl carbonate chloride (0.55 ml, 4.20 mmol) was added to a solution of 4-[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]butanoic acid (2.0 g, 3.65 mmol) obtained in Example 121-(2) and triethylamine (0.53 ml, 3.83 mmol) in dimethylacetamide (20 ml) at 0°C, and the mixture was stirred at 0°C for 30 minutes. To the reaction solution at 0°C, 4-amino-3-oxobutanoic acid hydrochloride (0.71 g, 4.02 mmol) obtained in Example 1-(2) was added and pyridine (0.47 ml, 5.84 mmol) was then added dropwise.

After stirring at room temperature for 1 hour, the reaction solution was diluted with ethyl acetate. This was washed with 1N aqueous hydrochloric acid, a 5% aqueous potassium hydrogen sulfate solution, an aqueous saturated sodium hydrogen carbonate solution and an aqueous saturated sodium chloride solution, dried over magnesium sulfate, and then concentrated under reduced pressure. The residue was dissolved in THF (20 ml), Lawesson's reagent (1.8 g, 4.44 mmol) was added thereto, and the mixture was stirred at 70°C for 1 hour. The reaction solution was diluted with ethyl acetate, washed with water and an aqueous saturated sodium chloride solution, dried over magnesium sulfate, and then concentrated under reduced pressure. The residue was dissolved in ethanol (20 ml), a 1N aqueous sodium hydroxide solution (10 ml) was added, and the mixture was stirred for 4 hours. The aqueous layer was acidified with a 5% aqueous potassium hydrogen sulfate solution and then extracted with ethyl acetate. The organic layer was washed with water, dried over magnesium sulfate, and then concentrated under reduced pressure. The residue was purified by silica gel column chromatography to obtain the title compound (1.0 g, 1.66 mmol, 43%).

¹H-NMR (DMSO-d₆) δ: 0.76 (3 H, s) 0.83 (3 H, s) 1.63-1.86 (4 H, m) 2.90 (2 H, t, J = 6.78 Hz) 3.02-3.20 (2 H, m) 3.51 (3 H, s) 3.64 (1 H, d, J = 14.13 Hz) 3.78-3.87 (m, 5 H) 4.31 (1 H, d, J = 14.13 Hz) 4.53 (1 H, t, J = 5.09 Hz) 6.05 (1 H, s) 6.38 (1 H, d, J = 2.45 Hz) 7.15 (2 H, d, J = 7.91 Hz) 7.21-7.28 (1 H, m) 7.41 (1 H, s) 7.51 (1 H, dd, J = 8.76, 2.54 Hz,) 7.66 (1 H, d, J = 8.85 Hz).

### Example 122

### 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid

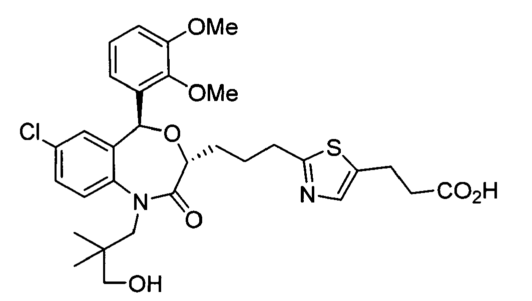

According to a similar manner to that of Example 121, the title compound was obtained.

¹H-NMR (DMSO-d₆) δ: 0.75 (3 H, s) 0.83 (3 H, s) 1.59-1.88 (4 H, m) 2.48-2.59 (2 H, m) 2.83-3.20 (6 H, m) 3.51 (3 H, s) 3.64 (1 H, d, J = 14.13 Hz) 3.78-3.86 (4 H, m) 4.31 (1 H, d, J = 14.13 Hz) 4.53 (1 H, t, J = 5.09 Hz) 6.05 (1 H, s) 6.37 (1 H, d, J = 2.45 Hz) 7.11-7.28 (m, 3 H) 7.51 (1 H, dd, J = 8.67, 2.45 Hz) 7.66 (1 H, d, J = 8.85 Hz).

### Example 123

### (2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepine-3-yl]propyl}-1,3-thiazol-4-yl)acetic acid

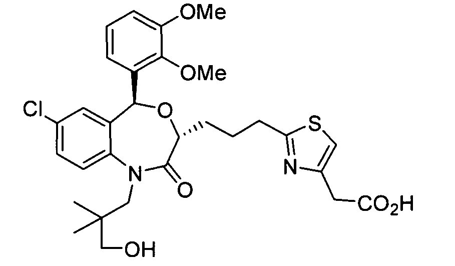

(1) Diethyl cyanomethylphosphonate (3.88 ml, 24 mmol) was added dropwise to a suspension of sodium hydride (0.88 g, 22 mmol) in THF (108 ml) at 0°C. After stirring for 30 minutes, 3-[(3R,5S)-7-chloro-5-(2,3-dimethoxyphenyl)-2-oxo-3-(2-oxoethyl)-2,3-dihydro-4,1-benzoxazepin-1(5H)-yl]-2,2-dimethylpropyl acetate (10.8 g, 20 mmol) was added. After stirring for 1 hour, the mixture was diluted with ethyl acetate, washed with saturated ammonium chloride and an aqueous saturated sodium chloride solution, dried over magnesium sulfate, and then concentrated under reduced pressure. The residue was dissolved in methanol (105 ml), magnesium (2.4 g, 100 mmol) was added, and the mixture was stirred for 2 hours. The reaction solution was diluted with ethyl acetate, washed with 1N aqueous hydrochloric acid and an aqueous saturated sodium chloride solution, dried over anhydrous magnesium sulfate, and then concentrated under reduced pressure. The residue was dissolved in methylene chloride (68 ml), triethylamine (3.69 ml, 26.6 mmol) and acetyl chloride (1.51 ml, 21.2 mmol) were added, and the mixture was stirred for 2 hours. The reaction solution was diluted with ethyl acetate, washed with an aqueous saturated sodium hydrogen carbonate solution and an aqueous saturated sodium chloride solution, dried over magnesium sulfate, and then concentrated under reduced pressure. The residue was purified by silica gel column chromatography to obtain 3-[(3R,5S)-7-chloro-3-(3-cyanopropyl)-5-(2,3-dimethoxyphenyl)-2-oxo-2,3-dihydro-4,1-benzoxazepin-1(5H)-yl]-2,2-dimethylpropyl acetate (5.5 g, 10.4 mmol, 52%).
   ¹H-NMR (CDCl₃) δ: 0.94 (3 H, s) 1.02 (3 H, s) 1.66-2.41 (9 H, m) 3.47-3.93 (10 H, m) 4.56 (1 H, d, J = 14.13 Hz) 6.25 (1 H, s) 6.64 (1 H, d, J = 2.26 Hz) 6.99 (1 H, dd, J = 7.25, 2.35 Hz) 7.14-7.39 (4 H, m).
(2) 3-[(3R,5S)-7-chloro-3-(3-cyanopropyl)-5-(2,3-dimethoxyphenyl)-2-oxo-2,3-dihydro-4,1-benzoxazepin-1(5H)-yl]-2,2-dimethylpropyl acetate (5.3 g, 10 mmol) obtained in Example 123-(1) was dissolved in a 4N hydrochloric acid-ethyl acetate solution (53 ml). Thereto O,O'-diethyl hydrogen dithiophosphate (2.01 ml, 12 mmol) was added and the mixture was stirred overnight. The reaction solution was washed with water, dried over magnesium sulfate, and then concentrated under reduced pressure. The residue was purified by silica gel column chromatography to obtain 2-[(3R,5S)-3-(4-amino-4-thioxobutyl)-7-chloro-5-(2,3-dimethoxyphenyl)-2-oxo-2,3-dihydro-4,1-benzoxazepin-1(5H)-yl]-1,1-dimethylethyl acetate (2.8 g, 4.97 mmol, 50%).
   MS (ES+) [M+1] 563
(3) 2-[(3R,5S)-3-(4-amino-4-thioxobutyl)-7-chloro-5-(2,3-dimethoxyphenyl)-2-oxo-2,3-dihydro-4,1-benzoxazepin-1(5H)-yl]-1,1-dimethylethyl acetate (0.563 g, 1.0 mmol) obtained in Example 123-(2) was dissolved in ethanol (10 ml). Thereto ethyl 4-chloro-3-oxobutanoate (0.243 ml, 1.8 mmol) was added and the mixture was stirred at 90°C for 2 hours. After the reaction solution was cooled to room temperature, a 1N aqueous sodium hydroxide solution (5 ml) was added thereto and the mixture was stirred for 5 hours. The aqueous layer was acidified with a 5% aqueous potassium hydrogen sulfate and then extracted with ethyl acetate. The organic layer was washed with water, dried over magnesium sulfate, and then concentrated under reduced pressure. The residue was purified by silica gel column chromatography to obtain the title compound (0.3 g, 0.498 mmol, 50%).
   ¹H-NMR (DMSO-d₆) δ: 0.76 (3 H, s) 0.83 (3 H, s) 1.63-1.86 (4 H, m) 2.90 (2 H, t, J = 6.78 Hz,) 3.02-3.20 (2 H, m) 3.51 (3 H, s) 3.64 (1 H, d, J = 14.13 Hz) 3.78-3.87 (5 H, m) 4.31 (1 H, d, J = 14.13 Hz) 4.53 (1 H, t, J = 5.09 Hz) 6.05 (1 H, s) 6.38 (1 H, d, J = 2.45 Hz) 7.15 (2 H, d, J = 7.91 Hz) 7.21-7.28 (1 H, m) 7.41 (1 H, s) 7.51 (1 H, dd, J = 8.76, 2.54 Hz) 7.66 (1 H, d, J = 8.85 Hz).

### Example 124

### 2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazole-4-carboxylic acid

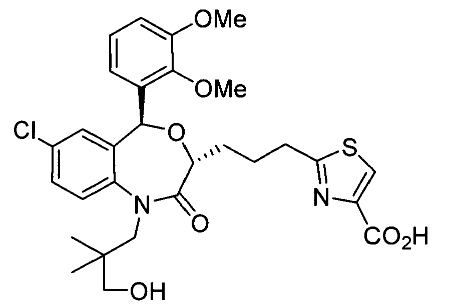

According to a similar manner to that of Example 123, the title compound was obtained.

¹H-NMR (DMSO-d₆) δ: 0.75 (3 H, s) 0.83 (3 H, s) 1.67-1.89 (4 H, m) 2.94-3.19 (4 H, m) 3.51 (3 H, s) 3.64 (1 H, d, J = 13.94 Hz) 3.78-3.88 (4 H, m) 4.31 (1 H, d, J = 14.13 Hz) 4.52 (1 H, t, J = 5.27 Hz) 6.05 (1 H, s) 6.37 (1 H, d, J = 2.45 Hz) 7.12-7.28 (3 H, m) 7.50 (1 H, dd, J = 8.76, 2.54 Hz,) 7.66 (1 H, d, J = 8.85 Hz).

### Example 125

### 3-(2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-4-methyl-1,3-thiazol-5-yl)propionic acid

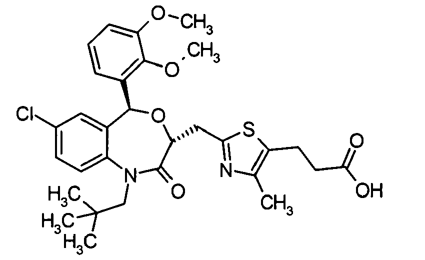

According to a similar manner to that of Example 1, the title compound was obtained.

¹H-NMR (CDCl₃) δ: 0.95 (9H, s), 2.28 (3H, s), 2.64 (2H, t, J = 7.6 Hz), 3.02 (2H, t, J = 7.6 Hz), 3.31-3.44 (2H, m), 3.52 (1H, dd, J = 15.0, 6.0 Hz), 3.63 (3H, s), 3.89 (3H, s), 4.24-4.30 (1H, m), 4.51 (1H, d, J = 13.8 Hz), 6.31 (1H, s), 6.57 (1H, d, J = 2.1 Hz), 6.97 (1H, dd, J = 6.6, 3.2 Hz), 7.14-7.22 (2H, m), 7.24-7.33 (2H, m).

### Example 126

### 2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-4-methyl-1,3-thiazole-5-carboxylic acid

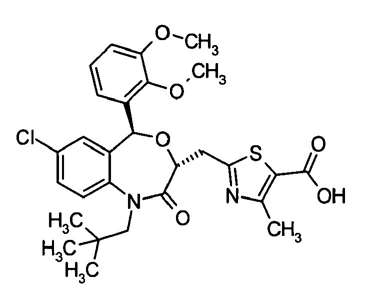

According to a similar manner to that of Example 16, the title compound was obtained.

¹H-NMR (CDCl₃) δ: 0.95 (9H, s), 2.68 (3H, s), 3.36 (1H, d, J = 13.9 Hz), 3.47 (1H, dd, J = 15.0, 6.9 Hz), 3.55-3.66 (4H, m), 3.89 (3H, s), 4.32 (1H, t, J = 6.4 Hz), 4.52 (1H, d, J = 13.9 Hz), 6.33 (1H, s), 6.59 (1H, d, J = 2.1 Hz), 6.99 (1H, dd, J = 7.5, 2.3 Hz), 7.13-7.23 (2H, m), 7.28-7.35 (2H, m).

### Example 127

### 2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,3-thiazole-4-carboxylic acid

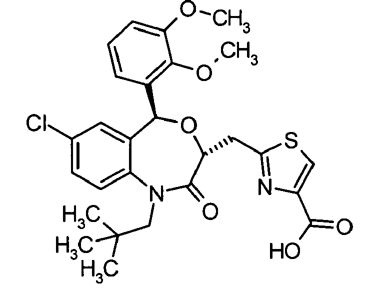

According to a similar manner to that of Example 22, the title compound was obtained.

¹H-NMR (CDCl₃) δ: 0.95 (9H, s), 3.35 (1H, d, J = 13.9 Hz), 3.50-3.69 (5H, m), 3.89 (3H, s), 4.34 (1H, t, J = 6.4 Hz), 4.50 (1H, d, J = 13.9 Hz), 6.33 (1H, s), 6.58 (1H, d, J = 2.1 Hz), 6.99 (1H, dd, J = 8.0, 1.4 Hz), 7.07-7.12 (1H, m), 7.18 (1H, t, J = 7.9 Hz), 7.25-7.35 (2H, m), 8.19 (1H, s) .

### Example 128

### (2-{[(3R,5S)-7-chloro-5-(2,3-dimethoxyphenyl)-1-(2,2-dimethylpropyl)-2-oxo-1,2,3,5-tetrahdyro-4,1-benzoxazepin-3-yl]methyl}-1,3-thiazol-4-yl)acetic acid

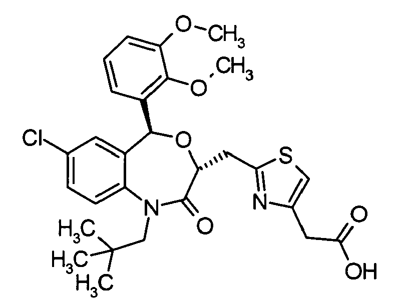

According to a similar manner to that of Example 16, the title compound was obtained.

¹H-NMR (CDCl₃) δ: 0.95 (9H, s), 3.38 (1H, d, J = 13. 9 Hz), 3.48 (1H, dd, J = 15.3, 6.6 Hz), 3.60-3.72 (4H, m), 3.79 (2H, s), 3.89 (3H, s), 4.34 (1H, t, J = 6.5 Hz), 4.49 (1H, d, J = 13.9 Hz), 6.33 (1H, s), 6.60 (1H, s), 6.99 (1H, dd, J = 6.9, 2.9 Hz), 7.02 (1H, s), 7.13-7.23 (2H, m), 7.33 (2H, s).

### Example 129

### 3-(2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-5-methyl-1,3-thiazol-4-yl)propionic acid

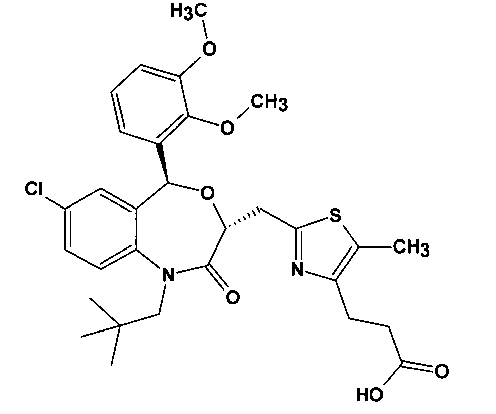

According to a similar manner to that of Example 20, the title compound was obtained.

¹H-NMR (CDCl₃) δ: 0.95 (9H, s), 2.32 (3H, s), 2.66-2.73 (2H, m), 2.82-2.90 (2H, m), 3.33-3.44 (2H, m), 3.58 (1H, dd, J = 16.2, 6.6 Hz), 3.63 (3H, s), 3.89 (3H, s), 4.27 (1H, t, J = 6.4 Hz), 4.49 (1H, d, J = 13.9 Hz), 6.31 (1H, s), 6.60 (1H, s), 6.99 (1H, dd, J = 5.8, 3.8 Hz), 7.15-7.23 (2H, m), 7.31-7.39 (2H, m, J = 1.9 Hz).

### Example 130

### 2-{2-[(3R,5S)-7-chloro-5-(2.3-dimethoxyphenyl)-1-(2,2-dimethylpropyl)-2-oxo-1,2,3-5-tetrahydro-4,1-benzoxazepin-3-yl]ethyl}-1,3-thiazole-4-carboxylic acid

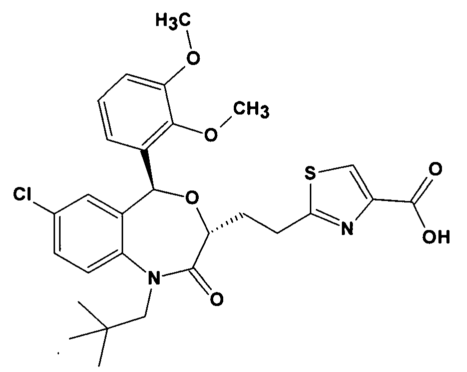

According to a similar manner to that of Example 22, the title compound was obtained.

¹H-NMR (CDCl₃) δ: 0.95 (9H, s), 2.22-2.49 (2H, m), 3.08-3.32 (2H, m), 3.35 (1H, d, J = 13.9 Hz), 3.63 (3H, s), 3.90 (3H, s), 3.94 (1H, dd, J = 7.5, 5.3 Hz), 4.50 (1H, d, J = 13.8 Hz), 6.28 (1H, s), 6.61 (1H, d, J = 2.3 Hz), 6.95-7.03 (1H, m), 7.16-7.24 (2H, m), 7.25-7.37 (2H, m), 8.11 (1H, s).

### Example 131

### 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid

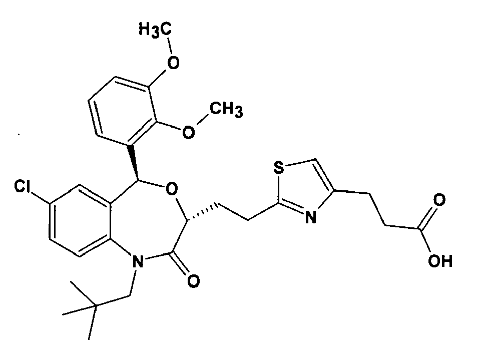

According to a similar manner to that of Example 88, the title compound was obtained.

¹H-NMR (CDCl₃) δ: 0.95 (9H, s), 2.18-2.44 (2H, m), 2.64-2.74 (2H, m), 2.94-3.03 (2H, m), 3.04-3.27 (2H, m), 3.36 (1H, d, J = 13.9 Hz), 3.63 (3H, s), 3.89 (3H, s), 3.94 (1H, dd, J = 7.3, 5.7 Hz), 4.52 (1H, d, J = 13.9 Hz), 6.28 (1H, s), 6.60 (1H, d, J = 2.1 Hz), 6.80 (1H, s), 6.99 (1H, dd, J = 7.0, 2.6 Hz), 7.16-7.36 (4H, m).

### Example 132

### 3-(2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-4-methyl-1,3-oxazol-5-yl)propionic acid

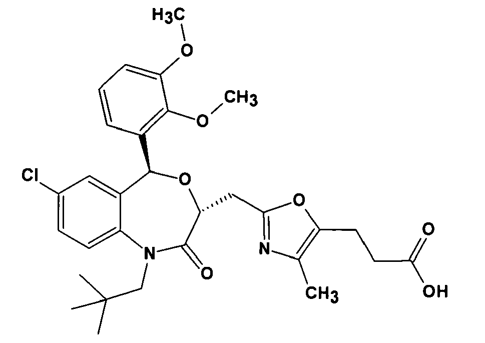

According to a similar manner to that of Example 29, the title compound was obtained.

¹H-NMR (CDCl₃) δ: 0. 94 (9H, s), 2. 05 (3H, s), 2. 63 (2H, t), 2.88 (2H, t, J = 7.4 Hz), 3.16-3.32 (2H, m), 3.36 (1H, d, J = 13.8 Hz), 3.62 (3H, s), 3.88 (3H, s), 4.38 (1H, t, J = 6.8 Hz), 4.51 (1H, d, J = 13.9 Hz), 6.30 (1H, s), 6.57 (1H, d, J = 1.5 Hz), 6.96 (1H, dd, J = 8.1, 1.3 Hz), 7.03 (1H, dd, J = 7.8, 1.2 Hz), 7.16 (1H, t, J = 7.9 Hz), 7.27-7.36 (2H, m).

### Example 133

### 3-(2-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-5-methyl-1,3-oxazol-4-yl)propionic acid

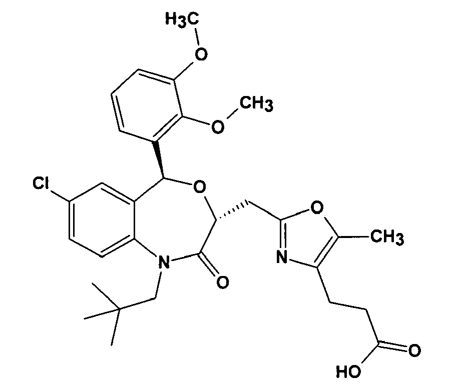

According to a similar manner to that of Example 38, the title compound was obtained.

¹H-NMR (CDCl₃) δ: 0.94 (9H, s), 2.22 (3H, s), 2.59-2.72 (4H, m), 3.17 (1H, dd, J = 16.0, 6.4 Hz), 3.31-3.45 (2H, m), 3.63 (3H, s), 3.89 (3H, s), 4.40 (1H, t, J = 6.8 Hz), 4.48 (1H, d, J = 13.9 Hz), 6.30 (1H, s), 6.59 (1H, d, J = 2.1 Hz), 6.97 (1H, dd, J = 7.9, 1.5 Hz), 7.08 (1H, dd, J = 7.7, 1.5 Hz), 7.16 (1H, t, J = 7.9 Hz), 7.32-7.42 (2H, m) .

### Example 134

### 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)propionic acid

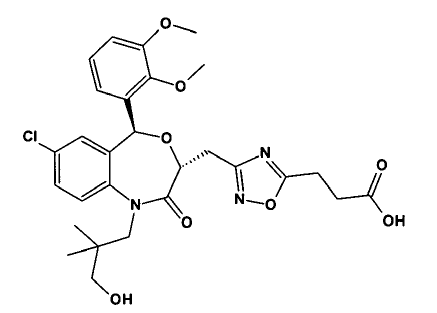

According to a similar manner to that of Example 60, the title compound was obtained.

¹H-NMR (CDCl₃) δ: 0.65 (3H, s), 1.04 4 (3H, s), 2.89 (2H, t, J = 7.2 Hz), 3.11-3.27 (4H, m), 3.34-3.44 (2H, m), 3.58-3.67 (4H, m), 3.89 (3H, s), 4.39-4.51 (2H, m), 6.19 (1H, s), 6.60 (1H, d, J = 1.7 Hz), 6.98 (1H, dd, J = 8.0, 1.6 Hz), 7.07-7.12 (1H, m), 7.18 (1H, t, J = 7.9 Hz), 7.32-7.41 (2H, m) .

### Example 135

### 4-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)butanoic acid

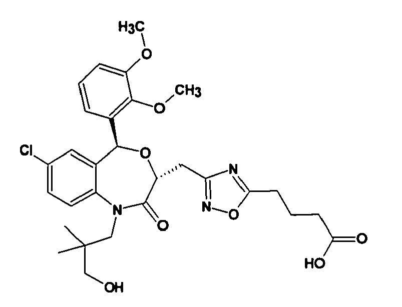

According to a similar manner to that of Example 60, the title compound was obtained.

¹H-NMR (CDCl₃) δ: 0.65 (3H, s), 1.05 (3H, s), 2.08-2.19 (3H, m), 2.49 (2H, t, J = 7.2 Hz), 2.95 (2H, t, J = 7.3 Hz), 3.12-3.28 (2H, m), 3.34-3.44 (2H, m), 3.59-3.67 (4H, m), 3.89 (3H, s), 4.41-4.52 (2H, m), 6.20 (1H, s), 6.60 (1H, d, J = 1.5 Hz), 6.98 (1H, dd, J = 8.0, 1.6 Hz), 7.09 (1H, dd, J = 7.8, 1.4 Hz), 7.17 (1H, t, J = 7.9 Hz), 7.35-7.38 (2H, m).

### Example 136

### 5-(3-([(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid

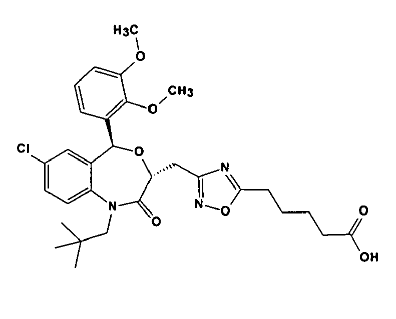

(1) 1-Ethyl-3-(3-dimethylaminopropyl)carbodiimide hydrochloride (23.0 g, 0.120 mol) and ammonium 1H-1,2,3-benzotriazol-1-olate (18.26 g, 0.120 mol) were added to a solution of [(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetic acid (46.19 g, 0.100 mol) in DMF (500 ml) and the mixture was stirred for 20 hours. The reaction solution was concentrated under reduced pressure, extracted with ethyl acetate, washed with water and an aqueous saturated sodium chloride solution, and then dried over magnesium sulfate. After concentration under reduced pressure, the residue was purified by silica gel column chromatography and then recrystallized from ethyl acetate-hexane to obtain 2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetamide (32.43 g) as a crystal.
   ¹H-NMR (CDCl₃) δ: 0.95 (9 H, s), 2.65 - 2.73 (1 H, m), 2.84 - 2.92 (1 H, m), 3.37 (1 H, d, J = 13.8 Hz), 3.62 (3 H, s), 3.89 (3 H, s), 4.39 (1 H, dd, J = 7.3, 5.9 Hz), 4.50 (1 H, d, J = 13.9 Hz), 5.27 (1 H, s), 5.86 (1 H, s), 6.28 (1 H, s), 6.61 (1 H, d, J = 2.1 Hz), 6.99 (1 H, dd, J = 6.7, 3.1 Hz), 7.18 - 7.23 (2 H, m), 7.31 - 7.36 (2 H, m).
(2) 1-1'-Carbonylbis-1H-imidazole (9.85 g, 0.0607 mol) and allyl bromide (20.7 ml, 0.243 mol) were added to a solution of 2-[(3R,5S)-7-chloro-5-(2,3-dimethoxyphenyl)-1-(2,2-dimethylpropyl)-2-oxo-1,2,3,5-tetrahydro-4,1-benzoxazepine-3-yl]acetamide (14.0 g, 0.0304 mol) obtained in Example 136-(1) in acetonitrile (140 ml), and the mixture was heated to reflux for 3 hours. After standing to cool, the reaction solution was extracted with ethyl acetate, washed with 1N aqueous hydrochloric acid and an aqueous saturated sodium chloride solution, and then dried over magnesium sulfate. The extract was filtered with silica gel and concentrated under reduced pressure. The residue was recrystallized from ethyl acetate-hexane to obtain [(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetonitrile (12.31 g).
   ¹H-NMR (CDCl₃) δ: 0. 95 (9 H, s), 2.78 - 2.88 (1 H, m), 2.89 - 2.99 (1 H, m), 3.38 (1 H, d, J = 13.9 Hz), 3.63 (3 H, s), 3.90 (3 H, s), 4.21 (1 H, dd, J = 7.6, 5.6 Hz), 4.51 (1 H, d, J = 13.9 Hz), 6.30 (1 H, s), 6.65 (1 H, d, J = 2.1 Hz), 7.01 (1 H, d, J = 7.9 Hz), 7.19 - 7.41 (4 H, m).
(3) Triethylamine (23.66 ml, 0.169 mol) was added dropwise to a solution of hydroxylamine hydrochloride (11.77 g, 0.169 mol) in DMSO (150 ml) under ice-cooling and the mixture was stirred for 30 minutes. Precipitates were filtered off. To the filtrate was added [(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetonitrile (15.0 g, 0.0339 mol) obtained in Example 136-(2) and the mixture was stirred at 80°C for 4 hours. The reaction solution was extracted with ethyl acetate, washed with water and an aqueous saturated sodium chloride solution, dried over magnesium sulfate, and then concentrated under reduced pressure to obtain 2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]-N'-hydroxyethaneimidoamide (17.5 g) as an amorphous substance, which was used in the next reaction without purification.
   ¹H-NMR (CDCl₃) δ: 0.95 (9 H, s), 2.56 - 2.64 (1 H, m), 2.79 (1 H, dd, J = 14.4, 7.1 Hz), 3.36 (1 H, d, J = 13.9 Hz), 3.60 - 3.65 (3 H, m), 3.89 (3 H, s), 4.07 - 4.14 (1 H, m), 4.50 (1 H, d, J = 13.9 Hz), 4.98 (2 H, s), 6.25 - 6.29 (1 H, m), 6.60 (1 H, d, J = 2.1 Hz), 6.99 (1 H, dd, J = 5.9, 3.9 Hz), 7.16 - 7.23 (2 H, m), 7.29 - 7.35 (2 H, m).
(4) Triethylamine (0.83 ml, 5.95 mmol) and methyl 6-chloro-6-oxohexanoate (0.69 g, 3.85 mmol) were added dropwise to a solution of 2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]-N'-hydroxyethaneimidoamide (1.67 g, 3.5 mmol) obtained in Example 136-(3) in THF (15 ml) under ice-cooling, and the mixture was stirred at room temperature for 2.5 hours. The reaction solution was concentrated under reduced pressure, water (15 ml) was added thereto, and the mixture was heated to reflux for 11 hours. The reaction solution was extracted with ethyl acetate, washed with water and an aqueous saturated sodium chloride solution, and dried over magnesium sulfate. After concentration under reduced pressure, the residue was purified by silica gel column chromatography to obtain methyl 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoate (1.04 g).
   ¹H-NMR (CDCl₃) δ: 0.95 (9 H, s), 1.63 - 1.91 (4 H, m), 2.36 (2 H, t, J = 7.2 Hz), 2.87 (2 H, t, J = 7.4 Hz), 3.20 - 3.42 (3 H, m), 3.62 (3 H, s), 3.67 (3 H, s), 3.88 (3 H, s), 4.43 (1 H, t, J = 6.8 Hz), 4.51 (1 H, d, J = 13.9 Hz), 6.31 (1 H, s), 6.58 (1 H, s), 6.97 (1 H, dd, J = 8.0, 1.6 Hz), 7.07 (1 H, dd, J = 7.7, 1.5 Hz), 7.15 (1 H, t, J = 7.9 Hz), 7.29 - 7.37 (2 H, m).
(5) A 2N aqueous sodium hydroxide solution (2.60 ml, 5.20 mmol) was added to a solution of methyl 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoate (1.04 g, 1.73 mmol) obtained in Example 136-(4) in ethanol (10 ml) and the mixture was stirred for 13 hours. The reaction solution was diluted with water and then washed with diethyl ether. The extract solution was acidified with 6N aqueous hydrochloric acid, extracted with ethyl acetate, washed with an aqueous saturated sodium chloride solution, dried over magnesium sulfate, and then concentrated under reduced pressure. The residue was purified by silica gel column chromatography to obtain 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid (0.89 g).
   ¹H-NMR (CDCl₃) δ: 0.95 (9H, s), 1.66-1.93 (4H, m), 2.39 (2H, t, J = 7.3 Hz), 2.88 (2H, t, J = 7.4 Hz), 3.19-3.41 (3H, m), 3.62 (3H, s), 3.88 (3H, s), 4.43 (1H, t, J = 6.8 Hz), 4.51 (1H, d, J = 13.9 Hz), 6.31 (1H, s), 6.58 (1H, d, J = 1.5 Hz), 6.96 (1H, dd, J = 7.9, 1.7 Hz), 7.07 (1H, dd, J = 7.8, 1.6 Hz), 7.14 (1H, t, J = 7.8 Hz), 7.28-7.37 (2H, m).

### Example 137

### 3-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)propionic acid

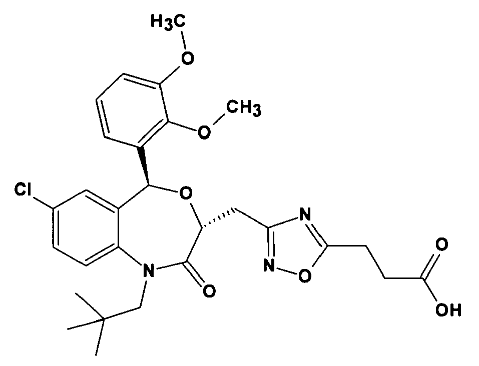

According to a similar manner to that of Example 136, the title compound was obtained.

¹H-NMR (CDCl₃) δ: 0.95 (9H, s), 2.90 (2H, t, J = 7.3 Hz), 3.16 (2H, t, J = 7.3 Hz), 3.21-3.40 (3H, m), 3.62 (3H, s), 3.88 (3H, s), 4.42 (1H, t, J = 6.7 Hz), 4.51 (1H, d, J = 13.9 Hz), 6.31 (1H, s), 6.59 (1H, d, J = 1.5 Hz), 6.97 (1H, dd, J = 8.0, 1.6 Hz), 7.06-7.11 (1H, m), 7.16 (1H, t, J = 7.9 Hz), 7.29-7.37 (2H, m).

### Example 138

### 4-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)butanoic acid

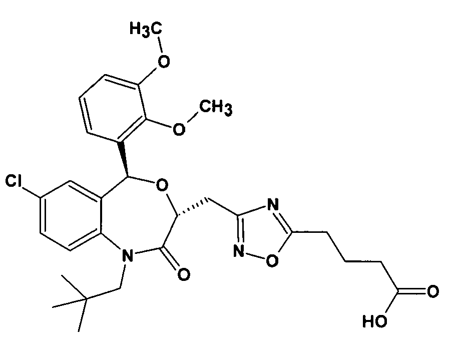

According to a similar manner to that of Example 136, the title compound was obtained.

¹H-NMR (CDCl₃) δ: 0.95 (9H, s), 2.08-2.20 (2H, m), 2.49 (2H, t, J = 7.1 Hz), 2.95 (2H, t, J = 7.3 Hz), 3.19-3.42 (3H, m), 3.62 (3H, s), 3.88 (3H, s), 4.44 (1H, t, J = 6.8 Hz), 4.51 (1H, d, J = 13.9 Hz), 6.31 (1H, s), 6.59 (1H, s), 6.97 (1H, dd, J = 8.0, 1.8 Hz), 7.08 (1H, dd, J = 7.8, 1.4 Hz), 7.16 (1H, t, J = 7.9 Hz), 7.30-7.36 (2H, m).

### Example 139

### 3-(3-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl)-1,2,4-oxadiazol-5-yl)propionic acid

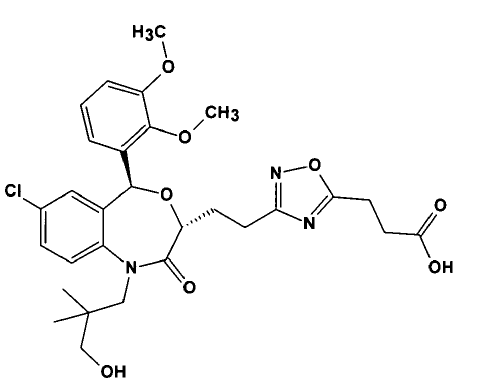

According to a similar manner to that of Example 60, the title compound was obtained.

¹H-NMR (CDCl₃) δ: 0.64 (3H, s), 1.05 (3H, s), 2.15-2.41 (2H, m), 2.77-2.98 (4H, m), 3.07-3.20 (3H, m), 3.37 (1H, d, J = 14.3 Hz), 3.59-3.66 (4H, m), 3.89 (3H, s), 3.95 (1H, dd, J = 7.7, 5.3 Hz), 4.46 (1H, d, J = 14.3 Hz), 6.15 (1H, s), 6.60 (1H, d, J = 2.3 Hz), 6.95-7.02 (1H, m), 7.17-7.22 (2H, m, J = 4.1, 4.1 Hz), 7.29-7.39 (2H, m).

### Example 140

### 3-(3-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,2,4-oxadiazol-5-yl)propionic acid

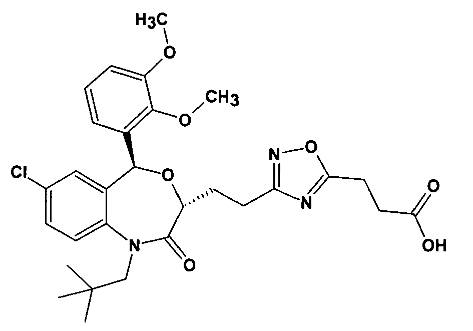

According to a similar manner to that of Example 136, the title compound was obtained.

¹H-NMR (CDCl₃) δ: 0.94 (9H, s), 2.14-2.41 (2H, m), 2.77-2.98 (4H, m), 3.11 (2H, t, J = 7.3 Hz), 3.34 (1H, d, J = 13.9 Hz), 3.63 (3H, s), 3.89 (3H, s), 3.93 (1H, dd, J = 7.9, 5.1 Hz), 4.51 (1H, d, J = 13.8 Hz), 6.27 (1H, s), 6.60 (1H, d, J = 2.1 Hz), 6.98 (1H, dd, J = 7.0, 2.6 Hz), 7.15-7.24 (2H, m), 7.28-7.36 (2H, m).

### Example 141

### 4-(3-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,2,4-oxadiazol-5-yl)butanoic acid

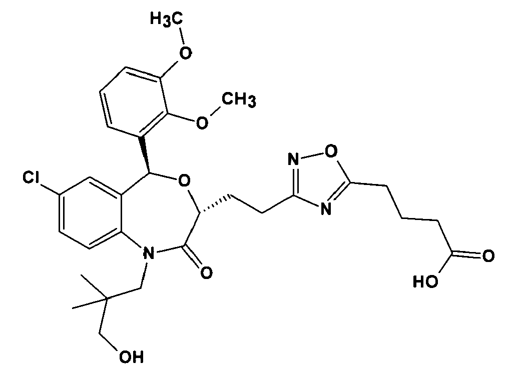

According to a similar manner to that of Example 60, the title compound was obtained.

¹H-NMR (CDCl₃) δ: 0.64 (3H, s), 1.05 (3H, s), 2.02-2.14 (3H, m), 2.18-2.42 (2H, m), 2.45 (2H, t, J = 7.2 Hz), 2.77-2.99 (4H, m), 3.15 (1H, d, J = 12.1 Hz), 3.37 (1H, d, J = 14.3 Hz), 3.58-3.66 (4H, m), 3.89 (3H, s), 3.95 (1H, dd, J = 7.8, 5.4 Hz), 4.46 (1H, d, J = 14.3 Hz), 6.15 (1H, s), 6.60 (1H, d, J = 2.3 Hz), 6.95-7.03 (1H, m), 7.16-7.23 (2H, m), 7.29-7.39 (2H, m).

### Example 142

### 3-(3-{3-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]propyl}-1,2,4-oxadiazol-5-yl)propionic acid

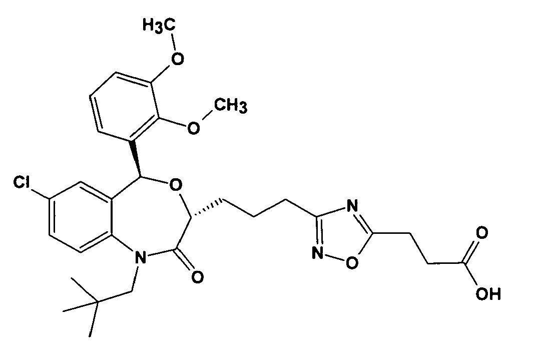

According to a similar manner to that of Example 136, the title compound was obtained.

¹H-NMR (CDCl₃) δ: 0.94 (9H, s), 1.70-2.03 (4H, m), 2.66-2.78 (2H, m), 2.90 (2H, t, J = 7.2 Hz), 3.15 (2H, t, J = 7.2 Hz), 3.34 (1H, d, J = 13.9 Hz), 3.62 (3H, s), 3.85 (1H, dd, J = 7.5, 4.8 Hz), 3.89 (3H, s), 4.50 (1H, d, J = 13.9 Hz), 6.24 (1H, s), 6.59 (1H, d, J = 2.3 Hz), 6.98 (1H, dd, J = 7.7, 2.1 Hz), 7.15-7.36 (4H, m).

### Example 143

### (3R)-4-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)-3-methylbutanoic acid

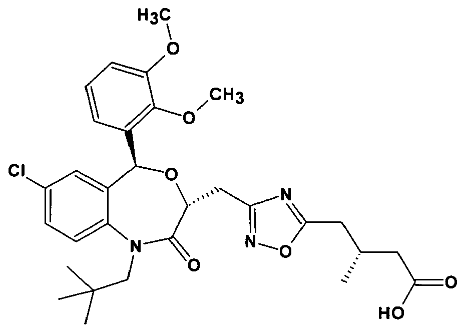

According to a similar manner to that of Example 136, the title compound was obtained.

¹H-NMR (CDCl₃) δ: 0.95 (9H, s), 1.07 (3H, d, J = 6.6 Hz), 2.33 (1H, dd, J = 15.6, 7.2 Hz), 2.41-2.63 (2H, m), 2.84 (1H, dd, J = 15.3, 6.9 Hz), 2.96 (1H, dd, J = 15.3, 6.6 Hz), 3.21-3.42 (3H, m), 3.62 (3H, s), 3.88 (3H, s), 4.45 (1H, t, J = 6.8 Hz), 4.51 (1H, d, J = 13.9 Hz), 6.31 (1H, s), 6.58 (1H, s), 6.96 (1H, dd, J = 7.9, 1.7 Hz), 7.06 (1H, dd, J = 7.9, 1.5 Hz), 7.14 (1H, t, J = 7.9 Hz), 7.29-7.37 (2H, m).

### Example 144

### {[(3-{[(3R,5S)-7-chloro-5-(2,3-dimethoxyphenyl)-1-(2,2-dimethylpropyl)-2-oxo-1,2,3-5-tetrahydro-4,1-benzoxazepin-3-yl]methyl}-1,2,4-oxadiazol-5-yl)methyl]thio}acetic acid

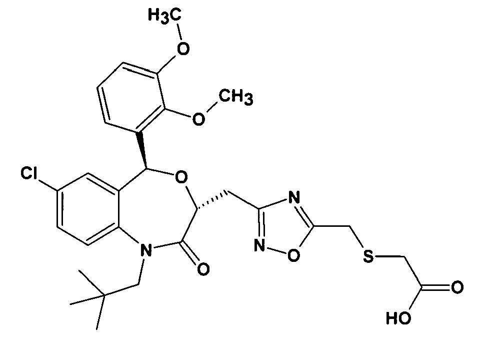

(1) Potassium thioacetate (0.76 g, 5.97 mmol) was added to a solution of (3R,5S)-7-chloro-3-{[5-(chloromethyl)-1,2,4-oxadiazol-3-yl]methyl}-5-(2,3-dimethoxyphenyl)-1-(2,2-dimethylpropyl)-1,5-dihydro-4,1-benzoxazepin-2(3H)-one (3.8 g, 4.98 mmol) synthesized as in Example 136 in DMF (30 ml), and the mixture was stirred at room temperature for 12 hours. The reaction solution was extracted with ethyl acetate, washed with water and an aqueous saturated sodium chloride solution, dried over magnesium sulfate, and then concentrated under reduced pressure. The residue was purified by silica gel column chromatography [developing solvent: hexane-ethyl acetate (4:1)] to obtain S-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)methyl] acetate (2.45 g, 4.26 mmol, 86%) as white noncrystalline powder.
   ¹H-NMR (CDCl₃) δ: 0. 95 (9H, s), 2.40 (3H, s), 3.30 (2H, dd, J = 6.7, 2.4 Hz), 3.37 (1H, d, J = 13.9 Hz), 3.62 (3H, s), 3.88 (3H, s), 4.28 (2H, s), 4.42 (1H, t, J = 6.8 Hz), 4.51 (1H, d, J = 13.8 Hz), 6.30 (1H, s), 6.58 (1H, d, J = 1.9 Hz), 6.97 (1H, dd, J = 8.1, 1.5 Hz), 7.05 (1H, dd, J = 7.8, 1.0 Hz), 7.17 (1H, t, J = 8.0 Hz), 7.28-7.37 (2H, m).
(2) A 2N aqueous sodium hydroxide solution (1.74 ml, 3.48 mmol) was added to a solution of S-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)methyl] acetate (1.0 g, 1.74 mmol) in methanol (10 ml), and the mixture was stirred for 1.5 hours. The reaction solution was extracted with ethyl acetate, washed with water and an aqueous saturated sodium chloride solution, dried over magnesium sulfate, and then concentrated under reduced pressure. Ethyl bromoacetate (0.12 ml, 1.04 mmol) and potassium carbonate (0.24 g, 1.74 mmol) were added to a solution of the residue in THF (10 ml), and the mixture was stirred of 17 hours. The reaction solution was extracted with ethyl acetate, washed with water and an aqueous saturated sodium chloride solution, dried over magnesium sulfate, and then concentrated under reduced pressure. The residue was dissolved in ethanol (10 ml), a 2N aqueous sodium hydroxide solution (1.16 ml, 2.33 mmol) was added, and the mixture was stirred for 5 hours. The reaction solution was diluted with water and then washed with diethyl ether. The extract was acidified with 1N aqueous hydrochloric acid, extracted with ethyl acetate, washed with an aqueous saturated sodium chloride solution, dried over magnesium sulfate, and then concentrated under reduced pressure to obtain {[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)methyl]thio}acetic acid (413 mg, 80%).
   ¹H-NMR (CDCl₃) δ: 0.95 (9H, s), 3.26 (1H, dd, J = 15 . 7 , 6.7 Hz), 3.34-3.44 (4H, m), 3.63 (3H, s), 3.89 (3H, s), 4.00 (2H, s), 4.44-4.54 (2H, m), 6.31 (1H, s), 6.59 (1H, s), 6.97 (1H, dd, J = 8.0, 1.6 Hz), 7.07 (1H, dd, J = 7.9, 1.5 Hz), 7.16 (1H, t, J = 8.0 Hz), 7.34 (2H, d, J = 1.3 Hz).

### Example 145

### 2-{[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}1,2,4-oxadiazol-5-yl)methyl]thio}-2-methylpropionic acid

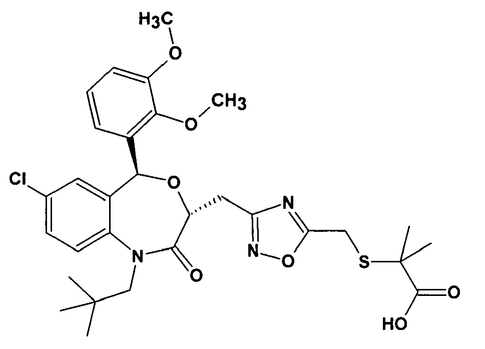

According to a similar manner to that of Example 144, the title compound was obtained.

¹H-NMR (CDCl₃) δ: 0.94 (9H, s), 1.54 (3H, s), 1.56 (3H, s), 3.20 (1H, dd, J = 15.4, 6.2 Hz), 3.33-3.47 (2H, m), 3.63 (3H, s), 3.89 (3H, s), 4.01 (2H, s), 4.44-4.53 (2H, m), 6.30 (1H, s), 6.60 (1H, s), 6.98 (1H, dd, J = 7.8, 1.8 Hz), 7.12 (1H, dd, J = 7.7, 1.7 Hz), 7.18 (1H, t, J = 7.8 Hz), 7.34 (2H, d, J = 0.9 Hz).

### Example 146

### {[1-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)-1-methylethyl]thio}acetic acid

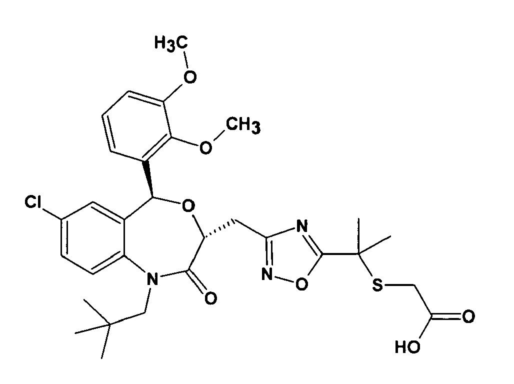

According to a similar manner to that of Example 144, the title compound was obtained.

¹H-NMR (CDCl₃) δ: 0.94 (9H, s), 1.78 (3H, s), 1.81 (3H, s), 3.15 (1H, dd, J = 15.8, 5.3 Hz), 3.28-3.53 (4H, m), 3.64 (3H, s), 3.89 (3H, s), 4.47 (1H, d, J = 13.9 Hz), 4.53 (1H, dd, J = 8.7, 5.3 Hz), 6.30 (1H, s), 6.62 (1H, s), 6.98 (1H, dd, J = 7.0, 2.8 Hz), 7.13-7.22 (2H, m), 7.36 (2H, d, J = 1.3 Hz).

### Example 147

### {[1-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)-1-methylethyl]thio}acetic acid

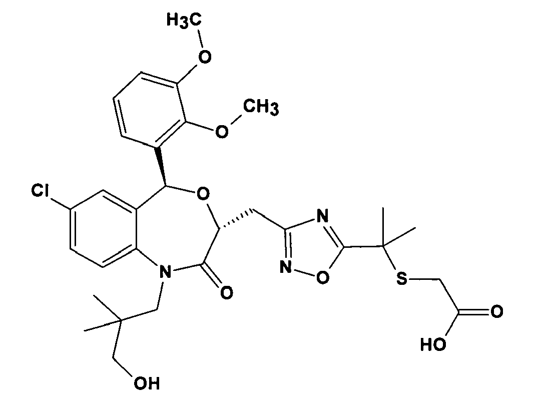

According to a similar manner to that of Example 144, the title compound was obtained.

¹H-NMR (CDCl₃) δ: 0.67 (3H, s), 1.04 (3H, s), 1.78 (3H, s), 1.79 (3H, s), 3.16-3.27 (2H, m), 3.35-3.49 (4H, m), 3.59-3.67 (4H, m), 3.89 (3H, s), 4.41-4.53 (2H, m), 6.19 (1H, s), 6.61 (1H, s), 6.99 (1H, dd, J = 7.9, 1.9 Hz), 7.11 (1H, dd, J = 7.8, 1.8 Hz), 7.18 (1H, t, J = 7.9 Hz), 7.34-7.43 (2H, m).

### Example 148

### {[(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)methyl}-1,2,4-oxadiazol-5-yl)methyl]thio}acetic acid

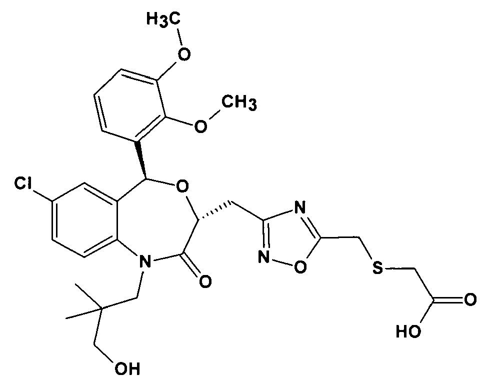

According to a similar manner to that of Example 144, the title compound was obtained.

¹H-NMR (CDCl₃) δ: 0.66 (3H, s), 1.05 (3H, s), 3.17-3.32 (2H, m), 3.34-3.48 (4H, m), 3.58-3.68 (4H, m), 3.89 (3H, s), 4.00 (2H, s), 4.42-4.53 (2H, m), 6.19 (1H, s), 6.61 (1H, d, J = 1. 3 Hz), 6.98 (1H, dd, J = 8.1, 1.5 Hz), 7.07 (1H, dd, J = 7.7, 1.5 Hz), 7.17 (1H, t, J = 8.0 Hz), 7.33-7.42 (2H, m).

### Example 149

### 2-{[(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)methyl]thio}-2-methylpropionic acid

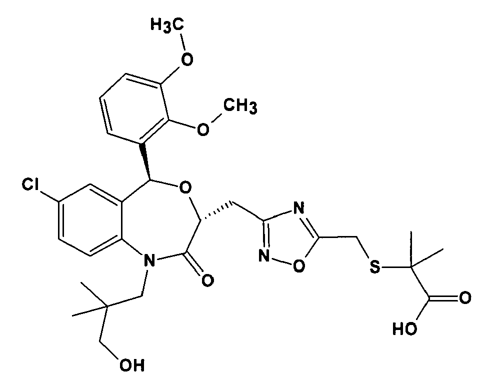

According to a similar manner to that of Example 144, the title compound was obtained.

¹H-NMR (CDCl₃) δ: 0.66 (3H, s), 1.04 (3H, s), 1.54 (3H, s), 1.54 (3H, s), 3.14-3.29 (2H, m), 3.34-3.47 (2H, m), 3.57-3.66 (4H, m), 3.89 (3H, s), 4.03 (2H, s), 4.42-4.52 (2H, m), 6.19 (1H, s), 6.60 (1H, d, J = 1.1 Hz), 6.98 (1H, dd, J = 8.1, 1.7 Hz), 7.09 (1H, dd, J = 7.7, 1.5 Hz), 7.17 (1H, t, J = 7.9 Hz), 7.33-7.41 (2H, m).

### Example 150

### [(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]acetic acid

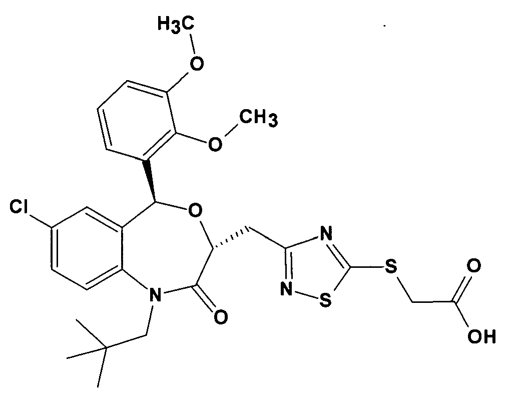

(1) Methyl iodide (0.65 ml, 10.48 mmol) was added to a solution of 2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethanethioamide (1.0 g, 2.10 mmol) obtained as in Example 16-(2) in acetone (10 ml), and the mixture was stirred for 14 hours. After the reaction solution was concentrated under reduced pressure, ammonium acetate (0.81 g, 10.48 mmol) and methanol (10 ml) were added thereto and the mixture was heated to reflux for 4 hours. After perchloromethylmercaptan (0.23 ml, 2.1 mmol) was added to a solution of the residue in dichloromethane, a solution of sodium hydroxide (0.375 g, 9.38 mmol) in water (3 ml) was slowly added dropwise thereto at -10°C. The reaction solution was stirred at -10°C for 2 hours and then at room temperature for 30 minutes. The reaction solution was extracted with ethyl acetate, washed successively with water and an aqueous saturated sodium chloride solution, dried over magnesium sulfate, and then concentrated under reduced pressure. The residue was purified by silica gel column chromatography [developing solvent: hexane-ethyl acetate (95:5-85:15)] to obtain (3R,5S)-7-chloro-3-[(5-chloro-1,2,4-thiadiazol-3-yl)methyl]-5-(2,3-dimethoxyphenyl)-1-(2,2,dimethylpropyl)-1,5-dihydro-4,1-benzoxazepin-2(3H)-one (0.46 g, 0.857 mmol, 41%) as a white noncrystalline solid.
   ¹H-NMR (CDCl₃) δ: 0.95 (9H, s), 3.33-3.48 (2H, m), 3.55-3.66 (4H, m), 3.89 (2H, s), 4.50 (1H, d, J = 13.8 Hz), 4.58 (1H, t, J = 6.7 Hz), 6.31 (1H, s), 6.59 (1H, d, J = 1.1 Hz), 6.93-7.04 (2H, m), 7.15 (1H, t, J = 8.0 Hz), 7.30-7.38 (2H, m).
(2) Ethyl thioglycolate (113 ml, 1.03 mmol) and potassium carbonate (178 mg, 1.29 mmol) were added to a solution of (3R,5S)-7-chloro-3-[(5-chloro-1,2,4-thiadiazol-3-yl)methyl]-5-(2,3-dimethoxyphenyl)-1-(2,2-dimethylpropyl)-1,5-dihydro-4,1-benzoxazepin-2(3H)-one (0.46 g, 0.857 mmol) obtained in Example 150-(1) in THF (10 ml), and the mixture was stirred at 45°C for 16 hours. The reaction solution was extracted with ethyl acetate, washed successively with water and an aqueous saturated sodium chloride solution, dried over magnesium sulfate, and then concentrated under reduced pressure. The residue was purified by silica gel column chromatography [developing solvent: hexane-ethyl acetate (95:5-85:15)] to obtain ethyl [(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]acetate (0.48 g, 0.774 mmol, 90%) as a white noncrystalline solid.
   ¹H-NMR (CDCl₃) δ: 0.95 (9H, s), 1.26 (3H, t, J = 7.2 Hz), 3.32-3.43 (2H, m), 3.57 (1H, dd, J = 15.6, 6.9 Hz), 3.63 (3H, s), 3.89 (3H, s), 4.01 (2H, s), 4.18 (2H, q, J = 7.2 Hz), 4.50 (1H, d, J = 13.9 Hz), 4.58 (1H, t, J = 6.7 Hz), 6.31 (1H, s), 6.59 (1H, s), 6.96 (1H, dd, J = 8.1, 1.5 Hz), 7.04 (1H, dd, J = 7.8, 1.4 Hz), 7.15 (1H, t, J = 8.0 Hz), 7.34 (2H, d, J = 1.3 Hz).
(3) A 2N aqueous sodium hydroxide solution (1.16 ml, 2.32 mmol) was added to a solution of ethyl [(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]acetate (0.48 g, 0.774 mmol) obtained in Example 150-(2) in a mixture of ethanol (5 ml) and THF (5 ml), and the mixture was stirred for 1 hour. The reaction solution was extracted with water and then washed with diethyl ether. The aqueous layer was acidified with 1N aqueous hydrochloric acid, extracted with ethyl acetate, washed with an aqueous saturated sodium chloride solution, dried over magnesium sulfate, and then concentrated under reduced pressure. The residue was recrystallized from ethanol-hexane to obtain [(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]acetic acid (374 mg, 0.632 mmol, 82%) as a colorless crystal.
   ¹H-NMR (CDCl₃) δ: 0.95 (9H, s), 3.34-3.45 (2H, m), 3.57-3.68 (4H, m), 3.89 (3H, s), 3.93 (2H, s), 4.49 (1H, d, J = 13.9 Hz), 4.56 (1H, dd, J = 7.2, 6.2 Hz), 6.31 (1H, s), 6.61 (1H, s), 6.97 (1H, dd, J = 8.1, 1.5 Hz), 7.04 (1H, dd, J = 7.7, 1.3 Hz), 7.16 (1H, t, J = 7.9 Hz), 7.33-7.38 (2H, m).

### Example 151

### 2-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-tihadiazol-5-yl)thio]-2-methylpropionic acid

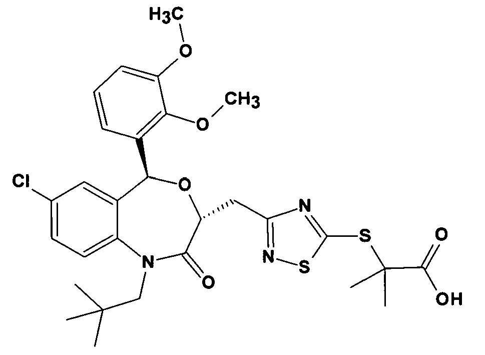

(1) Ethyl 3-mercaptopropionate (0.28 ml, 2.2 mmol) and potassium carbonate (0.21 g, 1.5 mmol) were added to a solution of (3R,5S)-7-chloro-3-[(5-chloro-1,2,4-thiadiazol-3-yl)methyl]-5-(2,3-dimethoxyphenyl)-1-(2,2-dimethylpropyl)-1,5-dihydro-4,1-benzoxazepin-2(3H)-one (0.54 g, 1.0 mmol) obtained in Example 150-(1) in THF (10 ml), and the mixture was stirred at 45°C for 13.5 hours. The reaction solution was extracted with ethyl acetate, washed successively with water and an aqueous saturated sodium chloride solution, dried over magnesium sulfate, and then concentrated under reduced pressure. The residue was dissolved in ethanol, a 2N aqueous sodium hydroxide solution (0.836 ml, 1.67 mmol) was added, and the mixture was stirred for 1 hour. The reaction solution was extracted with ethyl acetate, washed successively with water and an aqueous saturated sodium chloride solution, dried over magnesium sulfate, and then concentrated under reduced pressure. To the residue was added ethyl 2-bromo-2-methylpropionate (0.18 ml, 1.25 mmol), potassium carbonate (0.17 g, 1.25 mmol) and THF (10 ml), and the mixture was heated to reflux for 14 hours. The reaction solution was extracted with ethyl acetate, washed successively with water and an aqueous saturated sodium chloride solution, dried over magnesium sulfate, and then concentrated under reduced pressure. The residue was purified by silica gel column chromatography [developing solvent: hexane-ethyl acetate (85:15-80:20)] to obtain ethyl 2-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]-2-methylpropionate (0.47 g, 87%).
   ¹H-NMR (CDCl₃) δ: 0.95 (9H, s), 1.16 (3H, t, J = 7.1 Hz), 1.66 (3H, s), 1.68 (3H, s), 3.35-3.44 (2H, m), 3.55 (1H, dd, J = 15.6, 6.6 Hz), 3.62 (3H, s), 3.88 (3H, s), 4.06 (2H, ddd, J = 14.2, 7.1, 1.9 Hz), 4.51 (1H, d, J = 13.9 Hz), 4.59 (1H, t, J = 6.8 Hz), 6.30 (1H, s), 6.56 (1H, d, J = 1.9 Hz), 6.93-7.00 (1H, m), 7.13 (1H, t, J = 8.0 Hz), 7.31-7.39 (1H, m).
(2) A 2N aqueous sodium hydroxide solution (1.1 ml, 2.17 mmol) was added to a solution of ethyl 2-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]-2-methylpropionate (0.47 g, 0.725 mmol) obtained in Example 151-(1) in ethanol (10 ml), and the mixture was stirred for 2 hours. The reaction solution was extracted with water and then washed with diethyl ether. The aqueous layer was acidified with 1N aqueous hydrochloric acid, extracted with ethyl acetate, washed with an aqueous saturated sodium chloride solution, dried over magnesium sulfate, and then concentrated under reduced pressure to obtain 2-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-tihadiazol-5-yl)thio]2-methylpropionic acid (332 mg, 74%).
   ¹H-NMR (CDCl₃) δ: 0.95 (9H, s), 1.66 (3H, s), 1.68 (3H, s), 3.34-3.49 (2H, m), 3.56-3.67 (4H, m), 3.89 (3H, s), 4.49 (1H, d, J = 13.9 Hz), 4.57 (1H, t, J = 6.7 Hz), 6.31 (1H, s), 6.58 (1H, d, J = 2.1 Hz), 6.97 (2H, dd, J = 7.1, 5.9 Hz), 7.14 (1H, t, J = 7.9 Hz), 7.31-7.43 (2H, m).

### Example 152

### 4-[(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-thiadiazol-5-yl)thio]butanoic acid

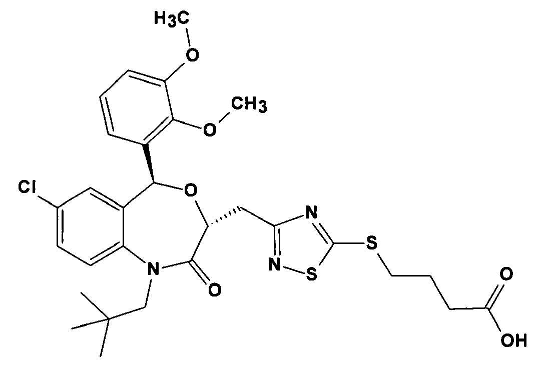

According to a similar manner to that of Example 151, the title compound was obtained.

¹H-NMR (CDCl₃) δ: 0.95 (9H, s), 2.03-2.15 (2H, m), 2.50 (2H, t, J = 7.1 Hz), 3.29 (2H, dt, J = 7.0, 2.3 Hz), 3.33-3.43 (2H, m), 3.58 (1H, dd, J = 15.6, 6.9 Hz), 3.63 (3H, s), 3.88 (3H, s), 4.50 (1H, d, J = 13.8 Hz), 4.61 (1H, t, J = 6.7 Hz), 6.31 (1H, s), 6.58 (1H, s), 6.96 (1H, dd, J = 8.1, 1.5 Hz), 7.03 (1H, dd, J = 7.9, 1.1 Hz), 7.14 (1H, t, J = 7.9 Hz), 7.33 (2H, d, J = 1.3 Hz).

### Example 153

### [(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-thiadiazol-5-yl)thio]acetic acid

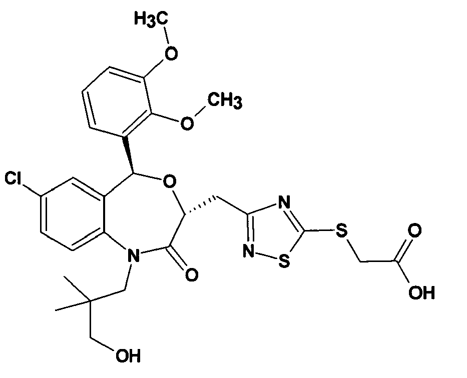

According to a similar manner to that of Example 150, the title compound was obtained.

¹H-NMR (CDCl₃) δ: 0.65 (3H, s), 1.04 (3H, s), 3.20 (1H, d, J = 11.9 Hz), 3.35 (1H, dd, J = 15.9, 5.4 Hz), 3.42 (1H, d, J = 14.3 Hz), 3.59-3.71 (5H, m), 3.89 (3H, s), 3.99 (2H, s), 4.46 (1H, d, J = 14.3 Hz), 4.58 (1H, dd, J = 7.9, 5.5 Hz), 6.19 (1H, s), 6.62 (1H, s), 6.98 (1H, dd, J = 8.0, 1.6 Hz), 7.09 (1H, dd, J = 7.9, 1.5 Hz), 7.17 (1H, t, J = 7.9 Hz), 7.35-7.44 (2H, m).

### Example 154

### 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid

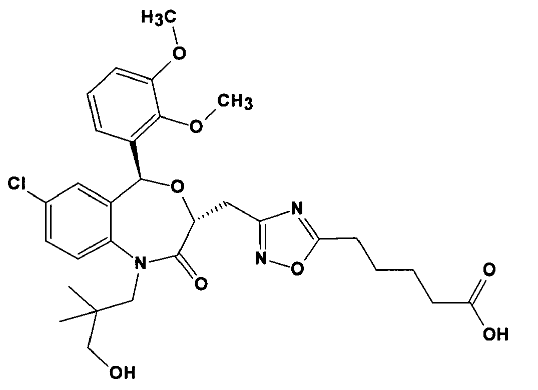

(1) Triethylamine (1.2 ml, 8.5 mmol) and methyl 6-chloro-6-oxohexanoate (0.98 g, 5.5 mmol) were added dropwise to a solution of 3-[(3R,5S)-3-[(2Z)-2-amino-2-(hydroxyimino)ethyl]-7-chloro-5-(2,3-dimethoxyphenyl)-2-oxo-2,3-dihydro-4,1-benzoxazepin-1(5H)-yl]-2,2-dimethylpropyl acetate (2.67 g, 5.0 mmol) obtained in Example 58-(1) in THF (25 ml) under ice-cooling, and the mixture was stirred under ice-cooling for 40 minutes and then at room temperature for 1.5 hours. The reaction solution was concentrated under reduced pressure, water (25 ml) was added, and the mixture was heated to reflux for 18 hours. The reaction solution was extracted with ethyl acetate, washed with water and an aqueous saturated sodium chloride solution, and dried over magnesium sulfate. After concentration under reduced pressure, the residue was purified by silica gel column chromatography to obtain methyl 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxodiazol-5-yl)pentanoate (1.73 g).
   ¹H-NMR (CDCl₃) δ: 0. 94 (3 H, s), 1. 03 (3 H, s), 1.65-1.90 (4 H, m), 2.03 (3 H, s), 2.35 (2 H, t, J = 7.2 Hz), 2.86 (2 H, t, J = 7.3 Hz), 3.29 (2 H, ddd, J = 30.5, 15.6, 6.7 Hz), 3.55 (1 H, d, J = 13.9 Hz), 3.61 (3 H, s), 3.67 (3 H, s), 3.74 (1 H, d, J = 11.1 Hz), 3.85 (1 H, d, J = 11.1 Hz), 3.88 (3 H, s), 4.42 (1 H, t, J = 6.7 Hz), 4.58 (1 H, d, J = 14.1 Hz), 6.30 (1 H, s), 6.61 (1 H, d, J = 1.5 Hz), 6.97 (1 H, dd, J = 7.9, 1.7 Hz), 7.07 (1 H, dd, J = 7.8, 1.6 Hz), 7.14 (1 H, t, J = 7.9 Hz), 7.29 - 7.38 (2 H, m).
(2) A 2N aqueous sodium hydroxide solution (5.25 ml, 10.5 mmol) was added to a solution of methyl 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxodiazol-5-yl)pentanoate (1.73 g, 2.63 mmol) obtained in Example 154-(1) in ethanol (17 ml), and the mixture was stirred for 2 hours. The reaction solution was diluted with water and then washed with diethyl ether. The extract was acidified with 6N aqueous hydrochloric acid, extracted with ethyl acetate, washed with an aqueous saturated sodium chloride solution, dried over magnesium sulfate, and then concentrated under reduced pressure. The residue was purified by silica gel column chromatography and then recrystallized from ethyl acetate-hexane to obtain 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}1,2,4-oxadiazol-5-yl)pentanoic acid (1.06 g).
   ¹H-NMR (CDCl₃) δ: 0. 65 (3H, s), 1. 04 (3H, s), 1. 66-1.93 (4H, m), 2.39 (2H, t, J = 7.2 Hz), 2.88 (2H, t, J = 7.4 Hz), 3.12-3.28 (2H, m), 3.33-3.44 (2H, m), 3.57-3.67 (4H, m), 3.89 (3H, s), 4.40-4.53 (2H, m), 6.19 (1H, s), 6.60 (1H, d, J = 1.7 Hz), 6.98 (1H, dd, J = 8.1, 1.7 Hz), 7.09 (1H, dd, J = 7.7, 1.5 Hz), 7.16 (1H, t, J = 7.9 Hz), 7.32-7.41 (2H, m).

### Example 155

### 4-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-5-yl)butanoic acid

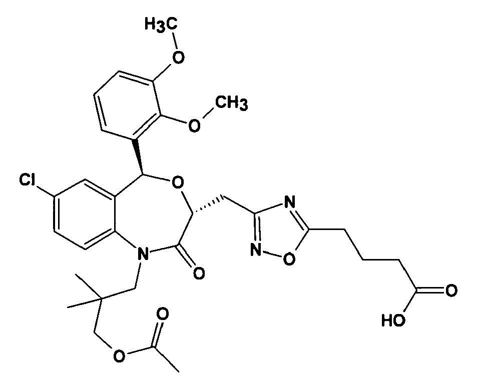

According to a similar manner to that of Example 14, the title compound was obtained.

¹H-NMR (CDCl₃) δ: 0. 94 (3H, s), 1.03 (3H, s), 2.03 (3H, s), 2.07-2.19 (2H, m), 2.48 (2H, t, J = 7.2 Hz), 2.95 (2H, t, J = 7.3 Hz), 3.24 (1H, dd, J = 15.6, 7.2 Hz), 3.36 (1H, dd, J = 15.6, 6.6 Hz), 3.55 (1H, d, J = 14.1 Hz), 3.61 (3H, s), 3.74 (1H, d, J = 11.1 Hz), 3.85 (1H, d, J = 11.3 Hz), 3.88 (3H, s), 4.43 (1H, t, J = 6.7 Hz), 4.57 (1H, d, J = 14.1 Hz), 6.30 (1H, s), 6.62 (1H, s), 6.97 (1H, dd, J = 8.0, 1.6 Hz), 7.05-7.10 (1H, m), 7.15 (1H, t, J = 7.8 Hz), 7.30-7.38 (2H, m).

### Example 156

### 5-(3-{[(3R,5S)-1-[3-acetoxy-2,2-dimethylpropyl]-7-chloro-5-(2,3-dimethoxyphenyl)-2-oxo-1,2,3,5-tetrahydro-4,1-benzoxazepin-3-yl]methyl}-1,2,4-oxodiazol-5-yl)pentanoic acid

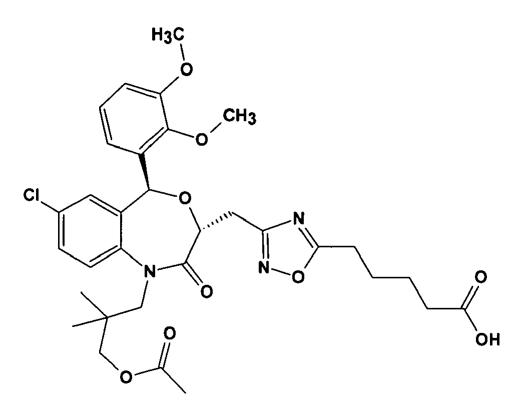

According to a similar manner to that of Example 14, the title compound was obtained.

¹H-NMR (CDCl₃) δ: 0.94 (3H, s), 1. 03 (3H, s), 1.67 - 1.77 (2H, m), 1.81 - 1.91 (2H, m), 2.03 (3H, s), 2.39 (2H, t, J = 7.2 Hz), 2.88 (2H, t, J = 7.4 Hz), 3.20 - 3.28 (1H, m), 3.30 - 3.38 (1H, m), 3.55 (1H, d, J = 14.1 Hz), 3.61 (3H, s), 3.72 - 3.77 (1H, m), 3.85 (1H, d, J = 11.1 Hz), 3.88 (3H, s), 4.42 (1H, t, J = 6.7 Hz), 4.57 (1H, d, J = 13.9 Hz), 6.30 (1H, s), 6.61 (1H, d, J = 1.7 Hz), 6.97 (1H, dd, J = 7.9, 1.7 Hz), 7.05 - 7.09 (1H, m), 7.14 (1H, t, J = 7.9 Hz), 7.30 - 7.37 (2H, m).

### Example 157

### 3-(3-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,2,4-oxadiazol-5-yl)propionic acid

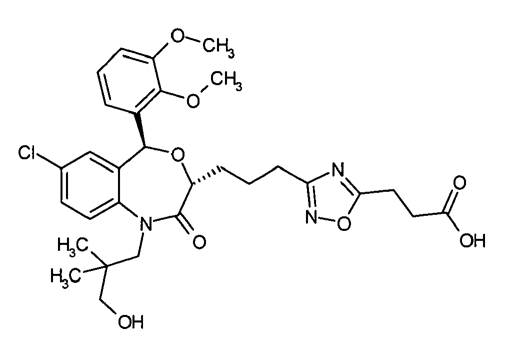

(1) (Diethoxyphosphoryl)acetonitrile (5.03 g, 28.4 mmol) was added dropwise to a suspension of sodium hydride (1.14 g, 28.4 mmol) in THF (110 ml) at 0°C. After stirring for 30 minutes, 3-[(3R,5S)-7-chloro-5-(2,3-dimethoxyphenyl)-2-oxo-3-(2-oxoethyl)-2,3-dihydro-4,1-benzoxazepin-1(5H)-yl]-2,2-dimethylpropyl acetate (11.01 g, 21.8 mmol) obtained in Example 115-(1) was added thereto. After stirring for 1 hour, the mixture was diluted with ethyl acetate, washed with an aqueous saturated ammonium chloride solution and an aqueous saturated sodium chloride solution, dried over magnesium sulfate, and then concentrated under reduced pressure. The residue was purified by silica gel column chromatography to obtain (2E)-4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-2-butenenitrile (8.15 g, 71%).
   ¹H-NMR (CDCl₃) δ: 0.95 (3 H, s), 1.02 (3 H, s), 2.03 (3 H, s), 2.64 - 3.10 (2 H, m), 3.49 - 3.76 (5 H, m), 3.81 - 4.00 (5 H, m), 4.56 (1 H, dd, J = 14.1, 5.1 Hz), 5.37 - 5.48 (1 H, m), 6.26 (1 H, d, J = 2.6 Hz), 6. 62 - 6.80 (2 H, m), 6.96 - 7.04 (1 H, m), 7.12 - 7.39 (4 H, m).
(2) (2E)-4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-2-butenenitrile (8.15 g, 12.4 mmol) obtained in Example 157-(1) was dissolved in methanol (80 ml), magnesium (1.13 g, 46.4 mmol) was added, and the mixture was stirred for 19 hours. The reaction solution was diluted with ethyl acetate, washed with 1N aqueous hydrochloric acid and an aqueous saturated sodium chloride solution, dried over anhydrous magnesium sulfate, and then concentrated under reduced pressure. The residue was dissolved in ethyl acetate (40 ml), triethylamine (5.1 ml, 36 mmol) and acetyl chloride (2.0 ml, 29.1 mmol) were added, and the mixture was stirred for 5 hours. The reaction solution was diluted with ethyl acetate, and the organic layer was washed with water and an aqueous saturated sodium chloride solution, dried over magnesium sulfate, and then concentrated under reduced pressure. The residue was purified by silica gel column chromatography to obtain 4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]butanenitrile (3.89 g, 91%).
   ¹H-NMR (CDCl₃) δ: 0.94 (3 H, s), 1.02 (3 H, s), 1.67 - 2.06 (7 H, m), 2.37 (2 H, t, J = 6.7 Hz), 3.52 (1 H, d, J = 14.1 Hz), 3.62 (3 H, s), 3.73 (1 H, d, J = 11.1 Hz), 3.81 - 3.88 (2 H, m), 3.89 (3 H, s), 4.56 (1 H, d, J = 13.9 Hz), 6.25 (1 H, s), 6.64 (1 H, d, J = 2.1 Hz), 7.00 (1 H, dd, J = 7.2, 2.3 Hz), 7.15 - 7.39 (4 H, m).
(3) Triethylamine (5.14 ml, 36.7 mmol) was added to a solution of hydroxyamine hydrochloride (2.55 g, 36.7 mmol) in dimethyl sulfoxide (40 ml), and the mixture was stirred for 30 minutes. Insoluble substances were filtered off. To the filtrate was added 4-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]butanenitrile (3.89 g, 7.35 mmol) obtained in Example 157-(2) and the mixture was stirred at 80°C for 6 hours. The reaction solution was diluted with ethyl acetate, and the organic layer was washed with water and an aqueous sodium chloride solution, dried over magnesium sulfate and then concentrated under reduced pressure to obtain 3-[(3R,5S)-3-[4-amino-4-(hydroxyimino)butyl]-7-chloro-5-(2,3-dimethoxyphenyl)-2-oxo-2,3-dihydro-4,1-benzoxazepin-1(5H)-yl]-2,2-dimethylpropyl acetate (4.33 g) as crude powder.
(4) According to a similar manner to that of Example 60, the title compound was obtained from 3-[(3R,5S)-3-[4-amino-4-(hydroxyimino)butyl]-7-chloro-5-(2,3-dimethoxyphenyl)-2-oxo-2,3-dihydro-4,1-benzoxazepin-1(5H)-yl]-2,2-dimethylpropyl acetate obtained in Example 157-(3).
   ¹H-NMR (CDCl₃) δ: 0. 63 (3 H, s), 1.04 (3 H, s), 1.70 - 2.02 (4 H, m), 2.66 - 2.78 (2 H, m), 2.89 (2 H, t, J = 7.2 Hz), 3.10 - 3.20 (3 H, m), 3.36 (1 H, d, J = 14.3 Hz), 3.57 - 3.66 (4 H, m), 3.83 - 3.92 (4 H, m), 4.45 (1 H, d, J = 14.3 Hz), 6.12 (1 H, s), 6.60 (1 H, d, J = 2.3 Hz), 6.99 (1 H, dd, J = 6.4, 3.4 Hz), 7.16 - 7.24 (2 H, m), 7.30 (1 H, d, J = 8.7 Hz), 7.36 (1 H, dd, J = 8.7, 2.4 Hz).

### Example 158

### 4-(3-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,2,4-oxadiazol-5-yl)butanoic acid

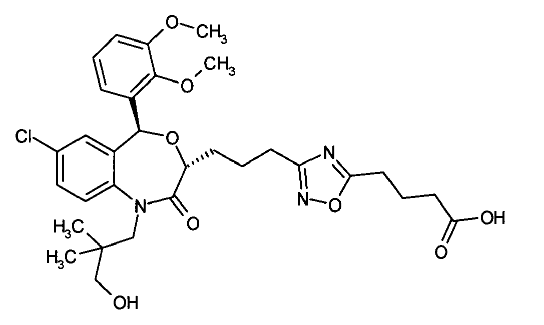

According to a similar manner to that of Example 157, the title compound was obtained.

¹H-NMR (CDCl₃) δ: 0.63 (3 H, s), 1.04 (3 H, s), 1.71 - 2.02 (4 H, m), 2.08 - 2.20 (2 H, m), 2.49 (2 H, t, J = 7.2 Hz), 2.74 (2 H, t, J = 6.4 Hz), 2.94 (2 H, t, J = 7.3 Hz), 3.15 (1 H, d, J = 12.1 Hz), 3.36 (1 H, d, J = 14.3 Hz), 3.57 - 3.66 (4 H, m), 3.83 - 3.92 (4 H, m), 4.46 (1 H, d, J = 14.3 Hz), 6.13 (1 H, s), 6.60 (1 H, d, J = 2.3 Hz), 6.99 (1 H, dd, J = 6.8, 3.0 Hz), 7.16 - 7.24 (2 H, m), 7.30 (1 H, d, J = 8.7 Hz), 7.36 (1 H, dd, J = 8.7, 2.1 Hz).

### Example 159

### 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,2,4-oxadiazol-5-yl)propionic acid

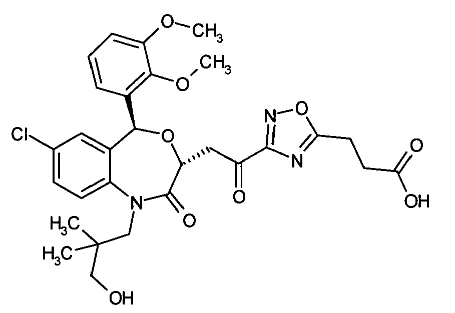

(1) Triethylamine (4.05 ml, 28.9 mmol) was added to a solution of hydroxyamine hydrochloride (2.0 g, 5.78 mmol) in dimethyl sulfoxide (40 ml), and the mixture was stirred for 30 minutes. Insoluble substances were filtered off. To the filtrate was added 3-[(3R,5S)-3-(2-{[tert-butyl(dimethyl)silyl]oxy}-2-cyanoethyl)-7-chloro-5-(2,3-dimethoxyphenyl)-2-oxo-2,3-dihydro-4,1-benzoxazepin-1(5H)-yl]-2,2-dimethylpropyl acetate (3.73 g, 5.78 mmol) obtained in Example 115-(2) and the mixture was stirred at 80°C for 2.5 hours. The reaction solution was diluted with ethyl acetate, and the organic layer was washed with water and an aqueous saturated sodium chloride solution, dried over magnesium sulfate and then concentrated under reduced pressure to obtain 3-[(3R,5S)-3-[(3Z)-3-amino-2-{[tert-butyl(dimethyl)silyl]oxy}-3-(hydroxyimino)propyl]-7-chloro-5-(2,3-dimethoxyphenyl)-2-oxo-2,3-dihydro-4,1-benzoxazepin-1(5H)-yl]-2,2-dimethylpropyl acetate as crude powder (4.07 g, 100%).
(2) Triethylamine (0.63 ml, 4.49 mmol) and ethyl 4-chloro-4-oxobutanoate (0.47 ml, 3.29 mmol) were added dropwise to a solution of 3-[(3R,5S)-3-[(3Z)-3-amino-2-{[tert-butyl(dimethyl)silyl]oxy}-3-(hydroxyimino)propyl]-7-chloro-5-(2,3-dimethoxyphenyl)-2-oxo-2,3-dihydro-4,1-benzoxazepin-1(5H)-yl]-2,2-dimethylpropyl acetate (2.03 g, 2.99 mmol) obtained in Example 159-(1) in THF (20 ml) under ice-cooling, and the mixture was stirred at room temperature for 4.5 hours. The reaction solution was concentrated under reduced pressure, water (20 ml) was added to the residue, and the mixture was heated to reflux for 17.5 hours. After the reaction solution was diluted with ethyl acetate, the organic layer was washed with water and an aqueous saturated chloride solution, dried over magnesium sulfate, and then concentrated under reduced pressure. The residue was purified by silica gel column chromatography to obtain ethyl 3-[3-(2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-1-{[tert-butyl(dimethyl)silyl]oxy}ethyl)-1,2-4-oxadiazol-5-yl]propionate (1.65 g, 70%).
   ¹H-NMR (CDCl₃) δ: -0.07 - 0.11 (6 H, m), 0.69-0.89 (9H, m), 1.04 (3 H, s), 1.09 - 1.15 (3 H, m), 1.29 - 1.38 (3 H, m), 2.09 - 2.14 (3 H, m), 2.32 - 2.60 (2 H, m), 2.85 - 2.95 (2 H, m), 3.18 - 3.29 (2 H, m), 3.61 (1 H, d, J = 14.1 Hz), 3.68 - 3.72 (3 H, m, J = 2.4 Hz), 3.80 - 3.87 (1 H, m), 3.90 - 4.00 (4 H, m), 4.15 - 4.33 (3 H, m), 4.66 (1 H, dd, J = 14.0, 8.2 Hz), 5.12 - 5.29 (1 H, m), 6.34 (1 H, d, J = 12.1 Hz), 6.72 (1 H, dd, J = 10.3, 2.2 Hz), 7.03 - 7.11 (1 H, m), 7.22 - 7.44 (4 H, m).
(3) A boron trifluoride-diethyl ether complex (0.53 ml, 4.19 mmol) was added to a solution of ethyl 3-[3-(2-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]-1-{[tert-butyl(dimethyl)silyl]oxy}ethyl)-1,2-4-oxadiazol-5-yl]propionate (1.65 g, 2.09 mmol) obtained in Example 159-(2) in acetonitrile (20 ml) under ice-cooling, and the mixture was stirred for 1 hour under ice-cooling. The reaction solution was diluted with ethyl acetate, and the organic layer was washed with water and an aqueous saturated sodium chloride solution, dried over magnesium sulfate and then concentrated under reduced pressure. To a solution of the residue in dichloromethane (15 ml), triethylamine (0.87 ml, 6.23 ml) was added and a solution of a sulfur trioxide-pyridine complex (0.83 g, 5.19 mmol) in DMSO (3.3 ml) that was prepared previously was then added dropwise. The mixture was stirred for 20 hours. The reaction solution was diluted with ethyl acetate, and the organic layer was washed with water and an aqueous saturated sodium chloride solution, dried over magnesium sulfate and then concentrated under reduced pressure. The residue was purified by silica gel column chromatography to obtain ethyl 3-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}-1,2,4-oxadiazol-5-yl)propionate (1.05 g, 75%) as crude powder.
   ¹H-NMR (CDCl₃) δ: 0.94 (3 H, s), 1.02 (3 H, s), 1.25 (3 H, t, J = 7.2 Hz), 2.02 (3 H, s), 2.90 (2 H, t, J = 7.3 Hz), 3.25 (2 H, t, J = 7.3 Hz), 3.42 (1 H, dd, J = 17.8, 5.4 Hz), 3.57 (1 H, d, J = 14.3 Hz), 3.61 (3 H, s), 3.73 (1 H, d, J = 11. 1 Hz), 3.80 - 3.93 (5 H, m), 4.16 (2 H, q, J = 7.2 Hz), 4.54 (1 H, d, J = 14.1 Hz), 4.62 (1 H, dd, J = 8.0, 5.4 Hz), 6.28 (1 H, s), 6.65 (1 H, s), 6.98 (1 H, dd, J = 6.4, 3.2 Hz), 7.13 - 7.21 (2 H, m), 7.36 (2 H, s).
(4) A 2N aqueous sodium hydroxide solution (2.35 ml, 4.68 mmol) was added to a solution of ethyl 3-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}-1,2,4-oxadiazol-5-yl)propionate (1.05 g, 1.562 mmol) obtained in Example 159-(3) in ethanol (10 ml), and the mixture was stirred for 30 minutes. The reaction solution was neutralized with 1N aqueous hydrochloric acid, extracted with ethyl acetate, washed with an aqueous saturated sodium chloride solution, dried over magnesium sulfate, and then concentrated under reduced pressure. The residue was purified by Gilson preparative HPLC to obtain 3-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,2,4-oxadiazol-5-yl)propionic acid (160 mg, 17%) as powder.
   ¹H-NMR (CDCl₃) δ: 0.65 (3 H, s), 1.01 - 1.07 (3 H, m), 2.98 (2 H, t, J = 7.1 Hz), 3.16 (1 H, d, J = 12.1 Hz), 3.25 (2 H, t, J = 7.0 Hz), 3.37 - 3.48 (2 H, m), 3.56 - 3.64 (4 H, m), 3.74 (1 H, d, J = 19.6 Hz), 3.82 - 3.96 (5 H, m), 4.43 (1 H, d, J = 14.3 Hz), 4.61 (1 H, dd, J = 8.3, 5.1 Hz), 6.16 (1 H, s), 6.63 (1 H, s), 6. 99 (1 H, dd, J = 7. 2, 2. 6 Hz), 7.13 - 7.23 (2 H, m), 7.40 (2 H, s).

### Example 160

### 4-(2-{2-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]ethyl}-1,3-thiazol-5-yl)butanoic acid

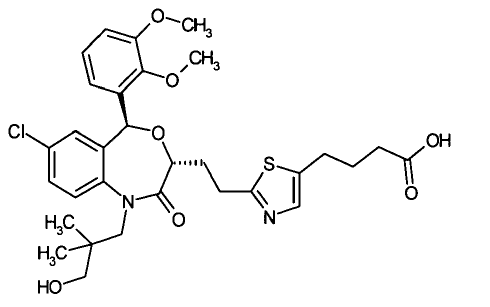

According to a similar manner to that of Example 105, the title compound was obtained

¹H-NMR (CDCl₃) δ: 0.64 (3H, s), 1. 05 (3H, s), 1. 93 (2H, quintet, J = 7.5 Hz), 2.17-2.42 (4H, m), 3.616 (2H, t, J = 7.5 Hz), 3.622 (1H, d, J = 12.3 Hz), 3.82-3.94 (1H, m), 3.89 (3H, s), 4.45 (1H, d, J = 14.1 Hz), 6.15 (1H, s), 6.61 (1H, d, J = 2.7 Hz), 6.99 (1H, dd, J = 2.4, 7.2 Hz), 7.15-7.25 (2H, m), 7.30 (1H, d, J = 8.7 Hz), 7.35 (1H, dd, J = 2.7, 8.7 Hz).

### Example 161

### 4-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)butanoic acid

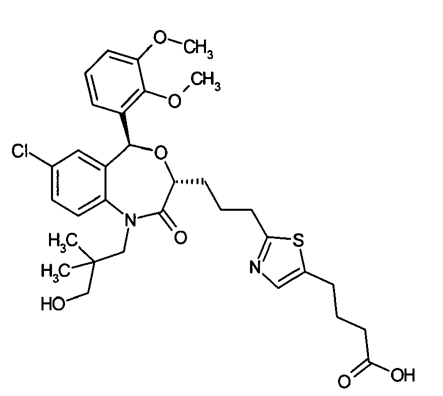

According to a similar manner to that of Example 122, the title compound was obtained.

¹H-NMR (CDCl₃) δ: 0. 63 (3H, s), 1.04 (3H, s), 1.75-2.05 (6H, m), 2.39 (2H, t, J = 7.5 Hz), 2.84 (2H, J = 7.5 Hz), 2.90-3.01 (2H, m), 3.15 (1H, d, J = 12.0 Hz), 3.35 (1H, d, J = 14.4 Hz), 3.61 (3H, s), 3.62 (1H, d, J = 12.0 Hz), 3.80-3.92 (1H, m), 3.89 (3H, s), 4.46 (1H, d, J = 14.4 Hz), 6.13 (1H, s), 6.60 (1H, d, J = 2.4 Hz), 6.99 (1H, dd, J = 2.7, 7.2 Hz), 7.15-7.25 (2H, m), 7.30 (1H, d, J = 8.4 Hz), 7.31 (1H, s), 7.36 (1H, dd, J = 2.4, 8.4 Hz).

### Example 162

### 4-(5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-oxadiazol-2-yl)butanoic acid

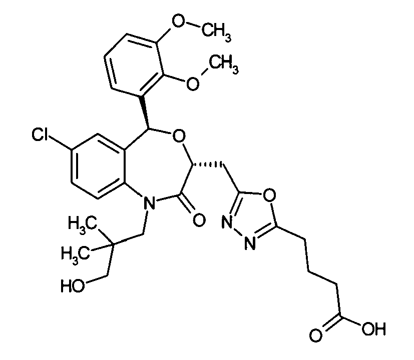

According to a similar manner to that of Example 55, the title compound was obtained.

¹H-NMR (CDCl₃) δ: 0.66 (3H, s), 1.05 (3H, s), 2.12 (2H, quintet, J = 7.2 Hz), 2.49 (2H, t, J = 7.2 Hz), 2.93 (2H, J = 7.2 Hz), 3.17 (1H, d, J = 12.3 Hz), 3.29 (1H, dd, J = 6.6, 15.9 Hz), 3.41 (1H, d, J = 14.4 Hz), 3.47 (1H, dd, J = 6.6, 15.9 Hz), 3.614 (1H, d, J = 12.3 Hz), 3.615 (3H, s), 3.89 (3H, s), 4.46 (1H, d, J = 14.4 Hz), 4.51 (1H, t, J = 6.6 Hz), 6.21 (1H, s), 6.61 (1H, d, J = 2.1 Hz), 6.99 (1H, dd, J = 1.8, 7.8 Hz), 7.08 (1H, dd, J = 1.8, 7.8 Hz), 7.18 (1H, t, J = 7.8 Hz), 7.34-7.43 (2H, m).

### Example 163

### 3-(5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-oxadiazol-2-ylpropionic acid

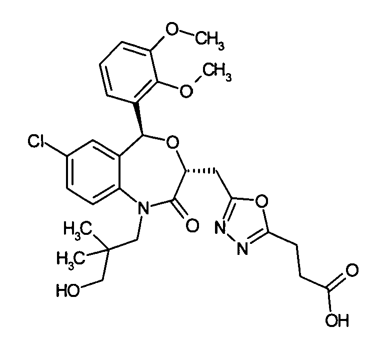

(1) Pyridine (0.067 ml, 0.83 mmol) and trifluoromethanesulfonic acid anhydride (0.13 ml, 0.79 mmol) were added to a solution of ethyl (4S)-4-({2-[(3R,5S)-1-(3-acetoxy-2,2-dimethylpropyl)-7-chloro-5-(2,3-dimethoxyphenyl)-1,2,3,5-tetrahydro-2-oxo-4,1-benzoxazepin-3-yl]acetyl}hydrazino)-4-oxobutanoate (0.25 g, 0.38 mmol) obtained in Example 57-(1) in dichloromethane (4 ml) at - 10°C, and the mixture was stirred for 1 hour. After further stirring at 0°C for 1 hour, an aqueous saturated sodium hydrogen carbonate solution (5 ml) was added thereto and the mixture was extracted with dichloromethane (80 ml). The organic layer was washed with water and an aqueous saturated sodium chloride solution, dried over magnesium sulfate, and then concentrated under reduced pressure. The resulting residue was purified by silica gel column chromatography [developing solvent: hexane-ethyl acetate (3:2)] to obtain ethyl 3-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3,4-oxadiazol-2-yl)propionate (0.16 g, 0.25 mmol, 66%) as colorless noncrystalline powder.
   ¹H-NMR (CDCl₃) δ: 0.95 (3H, s), 1.03 (3H, s), 1.26 (3H, t, J = 7.2 Hz), 2.03 (3H, s), 2.80-2.90 (2H, m), 3.14 (2H, t, J = 7.5 Hz), 3.29 (1H, dd, J = 7.2, 15.9 Hz), 3.43 (1H, dd, J = 6.6, 15.9 Hz), 3.55 (1H, d, J = 14.1 Hz), 3.62 (3H, s), 3.73 (1H, d, J = 10.8 Hz), 3.85 (1H, d, J = 10.8 Hz), 3.89 (3H, s), 4.16 (2H, q, J = 7.2 Hz), 4.42-4.93 (1H, m), 4.55 (1H, d, J = 14.1 Hz), 6.31 (1H, s), 6.63 (1H, d, J = 1.8 Hz), 6.98 (1H, dd, J = 1.5, 8.1 Hz), 7.07 (1H, dd, J = 1.5, 8.1), 7.18 (1H, t, J = 8.1 Hz), 7.32 (1H, d, J = 8.4 Hz), 7.36 (1H, dd, J = 1.8, 8.4 Hz)
(2) A mixture of ethyl 3-(5-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,3,4-oxadiazol-2-yl)propionate (0.1 g, 0.16 mmol) obtained in Example 163-(1), a 1N aqueous sodium hydroxide solution (0.6 ml) and ethanol (5 ml) was stirred at room temperature for 2 hours. The reaction solution was concentrated under reduced pressure, diluted with water (50 ml), and washed with diethyl ether (10 ml). The aqueous layer was acidified with 1N aqueous hydrochloric acid (1 ml) and then extracted with ethyl acetate (35 ml). The organic layer was washed with an aqueous saturated sodium chloride solution, dried over magnesium sulfate and then concentrated under reduced pressure to obtain 3-(5-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3,4-oxadiazol-2-ylpropionic acid (67 mg, 0.12 mmol, 75%) as a white crystal.
   ¹H-NMR (CDCl₃) δ: 0.65 (3H, s), 1.04 (3H, s), 2.89 (2H, t, J = 7.5 Hz), 3.14 (2H, t, J = 7.5 Hz), 3.17 (1H, d, J = 11.7 Hz), 3.29 (1H, dd, J = 6.3, 15.9 Hz), 3.61 (3H, s), 3.62 (1H, d, J = 11.7 Hz), 3.89 (3H, s), 4.46 (1H, d, J = 14.4 Hz), 4.44-4.52 (1H, m), 6.20 (1H, s), 6.61 (1H, d, J = 1.8 Hz), 6.99 (1H, dd, J = 1.5, 8.1 Hz), 7.07 (1H, dd, J = 1.5, 8.1 Hz), 7.18 (1H, t, J = 8.1 Hz), 7.32-7.42 (2H, m).

### Example 164

### 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)-3-hydroxypropionic acid

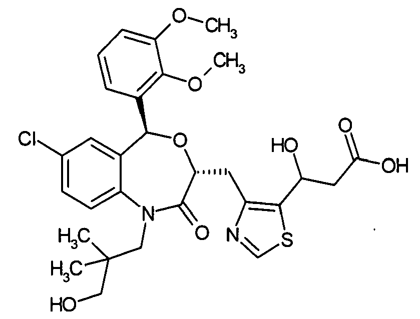

According to a similar manner to that of Example 18, the title compound was obtained.

¹H-NMR (CDCl₃) δ: 0.64 (3H, s), 1.01 (3/2H, s), 1.04 (3/2H, s), 2.67-2.87 (2H, m), 3.10-3.22 (2H, m), 3.28-3.40 (2H, m), 3.49 (1H, dd, J = 7.8, 14.1 Hz), 3.62 (3H, s), 3.59-3.68 (1H, m), 3.88 (3/2H, s), 3.89 (3/2H, s), 4.37-4.54 (2H, m), 5.56 (1/2H, t, J = 5.7 Hz), 5.65 (1/2H, dd, J = 4.5, 8.7 Hz), 6.18 (1H, s), 6.52 (1/2H, d, J = 2.1 Hz), 6.57 (1/2H, d, J = 2.1 Hz), 6.86-7.04 (2H, m), 7.10-7.20 (1H, m), 7.25-7.36 (2H, m), 8.58 (1/2H, s), 8.61 (1/2H, s).

### Example 165

### (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acrylic acid

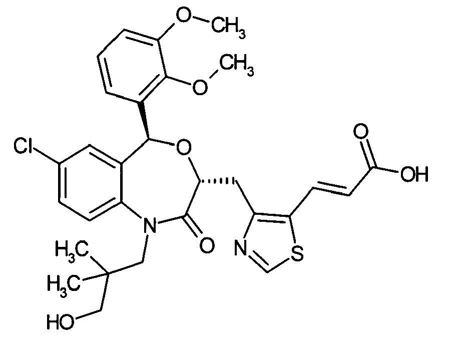

According to a similar manner to that of Example 19, the title compound was obtained.

¹H-NMR (CDCl₃) δ: 0.64 (3H, s), 1.05 (3H, s), 3.19 (1H, d, J = 12.0 Hz), 3.32-3.51 (3H, m), 3.61 (3H, s), 3.67 (1H, d, J = 12.0 Hz), 3.87 (3H, s), 4.39 (1H, t, J = 6.0 Hz), 4.47 (1H, d, J = 14.4 Hz), 6.169 (1H, d, J = 15.6 Hz), 6.173 (1H, s), 6.54 (1H, d, J = 1.8 Hz), 6.85-6.97 (2H, m), 7.09 (1H, t, J = 8.1 Hz), 7.26-7.37 (2H, m), 8.03 (1H, d, J = 15.6 Hz), 8.67 (1H, s)

### Example 166

### 3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)propionic acid

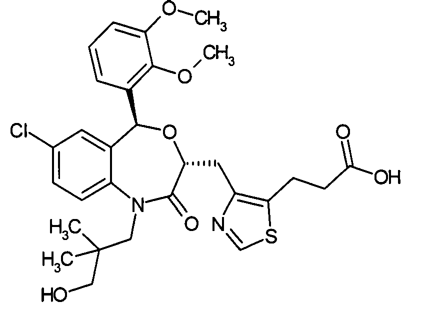

According to a similar manner to that of Example 88, the title compound was obtained.

¹H-NMR (CDCl₃) δ: 0.63 (3H, s), 1. 04 (3H, s), 2.66 (2H, t, J = 7.5 Hz), 3.10-3.40 (6H, m), 3.61 (3H, s), 3.66 (1H, d, J = 12.0 Hz), 3.88 (3H, s), 4.38-4.46 (2H, m), 6.17 (1H, s), 6.53 (1H, d, J = 2.4 Hz), 6.90-7.00 (2H, m), 7.13 (1H, t, J = 7.8 Hz), 7.26-7.38 (2H, m), 8.52 (1H, s).

### Example 167

### 4-(2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}-1,3-thiazol-5-yl)butanoic acid

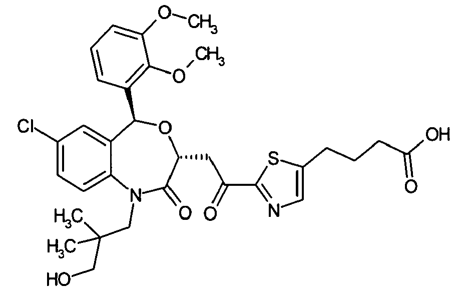

According to a similar manner to that of Example 115, the title compound was obtained.

¹H-NMR (CDCl₃) δ: 0.65 (3H, s), 1.04 (3H, s), 1.95-2.10 (2H, m), 2.43 (1H, t, J = 7.2 Hz), 2.97 (2H, t, J = 7.2 Hz), 3.14 (1H, d, J = 12.0 Hz), 3.41 (1H, d, J = 14.1 Hz), 3.55 (1H, dd, J = 4.8, 18.3 Hz), 3.61 (3H, s), 3.63 (1H, d, J = 12.0 Hz), 3.89 (3H, s), 3.95 (1H, dd, J = 8.4, 18.3 Hz), 4.48 (1H, d, J = 14.1 Hz), 4.58 (1H, dd, J = 4.8, 8.4 Hz), 6.17 (1H, s), 6.62 (1H, s), 6.95-7.00 (1H, m), 7.13-7.21 (2H, m), 7.36-7.42 (2H, m), 7.70 (1H, s).

### Example 168

### 3-(5-{[(3R,5S)-7-chloro-5-(2,3-dimethylphenyl)-1-(3-hydroxy-2,2-dimethylpropyl)-2-oxo-1,2,3,5-tetrahydro-4,1-benzoxazepin-3-yl]acetyl}-2-thienyl)propionic acid

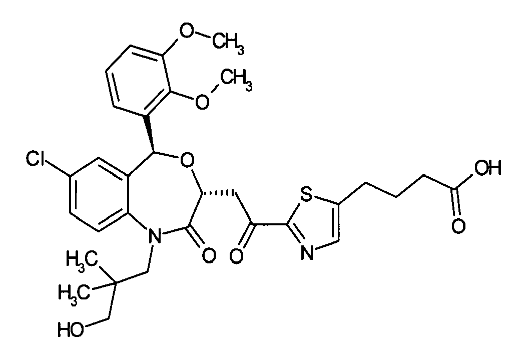

According to a similar manner to that of Example 119, the title compound was obtained.

¹H-NMR (CDCl₃) δ: 0.64 (3H, s), 1.04 (3H, s), 2.75 (2H, t, J = 7.2 Hz), 3.09-3.21 (3H, m), 3.27 (1H, dd, J = 4.5, 16.5 Hz), 3.40 (1H, d, J = 14.4 Hz), 3.58-3.74 (2H, m), 3.61 (3H, s), 3.89 (3H, s), 4.47 (1H, d, J = 14.4 Hz), 4.58 (1H, dd, J = 4.5, 8.4 Hz), 6.18 (1H, s), 6.62 (1H, s), 6.89 (1H, d, J = 3.6 Hz), 6.98 (1H, dd, J = 3.0, 7.2 Hz), 7.10-7.21 (2H, m), 7.35-7.42 (2H, m), 7.63 (1H, d, J = 3.6 Hz).

The agent for preventing or treating hyperlipemia of the present invention can be produced, for example, by the following formulations.

In the following formulations, ingredients (additives) other than the active ingredient may be products listed in Japanese Pharmacopoeia, Japanese Pharmaceutical Codex or Japanese Pharmaceutical Excipients.

### Preparation Example 1

### Capsule

| | |
|---|---|
| (1) (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid | 10 mg |
| (2) Lactose | 90 mg |
| (3) Microcrystalline cellulose | 70 mg |
| (4) Magnesium stearate | 10 mg |
| One capsule | 180 mg |

(1), (2) and (3) and 1/2 of (4) are mixed and then granulated. Thereto is added the remainder of (4) and the whole is encapsulated into a gelatin capsule.

### Preparation Example 2

### Capsule

| | |
|---|---|
| (1) (2-{[(3R,5S)-7-chloro-5-(2,3-dimethoxyphenyl)-1-neopentyl-2-oxo-1,2,3,5-tetrahydro-4,1-benzoxazepin-3-yl]methyl}-1,3-thiazol-5-yl)acetic acid | 10 mg |
| (2) Lactose | 90 mg |
| (3) Microcrystalline cellulose | 70 mg |
| (3) Magnesium stearate | 10 mg |
| One capsule | 180 mg |

(1), (2) and (3) and 1/2 of (4) are mixed and then granulated. Thereto is added the remainder of (4) and the whole is encapsulated into a gelatin capsule.

### Preparation Example 3

### Capsule

| | |
|---|---|
| (1) (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid | 10 mg |
| (2) Lactose | 90 mg |
| (3) Microcrystalline cellulose | 70 mg |
| (4) Magnesium stearate | 10 mg |
| One capsule | 180 mg |

(1), (2) and (3) and 1/2 of (4) are mixed and then granulated. Thereto is added the remainder of (4) and the whole is encapsulated into a gelatin capsule.

### Preparation Example 4

### Capsule

| | |
|---|---|
| (1) (5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid | 10 mg |
| (2) Lactose | 90 mg |
| (3) Microcrystalline cellulose | 70 mg |
| (4) Magnesium stearate | 10 mg |
| One capsule | 180 mg |

(1), (2) and (3) and 1/2 of (4) are mixed and then granulated. Thereto is added the remainder of (4) and the whole is encapsulated into a gelatin capsule.

### Preparation Example 5

### Tablet

| | |
|---|---|
| (1) (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2), (3), 2/3 of (4) and 1/2 of (5) are mixed and then granulated. To the granule added are the remainders of (4) and (5) and the mixture is compressed into a tablet.

### Preparation Example 6

### Tablet

| | |
|---|---|
| (1) (2-{[(3R,5S)-7-chloro-5-(2,3-dimethoxyphenyl)-1-neopentyl-2-oxo-1,2,3,5-tetrahydro-4,1-benzoxazepin-3-yl]methyl}-1,3-thiazol-5-yl)acetic acid | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2), (3), 2/3 of (4) and 1/2 of (5) are mixed and then granulated. To the granule added are the remainders of (4) and (5) and the mixture is compressed into a tablet.

### Preparation Example 7

### Tablet

| | |
|---|---|
| (1) (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2), (3), 2/3 of (4) and 1/2 of (5) are mixed and then granulated. To the granule added are the remainders of (4) and (5) and the mixture is compressed into a tablet.

### Preparation Example 8

### Tablet

| | |
|---|---|
| (1) (5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2), (3), 2/3 of (4) and 1/2 of (5) are mixed and then granulated. To the granule added are the remainders of (4) and (5) and the mixture is compressed into a tablet.

### Preparation Example 9

### Injection

| | |
|---|---|
| (1) (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-thiazol-5-yl)acetic acid | 10 mg |
| (2) Inositol | 100 mg |
| (3) Benzyl alcohol | 20 mg |
| One ampule | 130 mg |

(1), (2) and (3) are dissolved in distilled water for injection so that the total volume is 2 ml and then filled into an ampule. All steps are performed under sterile condition.

### Preparation Example 10

### Injection

| | |
|---|---|
| (1) (2-{[(3R,5S)-7-chloro-5-(2,3-dimethoxyphenyl)-1-neopentyl-2-oxo-1,2,3,5-tetrahydro-4,1-benzoxazepin-3-yl]methyl}-1,3-thiazol-5-yl)acetic acid | 10 mg |
| (2) Inositol | 100 mg |
| (3) Benzyl alcohol | 20 mg |
| One ampule | 130 mg |

(1), (2) and (3) are dissolved in distilled water for injection so that the total volume is 2 ml and then filled into an ampule. All steps are performed under sterile condition.

### Preparation Example 11

### Injection

| | |
|---|---|
| (1) (2E)-3-(4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)propionic acid | 10 mg |
| (2) Inositol | 100 mg |
| (3) Benzyl alcohol | 20 mg |
| One ampule | 130 mg |

(1), (2) and (3) are dissolved in distilled water for injection so that the total volume is 2 ml and then filled into an ampule. All steps are performed under sterile condition.

### Preparation Example 12

### Injection

| | |
|---|---|
| (1) (5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid | 10 mg |
| (2) Inositol | 100 mg |
| (3) Benzyl alcohol | 20 mg |
| One ampule | 130 mg |

(1), (2) and (3) are dissolved in distilled water for injection so that the total volume is 2 ml and then filled into an ampule. All steps are performed under sterile condition.

### Experimental Example 1

### Squalene synthase inhibitory activity

### Measuring method

Squalene synthase inhibitory activity was measured using an enzyme solution obtained according to the following preparation method.

That is, an enzyme solution (protein 0.8 µg) prepared according to the following preparation method was added to a solution (total amount 50 µl) containing 5 µM [1-3H] farnesyl pyrophosphate (specific activity 25 µCi/mole), 1mM NADPH (reduced-type nicotinamide adenine dinucleotide phosphate), 5mM MgCl₂, 6mM glutathione, 100mM potassium phosphate buffer (pH7.4) and a test drug (added as an aqueous solution or a DMSO solution) and reacted at 37°C for 45 minutes. After stopping the reaction by addition of 150 µl of chloroform/methanol (1:2) mixed solution, 50 µl of chloroform and 50 µl of a 3N sodium hydroxide solution were added. Then, 50 µl of the chloroform layer (lower layer) containing the reaction product whose main component was squalene was mixed with 3 ml of a toluene-based liquid scintillator and its radioactivity was measured with a liquid scintillation counter.

Squalene synthase inhibitory activity was expressed as the concentration of a test drug required to inhibit 50% of radioactivity incorporated into the chloroform layer (IC₅₀, molar concentration (M)). All compounds described in Examples exhibited potent squalene synthesis inhibitory activity having IC₅₀ of 1 µM or lower.

### Preparation of a human enzyme solution

Human liver cancer cell HepG2 (about 1×10⁹cells) obtained by culturing in Dulbecco's modified Eagle medium containing 10% bovine fetal serum (37°C in the presence of 5%CO₂) was suspended in 10 ml of an ice-cooled buffer [100mM potassium phosphate buffer (pH7.4), 30mM nicotinamide, 2.5mM MgCl₂], disrupted by sonication (30 seconds, twice) and then centrifuged at 10000 x g for 20 minutes (4°C). The resulting supernatant was further centrifuged at 105000 x g for 90 minutes (4°C). The precipitate was suspended in an ice-cooled 100mM potassium phosphate buffer (pH7.4) and then centrifuged at 105000 x g for 90 minutes (4°C). The precipitate was suspended in an ice-cooled 100mM potassium phosphate buffer (pH7.4) (protein concentration about 4 mg/ml). This suspension was used as an enzyme solution.

As seen from the above results, the compound of the present invention has superior squalene synthase inhibitory activity.

### Industrial Applicability

Since the compound of the present invention has squalene synthase inhibitory activity, cholesterol lowering activity and triglyceride lowering activity and has high selectivity for transition to a target organ and a wide safety margin, it is useful as an agent for preventing or treating hyperlipemia as a lipid lowering agent and is also useful for preventing or treating arteriosclerosis.

## Claims

1. A compound represented by the formula [1]: 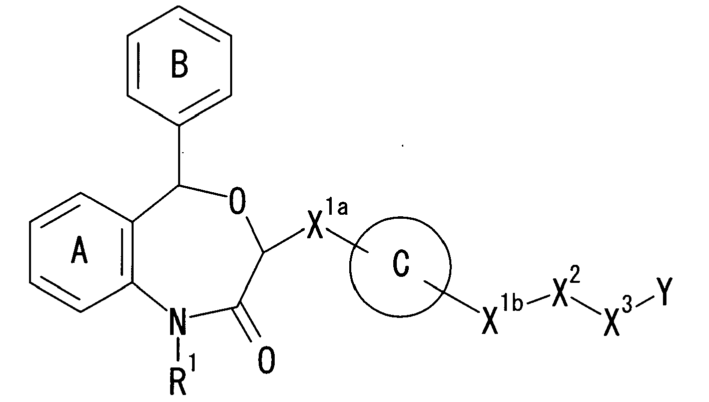 wherein ring A and ring B each represent an optionally substituted benzene ring, ring C represents an optionally further substituted aromatic ring, R¹ represents a lower alkyl group optionally substituted with an optionally substituted hydroxyl group, X^{1a} represents a bond or optionally substituted lower alkylene, X^{1b} represents a bond or optionally substituted lower alkylene, X² represents a bond, -O- or -S-, X³ represents a bond or an optionally substituted divalent hydrocarbon group, and Y represents an optionally esterified or amidated carboxyl group, or a salt thereof.

2. The compound according to claim 1, wherein X^{1b} is a bond and Y is an optionally esterified carboxyl group.

3. The compound according to claim 1, wherein ring A is a benzene ring substituted with halogen atom(s).

4. The compound according to claim 1, wherein ring B is a benzene ring substituted with lower alkoxy group(s).

5. The compound according to claim 1, wherein ring C is an optionally further substituted monocyclic aromatic heterocyclic ring.

6. The compound according to claim 1, wherein ring C is an optionally further substituted benzene ring.

7. The compound according to claim 1, wherein ring C is an optionally further substituted aromatic ring having no hydrogen atom that may be deprotonated.

8. The compound according to claim 1, wherein X^{1a} is C₁₋₃ alkylene.

9. The compound according to claim 1, wherein X² is a bond.

10. The compound according to claim 1, wherein X³ is C₁₋₄ alkylene.

11. The compound according to claim 1, wherein the formula [I] is the formula [Ia]: 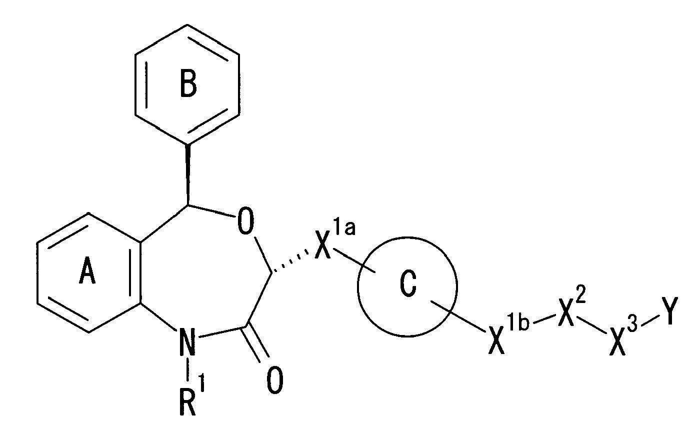 wherein respective symbols are as defined in claim 1.

12. 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid, or a salt thereof.

13. (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid, (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid, or a salt thereof.

14. 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-5-yl)pentanoic acid, or a salt thereof.

15. A prodrug of the compound according to claim 1.

16. A medicine comprising the compound according to claim 1 or a prodrug thereof.

17. A medicine comprising a combination of the compound according to claim 1 or a prodrug thereof and a cholesterol lowering agent.

18. The medicine according to claim 16 or 17, which is a squalene synthase inhibitor.

19. The medicine according to claim 16 or 17, which is a triglyceride lowering agent.

20. The medicine according to claim 16 or 17, which is a lipid lowering agent.

21. The medicine according to claim 16 or 17, which is an agent for preventing or treating hyperlipemia.

22. The medicine according to claim 16 or 17, which is a high density lipoprotein-cholesterol level elevating agent.

23. A process for preparing a compound represented by the formula [I']: 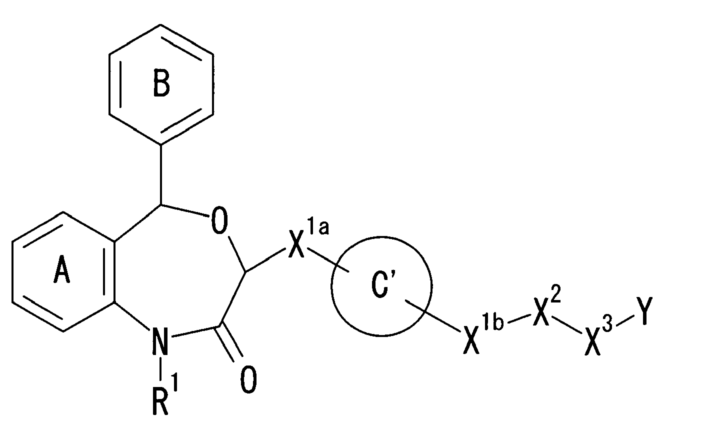 wherein ring C' represents an optionally further substituted aromatic heterocyclic ring and other symbols are as defined in claim 1, or a salt thereof, which comprises reacting a compound represented by the formula: 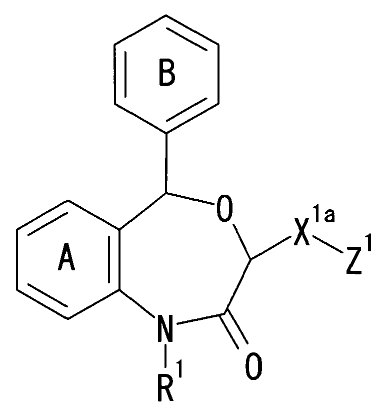 wherein Z¹ represents a functional group involved in an aromatic heterocyclic ring forming reaction and other symbols are as defined in claim 1, or a salt thereof, with a compound represented by the formula: 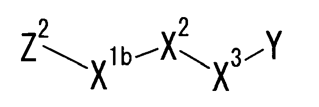 wherein Z² represents a functional group involved in an aromatic heterocyclic ring forming reaction and other symbols are as defined in claim 1, or a salt thereof.

24. A method of inhibiting squalene synthase in a mammal, which comprises administering an effective amount of the compound according to claim 1 or a prodrug thereof to said mammal.

25. A method of lowering triglyceride level in a mammal, which comprises administering an effective amount of the compound according to claim 1 or a prodrug thereof to said mammal.

26. A method of lowering lipid level in a mammal, which comprises administering an effective amount of the compound according to claim 1 or a prodrug thereof to said mammal.

27. A method of preventing or treating hyperlipemia in a mammal, which comprises administering an effective amount of the compound according to claim 1 or a prodrug thereof to said mammal.

28. A method of elevating high density lipoprotein-cholesterol level in a mammal, which comprises administering an effective amount of the compound according to claim 1 or a prodrug thereof to said mammal.

29. Use of the compound according to claim 1 or a prodrug for manufacture of a squalene synthase inhibitor.

30. Use of the compound according to claim 1 or a prodrug thereof for manufacture of a triglyceride lowering agent.

31. Use of the compound according to claim 1 or a prodrug thereof for manufacture of a lipid lowering agent.

32. Use of the compound according to claim 1 or a prodrug thereof for manufacture of an agent for preventing or treating hyperlipemia.

33. Use of the compound according to claim 1 or a prodrug thereof for manufacture of a high density lipoprotein-cholesterol level elevating agent.
